# EUROPEAN PATENT APPLICATION

(11) **EP 3 985 129 A1**
(43) Date of publication of application: **20.04.2022**
(21) Application number: 20202627.4
(22) Date of filing: 19.10.2020
(51) Int. Cl.: C12Q 1/6876, C12Q 1/70

(54) **METHOD FOR PARALLEL NUCLEIC ACID ANALYSIS**

(71) Applicant: IMBA-Institut für Molekulare Biotechnologie GmbH, 1030 Wien (AT)
(72) Inventor: The designation of the inventor has not yet been filed
(74) Representative: SONN Patentanwälte OG

(57) **Abstract**

The present invention provides a method for detecting a nucleic acid of interest in a plurality of samples, comprising the steps of a) providing analyte nucleic acids from a plurality of samples in separate containers for each sample, wherein the containers are arranged into an array of subsets, wherein the array comprises two or more subsets; b) amplifying nucleic acids by a primer extension reaction using at least one pair of primers hybridized to the analyte nucleic acids, wherein a pair of primers comprises a forward and a reverse primer, wherein the forward and reverse primers both comprise an adaptor sequence, an sample identifier sequence and a binding sequence for hybridization to the analyte nucleic acids, respectively; c) combining the amplified nucleic acids of step b) of containers of two or more subsets to an array of combined containers, wherein containers of one subset, but not of another subset, are combined to a combined container; d) amplifying nucleic acids by a primer extension reaction using at least one pair of further primers hybridized to the amplified nucleic acids of combined containers of step c), wherein a pair of further primers comprises a further forward and a further reverse primer, wherein the further forward and further reverse primers both comprise a subset identifier sequence and a sequence for hybridization to the adaptor sequence; e) determining the sequences of the amplified nucleic acids of step d); f) assigning a determined sequence of a nucleic acid of interest of step e) to a sample through association to a subset and container with the subset identifier sequences and the sample identifier sequences. Further provided are sets of primers for such a method.

SEQUENCE LISTING

<110>  IMBA - INSTITUT FÜR MOLEKULARE BIOTECHNOLOGIE GMBH

<120>  Method for parallel nucleic acid analysis

<130>  r77107

<160>  990

<170>  PatentIn version 3.5

<210>  1
<211>  51
<212>  DNA
<213>  Artificial Sequence

<220>
<223>  primer

<220>
<221>  misc_feature
<222>  23
<223>  n is a, c, g, or t

<400>  1
ctacacgacg ctcttccgat ctnccaatac tgaccccaaa atcagcgaaa t          51

<210>  2
<211>  52
<212>  DNA
<213>  Artificial Sequence

<220>
<223>  primer

<220>
<221>  misc_feature
<222>  (23)..(24)
<223>  n is a, c, g, or t

<400>  2
ctacacgacg ctcttccgat ctnntcgcgc atgaccccaa aatcagcgaa at          52

<210>  3
<211>  53
<212>  DNA
<213>  Artificial Sequence

<220>
<223>  primer

<220>
<221>  misc_feature

<222>  (23)..(25)
<223>  n is a, c, g, or t

<400>  3
ctacacgacg ctcttccgat ctnnngtgtg aacgacccca aaatcagcga aat          53



<210>  4
<211>  54
<212>  DNA
<213>  Artificial Sequence

<220>
<223>  primer


<220>
<221>  misc_feature
<222>  (23)..(26)
<223>  n is a, c, g, or t

<400>  4
ctacacgacg ctcttccgat ctnnnnggtg gcacgacccc aaaatcagcg aaat         54



<210>  5
<211>  51
<212>  DNA
<213>  Artificial Sequence

<220>
<223>  primer


<220>
<221>  misc_feature
<222>  23
<223>  n is a, c, g, or t

<400>  5
ctacacgacg ctcttccgat ctntaggtgc tgaccccaaa atcagcgaaa t            51



<210>  6
<211>  52
<212>  DNA
<213>  Artificial Sequence

<220>
<223>  primer


<220>
<221>  misc_feature
<222>  (23)..(24)
<223>  n is a, c, g, or t

<400>  6
ctacacgacg ctcttccgat ctnngagcgg ttgaccccaa aatcagcgaa at           52

<210>  7
<211>  53
<212>  DNA
<213>  Artificial Sequence

<220>
<223>  primer

<220>
<221>  misc_feature
<222>  (23)..(25)
<223>  n is a, c, g, or t

<400>  7
ctacacgacg ctcttccgat ctnnnaggat taggacccca aaatcagcga aat          53

<210>  8
<211>  54
<212>  DNA
<213>  Artificial Sequence

<220>
<223>  primer

<220>
<221>  misc_feature
<222>  (23)..(26)
<223>  n is a, c, g, or t

<400>  8
ctacacgacg ctcttccgat ctnnnngtga gccagacccc aaaatcagcg aaat          54

<210>  9
<211>  51
<212>  DNA
<213>  Artificial Sequence

<220>
<223>  primer

<220>
<221>  misc_feature
<222>  23
<223>  n is a, c, g, or t

<400>  9
ctacacgacg ctcttccgat ctnaattatg cgaccccaaa atcagcgaaa t          51

<210>  10
<211>  52
<212>  DNA
<213>  Artificial Sequence

<220>
<223>  primer


<220>
<221>  misc_feature
<222>  (23)..(24)
<223>  n is a, c, g, or t

<400>  10
ctacacgacg ctcttccgat ctnnttacgc cggaccccaa aatcagcgaa at          52


<210>  11
<211>  53
<212>  DNA
<213>  Artificial Sequence

<220>
<223>  primer


<220>
<221>  misc_feature
<222>  (23)..(25)
<223>  n is a, c, g, or t

<400>  11
ctacacgacg ctcttccgat ctnnngtagt gatgacccca aaatcagcga aat          53


<210>  12
<211>  54
<212>  DNA
<213>  Artificial Sequence

<220>
<223>  primer


<220>
<221>  misc_feature
<222>  (23)..(26)
<223>  n is a, c, g, or t

<400>  12
ctacacgacg ctcttccgat ctnnnntggt tgcagacccc aaaatcagcg aaat          54


<210>  13
<211>  51
<212>  DNA
<213>  Artificial Sequence

<220>
<223>  primer


<220>

<221>  misc_feature
<222>  23
<223>  n is a, c, g, or t

<400>  13
ctacacgacg ctcttccgat ctngtccgac cgaccccaaa atcagcgaaa t          51



<210>  14
<211>  52
<212>  DNA
<213>  Artificial Sequence

<220>
<223>  primer


<220>
<221>  misc_feature
<222>  (23)..(24)
<223>  n is a, c, g, or t

<400>  14
ctacacgacg ctcttccgat ctnngccagc cggaccccaa aatcagcgaa at          52



<210>  15
<211>  53
<212>  DNA
<213>  Artificial Sequence

<220>
<223>  primer


<220>
<221>  misc_feature
<222>  (23)..(25)
<223>  n is a, c, g, or t

<400>  15
ctacacgacg ctcttccgat ctnnnctcgt gtcgacccca aaatcagcga aat          53



<210>  16
<211>  54
<212>  DNA
<213>  Artificial Sequence

<220>
<223>  primer


<220>
<221>  misc_feature
<222>  (23)..(26)
<223>  n is a, c, g, or t

<400>  16
ctacacgacg ctcttccgat ctnnnngccg tggcgacccc aaaatcagcg aaat          54

<210>  17
<211>  51
<212>  DNA
<213>  Artificial Sequence

<220>
<223>  primer


<220>
<221>  misc_feature
<222>  23
<223>  n is a, c, g, or t

<400>  17
ctacacgacg ctcttccgat ctngtagcca cgaccccaaa atcagcgaaa t                51


<210>  18
<211>  52
<212>  DNA
<213>  Artificial Sequence

<220>
<223>  primer


<220>
<221>  misc_feature
<222>  (23)..(24)
<223>  n is a, c, g, or t

<400>  18
ctacacgacg ctcttccgat ctnngactta tagaccccaa aatcagcgaa at               52


<210>  19
<211>  53
<212>  DNA
<213>  Artificial Sequence

<220>
<223>  primer


<220>
<221>  misc_feature
<222>  (23)..(25)
<223>  n is a, c, g, or t

<400>  19
ctacacgacg ctcttccgat ctnnngaacg tcggacccca aaatcagcga aat              53


<210>  20
<211>  54
<212>  DNA

<213>  Artificial Sequence

<220>
<223>  primer



<220>
<221>  misc_feature
<222>  (23)..(26)
<223>  n is a, c, g, or t

<400>  20
ctacacgacg ctcttccgat ctnnnnatgc gtaggacccc aaaatcagcg aaat          54




<210>  21
<211>  51
<212>  DNA
<213>  Artificial Sequence

<220>
<223>  primer



<220>
<221>  misc_feature
<222>  23
<223>  n is a, c, g, or t

<400>  21
ctacacgacg ctcttccgat ctnaattaga tgaccccaaa atcagcgaaa t             51




<210>  22
<211>  52
<212>  DNA
<213>  Artificial Sequence

<220>
<223>  primer



<220>
<221>  misc_feature
<222>  (23)..(24)
<223>  n is a, c, g, or t

<400>  22
ctacacgacg ctcttccgat ctnnttacga tagaccccaa aatcagcgaa at            52




<210>  23
<211>  53
<212>  DNA
<213>  Artificial Sequence

<220>
<223>  primer

<220>
<221>  misc_feature
<222>  (23)..(25)
<223>  n is a, c, g, or t

<400>  23
ctacacgacg ctcttccgat ctnnnctgta caagacccca aaatcagcga aat          53



<210>  24
<211>  54
<212>  DNA
<213>  Artificial Sequence

<220>
<223>  primer


<220>
<221>  misc_feature
<222>  (23)..(26)
<223>  n is a, c, g, or t

<400>  24
ctacacgacg ctcttccgat ctnnnncgtg gttggacccc aaaatcagcg aaat          54



<210>  25
<211>  51
<212>  DNA
<213>  Artificial Sequence

<220>
<223>  primer


<220>
<221>  misc_feature
<222>  23
<223>  n is a, c, g, or t

<400>  25
ctacacgacg ctcttccgat ctnaataacg tgaccccaaa atcagcgaaa t          51



<210>  26
<211>  52
<212>  DNA
<213>  Artificial Sequence

<220>
<223>  primer


<220>
<221>  misc_feature
<222>  (23)..(24)
<223>  n is a, c, g, or t

<400>  26

ctacacgacg ctcttccgat ctnnttcttg aagaccccaa aatcagcgaa at 52

<210> 27
<211> 53
<212> DNA
<213> Artificial Sequence

<220>
<223> primer

<220>
<221> misc_feature
<222> (23)..(25)
<223> n is a, c, g, or t

<400> 27
ctacacgacg ctcttccgat ctnnnggcag atcgacccca aaatcagcga aat 53

<210> 28
<211> 54
<212> DNA
<213> Artificial Sequence

<220>
<223> primer

<220>
<221> misc_feature
<222> (23)..(26)
<223> n is a, c, g, or t

<400> 28
ctacacgacg ctcttccgat ctnnnnctat gttagacccc aaaatcagcg aaat 54

<210> 29
<211> 51
<212> DNA
<213> Artificial Sequence

<220>
<223> primer

<220>
<221> misc_feature
<222> 23
<223> n is a, c, g, or t

<400> 29
ctacacgacg ctcttccgat ctngttgacg cgaccccaaa atcagcgaaa t 51

<210> 30
<211> 52

<212>  DNA
<213>  Artificial Sequence

<220>
<223>  primer


<220>
<221>  misc_feature
<222>  (23)..(24)
<223>  n is a, c, g, or t

<400>  30
ctacacgacg ctcttccgat ctnnatctac gagaccccaa aatcagcgaa at          52



<210>  31
<211>  53
<212>  DNA
<213>  Artificial Sequence

<220>
<223>  primer


<220>
<221>  misc_feature
<222>  (23)..(25)
<223>  n is a, c, g, or t

<400>  31
ctacacgacg ctcttccgat ctnnnctcga caggacccca aaatcagcga aat          53



<210>  32
<211>  54
<212>  DNA
<213>  Artificial Sequence

<220>
<223>  primer


<220>
<221>  misc_feature
<222>  (23)..(26)
<223>  n is a, c, g, or t

<400>  32
ctacacgacg ctcttccgat ctnnnngagg ctgcgacccc aaaatcagcg aaat          54



<210>  33
<211>  51
<212>  DNA
<213>  Artificial Sequence

<220>
<223>  primer

<220>
<221>  misc_feature
<222>  23
<223>  n is a, c, g, or t

<400>  33
ctacacgacg ctcttccgat ctncctcgta ggaccccaaa atcagcgaaa t                51

<210>  34
<211>  52
<212>  DNA
<213>  Artificial Sequence

<220>
<223>  primer

<220>
<221>  misc_feature
<222>  (23)..(24)
<223>  n is a, c, g, or t

<400>  34
ctacacgacg ctcttccgat ctnncatagg cagaccccaa aatcagcgaa at               52

<210>  35
<211>  53
<212>  DNA
<213>  Artificial Sequence

<220>
<223>  primer

<220>
<221>  misc_feature
<222>  (23)..(25)
<223>  n is a, c, g, or t

<400>  35
ctacacgacg ctcttccgat ctnnnagatg aacgacccca aaatcagcga aat              53

<210>  36
<211>  54
<212>  DNA
<213>  Artificial Sequence

<220>
<223>  primer

<220>
<221>  misc_feature
<222>  (23)..(26)
<223>  n is a, c, g, or t

<400>  36
ctacacgacg ctcttccgat ctnnnnccga gtatgacccc aaaatcagcg aaat          54

<210>  37
<211>  51
<212>  DNA
<213>  Artificial Sequence

<220>
<223>  primer

<220>
<221>  misc_feature
<222>  23
<223>  n is a, c, g, or t

<400>  37
ctacacgacg ctcttccgat ctnaatattg agaccccaaa atcagcgaaa t          51

<210>  38
<211>  52
<212>  DNA
<213>  Artificial Sequence

<220>
<223>  primer

<220>
<221>  misc_feature
<222>  (23)..(24)
<223>  n is a, c, g, or t

<400>  38
ctacacgacg ctcttccgat ctnngtatac cggaccccaa aatcagcgaa at          52

<210>  39
<211>  53
<212>  DNA
<213>  Artificial Sequence

<220>
<223>  primer

<220>
<221>  misc_feature
<222>  (23)..(25)
<223>  n is a, c, g, or t

<400>  39
ctacacgacg ctcttccgat ctnnngatcc aacgacccca aaatcagcga aat          53

<210>  40

<211>  54
<212>  DNA
<213>  Artificial Sequence

<220>
<223>  primer


<220>
<221>  misc_feature
<222>  (23)..(26)
<223>  n is a, c, g, or t

<400>  40
ctacacgacg ctcttccgat ctnnnnagat acgcgacccc aaaatcagcg aaat          54


<210>  41
<211>  51
<212>  DNA
<213>  Artificial Sequence

<220>
<223>  primer


<220>
<221>  misc_feature
<222>  23
<223>  n is a, c, g, or t

<400>  41
ctacacgacg ctcttccgat ctnggtatct tgaccccaaa atcagcgaaa t          51


<210>  42
<211>  52
<212>  DNA
<213>  Artificial Sequence

<220>
<223>  primer


<220>
<221>  misc_feature
<222>  (23)..(24)
<223>  n is a, c, g, or t

<400>  42
ctacacgacg ctcttccgat ctnncctctg gcgaccccaa aatcagcgaa at          52


<210>  43
<211>  51
<212>  DNA
<213>  Artificial Sequence

<220>
<223>  primer

<220>
<221>  misc_feature
<222>  23
<223>  n is a, c, g, or t

<400>  43
ctacacgacg ctcttccgat ctnccattgt ggaccccaaa atcagcgaaa t                51


<210>  44
<211>  52
<212>  DNA
<213>  Artificial Sequence

<220>
<223>  primer


<220>
<221>  misc_feature
<222>  (23)..(24)
<223>  n is a, c, g, or t

<400>  44
ctacacgacg ctcttccgat ctnnactacg gtgaccccaa aatcagcgaa at               52


<210>  45
<211>  53
<212>  DNA
<213>  Artificial Sequence

<220>
<223>  primer


<220>
<221>  misc_feature
<222>  (23)..(25)
<223>  n is a, c, g, or t

<400>  45
ctacacgacg ctcttccgat ctnnnaagtg ctagacccca aaatcagcga aat              53


<210>  46
<211>  54
<212>  DNA
<213>  Artificial Sequence

<220>
<223>  primer


<220>
<221>  misc_feature
<222>  (23)..(26)
<223>  n is a, c, g, or t

<400>  46
ctacacgacg ctcttccgat ctnnnngccg aacggacccc aaaatcagcg aaat          54

<210>  47
<211>  51
<212>  DNA
<213>  Artificial Sequence

<220>
<223>  primer

<220>
<221>  misc_feature
<222>  23
<223>  n is a, c, g, or t

<400>  47
ctacacgacg ctcttccgat ctntgtccac ggaccccaaa atcagcgaaa t             51

<210>  48
<211>  52
<212>  DNA
<213>  Artificial Sequence

<220>
<223>  primer

<220>
<221>  misc_feature
<222>  (23)..(24)
<223>  n is a, c, g, or t

<400>  48
ctacacgacg ctcttccgat ctnngacaca ctgaccccaa aatcagcgaa at            52

<210>  49
<211>  53
<212>  DNA
<213>  Artificial Sequence

<220>
<223>  primer

<220>
<221>  misc_feature
<222>  (23)..(25)
<223>  n is a, c, g, or t

<400>  49
ctacacgacg ctcttccgat ctnnnaatat gctgacccca aaatcagcga aat           53

<210>  50
<211>  54
<212>  DNA
<213>  Artificial Sequence

<220>
<223>  primer


<220>
<221>  misc_feature
<222>  (23)..(26)
<223>  n is a, c, g, or t

<400>  50
ctacacgacg ctcttccgat ctnnnnttct catagacccc aaaatcagcg aaat          54


<210>  51
<211>  51
<212>  DNA
<213>  Artificial Sequence

<220>
<223>  primer


<220>
<221>  misc_feature
<222>  23
<223>  n is a, c, g, or t

<400>  51
ctacacgacg ctcttccgat ctntctgtga tgaccccaaa atcagcgaaa t          51


<210>  52
<211>  52
<212>  DNA
<213>  Artificial Sequence

<220>
<223>  primer


<220>
<221>  misc_feature
<222>  (23)..(24)
<223>  n is a, c, g, or t

<400>  52
ctacacgacg ctcttccgat ctnnccgaac ttgaccccaa aatcagcgaa at          52


<210>  53
<211>  53
<212>  DNA
<213>  Artificial Sequence

<220>

<223>  primer

<220>
<221>  misc_feature
<222>  (23)..(25)
<223>  n is a, c, g, or t

<400>  53
ctacacgacg ctcttccgat ctnnngtcta acagacccca aaatcagcga aat          53

<210>  54
<211>  54
<212>  DNA
<213>  Artificial Sequence

<220>
<223>  primer

<220>
<221>  misc_feature
<222>  (23)..(26)
<223>  n is a, c, g, or t

<400>  54
ctacacgacg ctcttccgat ctnnnngacg ccatgacccc aaaatcagcg aaat          54

<210>  55
<211>  51
<212>  DNA
<213>  Artificial Sequence

<220>
<223>  primer

<220>
<221>  misc_feature
<222>  23
<223>  n is a, c, g, or t

<400>  55
ctacacgacg ctcttccgat ctngccaatg tgaccccaaa atcagcgaaa t          51

<210>  56
<211>  52
<212>  DNA
<213>  Artificial Sequence

<220>
<223>  primer

<220>
<221>  misc_feature
<222>  (23)..(24)

<223>  n is a, c, g, or t

<400>  56
ctacacgacg ctcttccgat ctnnccaacg tcgaccccaa aatcagcgaa at          52

<210>  57
<211>  53
<212>  DNA
<213>  Artificial Sequence

<220>
<223>  primer

<220>
<221>  misc_feature
<222>  (23)..(25)
<223>  n is a, c, g, or t

<400>  57
ctacacgacg ctcttccgat ctnnngtaga taagacccca aaatcagcga aat          53

<210>  58
<211>  54
<212>  DNA
<213>  Artificial Sequence

<220>
<223>  primer

<220>
<221>  misc_feature
<222>  (23)..(26)
<223>  n is a, c, g, or t

<400>  58
ctacacgacg ctcttccgat ctnnnnctta cggcgacccc aaaatcagcg aaat          54

<210>  59
<211>  51
<212>  DNA
<213>  Artificial Sequence

<220>
<223>  primer

<220>
<221>  misc_feature
<222>  23
<223>  n is a, c, g, or t

<400>  59
ctacacgacg ctcttccgat ctnccaagtg cgaccccaaa atcagcgaaa t          51

<210>  60
<211>  52
<212>  DNA
<213>  Artificial Sequence

<220>
<223>  primer


<220>
<221>  misc_feature
<222>  (23)..(24)
<223>  n is a, c, g, or t

<400>  60
ctacacgacg ctcttccgat ctnnctaact cagaccccaa aatcagcgaa at          52


<210>  61
<211>  53
<212>  DNA
<213>  Artificial Sequence

<220>
<223>  primer


<220>
<221>  misc_feature
<222>  (23)..(25)
<223>  n is a, c, g, or t

<400>  61
ctacacgacg ctcttccgat ctnnnaatat ctggacccca aaatcagcga aat          53


<210>  62
<211>  54
<212>  DNA
<213>  Artificial Sequence

<220>
<223>  primer


<220>
<221>  misc_feature
<222>  (23)..(26)
<223>  n is a, c, g, or t

<400>  62
ctacacgacg ctcttccgat ctnnnnttat atcagacccc aaaatcagcg aaat          54


<210>  63
<211>  51
<212>  DNA
<213>  Artificial Sequence

<220>
<223>  primer

<220>
<221>  misc_feature
<222>  23
<223>  n is a, c, g, or t

<400>  63
ctacacgacg ctcttccgat ctnctgcgga tgaccccaaa atcagcgaaa t                51

<210>  64
<211>  52
<212>  DNA
<213>  Artificial Sequence

<220>
<223>  primer

<220>
<221>  misc_feature
<222>  (23)..(24)
<223>  n is a, c, g, or t

<400>  64
ctacacgacg ctcttccgat ctnngcggct tggaccccaa aatcagcgaa at               52

<210>  65
<211>  53
<212>  DNA
<213>  Artificial Sequence

<220>
<223>  primer

<220>
<221>  misc_feature
<222>  (23)..(25)
<223>  n is a, c, g, or t

<400>  65
ctacacgacg ctcttccgat ctnnngagtt gatgacccca aaatcagcga aat              53

<210>  66
<211>  54
<212>  DNA
<213>  Artificial Sequence

<220>
<223>  primer

<220>
<221>  misc_feature

<222>  (23)..(26)
<223>  n is a, c, g, or t

<400>  66
ctacacgacg ctcttccgat ctnnnngcac tgaggacccc aaaatcagcg aaat          54




<210>  67
<211>  51
<212>  DNA
<213>  Artificial Sequence

<220>
<223>  primer


<220>
<221>  misc_feature
<222>  23
<223>  n is a, c, g, or t

<400>  67
ctacacgacg ctcttccgat ctngaccacc tgaccccaaa atcagcgaaa t             51




<210>  68
<211>  51
<212>  DNA
<213>  Artificial Sequence

<220>
<223>  primer


<220>
<221>  misc_feature
<222>  23
<223>  n is a, c, g, or t

<400>  68
ctacacgacg ctcttccgat ctnaatagca agaccccaaa atcagcgaaa t             51




<210>  69
<211>  51
<212>  DNA
<213>  Artificial Sequence

<220>
<223>  primer


<220>
<221>  misc_feature
<222>  23
<223>  n is a, c, g, or t

<400>  69
ctacacgacg ctcttccgat ctncctaccg ggaccccaaa atcagcgaaa t             51

<210>  70
<211>  52
<212>  DNA
<213>  Artificial Sequence

<220>
<223>  primer



<220>
<221>  misc_feature
<222>  (23)..(24)
<223>  n is a, c, g, or t

<400>  70
ctacacgacg ctcttccgat ctnnggagga tggaccccaa aatcagcgaa at          52




<210>  71
<211>  53
<212>  DNA
<213>  Artificial Sequence

<220>
<223>  primer



<220>
<221>  misc_feature
<222>  (23)..(25)
<223>  n is a, c, g, or t

<400>  71
ctacacgacg ctcttccgat ctnnncgctg aatgacccca aaatcagcga aat          53




<210>  72
<211>  54
<212>  DNA
<213>  Artificial Sequence

<220>
<223>  primer



<220>
<221>  misc_feature
<222>  (23)..(26)
<223>  n is a, c, g, or t

<400>  72
ctacacgacg ctcttccgat ctnnnntgtg acgagacccc aaaatcagcg aaat          54




<210>  73
<211>  51
<212>  DNA
<213>  Artificial Sequence

<220>
<223>  primer


<220>
<221>  misc_feature
<222>  23
<223>  n is a, c, g, or t

<400>  73
ctacacgacg ctcttccgat ctnaatagat tgaccccaaa atcagcgaaa t          51



<210>  74
<211>  52
<212>  DNA
<213>  Artificial Sequence

<220>
<223>  primer


<220>
<221>  misc_feature
<222>  (23)..(24)
<223>  n is a, c, g, or t

<400>  74
ctacacgacg ctcttccgat ctnnttagcg cagaccccaa aatcagcgaa at          52



<210>  75
<211>  53
<212>  DNA
<213>  Artificial Sequence

<220>
<223>  primer


<220>
<221>  misc_feature
<222>  (23)..(25)
<223>  n is a, c, g, or t

<400>  75
ctacacgacg ctcttccgat ctnnngcggc cgtgacccca aaatcagcga aat          53



<210>  76
<211>  54
<212>  DNA
<213>  Artificial Sequence

<220>
<223>  primer


<220>

<221>  misc_feature
<222>  (23)..(26)
<223>  n is a, c, g, or t

<400>  76
ctacacgacg ctcttccgat ctnnnncagt aaccgacccc aaaatcagcg aaat          54

<210>  77
<211>  51
<212>  DNA
<213>  Artificial Sequence

<220>
<223>  primer

<220>
<221>  misc_feature
<222>  23
<223>  n is a, c, g, or t

<400>  77
ctacacgacg ctcttccgat ctngcctagt agaccccaaa atcagcgaaa t             51

<210>  78
<211>  52
<212>  DNA
<213>  Artificial Sequence

<220>
<223>  primer

<220>
<221>  misc_feature
<222>  (23)..(24)
<223>  n is a, c, g, or t

<400>  78
ctacacgacg ctcttccgat ctnncacggc gcgaccccaa aatcagcgaa at            52

<210>  79
<211>  53
<212>  DNA
<213>  Artificial Sequence

<220>
<223>  primer

<220>
<221>  misc_feature
<222>  (23)..(25)
<223>  n is a, c, g, or t

<400>  79
ctacacgacg ctcttccgat ctnnnggtgc agagacccca aaatcagcga aat           53

<210>  80
<211>  54
<212>  DNA
<213>  Artificial Sequence

<220>
<223>  primer


<220>
<221>  misc_feature
<222>  (23)..(26)
<223>  n is a, c, g, or t

<400>  80
ctacacgacg ctcttccgat ctnnnntcgc tgacgacccc aaaatcagcg aaat          54


<210>  81
<211>  51
<212>  DNA
<213>  Artificial Sequence

<220>
<223>  primer


<220>
<221>  misc_feature
<222>  23
<223>  n is a, c, g, or t

<400>  81
ctacacgacg ctcttccgat ctncagccag tgaccccaaa atcagcgaaa t          51


<210>  82
<211>  52
<212>  DNA
<213>  Artificial Sequence

<220>
<223>  primer


<220>
<221>  misc_feature
<222>  (23)..(24)
<223>  n is a, c, g, or t

<400>  82
ctacacgacg ctcttccgat ctnncgtcaa ccgaccccaa aatcagcgaa at          52


<210>  83
<211>  51
<212>  DNA

<213>  Artificial Sequence

<220>
<223>  primer



<220>
<221>  misc_feature
<222>  23
<223>  n is a, c, g, or t

<400>  83
ctacacgacg ctcttccgat ctngccggcg agaccccaaa atcagcgaaa t                51




<210>  84
<211>  52
<212>  DNA
<213>  Artificial Sequence

<220>
<223>  primer



<220>
<221>  misc_feature
<222>  (23)..(24)
<223>  n is a, c, g, or t

<400>  84
ctacacgacg ctcttccgat ctnngcctcc gggaccccaa aatcagcgaa at               52




<210>  85
<211>  53
<212>  DNA
<213>  Artificial Sequence

<220>
<223>  primer



<220>
<221>  misc_feature
<222>  (23)..(25)
<223>  n is a, c, g, or t

<400>  85
ctacacgacg ctcttccgat ctnnnaatag tccgacccca aaatcagcga aat              53




<210>  86
<211>  54
<212>  DNA
<213>  Artificial Sequence

<220>
<223>  primer

<220>
<221>  misc_feature
<222>  (23)..(26)
<223>  n is a, c, g, or t

<400>  86
ctacacgacg ctcttccgat ctnnnnttag acgtgacccc aaaatcagcg aaat          54

<210>  87
<211>  51
<212>  DNA
<213>  Artificial Sequence

<220>
<223>  primer

<220>
<221>  misc_feature
<222>  23
<223>  n is a, c, g, or t

<400>  87
ctacacgacg ctcttccgat ctngtggact agaccccaaa atcagcgaaa t             51

<210>  88
<211>  52
<212>  DNA
<213>  Artificial Sequence

<220>
<223>  primer

<220>
<221>  misc_feature
<222>  (23)..(24)
<223>  n is a, c, g, or t

<400>  88
ctacacgacg ctcttccgat ctnncacgga cggaccccaa aatcagcgaa at            52

<210>  89
<211>  53
<212>  DNA
<213>  Artificial Sequence

<220>
<223>  primer

<220>
<221>  misc_feature
<222>  (23)..(25)
<223>  n is a, c, g, or t

<400>  89

ctacacgacg ctcttccgat ctnnncacta gaggacccca aaatcagcga aat 53

<210> 90
<211> 54
<212> DNA
<213> Artificial Sequence

<220>
<223> primer

<220>
<221> misc_feature
<222> (23)..(26)
<223> n is a, c, g, or t

<400> 90
ctacacgacg ctcttccgat ctnnnngcag atgggacccc aaaatcagcg aaat 54

<210> 91
<211> 51
<212> DNA
<213> Artificial Sequence

<220>
<223> primer

<220>
<221> misc_feature
<222> 23
<223> n is a, c, g, or t

<400> 91
ctacacgacg ctcttccgat ctnctctcac ggaccccaaa atcagcgaaa t 51

<210> 92
<211> 52
<212> DNA
<213> Artificial Sequence

<220>
<223> primer

<220>
<221> misc_feature
<222> (23)..(24)
<223> n is a, c, g, or t

<400> 92
ctacacgacg ctcttccgat ctnnggaatc acgaccccaa aatcagcgaa at 52

<210> 93
<211> 53

<212>  DNA
<213>  Artificial Sequence

<220>
<223>  primer


<220>
<221>  misc_feature
<222>  (23)..(25)
<223>  n is a, c, g, or t

<400>  93
ctacacgacg ctcttccgat ctnnncgttg acggacccca aaatcagcga aat          53



<210>  94
<211>  54
<212>  DNA
<213>  Artificial Sequence

<220>
<223>  primer


<220>
<221>  misc_feature
<222>  (23)..(26)
<223>  n is a, c, g, or t

<400>  94
ctacacgacg ctcttccgat ctnnnncatc aggtgacccc aaaatcagcg aaat          54



<210>  95
<211>  51
<212>  DNA
<213>  Artificial Sequence

<220>
<223>  primer


<220>
<221>  misc_feature
<222>  23
<223>  n is a, c, g, or t

<400>  95
ctacacgacg ctcttccgat ctncgttgta agaccccaaa atcagcgaaa t          51



<210>  96
<211>  52
<212>  DNA
<213>  Artificial Sequence

<220>
<223>  primer

<220>
<221>  misc_feature
<222>  (23)..(24)
<223>  n is a, c, g, or t

<400>  96
ctacacgacg ctcttccgat ctnnggcacg gtgaccccaa aatcagcgaa at          52

<210>  97
<211>  53
<212>  DNA
<213>  Artificial Sequence

<220>
<223>  primer

<220>
<221>  misc_feature
<222>  23
<223>  n is a, c, g, or t

<400>  97
ctacacgacg ctcttccgat ctnccaatac tgggagcctt gaatacacca aaa          53

<210>  98
<211>  54
<212>  DNA
<213>  Artificial Sequence

<220>
<223>  primer

<220>
<221>  misc_feature
<222>  (23)..(24)
<223>  n is a, c, g, or t

<400>  98
ctacacgacg ctcttccgat ctnntcgcgc atgggagcct tgaatacacc aaaa          54

<210>  99
<211>  55
<212>  DNA
<213>  Artificial Sequence

<220>
<223>  primer

<220>
<221>  misc_feature
<222>  (23)..(25)
<223>  n is a, c, g, or t

<400>  99
ctacacgacg ctcttccgat ctnnngtgtg aacgggagcc ttgaatacac caaaa          55

<210>  100
<211>  56
<212>  DNA
<213>  Artificial Sequence

<220>
<223>  primer

<220>
<221>  misc_feature
<222>  (23)..(26)
<223>  n is a, c, g, or t

<400>  100
ctacacgacg ctcttccgat ctnnnnggtg gcacgggagc cttgaataca ccaaaa          56

<210>  101
<211>  53
<212>  DNA
<213>  Artificial Sequence

<220>
<223>  primer

<220>
<221>  misc_feature
<222>  23
<223>  n is a, c, g, or t

<400>  101
ctacacgacg ctcttccgat ctntaggtgc tgggagcctt gaatacacca aaa          53

<210>  102
<211>  54
<212>  DNA
<213>  Artificial Sequence

<220>
<223>  primer

<220>
<221>  misc_feature
<222>  (23)..(24)
<223>  n is a, c, g, or t

<400>  102
ctacacgacg ctcttccgat ctnngagcgg ttgggagcct tgaatacacc aaaa          54

<210>  103

<211>  55
<212>  DNA
<213>  Artificial Sequence

<220>
<223>  primer


<220>
<221>  misc_feature
<222>  (23)..(25)
<223>  n is a, c, g, or t

<400>  103
ctacacgacg ctcttccgat ctnnnaggat taggggagcc ttgaatacac caaaa          55



<210>  104
<211>  56
<212>  DNA
<213>  Artificial Sequence

<220>
<223>  primer


<220>
<221>  misc_feature
<222>  (23)..(26)
<223>  n is a, c, g, or t

<400>  104
ctacacgacg ctcttccgat ctnnnngtga gccagggagc cttgaataca ccaaaa         56



<210>  105
<211>  53
<212>  DNA
<213>  Artificial Sequence

<220>
<223>  primer


<220>
<221>  misc_feature
<222>  23
<223>  n is a, c, g, or t

<400>  105
ctacacgacg ctcttccgat ctnaattatg cgggagcctt gaatacacca aaa            53



<210>  106
<211>  54
<212>  DNA
<213>  Artificial Sequence

<220>
<223>  primer

<220>
<221>  misc_feature
<222>  (23)..(24)
<223>  n is a, c, g, or t

<400>  106
ctacacgacg ctcttccgat ctnnttacgc cggggagcct tgaatacacc aaaa          54

<210>  107
<211>  55
<212>  DNA
<213>  Artificial Sequence

<220>
<223>  primer

<220>
<221>  misc_feature
<222>  (23)..(25)
<223>  n is a, c, g, or t

<400>  107
ctacacgacg ctcttccgat ctnnngtagt gatgggagcc ttgaatacac caaaa         55

<210>  108
<211>  56
<212>  DNA
<213>  Artificial Sequence

<220>
<223>  primer

<220>
<221>  misc_feature
<222>  (23)..(26)
<223>  n is a, c, g, or t

<400>  108
ctacacgacg ctcttccgat ctnnnntggt tgcagggagc cttgaataca ccaaaa        56

<210>  109
<211>  53
<212>  DNA
<213>  Artificial Sequence

<220>
<223>  primer

<220>
<221>  misc_feature
<222>  23
<223>  n is a, c, g, or t

<400>  109
ctacacgacg ctcttccgat ctngtccgac cgggagcctt gaatacacca aaa          53

<210>  110
<211>  54
<212>  DNA
<213>  Artificial Sequence

<220>
<223>  primer

<220>
<221>  misc_feature
<222>  (23)..(24)
<223>  n is a, c, g, or t

<400>  110
ctacacgacg ctcttccgat ctnngccagc cggggagcct tgaatacacc aaaa          54

<210>  111
<211>  55
<212>  DNA
<213>  Artificial Sequence

<220>
<223>  primer

<220>
<221>  misc_feature
<222>  (23)..(25)
<223>  n is a, c, g, or t

<400>  111
ctacacgacg ctcttccgat ctnnnctcgt gtcgggagcc ttgaatacac caaaa          55

<210>  112
<211>  56
<212>  DNA
<213>  Artificial Sequence

<220>
<223>  primer

<220>
<221>  misc_feature
<222>  (23)..(26)
<223>  n is a, c, g, or t

<400>  112
ctacacgacg ctcttccgat ctnnnngccg tggcgggagc cttgaataca ccaaaa          56

<210>  113
<211>  53
<212>  DNA
<213>  Artificial Sequence

<220>
<223>  primer


<220>
<221>  misc_feature
<222>  23
<223>  n is a, c, g, or t

<400>  113
ctacacgacg ctcttccgat ctngtagcca cgggagcctt gaatacacca aaa                53


<210>  114
<211>  54
<212>  DNA
<213>  Artificial Sequence

<220>
<223>  primer


<220>
<221>  misc_feature
<222>  (23)..(24)
<223>  n is a, c, g, or t

<400>  114
ctacacgacg ctcttccgat ctnngactta tagggagcct tgaatacacc aaaa               54


<210>  115
<211>  55
<212>  DNA
<213>  Artificial Sequence

<220>
<223>  primer


<220>
<221>  misc_feature
<222>  (23)..(25)
<223>  n is a, c, g, or t

<400>  115
ctacacgacg ctcttccgat ctnnngaacg tcggggagcc ttgaatacac caaaa              55


<210>  116
<211>  56
<212>  DNA
<213>  Artificial Sequence

<220>

<223>  primer

<220>
<221>  misc_feature
<222>  (23)..(26)
<223>  n is a, c, g, or t

<400>  116
ctacacgacg ctcttccgat ctnnnnatgc gtaggggagc cttgaataca ccaaaa          56

<210>  117
<211>  53
<212>  DNA
<213>  Artificial Sequence

<220>
<223>  primer

<220>
<221>  misc_feature
<222>  23
<223>  n is a, c, g, or t

<400>  117
ctacacgacg ctcttccgat ctnaattaga tgggagcctt gaatacacca aaa             53

<210>  118
<211>  54
<212>  DNA
<213>  Artificial Sequence

<220>
<223>  primer

<220>
<221>  misc_feature
<222>  (23)..(24)
<223>  n is a, c, g, or t

<400>  118
ctacacgacg ctcttccgat ctnnttacga tagggagcct tgaatacacc aaaa            54

<210>  119
<211>  55
<212>  DNA
<213>  Artificial Sequence

<220>
<223>  primer

<220>
<221>  misc_feature
<222>  (23)..(25)

<223>  n is a, c, g, or t

<400>  119
ctacacgacg ctcttccgat ctnnnctgta caagggagcc ttgaatacac caaaa          55

<210>  120
<211>  56
<212>  DNA
<213>  Artificial Sequence

<220>
<223>  primer

<220>
<221>  misc_feature
<222>  (23)..(26)
<223>  n is a, c, g, or t

<400>  120
ctacacgacg ctcttccgat ctnnnncgtg gttggggagc cttgaataca ccaaaa         56

<210>  121
<211>  53
<212>  DNA
<213>  Artificial Sequence

<220>
<223>  primer

<220>
<221>  misc_feature
<222>  23
<223>  n is a, c, g, or t

<400>  121
ctacacgacg ctcttccgat ctnaataacg tgggagcctt gaatacacca aaa            53

<210>  122
<211>  54
<212>  DNA
<213>  Artificial Sequence

<220>
<223>  primer

<220>
<221>  misc_feature
<222>  (23)..(24)
<223>  n is a, c, g, or t

<400>  122
ctacacgacg ctcttccgat ctnnttcttg aagggagcct tgaatacacc aaaa           54

<210>  123
<211>  55
<212>  DNA
<213>  Artificial Sequence

<220>
<223>  primer

<220>
<221>  misc_feature
<222>  (23)..(25)
<223>  n is a, c, g, or t

<400>  123
ctacacgacg ctcttccgat ctnnnggcag atcgggagcc ttgaatacac caaaa          55

<210>  124
<211>  56
<212>  DNA
<213>  Artificial Sequence

<220>
<223>  primer

<220>
<221>  misc_feature
<222>  (23)..(26)
<223>  n is a, c, g, or t

<400>  124
ctacacgacg ctcttccgat ctnnnnctat gttagggagc cttgaataca ccaaaa          56

<210>  125
<211>  53
<212>  DNA
<213>  Artificial Sequence

<220>
<223>  primer

<220>
<221>  misc_feature
<222>  23
<223>  n is a, c, g, or t

<400>  125
ctacacgacg ctcttccgat ctngttgacg cgggagcctt gaatacacca aaa          53

<210>  126
<211>  54
<212>  DNA
<213>  Artificial Sequence

<220>
<223>  primer


<220>
<221>  misc_feature
<222>  (23)..(24)
<223>  n is a, c, g, or t

<400>  126
ctacacgacg ctcttccgat ctnnatctac gagggagcct tgaatacacc aaaa          54



<210>  127
<211>  55
<212>  DNA
<213>  Artificial Sequence

<220>
<223>  primer


<220>
<221>  misc_feature
<222>  (23)..(25)
<223>  n is a, c, g, or t

<400>  127
ctacacgacg ctcttccgat ctnnnctcga caggggagcc ttgaatacac caaaa          55



<210>  128
<211>  56
<212>  DNA
<213>  Artificial Sequence

<220>
<223>  primer


<220>
<221>  misc_feature
<222>  (23)..(26)
<223>  n is a, c, g, or t

<400>  128
ctacacgacg ctcttccgat ctnnnngagg ctgcgggagc cttgaataca ccaaaa          56



<210>  129
<211>  53
<212>  DNA
<213>  Artificial Sequence

<220>
<223>  primer


<220>
<221>  misc_feature

<222>  23
<223>  n is a, c, g, or t

<400>  129
ctacacgacg ctcttccgat ctncctcgta ggggagcctt gaatacacca aaa          53

<210>  130
<211>  54
<212>  DNA
<213>  Artificial Sequence

<220>
<223>  primer

<220>
<221>  misc_feature
<222>  (23)..(24)
<223>  n is a, c, g, or t

<400>  130
ctacacgacg ctcttccgat ctnncatagg cagggagcct tgaatacacc aaaa          54

<210>  131
<211>  55
<212>  DNA
<213>  Artificial Sequence

<220>
<223>  primer

<220>
<221>  misc_feature
<222>  (23)..(25)
<223>  n is a, c, g, or t

<400>  131
ctacacgacg ctcttccgat ctnnnagatg aacgggagcc ttgaatacac caaaa          55

<210>  132
<211>  56
<212>  DNA
<213>  Artificial Sequence

<220>
<223>  primer

<220>
<221>  misc_feature
<222>  (23)..(26)
<223>  n is a, c, g, or t

<400>  132
ctacacgacg ctcttccgat ctnnnnccga gtatgggagc cttgaataca ccaaaa          56

<210>  133
<211>  53
<212>  DNA
<213>  Artificial Sequence

<220>
<223>  primer


<220>
<221>  misc_feature
<222>  23
<223>  n is a, c, g, or t

<400>  133
ctacacgacg ctcttccgat ctnaatattg agggagcctt gaatacacca aaa          53


<210>  134
<211>  54
<212>  DNA
<213>  Artificial Sequence

<220>
<223>  primer


<220>
<221>  misc_feature
<222>  (23)..(24)
<223>  n is a, c, g, or t

<400>  134
ctacacgacg ctcttccgat ctnngtatac cggggagcct tgaatacacc aaaa          54


<210>  135
<211>  55
<212>  DNA
<213>  Artificial Sequence

<220>
<223>  primer


<220>
<221>  misc_feature
<222>  (23)..(25)
<223>  n is a, c, g, or t

<400>  135
ctacacgacg ctcttccgat ctnnngatcc aacgggagcc ttgaatacac caaaa          55


<210>  136
<211>  56
<212>  DNA
<213>  Artificial Sequence

<220>
<223>  primer


<220>
<221>  misc_feature
<222>  (23)..(26)
<223>  n is a, c, g, or t

<400>  136
ctacacgacg ctcttccgat ctnnnnagat acgcgggagc cttgaataca ccaaaa          56



<210>  137
<211>  53
<212>  DNA
<213>  Artificial Sequence

<220>
<223>  primer


<220>
<221>  misc_feature
<222>  23
<223>  n is a, c, g, or t

<400>  137
ctacacgacg ctcttccgat ctnggtatct tgggagcctt gaatacacca aaa             53



<210>  138
<211>  54
<212>  DNA
<213>  Artificial Sequence

<220>
<223>  primer


<220>
<221>  misc_feature
<222>  (23)..(24)
<223>  n is a, c, g, or t

<400>  138
ctacacgacg ctcttccgat ctnncctctg gcgggagcct tgaatacacc aaaa            54



<210>  139
<211>  53
<212>  DNA
<213>  Artificial Sequence

<220>
<223>  primer


<220>

<221>  misc_feature
<222>  23
<223>  n is a, c, g, or t

<400>  139
ctacacgacg ctcttccgat ctnccattgt ggggagcctt gaatacacca aaa          53



<210>  140
<211>  54
<212>  DNA
<213>  Artificial Sequence

<220>
<223>  primer


<220>
<221>  misc_feature
<222>  (23)..(24)
<223>  n is a, c, g, or t

<400>  140
ctacacgacg ctcttccgat ctnnactacg gtgggagcct tgaatacacc aaaa          54



<210>  141
<211>  55
<212>  DNA
<213>  Artificial Sequence

<220>
<223>  primer


<220>
<221>  misc_feature
<222>  (23)..(25)
<223>  n is a, c, g, or t

<400>  141
ctacacgacg ctcttccgat ctnnnaagtg ctagggagcc ttgaatacac caaaa          55



<210>  142
<211>  56
<212>  DNA
<213>  Artificial Sequence

<220>
<223>  primer


<220>
<221>  misc_feature
<222>  (23)..(26)
<223>  n is a, c, g, or t

<400>  142
ctacacgacg ctcttccgat ctnnnngccg aacggggagc cttgaataca ccaaaa          56

<210>  143
<211>  53
<212>  DNA
<213>  Artificial Sequence

<220>
<223>  primer


<220>
<221>  misc_feature
<222>  23
<223>  n is a, c, g, or t

<400>  143
ctacacgacg ctcttccgat ctntgtccac ggggagcctt gaatacacca aaa          53



<210>  144
<211>  54
<212>  DNA
<213>  Artificial Sequence

<220>
<223>  primer


<220>
<221>  misc_feature
<222>  (23)..(24)
<223>  n is a, c, g, or t

<400>  144
ctacacgacg ctcttccgat ctnngacaca ctgggagcct tgaatacacc aaaa          54



<210>  145
<211>  55
<212>  DNA
<213>  Artificial Sequence

<220>
<223>  primer


<220>
<221>  misc_feature
<222>  (23)..(25)
<223>  n is a, c, g, or t

<400>  145
ctacacgacg ctcttccgat ctnnnaatat gctgggagcc ttgaatacac caaaa          55



<210>  146
<211>  56
<212>  DNA

<213>  Artificial Sequence

<220>
<223>  primer


<220>
<221>  misc_feature
<222>  (23)..(26)
<223>  n is a, c, g, or t

<400>  146
ctacacgacg ctcttccgat ctnnnnttct catagggagc cttgaataca ccaaaa          56



<210>  147
<211>  53
<212>  DNA
<213>  Artificial Sequence

<220>
<223>  primer


<220>
<221>  misc_feature
<222>  23
<223>  n is a, c, g, or t

<400>  147
ctacacgacg ctcttccgat ctntctgtga tgggagcctt gaatacacca aaa          53



<210>  148
<211>  54
<212>  DNA
<213>  Artificial Sequence

<220>
<223>  primer


<220>
<221>  misc_feature
<222>  (23)..(24)
<223>  n is a, c, g, or t

<400>  148
ctacacgacg ctcttccgat ctnnccgaac ttgggagcct tgaatacacc aaaa          54



<210>  149
<211>  55
<212>  DNA
<213>  Artificial Sequence

<220>
<223>  primer

<220>
<221>  misc_feature
<222>  (23)..(25)
<223>  n is a, c, g, or t

<400>  149
ctacacgacg ctcttccgat ctnnngtcta acagggagcc ttgaatacac caaaa          55



<210>  150
<211>  56
<212>  DNA
<213>  Artificial Sequence

<220>
<223>  primer


<220>
<221>  misc_feature
<222>  (23)..(26)
<223>  n is a, c, g, or t

<400>  150
ctacacgacg ctcttccgat ctnnnngacg ccatgggagc cttgaataca ccaaaa         56



<210>  151
<211>  53
<212>  DNA
<213>  Artificial Sequence

<220>
<223>  primer


<220>
<221>  misc_feature
<222>  23
<223>  n is a, c, g, or t

<400>  151
ctacacgacg ctcttccgat ctngccaatg tgggagcctt gaatacacca aaa            53



<210>  152
<211>  54
<212>  DNA
<213>  Artificial Sequence

<220>
<223>  primer


<220>
<221>  misc_feature
<222>  (23)..(24)
<223>  n is a, c, g, or t

<400>  152

ctacacgacg ctcttccgat ctnnccaacg tcgggagcct tgaatacacc aaaa 54

<210> 153
<211> 55
<212> DNA
<213> Artificial Sequence

<220>
<223> primer

<220>
<221> misc_feature
<222> (23)..(25)
<223> n is a, c, g, or t

<400> 153
ctacacgacg ctcttccgat ctnnngtaga taagggagcc ttgaatacac caaaa 55

<210> 154
<211> 56
<212> DNA
<213> Artificial Sequence

<220>
<223> primer

<220>
<221> misc_feature
<222> (23)..(26)
<223> n is a, c, g, or t

<400> 154
ctacacgacg ctcttccgat ctnnnnctta cggcgggagc cttgaataca ccaaaa 56

<210> 155
<211> 53
<212> DNA
<213> Artificial Sequence

<220>
<223> primer

<220>
<221> misc_feature
<222> 23
<223> n is a, c, g, or t

<400> 155
ctacacgacg ctcttccgat ctnccaagtg cgggagcctt gaatacacca aaa 53

<210> 156
<211> 54

<212>  DNA
<213>  Artificial Sequence

<220>
<223>  primer


<220>
<221>  misc_feature
<222>  (23)..(24)
<223>  n is a, c, g, or t

<400>  156
ctacacgacg ctcttccgat ctnnctaact cagggagcct tgaatacacc aaaa          54



<210>  157
<211>  55
<212>  DNA
<213>  Artificial Sequence

<220>
<223>  primer


<220>
<221>  misc_feature
<222>  (23)..(25)
<223>  n is a, c, g, or t

<400>  157
ctacacgacg ctcttccgat ctnnnaatat ctggggagcc ttgaatacac caaaa          55



<210>  158
<211>  56
<212>  DNA
<213>  Artificial Sequence

<220>
<223>  primer


<220>
<221>  misc_feature
<222>  (23)..(26)
<223>  n is a, c, g, or t

<400>  158
ctacacgacg ctcttccgat ctnnnnttat atcagggagc cttgaataca ccaaaa          56



<210>  159
<211>  53
<212>  DNA
<213>  Artificial Sequence

<220>
<223>  primer

<220>
<221>  misc_feature
<222>  23
<223>  n is a, c, g, or t

<400>  159
ctacacgacg ctcttccgat ctnctgcgga tgggagcctt gaatacacca aaa                53

<210>  160
<211>  54
<212>  DNA
<213>  Artificial Sequence

<220>
<223>  primer

<220>
<221>  misc_feature
<222>  (23)..(24)
<223>  n is a, c, g, or t

<400>  160
ctacacgacg ctcttccgat ctnngcggct tggggagcct tgaatacacc aaaa               54

<210>  161
<211>  55
<212>  DNA
<213>  Artificial Sequence

<220>
<223>  primer

<220>
<221>  misc_feature
<222>  (23)..(25)
<223>  n is a, c, g, or t

<400>  161
ctacacgacg ctcttccgat ctnnngagtt gatgggagcc ttgaatacac caaaa              55

<210>  162
<211>  56
<212>  DNA
<213>  Artificial Sequence

<220>
<223>  primer

<220>
<221>  misc_feature
<222>  (23)..(26)
<223>  n is a, c, g, or t

<400>  162
ctacacgacg ctcttccgat ctnnnngcac tgaggggagc cttgaataca ccaaaa          56

<210>  163
<211>  53
<212>  DNA
<213>  Artificial Sequence

<220>
<223>  primer

<220>
<221>  misc_feature
<222>  23
<223>  n is a, c, g, or t

<400>  163
ctacacgacg ctcttccgat ctngaccacc tgggagcctt gaatacacca aaa          53

<210>  164
<211>  53
<212>  DNA
<213>  Artificial Sequence

<220>
<223>  primer

<220>
<221>  misc_feature
<222>  23
<223>  n is a, c, g, or t

<400>  164
ctacacgacg ctcttccgat ctnaatagca agggagcctt gaatacacca aaa          53

<210>  165
<211>  53
<212>  DNA
<213>  Artificial Sequence

<220>
<223>  primer

<220>
<221>  misc_feature
<222>  23
<223>  n is a, c, g, or t

<400>  165
ctacacgacg ctcttccgat ctncctaccg ggggagcctt gaatacacca aaa          53

<210>  166

<211>  54
<212>  DNA
<213>  Artificial Sequence

<220>
<223>  primer


<220>
<221>  misc_feature
<222>  (23)..(24)
<223>  n is a, c, g, or t

<400>  166
ctacacgacg ctcttccgat ctnnggagga tggggagcct tgaatacacc aaaa          54



<210>  167
<211>  55
<212>  DNA
<213>  Artificial Sequence

<220>
<223>  primer


<220>
<221>  misc_feature
<222>  (23)..(25)
<223>  n is a, c, g, or t

<400>  167
ctacacgacg ctcttccgat ctnnncgctg aatgggagcc ttgaatacac caaaa          55



<210>  168
<211>  56
<212>  DNA
<213>  Artificial Sequence

<220>
<223>  primer


<220>
<221>  misc_feature
<222>  (23)..(26)
<223>  n is a, c, g, or t

<400>  168
ctacacgacg ctcttccgat ctnnnntgtg acgagggagc cttgaataca ccaaaa          56



<210>  169
<211>  53
<212>  DNA
<213>  Artificial Sequence

<220>
<223>  primer

<220>
<221>  misc_feature
<222>  23
<223>  n is a, c, g, or t

<400>  169
ctacacgacg ctcttccgat ctnaatagat tgggagcctt gaatacacca aaa          53




<210>  170
<211>  54
<212>  DNA
<213>  Artificial Sequence

<220>
<223>  primer



<220>
<221>  misc_feature
<222>  (23)..(24)
<223>  n is a, c, g, or t

<400>  170
ctacacgacg ctcttccgat ctnnttagcg cagggagcct tgaatacacc aaaa          54




<210>  171
<211>  55
<212>  DNA
<213>  Artificial Sequence

<220>
<223>  primer



<220>
<221>  misc_feature
<222>  (23)..(25)
<223>  n is a, c, g, or t

<400>  171
ctacacgacg ctcttccgat ctnnngcggc cgtgggagcc ttgaatacac caaaa          55




<210>  172
<211>  56
<212>  DNA
<213>  Artificial Sequence

<220>
<223>  primer



<220>
<221>  misc_feature
<222>  (23)..(26)
<223>  n is a, c, g, or t

<400>  172
ctacacgacg ctcttccgat ctnnnncagt aaccgggagc cttgaataca ccaaaa          56

<210>  173
<211>  53
<212>  DNA
<213>  Artificial Sequence

<220>
<223>  primer

<220>
<221>  misc_feature
<222>  23
<223>  n is a, c, g, or t

<400>  173
ctacacgacg ctcttccgat ctngcctagt agggagcctt gaatacacca aaa             53

<210>  174
<211>  54
<212>  DNA
<213>  Artificial Sequence

<220>
<223>  primer

<220>
<221>  misc_feature
<222>  (23)..(24)
<223>  n is a, c, g, or t

<400>  174
ctacacgacg ctcttccgat ctnncacggc gcgggagcct tgaatacacc aaaa            54

<210>  175
<211>  55
<212>  DNA
<213>  Artificial Sequence

<220>
<223>  primer

<220>
<221>  misc_feature
<222>  (23)..(25)
<223>  n is a, c, g, or t

<400>  175
ctacacgacg ctcttccgat ctnnnggtgc agagggagcc ttgaatacac caaaa           55

<210>  176
<211>  56
<212>  DNA
<213>  Artificial Sequence

<220>
<223>  primer



<220>
<221>  misc_feature
<222>  (23)..(26)
<223>  n is a, c, g, or t

<400>  176
ctacacgacg ctcttccgat ctnnnntcgc tgacgggagc cttgaataca ccaaaa          56




<210>  177
<211>  53
<212>  DNA
<213>  Artificial Sequence

<220>
<223>  primer



<220>
<221>  misc_feature
<222>  23
<223>  n is a, c, g, or t

<400>  177
ctacacgacg ctcttccgat ctncagccag tgggagcctt gaatacacca aaa             53




<210>  178
<211>  54
<212>  DNA
<213>  Artificial Sequence

<220>
<223>  primer



<220>
<221>  misc_feature
<222>  (23)..(24)
<223>  n is a, c, g, or t

<400>  178
ctacacgacg ctcttccgat ctnncgtcaa ccgggagcct tgaatacacc aaaa            54




<210>  179
<211>  53
<212>  DNA
<213>  Artificial Sequence

<220>

<223>  primer

<220>
<221>  misc_feature
<222>  23
<223>  n is a, c, g, or t

<400>  179
ctacacgacg ctcttccgat ctngccggcg agggagcctt gaatacacca aaa          53

<210>  180
<211>  54
<212>  DNA
<213>  Artificial Sequence

<220>
<223>  primer

<220>
<221>  misc_feature
<222>  (23)..(24)
<223>  n is a, c, g, or t

<400>  180
ctacacgacg ctcttccgat ctnngcctcc gggggagcct tgaatacacc aaaa          54

<210>  181
<211>  55
<212>  DNA
<213>  Artificial Sequence

<220>
<223>  primer

<220>
<221>  misc_feature
<222>  (23)..(25)
<223>  n is a, c, g, or t

<400>  181
ctacacgacg ctcttccgat ctnnnaatag tccgggagcc ttgaatacac caaaa          55

<210>  182
<211>  56
<212>  DNA
<213>  Artificial Sequence

<220>
<223>  primer

<220>
<221>  misc_feature
<222>  (23)..(26)

<223>  n is a, c, g, or t

<400>  182
ctacacgacg ctcttccgat ctnnnnttag acgtgggagc cttgaataca ccaaaa          56




<210>  183
<211>  53
<212>  DNA
<213>  Artificial Sequence

<220>
<223>  primer



<220>
<221>  misc_feature
<222>  23
<223>  n is a, c, g, or t

<400>  183
ctacacgacg ctcttccgat ctngtggact agggagcctt gaatacacca aaa             53




<210>  184
<211>  54
<212>  DNA
<213>  Artificial Sequence

<220>
<223>  primer



<220>
<221>  misc_feature
<222>  (23)..(24)
<223>  n is a, c, g, or t

<400>  184
ctacacgacg ctcttccgat ctnncacgga cggggagcct tgaatacacc aaaa            54




<210>  185
<211>  55
<212>  DNA
<213>  Artificial Sequence

<220>
<223>  primer



<220>
<221>  misc_feature
<222>  (23)..(25)
<223>  n is a, c, g, or t

<400>  185
ctacacgacg ctcttccgat ctnnncacta gaggggagcc ttgaatacac caaaa           55

<210>  186
<211>  56
<212>  DNA
<213>  Artificial Sequence

<220>
<223>  primer

<220>
<221>  misc_feature
<222>  (23)..(26)
<223>  n is a, c, g, or t

<400>  186
ctacacgacg ctcttccgat ctnnnngcag atgggggagc cttgaataca ccaaaa          56

<210>  187
<211>  53
<212>  DNA
<213>  Artificial Sequence

<220>
<223>  primer

<220>
<221>  misc_feature
<222>  23
<223>  n is a, c, g, or t

<400>  187
ctacacgacg ctcttccgat ctnctctcac ggggagcctt gaatacacca aaa          53

<210>  188
<211>  54
<212>  DNA
<213>  Artificial Sequence

<220>
<223>  primer

<220>
<221>  misc_feature
<222>  (23)..(24)
<223>  n is a, c, g, or t

<400>  188
ctacacgacg ctcttccgat ctnnggaatc acgggagcct tgaatacacc aaaa          54

<210>  189
<211>  55
<212>  DNA
<213>  Artificial Sequence

<220>
<223>  primer


<220>
<221>  misc_feature
<222>  (23)..(25)
<223>  n is a, c, g, or t

<400>  189
ctacacgacg ctcttccgat ctnnncgttg acggggagcc ttgaatacac caaaa          55



<210>  190
<211>  56
<212>  DNA
<213>  Artificial Sequence

<220>
<223>  primer


<220>
<221>  misc_feature
<222>  (23)..(26)
<223>  n is a, c, g, or t

<400>  190
ctacacgacg ctcttccgat ctnnnncatc aggtgggagc cttgaataca ccaaaa          56



<210>  191
<211>  53
<212>  DNA
<213>  Artificial Sequence

<220>
<223>  primer


<220>
<221>  misc_feature
<222>  23
<223>  n is a, c, g, or t

<400>  191
ctacacgacg ctcttccgat ctncgttgta agggagcctt gaatacacca aaa          53



<210>  192
<211>  54
<212>  DNA
<213>  Artificial Sequence

<220>
<223>  primer


<220>
<221>  misc_feature

<222>  (23)..(24)
<223>  n is a, c, g, or t

<400>  192
ctacacgacg ctcttccgat ctnnggcacg gtgggagcct tgaatacacc aaaa          54




<210>  193
<211>  52
<212>  DNA
<213>  Artificial Sequence

<220>
<223>  primer


<220>
<221>  misc_feature
<222>  23
<223>  n is a, c, g, or t

<400>  193
ctacacgacg ctcttccgat ctnccaatac tggcattcgt attgcgccgc ta            52




<210>  194
<211>  53
<212>  DNA
<213>  Artificial Sequence

<220>
<223>  primer


<220>
<221>  misc_feature
<222>  (23)..(24)
<223>  n is a, c, g, or t

<400>  194
ctacacgacg ctcttccgat ctnntcgcgc atggcattcg tattgcgccg cta           53




<210>  195
<211>  54
<212>  DNA
<213>  Artificial Sequence

<220>
<223>  primer


<220>
<221>  misc_feature
<222>  (23)..(25)
<223>  n is a, c, g, or t

<400>  195
ctacacgacg ctcttccgat ctnnngtgtg aacggcattc gtattgcgcc gcta          54

<210>  196
<211>  55
<212>  DNA
<213>  Artificial Sequence

<220>
<223>  primer

<220>
<221>  misc_feature
<222>  (23)..(26)
<223>  n is a, c, g, or t

<400>  196
ctacacgacg ctcttccgat ctnnnnggtg gcacggcatt cgtattgcgc cgcta          55

<210>  197
<211>  52
<212>  DNA
<213>  Artificial Sequence

<220>
<223>  primer

<220>
<221>  misc_feature
<222>  23
<223>  n is a, c, g, or t

<400>  197
ctacacgacg ctcttccgat ctntaggtgc tggcattcgt attgcgccgc ta          52

<210>  198
<211>  53
<212>  DNA
<213>  Artificial Sequence

<220>
<223>  primer

<220>
<221>  misc_feature
<222>  (23)..(24)
<223>  n is a, c, g, or t

<400>  198
ctacacgacg ctcttccgat ctnngagcgg ttggcattcg tattgcgccg cta          53

<210>  199
<211>  54
<212>  DNA
<213>  Artificial Sequence

<220>
<223>  primer


<220>
<221>  misc_feature
<222>  (23)..(25)
<223>  n is a, c, g, or t

<400>  199
ctacacgacg ctcttccgat ctnnnaggat tagggcattc gtattgcgcc gcta          54



<210>  200
<211>  55
<212>  DNA
<213>  Artificial Sequence

<220>
<223>  primer


<220>
<221>  misc_feature
<222>  (23)..(26)
<223>  n is a, c, g, or t

<400>  200
ctacacgacg ctcttccgat ctnnnngtga gccaggcatt cgtattgcgc cgcta         55



<210>  201
<211>  52
<212>  DNA
<213>  Artificial Sequence

<220>
<223>  primer


<220>
<221>  misc_feature
<222>  23
<223>  n is a, c, g, or t

<400>  201
ctacacgacg ctcttccgat ctnaattatg cggcattcgt attgcgccgc ta            52



<210>  202
<211>  53
<212>  DNA
<213>  Artificial Sequence

<220>
<223>  primer


<220>

<221>  misc_feature
<222>  (23)..(24)
<223>  n is a, c, g, or t

<400>  202
ctacacgacg ctcttccgat ctnnttacgc cgggcattcg tattgcgccg cta          53



<210>  203
<211>  54
<212>  DNA
<213>  Artificial Sequence

<220>
<223>  primer


<220>
<221>  misc_feature
<222>  (23)..(25)
<223>  n is a, c, g, or t

<400>  203
ctacacgacg ctcttccgat ctnnngtagt gatggcattc gtattgcgcc gcta          54



<210>  204
<211>  55
<212>  DNA
<213>  Artificial Sequence

<220>
<223>  primer


<220>
<221>  misc_feature
<222>  (23)..(26)
<223>  n is a, c, g, or t

<400>  204
ctacacgacg ctcttccgat ctnnnntggt tgcaggcatt cgtattgcgc cgcta          55



<210>  205
<211>  52
<212>  DNA
<213>  Artificial Sequence

<220>
<223>  primer


<220>
<221>  misc_feature
<222>  23
<223>  n is a, c, g, or t

<400>  205
ctacacgacg ctcttccgat ctngtccgac cggcattcgt attgcgccgc ta          52

<210>  206
<211>  53
<212>  DNA
<213>  Artificial Sequence

<220>
<223>  primer


<220>
<221>  misc_feature
<222>  (23)..(24)
<223>  n is a, c, g, or t

<400>  206
ctacacgacg ctcttccgat ctnngccagc cgggcattcg tattgcgccg cta          53


<210>  207
<211>  54
<212>  DNA
<213>  Artificial Sequence

<220>
<223>  primer


<220>
<221>  misc_feature
<222>  (23)..(25)
<223>  n is a, c, g, or t

<400>  207
ctacacgacg ctcttccgat ctnnnctcgt gtcggcattc gtattgcgcc gcta          54


<210>  208
<211>  55
<212>  DNA
<213>  Artificial Sequence

<220>
<223>  primer


<220>
<221>  misc_feature
<222>  (23)..(26)
<223>  n is a, c, g, or t

<400>  208
ctacacgacg ctcttccgat ctnnnngccg tggcggcatt cgtattgcgc cgcta          55


<210>  209
<211>  52
<212>  DNA

<213>  Artificial Sequence

<220>
<223>  primer

<220>
<221>  misc_feature
<222>  23
<223>  n is a, c, g, or t

<400>  209
ctacacgacg ctcttccgat ctngtagcca cggcattcgt attgcgccgc ta          52

<210>  210
<211>  53
<212>  DNA
<213>  Artificial Sequence

<220>
<223>  primer

<220>
<221>  misc_feature
<222>  (23)..(24)
<223>  n is a, c, g, or t

<400>  210
ctacacgacg ctcttccgat ctnngactta taggcattcg tattgcgccg cta          53

<210>  211
<211>  54
<212>  DNA
<213>  Artificial Sequence

<220>
<223>  primer

<220>
<221>  misc_feature
<222>  (23)..(25)
<223>  n is a, c, g, or t

<400>  211
ctacacgacg ctcttccgat ctnnngaacg tcgggcattc gtattgcgcc gcta          54

<210>  212
<211>  55
<212>  DNA
<213>  Artificial Sequence

<220>
<223>  primer

<220>
<221>  misc_feature
<222>  (23)..(26)
<223>  n is a, c, g, or t

<400>  212
ctacacgacg ctcttccgat ctnnnnatgc gtagggcatt cgtattgcgc cgcta          55



<210>  213
<211>  52
<212>  DNA
<213>  Artificial Sequence

<220>
<223>  primer


<220>
<221>  misc_feature
<222>  23
<223>  n is a, c, g, or t

<400>  213
ctacacgacg ctcttccgat ctnaattaga tggcattcgt attgcgccgc ta             52



<210>  214
<211>  53
<212>  DNA
<213>  Artificial Sequence

<220>
<223>  primer


<220>
<221>  misc_feature
<222>  (23)..(24)
<223>  n is a, c, g, or t

<400>  214
ctacacgacg ctcttccgat ctnnttacga taggcattcg tattgcgccg cta            53



<210>  215
<211>  54
<212>  DNA
<213>  Artificial Sequence

<220>
<223>  primer


<220>
<221>  misc_feature
<222>  (23)..(25)
<223>  n is a, c, g, or t

<400>  215

ctacacgacg ctcttccgat ctnnnctgta caaggcattc gtattgcgcc gcta 54

<210> 216
<211> 55
<212> DNA
<213> Artificial Sequence

<220>
<223> primer

<220>
<221> misc_feature
<222> (23)..(26)
<223> n is a, c, g, or t

<400> 216
ctacacgacg ctcttccgat ctnnnncgtg gttgggcatt cgtattgcgc cgcta 55

<210> 217
<211> 52
<212> DNA
<213> Artificial Sequence

<220>
<223> primer

<220>
<221> misc_feature
<222> 23
<223> n is a, c, g, or t

<400> 217
ctacacgacg ctcttccgat ctnaataacg tggcattcgt attgcgccgc ta 52

<210> 218
<211> 53
<212> DNA
<213> Artificial Sequence

<220>
<223> primer

<220>
<221> misc_feature
<222> (23)..(24)
<223> n is a, c, g, or t

<400> 218
ctacacgacg ctcttccgat ctnnttcttg aaggcattcg tattgcgccg cta 53

<210> 219
<211> 54

<212>  DNA
<213>  Artificial Sequence

<220>
<223>  primer


<220>
<221>  misc_feature
<222>  (23)..(25)
<223>  n is a, c, g, or t

<400>  219
ctacacgacg ctcttccgat ctnnnggcag atcggcattc gtattgcgcc gcta          54



<210>  220
<211>  55
<212>  DNA
<213>  Artificial Sequence

<220>
<223>  primer


<220>
<221>  misc_feature
<222>  (23)..(26)
<223>  n is a, c, g, or t

<400>  220
ctacacgacg ctcttccgat ctnnnnctat gttaggcatt cgtattgcgc cgcta          55



<210>  221
<211>  52
<212>  DNA
<213>  Artificial Sequence

<220>
<223>  primer


<220>
<221>  misc_feature
<222>  23
<223>  n is a, c, g, or t

<400>  221
ctacacgacg ctcttccgat ctngttgacg cggcattcgt attgcgccgc ta          52



<210>  222
<211>  53
<212>  DNA
<213>  Artificial Sequence

<220>
<223>  primer

<220>
<221>  misc_feature
<222>  (23)..(24)
<223>  n is a, c, g, or t

<400>  222
ctacacgacg ctcttccgat ctnnatctac gaggcattcg tattgcgccg cta          53



<210>  223
<211>  54
<212>  DNA
<213>  Artificial Sequence

<220>
<223>  primer


<220>
<221>  misc_feature
<222>  (23)..(25)
<223>  n is a, c, g, or t

<400>  223
ctacacgacg ctcttccgat ctnnnctcga cagggcattc gtattgcgcc gcta          54



<210>  224
<211>  55
<212>  DNA
<213>  Artificial Sequence

<220>
<223>  primer


<220>
<221>  misc_feature
<222>  (23)..(26)
<223>  n is a, c, g, or t

<400>  224
ctacacgacg ctcttccgat ctnnnngagg ctgcggcatt cgtattgcgc cgcta          55



<210>  225
<211>  52
<212>  DNA
<213>  Artificial Sequence

<220>
<223>  primer


<220>
<221>  misc_feature
<222>  23
<223>  n is a, c, g, or t

<400>  225
ctacacgacg ctcttccgat ctncctcgta gggcattcgt attgcgccgc ta          52

<210>  226
<211>  53
<212>  DNA
<213>  Artificial Sequence

<220>
<223>  primer

<220>
<221>  misc_feature
<222>  (23)..(24)
<223>  n is a, c, g, or t

<400>  226
ctacacgacg ctcttccgat ctnncatagg caggcattcg tattgcgccg cta          53

<210>  227
<211>  54
<212>  DNA
<213>  Artificial Sequence

<220>
<223>  primer

<220>
<221>  misc_feature
<222>  (23)..(25)
<223>  n is a, c, g, or t

<400>  227
ctacacgacg ctcttccgat ctnnnagatg aacggcattc gtattgcgcc gcta          54

<210>  228
<211>  55
<212>  DNA
<213>  Artificial Sequence

<220>
<223>  primer

<220>
<221>  misc_feature
<222>  (23)..(26)
<223>  n is a, c, g, or t

<400>  228
ctacacgacg ctcttccgat ctnnnnccga gtatggcatt cgtattgcgc cgcta          55

<210>  229

<211>  52
<212>  DNA
<213>  Artificial Sequence

<220>
<223>  primer


<220>
<221>  misc_feature
<222>  23
<223>  n is a, c, g, or t

<400>  229
ctacacgacg ctcttccgat ctnaatattg aggcattcgt attgcgccgc ta          52


<210>  230
<211>  53
<212>  DNA
<213>  Artificial Sequence

<220>
<223>  primer


<220>
<221>  misc_feature
<222>  (23)..(24)
<223>  n is a, c, g, or t

<400>  230
ctacacgacg ctcttccgat ctnngtatac cgggcattcg tattgcgccg cta          53


<210>  231
<211>  54
<212>  DNA
<213>  Artificial Sequence

<220>
<223>  primer


<220>
<221>  misc_feature
<222>  (23)..(25)
<223>  n is a, c, g, or t

<400>  231
ctacacgacg ctcttccgat ctnnngatcc aacggcattc gtattgcgcc gcta          54


<210>  232
<211>  55
<212>  DNA
<213>  Artificial Sequence

<220>
<223>  primer

<220>
<221>  misc_feature
<222>  (23)..(26)
<223>  n is a, c, g, or t

<400>  232
ctacacgacg ctcttccgat ctnnnnagat acgcggcatt cgtattgcgc cgcta          55



<210>  233
<211>  52
<212>  DNA
<213>  Artificial Sequence

<220>
<223>  primer


<220>
<221>  misc_feature
<222>  23
<223>  n is a, c, g, or t

<400>  233
ctacacgacg ctcttccgat ctnggtatct tggcattcgt attgcgccgc ta          52



<210>  234
<211>  53
<212>  DNA
<213>  Artificial Sequence

<220>
<223>  primer


<220>
<221>  misc_feature
<222>  (23)..(24)
<223>  n is a, c, g, or t

<400>  234
ctacacgacg ctcttccgat ctnncctctg gcggcattcg tattgcgccg cta          53



<210>  235
<211>  52
<212>  DNA
<213>  Artificial Sequence

<220>
<223>  primer


<220>
<221>  misc_feature
<222>  23
<223>  n is a, c, g, or t

<400>  235
ctacacgacg ctcttccgat ctnccattgt gggcattcgt attgcgccgc ta                52

<210>  236
<211>  53
<212>  DNA
<213>  Artificial Sequence

<220>
<223>  primer

<220>
<221>  misc_feature
<222>  (23)..(24)
<223>  n is a, c, g, or t

<400>  236
ctacacgacg ctcttccgat ctnnactacg gtggcattcg tattgcgccg cta               53

<210>  237
<211>  54
<212>  DNA
<213>  Artificial Sequence

<220>
<223>  primer

<220>
<221>  misc_feature
<222>  (23)..(25)
<223>  n is a, c, g, or t

<400>  237
ctacacgacg ctcttccgat ctnnnaagtg ctaggcattc gtattgcgcc gcta              54

<210>  238
<211>  55
<212>  DNA
<213>  Artificial Sequence

<220>
<223>  primer

<220>
<221>  misc_feature
<222>  (23)..(26)
<223>  n is a, c, g, or t

<400>  238
ctacacgacg ctcttccgat ctnnnngccg aacgggcatt cgtattgcgc cgcta             55

<210>  239
<211>  52
<212>  DNA
<213>  Artificial Sequence

<220>
<223>  primer


<220>
<221>  misc_feature
<222>  23
<223>  n is a, c, g, or t

<400>  239
ctacacgacg ctcttccgat ctntgtccac gggcattcgt attgcgccgc ta                52



<210>  240
<211>  53
<212>  DNA
<213>  Artificial Sequence

<220>
<223>  primer


<220>
<221>  misc_feature
<222>  (23)..(24)
<223>  n is a, c, g, or t

<400>  240
ctacacgacg ctcttccgat ctnngacaca ctggcattcg tattgcgccg cta               53



<210>  241
<211>  54
<212>  DNA
<213>  Artificial Sequence

<220>
<223>  primer


<220>
<221>  misc_feature
<222>  (23)..(25)
<223>  n is a, c, g, or t

<400>  241
ctacacgacg ctcttccgat ctnnnaatat gctggcattc gtattgcgcc gcta              54



<210>  242
<211>  55
<212>  DNA
<213>  Artificial Sequence

<220>

<223>  primer

<220>
<221>  misc_feature
<222>  (23)..(26)
<223>  n is a, c, g, or t

<400>  242
ctacacgacg ctcttccgat ctnnnnttct cataggcatt cgtattgcgc cgcta          55

<210>  243
<211>  52
<212>  DNA
<213>  Artificial Sequence

<220>
<223>  primer

<220>
<221>  misc_feature
<222>  23
<223>  n is a, c, g, or t

<400>  243
ctacacgacg ctcttccgat ctntctgtga tggcattcgt attgcgccgc ta          52

<210>  244
<211>  53
<212>  DNA
<213>  Artificial Sequence

<220>
<223>  primer

<220>
<221>  misc_feature
<222>  (23)..(24)
<223>  n is a, c, g, or t

<400>  244
ctacacgacg ctcttccgat ctnnccgaac ttggcattcg tattgcgccg cta          53

<210>  245
<211>  54
<212>  DNA
<213>  Artificial Sequence

<220>
<223>  primer

<220>
<221>  misc_feature
<222>  (23)..(25)

<223>  n is a, c, g, or t

<400>  245
ctacacgacg ctcttccgat ctnnngtcta acaggcattc gtattgcgcc gcta          54

<210>  246
<211>  55
<212>  DNA
<213>  Artificial Sequence

<220>
<223>  primer

<220>
<221>  misc_feature
<222>  (23)..(26)
<223>  n is a, c, g, or t

<400>  246
ctacacgacg ctcttccgat ctnnnngacg ccatggcatt cgtattgcgc cgcta          55

<210>  247
<211>  52
<212>  DNA
<213>  Artificial Sequence

<220>
<223>  primer

<220>
<221>  misc_feature
<222>  23
<223>  n is a, c, g, or t

<400>  247
ctacacgacg ctcttccgat ctngccaatg tggcattcgt attgcgccgc ta          52

<210>  248
<211>  53
<212>  DNA
<213>  Artificial Sequence

<220>
<223>  primer

<220>
<221>  misc_feature
<222>  (23)..(24)
<223>  n is a, c, g, or t

<400>  248
ctacacgacg ctcttccgat ctnnccaacg tcggcattcg tattgcgccg cta          53

<210>  249
<211>  54
<212>  DNA
<213>  Artificial Sequence

<220>
<223>  primer


<220>
<221>  misc_feature
<222>  (23)..(25)
<223>  n is a, c, g, or t

<400>  249
ctacacgacg ctcttccgat ctnnngtaga taaggcattc gtattgcgcc gcta          54


<210>  250
<211>  55
<212>  DNA
<213>  Artificial Sequence

<220>
<223>  primer


<220>
<221>  misc_feature
<222>  (23)..(26)
<223>  n is a, c, g, or t

<400>  250
ctacacgacg ctcttccgat ctnnnnctta cggcggcatt cgtattgcgc cgcta          55


<210>  251
<211>  52
<212>  DNA
<213>  Artificial Sequence

<220>
<223>  primer


<220>
<221>  misc_feature
<222>  23
<223>  n is a, c, g, or t

<400>  251
ctacacgacg ctcttccgat ctnccaagtg cggcattcgt attgcgccgc ta          52


<210>  252
<211>  53
<212>  DNA
<213>  Artificial Sequence

<220>
<223>  primer


<220>
<221>  misc_feature
<222>  (23)..(24)
<223>  n is a, c, g, or t

<400>  252
ctacacgacg ctcttccgat ctnnctaact caggcattcg tattgcgccg cta          53



<210>  253
<211>  54
<212>  DNA
<213>  Artificial Sequence

<220>
<223>  primer


<220>
<221>  misc_feature
<222>  (23)..(25)
<223>  n is a, c, g, or t

<400>  253
ctacacgacg ctcttccgat ctnnnaatat ctgggcattc gtattgcgcc gcta          54



<210>  254
<211>  55
<212>  DNA
<213>  Artificial Sequence

<220>
<223>  primer


<220>
<221>  misc_feature
<222>  (23)..(26)
<223>  n is a, c, g, or t

<400>  254
ctacacgacg ctcttccgat ctnnnnttat atcaggcatt cgtattgcgc cgcta          55



<210>  255
<211>  52
<212>  DNA
<213>  Artificial Sequence

<220>
<223>  primer


<220>
<221>  misc_feature

<222>  23
<223>  n is a, c, g, or t

<400>  255
ctacacgacg ctcttccgat ctnctgcgga tggcattcgt attgcgccgc ta                52




<210>  256
<211>  53
<212>  DNA
<213>  Artificial Sequence

<220>
<223>  primer


<220>
<221>  misc_feature
<222>  (23)..(24)
<223>  n is a, c, g, or t

<400>  256
ctacacgacg ctcttccgat ctnngcggct tgggcattcg tattgcgccg cta               53




<210>  257
<211>  54
<212>  DNA
<213>  Artificial Sequence

<220>
<223>  primer


<220>
<221>  misc_feature
<222>  (23)..(25)
<223>  n is a, c, g, or t

<400>  257
ctacacgacg ctcttccgat ctnnngagtt gatggcattc gtattgcgcc gcta              54




<210>  258
<211>  55
<212>  DNA
<213>  Artificial Sequence

<220>
<223>  primer


<220>
<221>  misc_feature
<222>  (23)..(26)
<223>  n is a, c, g, or t

<400>  258
ctacacgacg ctcttccgat ctnnnngcac tgagggcatt cgtattgcgc cgcta             55

<210>  259
<211>  52
<212>  DNA
<213>  Artificial Sequence

<220>
<223>  primer


<220>
<221>  misc_feature
<222>  23
<223>  n is a, c, g, or t

<400>  259
ctacacgacg ctcttccgat ctngaccacc tggcattcgt attgcgccgc ta          52


<210>  260
<211>  52
<212>  DNA
<213>  Artificial Sequence

<220>
<223>  primer


<220>
<221>  misc_feature
<222>  23
<223>  n is a, c, g, or t

<400>  260
ctacacgacg ctcttccgat ctnaatagca aggcattcgt attgcgccgc ta          52


<210>  261
<211>  52
<212>  DNA
<213>  Artificial Sequence

<220>
<223>  primer


<220>
<221>  misc_feature
<222>  23
<223>  n is a, c, g, or t

<400>  261
ctacacgacg ctcttccgat ctncctaccg gggcattcgt attgcgccgc ta          52


<210>  262
<211>  53
<212>  DNA
<213>  Artificial Sequence

<220>
<223>  primer


<220>
<221>  misc_feature
<222>  (23)..(24)
<223>  n is a, c, g, or t

<400>  262
ctacacgacg ctcttccgat ctnnggagga tgggcattcg tattgcgccg cta          53



<210>  263
<211>  54
<212>  DNA
<213>  Artificial Sequence

<220>
<223>  primer


<220>
<221>  misc_feature
<222>  (23)..(25)
<223>  n is a, c, g, or t

<400>  263
ctacacgacg ctcttccgat ctnnncgctg aatggcattc gtattgcgcc gcta          54



<210>  264
<211>  55
<212>  DNA
<213>  Artificial Sequence

<220>
<223>  primer


<220>
<221>  misc_feature
<222>  (23)..(26)
<223>  n is a, c, g, or t

<400>  264
ctacacgacg ctcttccgat ctnnnntgtg acgaggcatt cgtattgcgc cgcta          55



<210>  265
<211>  52
<212>  DNA
<213>  Artificial Sequence

<220>
<223>  primer


<220>

<221>  misc_feature
<222>  23
<223>  n is a, c, g, or t

<400>  265
ctacacgacg ctcttccgat ctnaatagat tggcattcgt attgcgccgc ta          52

<210>  266
<211>  53
<212>  DNA
<213>  Artificial Sequence

<220>
<223>  primer

<220>
<221>  misc_feature
<222>  (23)..(24)
<223>  n is a, c, g, or t

<400>  266
ctacacgacg ctcttccgat ctnnttagcg caggcattcg tattgcgccg cta          53

<210>  267
<211>  54
<212>  DNA
<213>  Artificial Sequence

<220>
<223>  primer

<220>
<221>  misc_feature
<222>  (23)..(25)
<223>  n is a, c, g, or t

<400>  267
ctacacgacg ctcttccgat ctnnngcggc cgtggcattc gtattgcgcc gcta          54

<210>  268
<211>  55
<212>  DNA
<213>  Artificial Sequence

<220>
<223>  primer

<220>
<221>  misc_feature
<222>  (23)..(26)
<223>  n is a, c, g, or t

<400>  268
ctacacgacg ctcttccgat ctnnnncagt aaccggcatt cgtattgcgc cgcta          55

<210>  269
<211>  52
<212>  DNA
<213>  Artificial Sequence

<220>
<223>  primer

<220>
<221>  misc_feature
<222>  23
<223>  n is a, c, g, or t

<400>  269
ctacacgacg ctcttccgat ctngcctagt aggcattcgt attgcgccgc ta          52

<210>  270
<211>  53
<212>  DNA
<213>  Artificial Sequence

<220>
<223>  primer

<220>
<221>  misc_feature
<222>  (23)..(24)
<223>  n is a, c, g, or t

<400>  270
ctacacgacg ctcttccgat ctnncacggc gcggcattcg tattgcgccg cta          53

<210>  271
<211>  54
<212>  DNA
<213>  Artificial Sequence

<220>
<223>  primer

<220>
<221>  misc_feature
<222>  (23)..(25)
<223>  n is a, c, g, or t

<400>  271
ctacacgacg ctcttccgat ctnnnggtgc agaggcattc gtattgcgcc gcta          54

<210>  272
<211>  55
<212>  DNA

<213>  Artificial Sequence

<220>
<223>  primer



<220>
<221>  misc_feature
<222>  (23)..(26)
<223>  n is a, c, g, or t

<400>  272
ctacacgacg ctcttccgat ctnnnntcgc tgacggcatt cgtattgcgc cgcta          55




<210>  273
<211>  52
<212>  DNA
<213>  Artificial Sequence

<220>
<223>  primer



<220>
<221>  misc_feature
<222>  23
<223>  n is a, c, g, or t

<400>  273
ctacacgacg ctcttccgat ctncagccag tggcattcgt attgcgccgc ta          52




<210>  274
<211>  53
<212>  DNA
<213>  Artificial Sequence

<220>
<223>  primer



<220>
<221>  misc_feature
<222>  (23)..(24)
<223>  n is a, c, g, or t

<400>  274
ctacacgacg ctcttccgat ctnncgtcaa ccggcattcg tattgcgccg cta          53




<210>  275
<211>  52
<212>  DNA
<213>  Artificial Sequence

<220>
<223>  primer

<220>
<221>  misc_feature
<222>  23
<223>  n is a, c, g, or t

<400>  275
ctacacgacg ctcttccgat ctngccggcg aggcattcgt attgcgccgc ta                52

<210>  276
<211>  53
<212>  DNA
<213>  Artificial Sequence

<220>
<223>  primer

<220>
<221>  misc_feature
<222>  (23)..(24)
<223>  n is a, c, g, or t

<400>  276
ctacacgacg ctcttccgat ctnngcctcc ggggcattcg tattgcgccg cta               53

<210>  277
<211>  54
<212>  DNA
<213>  Artificial Sequence

<220>
<223>  primer

<220>
<221>  misc_feature
<222>  (23)..(25)
<223>  n is a, c, g, or t

<400>  277
ctacacgacg ctcttccgat ctnnnaatag tccggcattc gtattgcgcc gcta              54

<210>  278
<211>  55
<212>  DNA
<213>  Artificial Sequence

<220>
<223>  primer

<220>
<221>  misc_feature
<222>  (23)..(26)
<223>  n is a, c, g, or t

<400>  278

ctacacgacg ctcttccgat ctnnnnttag acgtggcatt cgtattgcgc cgcta 55

<210> 279
<211> 52
<212> DNA
<213> Artificial Sequence

<220>
<223> primer

<220>
<221> misc_feature
<222> 23
<223> n is a, c, g, or t

<400> 279
ctacacgacg ctcttccgat ctngtggact aggcattcgt attgcgccgc ta 52

<210> 280
<211> 53
<212> DNA
<213> Artificial Sequence

<220>
<223> primer

<220>
<221> misc_feature
<222> (23)..(24)
<223> n is a, c, g, or t

<400> 280
ctacacgacg ctcttccgat ctnncacgga cgggcattcg tattgcgccg cta 53

<210> 281
<211> 54
<212> DNA
<213> Artificial Sequence

<220>
<223> primer

<220>
<221> misc_feature
<222> (23)..(25)
<223> n is a, c, g, or t

<400> 281
ctacacgacg ctcttccgat ctnnncacta gagggcattc gtattgcgcc gcta 54

<210> 282
<211> 55

<212>  DNA
<213>  Artificial Sequence

<220>
<223>  primer


<220>
<221>  misc_feature
<222>  (23)..(26)
<223>  n is a, c, g, or t

<400>  282
ctacacgacg ctcttccgat ctnnnngcag atggggcatt cgtattgcgc cgcta          55



<210>  283
<211>  52
<212>  DNA
<213>  Artificial Sequence

<220>
<223>  primer


<220>
<221>  misc_feature
<222>  23
<223>  n is a, c, g, or t

<400>  283
ctacacgacg ctcttccgat ctnctctcac gggcattcgt attgcgccgc ta             52



<210>  284
<211>  53
<212>  DNA
<213>  Artificial Sequence

<220>
<223>  primer


<220>
<221>  misc_feature
<222>  (23)..(24)
<223>  n is a, c, g, or t

<400>  284
ctacacgacg ctcttccgat ctnnggaatc acggcattcg tattgcgccg cta            53



<210>  285
<211>  54
<212>  DNA
<213>  Artificial Sequence

<220>
<223>  primer

<220>
<221>  misc_feature
<222>  (23)..(25)
<223>  n is a, c, g, or t

<400>  285
ctacacgacg ctcttccgat ctnnncgttg acgggcattc gtattgcgcc gcta          54

<210>  286
<211>  55
<212>  DNA
<213>  Artificial Sequence

<220>
<223>  primer

<220>
<221>  misc_feature
<222>  (23)..(26)
<223>  n is a, c, g, or t

<400>  286
ctacacgacg ctcttccgat ctnnnncatc aggtggcatt cgtattgcgc cgcta          55

<210>  287
<211>  52
<212>  DNA
<213>  Artificial Sequence

<220>
<223>  primer

<220>
<221>  misc_feature
<222>  23
<223>  n is a, c, g, or t

<400>  287
ctacacgacg ctcttccgat ctncgttgta aggcattcgt attgcgccgc ta          52

<210>  288
<211>  53
<212>  DNA
<213>  Artificial Sequence

<220>
<223>  primer

<220>
<221>  misc_feature
<222>  (23)..(24)
<223>  n is a, c, g, or t

<400>  288
ctacacgacg ctcttccgat ctnnggcacg gtggcattcg tattgcgccg cta          53

<210>  289
<211>  49
<212>  DNA
<213>  Artificial Sequence

<220>
<223>  primer

<220>
<221>  misc_feature
<222>  23
<223>  n is a, c, g, or t

<400>  289
ctacacgacg ctcttccgat ctnccaatac tctcatggar tggctaaag               49

<210>  290
<211>  50
<212>  DNA
<213>  Artificial Sequence

<220>
<223>  primer

<220>
<221>  misc_feature
<222>  (23)..(24)
<223>  n is a, c, g, or t

<400>  290
ctacacgacg ctcttccgat ctnntcgcgc atctcatgga rtggctaaag              50

<210>  291
<211>  51
<212>  DNA
<213>  Artificial Sequence

<220>
<223>  primer

<220>
<221>  misc_feature
<222>  (23)..(25)
<223>  n is a, c, g, or t

<400>  291
ctacacgacg ctcttccgat ctnnngtgtg aacctcatgg artggctaaa g            51

<210>  292

<211>  52
<212>  DNA
<213>  Artificial Sequence

<220>
<223>  primer


<220>
<221>  misc_feature
<222>  (23)..(26)
<223>  n is a, c, g, or t

<400>  292
ctacacgacg ctcttccgat ctnnnnggtg gcacctcatg gartggctaa ag                52



<210>  293
<211>  49
<212>  DNA
<213>  Artificial Sequence

<220>
<223>  primer


<220>
<221>  misc_feature
<222>  23
<223>  n is a, c, g, or t

<400>  293
ctacacgacg ctcttccgat ctntaggtgc tctcatggar tggctaaag                49



<210>  294
<211>  50
<212>  DNA
<213>  Artificial Sequence

<220>
<223>  primer


<220>
<221>  misc_feature
<222>  (23)..(24)
<223>  n is a, c, g, or t

<400>  294
ctacacgacg ctcttccgat ctnngagcgg ttctcatgga rtggctaaag                50



<210>  295
<211>  51
<212>  DNA
<213>  Artificial Sequence

<220>
<223>  primer

<220>
<221>  misc_feature
<222>  (23)..(25)
<223>  n is a, c, g, or t

<400>  295
ctacacgacg ctcttccgat ctnnnaggat tagctcatgg artggctaaa g                51

<210>  296
<211>  52
<212>  DNA
<213>  Artificial Sequence

<220>
<223>  primer

<220>
<221>  misc_feature
<222>  (23)..(26)
<223>  n is a, c, g, or t

<400>  296
ctacacgacg ctcttccgat ctnnnngtga gccactcatg gartggctaa ag               52

<210>  297
<211>  49
<212>  DNA
<213>  Artificial Sequence

<220>
<223>  primer

<220>
<221>  misc_feature
<222>  23
<223>  n is a, c, g, or t

<400>  297
ctacacgacg ctcttccgat ctnaattatg cctcatggar tggctaaag                   49

<210>  298
<211>  50
<212>  DNA
<213>  Artificial Sequence

<220>
<223>  primer

<220>
<221>  misc_feature
<222>  (23)..(24)
<223>  n is a, c, g, or t

<400>  298
ctacacgacg ctcttccgat ctnnttacgc cgctcatgga rtggctaaag                50

<210>  299
<211>  51
<212>  DNA
<213>  Artificial Sequence

<220>
<223>  primer

<220>
<221>  misc_feature
<222>  (23)..(25)
<223>  n is a, c, g, or t

<400>  299
ctacacgacg ctcttccgat ctnnngtagt gatctcatgg artggctaaa g              51

<210>  300
<211>  52
<212>  DNA
<213>  Artificial Sequence

<220>
<223>  primer

<220>
<221>  misc_feature
<222>  (23)..(26)
<223>  n is a, c, g, or t

<400>  300
ctacacgacg ctcttccgat ctnnnntggt tgcactcatg gartggctaa ag             52

<210>  301
<211>  49
<212>  DNA
<213>  Artificial Sequence

<220>
<223>  primer

<220>
<221>  misc_feature
<222>  23
<223>  n is a, c, g, or t

<400>  301
ctacacgacg ctcttccgat ctngtccgac cctcatggar tggctaaag                 49

<210>  302
<211>  50
<212>  DNA
<213>  Artificial Sequence

<220>
<223>  primer


<220>
<221>  misc_feature
<222>  (23)..(24)
<223>  n is a, c, g, or t

<400>  302
ctacacgacg ctcttccgat ctnngccagc cgctcatgga rtggctaaag                50


<210>  303
<211>  51
<212>  DNA
<213>  Artificial Sequence

<220>
<223>  primer


<220>
<221>  misc_feature
<222>  (23)..(25)
<223>  n is a, c, g, or t

<400>  303
ctacacgacg ctcttccgat ctnnnctcgt gtcctcatgg artggctaaa g              51


<210>  304
<211>  52
<212>  DNA
<213>  Artificial Sequence

<220>
<223>  primer


<220>
<221>  misc_feature
<222>  (23)..(26)
<223>  n is a, c, g, or t

<400>  304
ctacacgacg ctcttccgat ctnnnngccg tggcctcatg gartggctaa ag             52


<210>  305
<211>  49
<212>  DNA
<213>  Artificial Sequence

<220>

<223>  primer

<220>
<221>  misc_feature
<222>  23
<223>  n is a, c, g, or t

<400>  305
ctacacgacg ctcttccgat ctngtagcca cctcatggar tggctaaag                49

<210>  306
<211>  50
<212>  DNA
<213>  Artificial Sequence

<220>
<223>  primer

<220>
<221>  misc_feature
<222>  (23)..(24)
<223>  n is a, c, g, or t

<400>  306
ctacacgacg ctcttccgat ctnngactta tactcatgga rtggctaaag               50

<210>  307
<211>  51
<212>  DNA
<213>  Artificial Sequence

<220>
<223>  primer

<220>
<221>  misc_feature
<222>  (23)..(25)
<223>  n is a, c, g, or t

<400>  307
ctacacgacg ctcttccgat ctnnngaacg tcgctcatgg artggctaaa g             51

<210>  308
<211>  52
<212>  DNA
<213>  Artificial Sequence

<220>
<223>  primer

<220>
<221>  misc_feature
<222>  (23)..(26)

<223>  n is a, c, g, or t

<400>  308
ctacacgacg ctcttccgat ctnnnnatgc gtagctcatg gartggctaa ag                52

<210>  309
<211>  49
<212>  DNA
<213>  Artificial Sequence

<220>
<223>  primer

<220>
<221>  misc_feature
<222>  23
<223>  n is a, c, g, or t

<400>  309
ctacacgacg ctcttccgat ctnaattaga tctcatggar tggctaaag                49

<210>  310
<211>  50
<212>  DNA
<213>  Artificial Sequence

<220>
<223>  primer

<220>
<221>  misc_feature
<222>  (23)..(24)
<223>  n is a, c, g, or t

<400>  310
ctacacgacg ctcttccgat ctnnttacga tactcatgga rtggctaaag                50

<210>  311
<211>  51
<212>  DNA
<213>  Artificial Sequence

<220>
<223>  primer

<220>
<221>  misc_feature
<222>  (23)..(25)
<223>  n is a, c, g, or t

<400>  311
ctacacgacg ctcttccgat ctnnnctgta caactcatgg artggctaaa g                51

<210>  312
<211>  52
<212>  DNA
<213>  Artificial Sequence

<220>
<223>  primer

<220>
<221>  misc_feature
<222>  (23)..(26)
<223>  n is a, c, g, or t

<400>  312
ctacacgacg ctcttccgat ctnnnncgtg gttgctcatg gartggctaa ag          52

<210>  313
<211>  49
<212>  DNA
<213>  Artificial Sequence

<220>
<223>  primer

<220>
<221>  misc_feature
<222>  23
<223>  n is a, c, g, or t

<400>  313
ctacacgacg ctcttccgat ctnaataacg tctcatggar tggctaaag          49

<210>  314
<211>  50
<212>  DNA
<213>  Artificial Sequence

<220>
<223>  primer

<220>
<221>  misc_feature
<222>  (23)..(24)
<223>  n is a, c, g, or t

<400>  314
ctacacgacg ctcttccgat ctnnttcttg aactcatgga rtggctaaag          50

<210>  315
<211>  51
<212>  DNA
<213>  Artificial Sequence

<220>
<223>  primer


<220>
<221>  misc_feature
<222>  (23)..(25)
<223>  n is a, c, g, or t

<400>  315
ctacacgacg ctcttccgat ctnnnggcag atcctcatgg artggctaaa g                51



<210>  316
<211>  52
<212>  DNA
<213>  Artificial Sequence

<220>
<223>  primer


<220>
<221>  misc_feature
<222>  (23)..(26)
<223>  n is a, c, g, or t

<400>  316
ctacacgacg ctcttccgat ctnnnnctat gttactcatg gartggctaa ag               52



<210>  317
<211>  49
<212>  DNA
<213>  Artificial Sequence

<220>
<223>  primer


<220>
<221>  misc_feature
<222>  23
<223>  n is a, c, g, or t

<400>  317
ctacacgacg ctcttccgat ctngttgacg cctcatggar tggctaaag                   49



<210>  318
<211>  50
<212>  DNA
<213>  Artificial Sequence

<220>
<223>  primer


<220>
<221>  misc_feature

<222>  (23)..(24)
<223>  n is a, c, g, or t

<400>  318
ctacacgacg ctcttccgat ctnnatctac gactcatgga rtggctaaag          50




<210>  319
<211>  51
<212>  DNA
<213>  Artificial Sequence

<220>
<223>  primer


<220>
<221>  misc_feature
<222>  (23)..(25)
<223>  n is a, c, g, or t

<400>  319
ctacacgacg ctcttccgat ctnnnctcga cagctcatgg artggctaaa g          51




<210>  320
<211>  52
<212>  DNA
<213>  Artificial Sequence

<220>
<223>  primer


<220>
<221>  misc_feature
<222>  (23)..(26)
<223>  n is a, c, g, or t

<400>  320
ctacacgacg ctcttccgat ctnnnngagg ctgcctcatg gartggctaa ag          52




<210>  321
<211>  49
<212>  DNA
<213>  Artificial Sequence

<220>
<223>  primer


<220>
<221>  misc_feature
<222>  23
<223>  n is a, c, g, or t

<400>  321
ctacacgacg ctcttccgat ctncctcgta gctcatggar tggctaaag          49

<210>  322
<211>  50
<212>  DNA
<213>  Artificial Sequence

<220>
<223>  primer


<220>
<221>  misc_feature
<222>  (23)..(24)
<223>  n is a, c, g, or t

<400>  322
ctacacgacg ctcttccgat ctnncatagg cactcatgga rtggctaaag                50


<210>  323
<211>  51
<212>  DNA
<213>  Artificial Sequence

<220>
<223>  primer


<220>
<221>  misc_feature
<222>  (23)..(25)
<223>  n is a, c, g, or t

<400>  323
ctacacgacg ctcttccgat ctnnnagatg aacctcatgg artggctaaa g              51


<210>  324
<211>  52
<212>  DNA
<213>  Artificial Sequence

<220>
<223>  primer


<220>
<221>  misc_feature
<222>  (23)..(26)
<223>  n is a, c, g, or t

<400>  324
ctacacgacg ctcttccgat ctnnnnccga gtatctcatg gartggctaa ag             52


<210>  325
<211>  49
<212>  DNA
<213>  Artificial Sequence

<220>
<223>  primer


<220>
<221>  misc_feature
<222>  23
<223>  n is a, c, g, or t

<400>  325
ctacacgacg ctcttccgat ctnaatattg actcatggar tggctaaag                    49



<210>  326
<211>  50
<212>  DNA
<213>  Artificial Sequence

<220>
<223>  primer


<220>
<221>  misc_feature
<222>  (23)..(24)
<223>  n is a, c, g, or t

<400>  326
ctacacgacg ctcttccgat ctnngtatac cgctcatgga rtggctaaag                   50



<210>  327
<211>  51
<212>  DNA
<213>  Artificial Sequence

<220>
<223>  primer


<220>
<221>  misc_feature
<222>  (23)..(25)
<223>  n is a, c, g, or t

<400>  327
ctacacgacg ctcttccgat ctnnngatcc aacctcatgg artggctaaa g                 51



<210>  328
<211>  52
<212>  DNA
<213>  Artificial Sequence

<220>
<223>  primer


<220>

<221>  misc_feature
<222>  (23)..(26)
<223>  n is a, c, g, or t

<400>  328
ctacacgacg ctcttccgat ctnnnnagat acgcctcatg gartggctaa ag                52

<210>  329
<211>  49
<212>  DNA
<213>  Artificial Sequence

<220>
<223>  primer

<220>
<221>  misc_feature
<222>  23
<223>  n is a, c, g, or t

<400>  329
ctacacgacg ctcttccgat ctnggtatct tctcatggar tggctaaag                    49

<210>  330
<211>  50
<212>  DNA
<213>  Artificial Sequence

<220>
<223>  primer

<220>
<221>  misc_feature
<222>  (23)..(24)
<223>  n is a, c, g, or t

<400>  330
ctacacgacg ctcttccgat ctnncctctg gcctcatgga rtggctaaag                   50

<210>  331
<211>  49
<212>  DNA
<213>  Artificial Sequence

<220>
<223>  primer

<220>
<221>  misc_feature
<222>  23
<223>  n is a, c, g, or t

<400>  331
ctacacgacg ctcttccgat ctnccattgt gctcatggar tggctaaag                    49

<210>  332
<211>  50
<212>  DNA
<213>  Artificial Sequence

<220>
<223>  primer


<220>
<221>  misc_feature
<222>  (23)..(24)
<223>  n is a, c, g, or t

<400>  332
ctacacgacg ctcttccgat ctnnactacg gtctcatgga rtggctaaag                50


<210>  333
<211>  51
<212>  DNA
<213>  Artificial Sequence

<220>
<223>  primer


<220>
<221>  misc_feature
<222>  (23)..(25)
<223>  n is a, c, g, or t

<400>  333
ctacacgacg ctcttccgat ctnnnaagtg ctactcatgg artggctaaa g              51


<210>  334
<211>  52
<212>  DNA
<213>  Artificial Sequence

<220>
<223>  primer


<220>
<221>  misc_feature
<222>  (23)..(26)
<223>  n is a, c, g, or t

<400>  334
ctacacgacg ctcttccgat ctnnnngccg aacgctcatg gartggctaa ag             52


<210>  335
<211>  49
<212>  DNA

<213>  Artificial Sequence

<220>
<223>  primer



<220>
<221>  misc_feature
<222>  23
<223>  n is a, c, g, or t

<400>  335
ctacacgacg ctcttccgat ctntgtccac gctcatggar tggctaaag                49



<210>  336
<211>  50
<212>  DNA
<213>  Artificial Sequence

<220>
<223>  primer



<220>
<221>  misc_feature
<222>  (23)..(24)
<223>  n is a, c, g, or t

<400>  336
ctacacgacg ctcttccgat ctnngacaca ctctcatgga rtggctaaag               50



<210>  337
<211>  51
<212>  DNA
<213>  Artificial Sequence

<220>
<223>  primer



<220>
<221>  misc_feature
<222>  (23)..(25)
<223>  n is a, c, g, or t

<400>  337
ctacacgacg ctcttccgat ctnnnaatat gctctcatgg artggctaaa g             51



<210>  338
<211>  52
<212>  DNA
<213>  Artificial Sequence

<220>
<223>  primer

<220>
<221>  misc_feature
<222>  (23)..(26)
<223>  n is a, c, g, or t

<400>  338
ctacacgacg ctcttccgat ctnnnnttct catactcatg gartggctaa ag          52

<210>  339
<211>  49
<212>  DNA
<213>  Artificial Sequence

<220>
<223>  primer

<220>
<221>  misc_feature
<222>  23
<223>  n is a, c, g, or t

<400>  339
ctacacgacg ctcttccgat ctntctgtga tctcatggar tggctaaag          49

<210>  340
<211>  50
<212>  DNA
<213>  Artificial Sequence

<220>
<223>  primer

<220>
<221>  misc_feature
<222>  (23)..(24)
<223>  n is a, c, g, or t

<400>  340
ctacacgacg ctcttccgat ctnnccgaac ttctcatgga rtggctaaag          50

<210>  341
<211>  51
<212>  DNA
<213>  Artificial Sequence

<220>
<223>  primer

<220>
<221>  misc_feature
<222>  (23)..(25)
<223>  n is a, c, g, or t

<400>  341

ctacacgacg ctcttccgat ctnnngtcta acactcatgg artggctaaa g 51

<210> 342
<211> 52
<212> DNA
<213> Artificial Sequence

<220>
<223> primer

<220>
<221> misc_feature
<222> (23)..(26)
<223> n is a, c, g, or t

<400> 342
ctacacgacg ctcttccgat ctnnnngacg ccatctcatg gartggctaa ag 52

<210> 343
<211> 49
<212> DNA
<213> Artificial Sequence

<220>
<223> primer

<220>
<221> misc_feature
<222> 23
<223> n is a, c, g, or t

<400> 343
ctacacgacg ctcttccgat ctngccaatg tctcatggar tggctaaag 49

<210> 344
<211> 50
<212> DNA
<213> Artificial Sequence

<220>
<223> primer

<220>
<221> misc_feature
<222> (23)..(24)
<223> n is a, c, g, or t

<400> 344
ctacacgacg ctcttccgat ctnnccaacg tcctcatgga rtggctaaag 50

<210> 345
<211> 51

<212>  DNA
<213>  Artificial Sequence

<220>
<223>  primer


<220>
<221>  misc_feature
<222>  (23)..(25)
<223>  n is a, c, g, or t

<400>  345
ctacacgacg ctcttccgat ctnnngtaga taactcatgg artggctaaa g                51


<210>  346
<211>  52
<212>  DNA
<213>  Artificial Sequence

<220>
<223>  primer


<220>
<221>  misc_feature
<222>  (23)..(26)
<223>  n is a, c, g, or t

<400>  346
ctacacgacg ctcttccgat ctnnnnctta cggcctcatg gartggctaa ag               52


<210>  347
<211>  49
<212>  DNA
<213>  Artificial Sequence

<220>
<223>  primer


<220>
<221>  misc_feature
<222>  23
<223>  n is a, c, g, or t

<400>  347
ctacacgacg ctcttccgat ctnccaagtg cctcatggar tggctaaag                   49


<210>  348
<211>  50
<212>  DNA
<213>  Artificial Sequence

<220>
<223>  primer

<220>
<221>  misc_feature
<222>  (23)..(24)
<223>  n is a, c, g, or t

<400>  348
ctacacgacg ctcttccgat ctnnctaact cactcatgga rtggctaaag          50

<210>  349
<211>  51
<212>  DNA
<213>  Artificial Sequence

<220>
<223>  primer

<220>
<221>  misc_feature
<222>  (23)..(25)
<223>  n is a, c, g, or t

<400>  349
ctacacgacg ctcttccgat ctnnnaatat ctgctcatgg artggctaaa g          51

<210>  350
<211>  52
<212>  DNA
<213>  Artificial Sequence

<220>
<223>  primer

<220>
<221>  misc_feature
<222>  (23)..(26)
<223>  n is a, c, g, or t

<400>  350
ctacacgacg ctcttccgat ctnnnnttat atcactcatg gartggctaa ag          52

<210>  351
<211>  49
<212>  DNA
<213>  Artificial Sequence

<220>
<223>  primer

<220>
<221>  misc_feature
<222>  23
<223>  n is a, c, g, or t

<400>  351
ctacacgacg ctcttccgat ctnctgcgga tctcatggar tggctaaag                49

<210>  352
<211>  50
<212>  DNA
<213>  Artificial Sequence

<220>
<223>  primer

<220>
<221>  misc_feature
<222>  (23)..(24)
<223>  n is a, c, g, or t

<400>  352
ctacacgacg ctcttccgat ctnngcggct tgctcatgga rtggctaaag               50

<210>  353
<211>  51
<212>  DNA
<213>  Artificial Sequence

<220>
<223>  primer

<220>
<221>  misc_feature
<222>  (23)..(25)
<223>  n is a, c, g, or t

<400>  353
ctacacgacg ctcttccgat ctnnngagtt gatctcatgg artggctaaa g             51

<210>  354
<211>  52
<212>  DNA
<213>  Artificial Sequence

<220>
<223>  primer

<220>
<221>  misc_feature
<222>  (23)..(26)
<223>  n is a, c, g, or t

<400>  354
ctacacgacg ctcttccgat ctnnnngcac tgagctcatg gartggctaa ag            52

<210>  355

<211>  49
<212>  DNA
<213>  Artificial Sequence

<220>
<223>  primer


<220>
<221>  misc_feature
<222>  23
<223>  n is a, c, g, or t

<400>  355
ctacacgacg ctcttccgat ctngaccacc tctcatggar tggctaaag                49



<210>  356
<211>  49
<212>  DNA
<213>  Artificial Sequence

<220>
<223>  primer


<220>
<221>  misc_feature
<222>  23
<223>  n is a, c, g, or t

<400>  356
ctacacgacg ctcttccgat ctnaatagca actcatggar tggctaaag                49



<210>  357
<211>  49
<212>  DNA
<213>  Artificial Sequence

<220>
<223>  primer


<220>
<221>  misc_feature
<222>  23
<223>  n is a, c, g, or t

<400>  357
ctacacgacg ctcttccgat ctncctaccg gctcatggar tggctaaag                49



<210>  358
<211>  50
<212>  DNA
<213>  Artificial Sequence

<220>
<223>  primer

<220>
<221>  misc_feature
<222>  (23)..(24)
<223>  n is a, c, g, or t

<400>  358
ctacacgacg ctcttccgat ctnnggagga tgctcatgga rtggctaaag                50

<210>  359
<211>  51
<212>  DNA
<213>  Artificial Sequence

<220>
<223>  primer

<220>
<221>  misc_feature
<222>  (23)..(25)
<223>  n is a, c, g, or t

<400>  359
ctacacgacg ctcttccgat ctnnncgctg aatctcatgg artggctaaa g              51

<210>  360
<211>  52
<212>  DNA
<213>  Artificial Sequence

<220>
<223>  primer

<220>
<221>  misc_feature
<222>  (23)..(26)
<223>  n is a, c, g, or t

<400>  360
ctacacgacg ctcttccgat ctnnnntgtg acgactcatg gartggctaa ag             52

<210>  361
<211>  49
<212>  DNA
<213>  Artificial Sequence

<220>
<223>  primer

<220>
<221>  misc_feature
<222>  23
<223>  n is a, c, g, or t

<400>  361
ctacacgacg ctcttccgat ctnaatagat tctcatggar tggctaaag                49

<210>  362
<211>  50
<212>  DNA
<213>  Artificial Sequence

<220>
<223>  primer

<220>
<221>  misc_feature
<222>  (23)..(24)
<223>  n is a, c, g, or t

<400>  362
ctacacgacg ctcttccgat ctnnttagcg cactcatgga rtggctaaag               50

<210>  363
<211>  51
<212>  DNA
<213>  Artificial Sequence

<220>
<223>  primer

<220>
<221>  misc_feature
<222>  (23)..(25)
<223>  n is a, c, g, or t

<400>  363
ctacacgacg ctcttccgat ctnnngcggc cgtctcatgg artggctaaa g             51

<210>  364
<211>  52
<212>  DNA
<213>  Artificial Sequence

<220>
<223>  primer

<220>
<221>  misc_feature
<222>  (23)..(26)
<223>  n is a, c, g, or t

<400>  364
ctacacgacg ctcttccgat ctnnnncagt aaccctcatg gartggctaa ag            52

<210>  365
<211>  49
<212>  DNA
<213>  Artificial Sequence

<220>
<223>  primer


<220>
<221>  misc_feature
<222>  23
<223>  n is a, c, g, or t

<400>  365
ctacacgacg ctcttccgat ctngcctagt actcatggar tggctaaag                49


<210>  366
<211>  50
<212>  DNA
<213>  Artificial Sequence

<220>
<223>  primer


<220>
<221>  misc_feature
<222>  (23)..(24)
<223>  n is a, c, g, or t

<400>  366
ctacacgacg ctcttccgat ctnncacggc gcctcatgga rtggctaaag               50


<210>  367
<211>  51
<212>  DNA
<213>  Artificial Sequence

<220>
<223>  primer


<220>
<221>  misc_feature
<222>  (23)..(25)
<223>  n is a, c, g, or t

<400>  367
ctacacgacg ctcttccgat ctnnnggtgc agactcatgg artggctaaa g             51


<210>  368
<211>  52
<212>  DNA
<213>  Artificial Sequence

<220>

<223>  primer

<220>
<221>  misc_feature
<222>  (23)..(26)
<223>  n is a, c, g, or t

<400>  368
ctacacgacg ctcttccgat ctnnnntcgc tgacctcatg gartggctaa ag          52

<210>  369
<211>  49
<212>  DNA
<213>  Artificial Sequence

<220>
<223>  primer

<220>
<221>  misc_feature
<222>  23
<223>  n is a, c, g, or t

<400>  369
ctacacgacg ctcttccgat ctncagccag tctcatggar tggctaaag          49

<210>  370
<211>  50
<212>  DNA
<213>  Artificial Sequence

<220>
<223>  primer

<220>
<221>  misc_feature
<222>  (23)..(24)
<223>  n is a, c, g, or t

<400>  370
ctacacgacg ctcttccgat ctnncgtcaa ccctcatgga rtggctaaag          50

<210>  371
<211>  49
<212>  DNA
<213>  Artificial Sequence

<220>
<223>  primer

<220>
<221>  misc_feature
<222>  23

<223>  n is a, c, g, or t

<400>  371
ctacacgacg ctcttccgat ctngccggcg actcatggar tggctaaag                    49

<210>  372
<211>  50
<212>  DNA
<213>  Artificial Sequence

<220>
<223>  primer

<220>
<221>  misc_feature
<222>  (23)..(24)
<223>  n is a, c, g, or t

<400>  372
ctacacgacg ctcttccgat ctnngcctcc ggctcatgga rtggctaaag                   50

<210>  373
<211>  51
<212>  DNA
<213>  Artificial Sequence

<220>
<223>  primer

<220>
<221>  misc_feature
<222>  (23)..(25)
<223>  n is a, c, g, or t

<400>  373
ctacacgacg ctcttccgat ctnnnaatag tccctcatgg artggctaaa g                 51

<210>  374
<211>  52
<212>  DNA
<213>  Artificial Sequence

<220>
<223>  primer

<220>
<221>  misc_feature
<222>  (23)..(26)
<223>  n is a, c, g, or t

<400>  374
ctacacgacg ctcttccgat ctnnnnttag acgtctcatg gartggctaa ag                52

<210>  375
<211>  49
<212>  DNA
<213>  Artificial Sequence

<220>
<223>  primer


<220>
<221>  misc_feature
<222>  23
<223>  n is a, c, g, or t

<400>  375
ctacacgacg ctcttccgat ctngtggact actcatggar tggctaaag                49


<210>  376
<211>  50
<212>  DNA
<213>  Artificial Sequence

<220>
<223>  primer


<220>
<221>  misc_feature
<222>  (23)..(24)
<223>  n is a, c, g, or t

<400>  376
ctacacgacg ctcttccgat ctnncacgga cgctcatgga rtggctaaag               50


<210>  377
<211>  51
<212>  DNA
<213>  Artificial Sequence

<220>
<223>  primer


<220>
<221>  misc_feature
<222>  (23)..(25)
<223>  n is a, c, g, or t

<400>  377
ctacacgacg ctcttccgat ctnnncacta gagctcatgg artggctaaa g             51


<210>  378
<211>  52
<212>  DNA
<213>  Artificial Sequence

<220>
<223>  primer


<220>
<221>  misc_feature
<222>  (23)..(26)
<223>  n is a, c, g, or t

<400>  378
ctacacgacg ctcttccgat ctnnnngcag atggctcatg gartggctaa ag                52



<210>  379
<211>  49
<212>  DNA
<213>  Artificial Sequence

<220>
<223>  primer


<220>
<221>  misc_feature
<222>  23
<223>  n is a, c, g, or t

<400>  379
ctacacgacg ctcttccgat ctnctctcac gctcatggar tggctaaag                    49



<210>  380
<211>  50
<212>  DNA
<213>  Artificial Sequence

<220>
<223>  primer


<220>
<221>  misc_feature
<222>  (23)..(24)
<223>  n is a, c, g, or t

<400>  380
ctacacgacg ctcttccgat ctnnggaatc acctcatgga rtggctaaag                   50



<210>  381
<211>  51
<212>  DNA
<213>  Artificial Sequence

<220>
<223>  primer


<220>
<221>  misc_feature

<222>  (23)..(25)
<223>  n is a, c, g, or t

<400>  381
ctacacgacg ctcttccgat ctnnncgttg acgctcatgg artggctaaa g                51

<210>  382
<211>  52
<212>  DNA
<213>  Artificial Sequence

<220>
<223>  primer

<220>
<221>  misc_feature
<222>  (23)..(26)
<223>  n is a, c, g, or t

<400>  382
ctacacgacg ctcttccgat ctnnnncatc aggtctcatg gartggctaa ag               52

<210>  383
<211>  49
<212>  DNA
<213>  Artificial Sequence

<220>
<223>  primer

<220>
<221>  misc_feature
<222>  23
<223>  n is a, c, g, or t

<400>  383
ctacacgacg ctcttccgat ctncgttgta actcatggar tggctaaag                   49

<210>  384
<211>  50
<212>  DNA
<213>  Artificial Sequence

<220>
<223>  primer

<220>
<221>  misc_feature
<222>  (23)..(24)
<223>  n is a, c, g, or t

<400>  384
ctacacgacg ctcttccgat ctnnggcacg gtctcatgga rtggctaaag                  50

<210>  385
<211>  48
<212>  DNA
<213>  Artificial Sequence

<220>
<223>  primer

<220>
<221>  misc_feature
<222>  23
<223>  n is a, c, g, or t

<400>  385
ctacacgacg ctcttccgat ctnccaatac tgccttctcc atcttctg                48

<210>  386
<211>  49
<212>  DNA
<213>  Artificial Sequence

<220>
<223>  primer

<220>
<221>  misc_feature
<222>  (23)..(24)
<223>  n is a, c, g, or t

<400>  386
ctacacgacg ctcttccgat ctnntcgcgc atgccttctc catcttctg               49

<210>  387
<211>  50
<212>  DNA
<213>  Artificial Sequence

<220>
<223>  primer

<220>
<221>  misc_feature
<222>  (23)..(25)
<223>  n is a, c, g, or t

<400>  387
ctacacgacg ctcttccgat ctnnngtgtg aacgccttct ccatcttctg              50

<210>  388
<211>  51
<212>  DNA
<213>  Artificial Sequence

<220>
<223>  primer

<220>
<221>  misc_feature
<222>  (23)..(26)
<223>  n is a, c, g, or t

<400>  388
ctacacgacg ctcttccgat ctnnnnggtg gcacgccttc tccatcttct g                51

<210>  389
<211>  48
<212>  DNA
<213>  Artificial Sequence

<220>
<223>  primer

<220>
<221>  misc_feature
<222>  23
<223>  n is a, c, g, or t

<400>  389
ctacacgacg ctcttccgat ctntaggtgc tgccttctcc atcttctg                48

<210>  390
<211>  49
<212>  DNA
<213>  Artificial Sequence

<220>
<223>  primer

<220>
<221>  misc_feature
<222>  (23)..(24)
<223>  n is a, c, g, or t

<400>  390
ctacacgacg ctcttccgat ctnngagcgg ttgccttctc catcttctg                49

<210>  391
<211>  50
<212>  DNA
<213>  Artificial Sequence

<220>
<223>  primer

<220>

<221>  misc_feature
<222>  (23)..(25)
<223>  n is a, c, g, or t

<400>  391
ctacacgacg ctcttccgat ctnnnaggat taggccttct ccatcttctg                50



<210>  392
<211>  51
<212>  DNA
<213>  Artificial Sequence

<220>
<223>  primer


<220>
<221>  misc_feature
<222>  (23)..(26)
<223>  n is a, c, g, or t

<400>  392
ctacacgacg ctcttccgat ctnnnngtga gccagccttc tccatcttct g              51



<210>  393
<211>  48
<212>  DNA
<213>  Artificial Sequence

<220>
<223>  primer


<220>
<221>  misc_feature
<222>  23
<223>  n is a, c, g, or t

<400>  393
ctacacgacg ctcttccgat ctnaattatg cgccttctcc atcttctg                  48



<210>  394
<211>  49
<212>  DNA
<213>  Artificial Sequence

<220>
<223>  primer


<220>
<221>  misc_feature
<222>  (23)..(24)
<223>  n is a, c, g, or t

<400>  394
ctacacgacg ctcttccgat ctnnttacgc cggccttctc catcttctg                 49

<210>  395
<211>  50
<212>  DNA
<213>  Artificial Sequence

<220>
<223>  primer


<220>
<221>  misc_feature
<222>  (23)..(25)
<223>  n is a, c, g, or t

<400>  395
ctacacgacg ctcttccgat ctnnngtagt gatgccttct ccatcttctg                50


<210>  396
<211>  51
<212>  DNA
<213>  Artificial Sequence

<220>
<223>  primer


<220>
<221>  misc_feature
<222>  (23)..(26)
<223>  n is a, c, g, or t

<400>  396
ctacacgacg ctcttccgat ctnnnntggt tgcagccttc tccatcttct g              51


<210>  397
<211>  48
<212>  DNA
<213>  Artificial Sequence

<220>
<223>  primer


<220>
<221>  misc_feature
<222>  23
<223>  n is a, c, g, or t

<400>  397
ctacacgacg ctcttccgat ctngtccgac cgccttctcc atcttctg                  48


<210>  398
<211>  49
<212>  DNA

<213>  Artificial Sequence

<220>
<223>  primer


<220>
<221>  misc_feature
<222>  (23)..(24)
<223>  n is a, c, g, or t

<400>  398
ctacacgacg ctcttccgat ctnngccagc cggccttctc catcttctg                49


<210>  399
<211>  50
<212>  DNA
<213>  Artificial Sequence

<220>
<223>  primer


<220>
<221>  misc_feature
<222>  (23)..(25)
<223>  n is a, c, g, or t

<400>  399
ctacacgacg ctcttccgat ctnnnctcgt gtcgccttct ccatcttctg               50


<210>  400
<211>  51
<212>  DNA
<213>  Artificial Sequence

<220>
<223>  primer


<220>
<221>  misc_feature
<222>  (23)..(26)
<223>  n is a, c, g, or t

<400>  400
ctacacgacg ctcttccgat ctnnnngccg tggcgccttc tccatcttct g             51


<210>  401
<211>  48
<212>  DNA
<213>  Artificial Sequence

<220>
<223>  primer

<220>
<221>  misc_feature
<222>  23
<223>  n is a, c, g, or t

<400>  401
ctacacgacg ctcttccgat ctngtagcca cgccttctcc atcttctg                48

<210>  402
<211>  49
<212>  DNA
<213>  Artificial Sequence

<220>
<223>  primer

<220>
<221>  misc_feature
<222>  (23)..(24)
<223>  n is a, c, g, or t

<400>  402
ctacacgacg ctcttccgat ctnngactta tagccttctc catcttctg               49

<210>  403
<211>  50
<212>  DNA
<213>  Artificial Sequence

<220>
<223>  primer

<220>
<221>  misc_feature
<222>  (23)..(25)
<223>  n is a, c, g, or t

<400>  403
ctacacgacg ctcttccgat ctnnngaacg tcggccttct ccatcttctg              50

<210>  404
<211>  51
<212>  DNA
<213>  Artificial Sequence

<220>
<223>  primer

<220>
<221>  misc_feature
<222>  (23)..(26)
<223>  n is a, c, g, or t

<400>  404

ctacacgacg ctcttccgat ctnnnnatgc gtaggccttc tccatcttct g 51

<210> 405
<211> 48
<212> DNA
<213> Artificial Sequence

<220>
<223> primer

<220>
<221> misc_feature
<222> 23
<223> n is a, c, g, or t

<400> 405
ctacacgacg ctcttccgat ctnaattaga tgccttctcc atcttctg 48

<210> 406
<211> 49
<212> DNA
<213> Artificial Sequence

<220>
<223> primer

<220>
<221> misc_feature
<222> (23)..(24)
<223> n is a, c, g, or t

<400> 406
ctacacgacg ctcttccgat ctnnttacga tagccttctc catcttctg 49

<210> 407
<211> 50
<212> DNA
<213> Artificial Sequence

<220>
<223> primer

<220>
<221> misc_feature
<222> (23)..(25)
<223> n is a, c, g, or t

<400> 407
ctacacgacg ctcttccgat ctnnnctgta caagccttct ccatcttctg 50

<210> 408
<211> 51

<212>  DNA
<213>  Artificial Sequence

<220>
<223>  primer


<220>
<221>  misc_feature
<222>  (23)..(26)
<223>  n is a, c, g, or t

<400>  408
ctacacgacg ctcttccgat ctnnnncgtg gttggccttc tccatcttct g                51


<210>  409
<211>  48
<212>  DNA
<213>  Artificial Sequence

<220>
<223>  primer


<220>
<221>  misc_feature
<222>  23
<223>  n is a, c, g, or t

<400>  409
ctacacgacg ctcttccgat ctnaataacg tgccttctcc atcttctg                48


<210>  410
<211>  49
<212>  DNA
<213>  Artificial Sequence

<220>
<223>  primer


<220>
<221>  misc_feature
<222>  (23)..(24)
<223>  n is a, c, g, or t

<400>  410
ctacacgacg ctcttccgat ctnnttcttg aagccttctc catcttctg                49


<210>  411
<211>  50
<212>  DNA
<213>  Artificial Sequence

<220>
<223>  primer

<220>
<221>  misc_feature
<222>  (23)..(25)
<223>  n is a, c, g, or t

<400>  411
ctacacgacg ctcttccgat ctnnnggcag atcgccttct ccatcttctg                50

<210>  412
<211>  51
<212>  DNA
<213>  Artificial Sequence

<220>
<223>  primer

<220>
<221>  misc_feature
<222>  (23)..(26)
<223>  n is a, c, g, or t

<400>  412
ctacacgacg ctcttccgat ctnnnnctat gttagccttc tccatcttct g              51

<210>  413
<211>  48
<212>  DNA
<213>  Artificial Sequence

<220>
<223>  primer

<220>
<221>  misc_feature
<222>  23
<223>  n is a, c, g, or t

<400>  413
ctacacgacg ctcttccgat ctngttgacg cgccttctcc atcttctg                  48

<210>  414
<211>  49
<212>  DNA
<213>  Artificial Sequence

<220>
<223>  primer

<220>
<221>  misc_feature
<222>  (23)..(24)
<223>  n is a, c, g, or t

<400>  414
ctacacgacg ctcttccgat ctnnatctac gagccttctc catcttctg                49

<210>  415
<211>  50
<212>  DNA
<213>  Artificial Sequence

<220>
<223>  primer

<220>
<221>  misc_feature
<222>  (23)..(25)
<223>  n is a, c, g, or t

<400>  415
ctacacgacg ctcttccgat ctnnnctcga caggccttct ccatcttctg               50

<210>  416
<211>  51
<212>  DNA
<213>  Artificial Sequence

<220>
<223>  primer

<220>
<221>  misc_feature
<222>  (23)..(26)
<223>  n is a, c, g, or t

<400>  416
ctacacgacg ctcttccgat ctnnnngagg ctgcgccttc tccatcttct g             51

<210>  417
<211>  48
<212>  DNA
<213>  Artificial Sequence

<220>
<223>  primer

<220>
<221>  misc_feature
<222>  23
<223>  n is a, c, g, or t

<400>  417
ctacacgacg ctcttccgat ctncctcgta ggccttctcc atcttctg                 48

<210>  418

<211>  49
<212>  DNA
<213>  Artificial Sequence

<220>
<223>  primer


<220>
<221>  misc_feature
<222>  (23)..(24)
<223>  n is a, c, g, or t

<400>  418
ctacacgacg ctcttccgat ctnncatagg cagccttctc catcttctg                49


<210>  419
<211>  50
<212>  DNA
<213>  Artificial Sequence

<220>
<223>  primer


<220>
<221>  misc_feature
<222>  (23)..(25)
<223>  n is a, c, g, or t

<400>  419
ctacacgacg ctcttccgat ctnnnagatg aacgccttct ccatcttctg               50


<210>  420
<211>  51
<212>  DNA
<213>  Artificial Sequence

<220>
<223>  primer


<220>
<221>  misc_feature
<222>  (23)..(26)
<223>  n is a, c, g, or t

<400>  420
ctacacgacg ctcttccgat ctnnnnccga gtatgccttc tccatcttct g             51


<210>  421
<211>  48
<212>  DNA
<213>  Artificial Sequence

<220>
<223>  primer

<220>
<221>  misc_feature
<222>  23
<223>  n is a, c, g, or t

<400>  421
ctacacgacg ctcttccgat ctnaatattg agccttctcc atcttctg                    48




<210>  422
<211>  49
<212>  DNA
<213>  Artificial Sequence

<220>
<223>  primer



<220>
<221>  misc_feature
<222>  (23)..(24)
<223>  n is a, c, g, or t

<400>  422
ctacacgacg ctcttccgat ctnngtatac cggccttctc catcttctg                   49




<210>  423
<211>  50
<212>  DNA
<213>  Artificial Sequence

<220>
<223>  primer



<220>
<221>  misc_feature
<222>  (23)..(25)
<223>  n is a, c, g, or t

<400>  423
ctacacgacg ctcttccgat ctnnngatcc aacgccttct ccatcttctg                  50




<210>  424
<211>  51
<212>  DNA
<213>  Artificial Sequence

<220>
<223>  primer



<220>
<221>  misc_feature
<222>  (23)..(26)
<223>  n is a, c, g, or t

<400>  424
ctacacgacg ctcttccgat ctnnnnagat acgcgccttc tccatcttct g                51

<210>  425
<211>  48
<212>  DNA
<213>  Artificial Sequence

<220>
<223>  primer

<220>
<221>  misc_feature
<222>  23
<223>  n is a, c, g, or t

<400>  425
ctacacgacg ctcttccgat ctnggtatct tgccttctcc atcttctg                48

<210>  426
<211>  49
<212>  DNA
<213>  Artificial Sequence

<220>
<223>  primer

<220>
<221>  misc_feature
<222>  (23)..(24)
<223>  n is a, c, g, or t

<400>  426
ctacacgacg ctcttccgat ctnncctctg gcgccttctc catcttctg               49

<210>  427
<211>  48
<212>  DNA
<213>  Artificial Sequence

<220>
<223>  primer

<220>
<221>  misc_feature
<222>  23
<223>  n is a, c, g, or t

<400>  427
ctacacgacg ctcttccgat ctnccattgt ggccttctcc atcttctg                48

<210>  428
<211>  49
<212>  DNA
<213>  Artificial Sequence

<220>
<223>  primer


<220>
<221>  misc_feature
<222>  (23)..(24)
<223>  n is a, c, g, or t

<400>  428
ctacacgacg ctcttccgat ctnnactacg gtgccttctc catcttctg                49


<210>  429
<211>  50
<212>  DNA
<213>  Artificial Sequence

<220>
<223>  primer


<220>
<221>  misc_feature
<222>  (23)..(25)
<223>  n is a, c, g, or t

<400>  429
ctacacgacg ctcttccgat ctnnnaagtg ctagccttct ccatcttctg               50


<210>  430
<211>  51
<212>  DNA
<213>  Artificial Sequence

<220>
<223>  primer


<220>
<221>  misc_feature
<222>  (23)..(26)
<223>  n is a, c, g, or t

<400>  430
ctacacgacg ctcttccgat ctnnnngccg aacggccttc tccatcttct g             51


<210>  431
<211>  48
<212>  DNA
<213>  Artificial Sequence

<220>

<223>  primer

<220>
<221>  misc_feature
<222>  23
<223>  n is a, c, g, or t

<400>  431
ctacacgacg ctcttccgat ctntgtccac ggccttctcc atcttctg                48

<210>  432
<211>  49
<212>  DNA
<213>  Artificial Sequence

<220>
<223>  primer

<220>
<221>  misc_feature
<222>  (23)..(24)
<223>  n is a, c, g, or t

<400>  432
ctacacgacg ctcttccgat ctnngacaca ctgccttctc catcttctg               49

<210>  433
<211>  50
<212>  DNA
<213>  Artificial Sequence

<220>
<223>  primer

<220>
<221>  misc_feature
<222>  (23)..(25)
<223>  n is a, c, g, or t

<400>  433
ctacacgacg ctcttccgat ctnnnaatat gctgccttct ccatcttctg              50

<210>  434
<211>  51
<212>  DNA
<213>  Artificial Sequence

<220>
<223>  primer

<220>
<221>  misc_feature
<222>  (23)..(26)

<223>  n is a, c, g, or t

<400>  434
ctacacgacg ctcttccgat ctnnnnttct catagccttc tccatcttct g                51

<210>  435
<211>  48
<212>  DNA
<213>  Artificial Sequence

<220>
<223>  primer

<220>
<221>  misc_feature
<222>  23
<223>  n is a, c, g, or t

<400>  435
ctacacgacg ctcttccgat ctntctgtga tgccttctcc atcttctg                48

<210>  436
<211>  49
<212>  DNA
<213>  Artificial Sequence

<220>
<223>  primer

<220>
<221>  misc_feature
<222>  (23)..(24)
<223>  n is a, c, g, or t

<400>  436
ctacacgacg ctcttccgat ctnnccgaac ttgccttctc catcttctg                49

<210>  437
<211>  50
<212>  DNA
<213>  Artificial Sequence

<220>
<223>  primer

<220>
<221>  misc_feature
<222>  (23)..(25)
<223>  n is a, c, g, or t

<400>  437
ctacacgacg ctcttccgat ctnnngtcta acagccttct ccatcttctg                50

<210>  438
<211>  51
<212>  DNA
<213>  Artificial Sequence

<220>
<223>  primer

<220>
<221>  misc_feature
<222>  (23)..(26)
<223>  n is a, c, g, or t

<400>  438
ctacacgacg ctcttccgat ctnnnngacg ccatgccttc tccatcttct g                51

<210>  439
<211>  48
<212>  DNA
<213>  Artificial Sequence

<220>
<223>  primer

<220>
<221>  misc_feature
<222>  23
<223>  n is a, c, g, or t

<400>  439
ctacacgacg ctcttccgat ctngccaatg tgccttctcc atcttctg                48

<210>  440
<211>  49
<212>  DNA
<213>  Artificial Sequence

<220>
<223>  primer

<220>
<221>  misc_feature
<222>  (23)..(24)
<223>  n is a, c, g, or t

<400>  440
ctacacgacg ctcttccgat ctnnccaacg tcgccttctc catcttctg                49

<210>  441
<211>  50
<212>  DNA
<213>  Artificial Sequence

<220>
<223>  primer


<220>
<221>  misc_feature
<222>  (23)..(25)
<223>  n is a, c, g, or t

<400>  441
ctacacgacg ctcttccgat ctnnngtaga taagccttct ccatcttctg                50



<210>  442
<211>  51
<212>  DNA
<213>  Artificial Sequence

<220>
<223>  primer


<220>
<221>  misc_feature
<222>  (23)..(26)
<223>  n is a, c, g, or t

<400>  442
ctacacgacg ctcttccgat ctnnnnctta cggcgccttc tccatcttct g              51



<210>  443
<211>  48
<212>  DNA
<213>  Artificial Sequence

<220>
<223>  primer


<220>
<221>  misc_feature
<222>  23
<223>  n is a, c, g, or t

<400>  443
ctacacgacg ctcttccgat ctnccaagtg cgccttctcc atcttctg                  48



<210>  444
<211>  49
<212>  DNA
<213>  Artificial Sequence

<220>
<223>  primer


<220>
<221>  misc_feature

<222>  (23)..(24)
<223>  n is a, c, g, or t

<400>  444
ctacacgacg ctcttccgat ctnnctaact cagccttctc catcttctg                49



<210>  445
<211>  50
<212>  DNA
<213>  Artificial Sequence

<220>
<223>  primer


<220>
<221>  misc_feature
<222>  (23)..(25)
<223>  n is a, c, g, or t

<400>  445
ctacacgacg ctcttccgat ctnnnaatat ctggccttct ccatcttctg               50



<210>  446
<211>  51
<212>  DNA
<213>  Artificial Sequence

<220>
<223>  primer


<220>
<221>  misc_feature
<222>  (23)..(26)
<223>  n is a, c, g, or t

<400>  446
ctacacgacg ctcttccgat ctnnnnttat atcagccttc tccatcttct g             51



<210>  447
<211>  48
<212>  DNA
<213>  Artificial Sequence

<220>
<223>  primer


<220>
<221>  misc_feature
<222>  23
<223>  n is a, c, g, or t

<400>  447
ctacacgacg ctcttccgat ctnctgcgga tgccttctcc atcttctg                 48

<210>  448
<211>  49
<212>  DNA
<213>  Artificial Sequence

<220>
<223>  primer


<220>
<221>  misc_feature
<222>  (23)..(24)
<223>  n is a, c, g, or t

<400>  448
ctacacgacg ctcttccgat ctnngcggct tggccttctc catcttctg                49


<210>  449
<211>  50
<212>  DNA
<213>  Artificial Sequence

<220>
<223>  primer


<220>
<221>  misc_feature
<222>  (23)..(25)
<223>  n is a, c, g, or t

<400>  449
ctacacgacg ctcttccgat ctnnngagtt gatgccttct ccatcttctg               50


<210>  450
<211>  51
<212>  DNA
<213>  Artificial Sequence

<220>
<223>  primer


<220>
<221>  misc_feature
<222>  (23)..(26)
<223>  n is a, c, g, or t

<400>  450
ctacacgacg ctcttccgat ctnnnngcac tgaggccttc tccatcttct g             51


<210>  451
<211>  48
<212>  DNA
<213>  Artificial Sequence

<220>
<223>  primer


<220>
<221>  misc_feature
<222>  23
<223>  n is a, c, g, or t

<400>  451
ctacacgacg ctcttccgat ctngaccacc tgccttctcc atcttctg                    48



<210>  452
<211>  48
<212>  DNA
<213>  Artificial Sequence

<220>
<223>  primer


<220>
<221>  misc_feature
<222>  23
<223>  n is a, c, g, or t

<400>  452
ctacacgacg ctcttccgat ctnaatagca agccttctcc atcttctg                    48



<210>  453
<211>  48
<212>  DNA
<213>  Artificial Sequence

<220>
<223>  primer


<220>
<221>  misc_feature
<222>  23
<223>  n is a, c, g, or t

<400>  453
ctacacgacg ctcttccgat ctncctaccg ggccttctcc atcttctg                    48



<210>  454
<211>  49
<212>  DNA
<213>  Artificial Sequence

<220>
<223>  primer


<220>

<221>  misc_feature
<222>  (23)..(24)
<223>  n is a, c, g, or t

<400>  454
ctacacgacg ctcttccgat ctnnggagga tggccttctc catcttctg                49

<210>  455
<211>  50
<212>  DNA
<213>  Artificial Sequence

<220>
<223>  primer

<220>
<221>  misc_feature
<222>  (23)..(25)
<223>  n is a, c, g, or t

<400>  455
ctacacgacg ctcttccgat ctnnncgctg aatgccttct ccatcttctg               50

<210>  456
<211>  51
<212>  DNA
<213>  Artificial Sequence

<220>
<223>  primer

<220>
<221>  misc_feature
<222>  (23)..(26)
<223>  n is a, c, g, or t

<400>  456
ctacacgacg ctcttccgat ctnnnntgtg acgagccttc tccatcttct g             51

<210>  457
<211>  48
<212>  DNA
<213>  Artificial Sequence

<220>
<223>  primer

<220>
<221>  misc_feature
<222>  23
<223>  n is a, c, g, or t

<400>  457
ctacacgacg ctcttccgat ctnaatagat tgccttctcc atcttctg                 48

<210>  458
<211>  49
<212>  DNA
<213>  Artificial Sequence

<220>
<223>  primer


<220>
<221>  misc_feature
<222>  (23)..(24)
<223>  n is a, c, g, or t

<400>  458
ctacacgacg ctcttccgat ctnnttagcg cagccttctc catcttctg                49


<210>  459
<211>  50
<212>  DNA
<213>  Artificial Sequence

<220>
<223>  primer


<220>
<221>  misc_feature
<222>  (23)..(25)
<223>  n is a, c, g, or t

<400>  459
ctacacgacg ctcttccgat ctnnngcggc cgtgccttct ccatcttctg               50


<210>  460
<211>  51
<212>  DNA
<213>  Artificial Sequence

<220>
<223>  primer


<220>
<221>  misc_feature
<222>  (23)..(26)
<223>  n is a, c, g, or t

<400>  460
ctacacgacg ctcttccgat ctnnnncagt aaccgccttc tccatcttct g             51


<210>  461
<211>  48
<212>  DNA

<213>  Artificial Sequence

<220>
<223>  primer


<220>
<221>  misc_feature
<222>  23
<223>  n is a, c, g, or t

<400>  461
ctacacgacg ctcttccgat ctngcctagt agccttctcc atcttctg                    48



<210>  462
<211>  49
<212>  DNA
<213>  Artificial Sequence

<220>
<223>  primer


<220>
<221>  misc_feature
<222>  (23)..(24)
<223>  n is a, c, g, or t

<400>  462
ctacacgacg ctcttccgat ctnncacggc gcgccttctc catcttctg                   49



<210>  463
<211>  50
<212>  DNA
<213>  Artificial Sequence

<220>
<223>  primer


<220>
<221>  misc_feature
<222>  (23)..(25)
<223>  n is a, c, g, or t

<400>  463
ctacacgacg ctcttccgat ctnnnggtgc agagccttct ccatcttctg                  50



<210>  464
<211>  51
<212>  DNA
<213>  Artificial Sequence

<220>
<223>  primer

<220>
<221>  misc_feature
<222>  (23)..(26)
<223>  n is a, c, g, or t

<400>  464
ctacacgacg ctcttccgat ctnnnntcgc tgacgccttc tccatcttct g          51

<210>  465
<211>  48
<212>  DNA
<213>  Artificial Sequence

<220>
<223>  primer

<220>
<221>  misc_feature
<222>  23
<223>  n is a, c, g, or t

<400>  465
ctacacgacg ctcttccgat ctncagccag tgccttctcc atcttctg          48

<210>  466
<211>  49
<212>  DNA
<213>  Artificial Sequence

<220>
<223>  primer

<220>
<221>  misc_feature
<222>  (23)..(24)
<223>  n is a, c, g, or t

<400>  466
ctacacgacg ctcttccgat ctnncgtcaa ccgccttctc catcttctg          49

<210>  467
<211>  48
<212>  DNA
<213>  Artificial Sequence

<220>
<223>  primer

<220>
<221>  misc_feature
<222>  23
<223>  n is a, c, g, or t

<400>  467

ctacacgacg ctcttccgat ctngccggcg agccttctcc atcttctg 48

<210> 468
<211> 49
<212> DNA
<213> Artificial Sequence

<220>
<223> primer

<220>
<221> misc_feature
<222> (23)..(24)
<223> n is a, c, g, or t

<400> 468
ctacacgacg ctcttccgat ctnngcctcc gggccttctc catcttctg 49

<210> 469
<211> 50
<212> DNA
<213> Artificial Sequence

<220>
<223> primer

<220>
<221> misc_feature
<222> (23)..(25)
<223> n is a, c, g, or t

<400> 469
ctacacgacg ctcttccgat ctnnnaatag tccgccttct ccatcttctg 50

<210> 470
<211> 51
<212> DNA
<213> Artificial Sequence

<220>
<223> primer

<220>
<221> misc_feature
<222> (23)..(26)
<223> n is a, c, g, or t

<400> 470
ctacacgacg ctcttccgat ctnnnnttag acgtgccttc tccatcttct g 51

<210> 471
<211> 48

<212>  DNA
<213>  Artificial Sequence

<220>
<223>  primer


<220>
<221>  misc_feature
<222>  23
<223>  n is a, c, g, or t

<400>  471
ctacacgacg ctcttccgat ctngtggact agccttctcc atcttctg                    48



<210>  472
<211>  49
<212>  DNA
<213>  Artificial Sequence

<220>
<223>  primer


<220>
<221>  misc_feature
<222>  (23)..(24)
<223>  n is a, c, g, or t

<400>  472
ctacacgacg ctcttccgat ctnncacgga cggccttctc catcttctg                   49



<210>  473
<211>  50
<212>  DNA
<213>  Artificial Sequence

<220>
<223>  primer


<220>
<221>  misc_feature
<222>  (23)..(25)
<223>  n is a, c, g, or t

<400>  473
ctacacgacg ctcttccgat ctnnncacta gaggccttct ccatcttctg                  50



<210>  474
<211>  51
<212>  DNA
<213>  Artificial Sequence

<220>
<223>  primer

<220>
<221>  misc_feature
<222>  (23)..(26)
<223>  n is a, c, g, or t

<400>  474
ctacacgacg ctcttccgat ctnnnngcag atgggccttc tccatcttct g                51

<210>  475
<211>  48
<212>  DNA
<213>  Artificial Sequence

<220>
<223>  primer

<220>
<221>  misc_feature
<222>  23
<223>  n is a, c, g, or t

<400>  475
ctacacgacg ctcttccgat ctnctctcac ggccttctcc atcttctg                48

<210>  476
<211>  49
<212>  DNA
<213>  Artificial Sequence

<220>
<223>  primer

<220>
<221>  misc_feature
<222>  (23)..(24)
<223>  n is a, c, g, or t

<400>  476
ctacacgacg ctcttccgat ctnnggaatc acgccttctc catcttctg                49

<210>  477
<211>  50
<212>  DNA
<213>  Artificial Sequence

<220>
<223>  primer

<220>
<221>  misc_feature
<222>  (23)..(25)
<223>  n is a, c, g, or t

<400>  477
ctacacgacg ctcttccgat ctnnncgttg acggccttct ccatcttctg                50

<210>  478
<211>  51
<212>  DNA
<213>  Artificial Sequence

<220>
<223>  primer

<220>
<221>  misc_feature
<222>  (23)..(26)
<223>  n is a, c, g, or t

<400>  478
ctacacgacg ctcttccgat ctnnnncatc aggtgccttc tccatcttct g              51

<210>  479
<211>  48
<212>  DNA
<213>  Artificial Sequence

<220>
<223>  primer

<220>
<221>  misc_feature
<222>  23
<223>  n is a, c, g, or t

<400>  479
ctacacgacg ctcttccgat ctncgttgta agccttctcc atcttctg                  48

<210>  480
<211>  49
<212>  DNA
<213>  Artificial Sequence

<220>
<223>  primer

<220>
<221>  misc_feature
<222>  (23)..(24)
<223>  n is a, c, g, or t

<400>  480
ctacacgacg ctcttccgat ctnnggcacg gtgccttctc catcttctg                 49

<210>  481

<211>  48
<212>  DNA
<213>  Artificial Sequence

<220>
<223>  primer


<220>
<221>  misc_feature
<222>  23
<223>  n is a, c, g, or t

<400>  481
ctacacgacg ctcttccgat ctnaattaat gtcctccggc ccctgaat                    48



<210>  482
<211>  49
<212>  DNA
<213>  Artificial Sequence

<220>
<223>  primer


<220>
<221>  misc_feature
<222>  (23)..(24)
<223>  n is a, c, g, or t

<400>  482
   ctacacgacg ctcttccgat ctnnttaatt cctcctccgg cccctgaat                  49



<210>  483
<211>  48
<212>  DNA
<213>  Artificial Sequence

<220>
<223>  primer


<220>
<221>  misc_feature
<222>  23
<223>  n is a, c, g, or t

<400>  483
ctacacgacg ctcttccgat ctnccaatac ttcctccggc ccctgaat                    48



<210>  484
<211>  49
<212>  DNA
<213>  Artificial Sequence

<220>
<223>  primer

<220>
<221>  misc_feature
<222>  (23)..(24)
<223>  n is a, c, g, or t

<400>  484
ctacacgacg ctcttccgat ctnntcgcgc attcctccgg cccctgaat                49

<210>  485
<211>  50
<212>  DNA
<213>  Artificial Sequence

<220>
<223>  primer

<220>
<221>  misc_feature
<222>  (23)..(25)
<223>  n is a, c, g, or t

<400>  485
ctacacgacg ctcttccgat ctnnngtgtg aactcctccg gcccctgaat               50

<210>  486
<211>  51
<212>  DNA
<213>  Artificial Sequence

<220>
<223>  primer

<220>
<221>  misc_feature
<222>  (23)..(26)
<223>  n is a, c, g, or t

<400>  486
ctacacgacg ctcttccgat ctnnnnggtg gcactcctcc ggcccctgaa t             51

<210>  487
<211>  48
<212>  DNA
<213>  Artificial Sequence

<220>
<223>  primer

<220>
<221>  misc_feature
<222>  23
<223>  n is a, c, g, or t

<400>  487
ctacacgacg ctcttccgat ctntaggtgc ttcctccggc ccctgaat                    48

<210>  488
<211>  49
<212>  DNA
<213>  Artificial Sequence

<220>
<223>  primer

<220>
<221>  misc_feature
<222>  (23)..(24)
<223>  n is a, c, g, or t

<400>  488
ctacacgacg ctcttccgat ctnngagcgg tttcctccgg cccctgaat                   49

<210>  489
<211>  50
<212>  DNA
<213>  Artificial Sequence

<220>
<223>  primer

<220>
<221>  misc_feature
<222>  (23)..(25)
<223>  n is a, c, g, or t

<400>  489
ctacacgacg ctcttccgat ctnnnaggat tagtcctccg gcccctgaat                  50

<210>  490
<211>  51
<212>  DNA
<213>  Artificial Sequence

<220>
<223>  primer

<220>
<221>  misc_feature
<222>  (23)..(26)
<223>  n is a, c, g, or t

<400>  490
ctacacgacg ctcttccgat ctnnnngtga gccatcctcc ggcccctgaa t                51

<210>  491
<211>  48
<212>  DNA
<213>  Artificial Sequence

<220>
<223>  primer


<220>
<221>  misc_feature
<222>  23
<223>  n is a, c, g, or t

<400>  491
ctacacgacg ctcttccgat ctnaattatg ctcctccggc ccctgaat                    48



<210>  492
<211>  49
<212>  DNA
<213>  Artificial Sequence

<220>
<223>  primer


<220>
<221>  misc_feature
<222>  (23)..(24)
<223>  n is a, c, g, or t

<400>  492
ctacacgacg ctcttccgat ctnnttacgc cgtcctccgg cccctgaat                   49



<210>  493
<211>  50
<212>  DNA
<213>  Artificial Sequence

<220>
<223>  primer


<220>
<221>  misc_feature
<222>  (23)..(25)
<223>  n is a, c, g, or t

<400>  493
ctacacgacg ctcttccgat ctnnngtagt gattcctccg gcccctgaat                  50



<210>  494
<211>  51
<212>  DNA
<213>  Artificial Sequence

<220>

<223>  primer

<220>
<221>  misc_feature
<222>  (23)..(26)
<223>  n is a, c, g, or t

<400>  494
ctacacgacg ctcttccgat ctnnnntggt tgcatcctcc ggcccctgaa t                51

<210>  495
<211>  48
<212>  DNA
<213>  Artificial Sequence

<220>
<223>  primer

<220>
<221>  misc_feature
<222>  23
<223>  n is a, c, g, or t

<400>  495
ctacacgacg ctcttccgat ctngtccgac ctcctccggc ccctgaat                    48

<210>  496
<211>  66
<212>  DNA
<213>  Artificial Sequence

<220>
<223>  primer

<400>  496
gtgactggag ttcagacgtg tgctcttccg atctagcttg gttctggtta ctgccagttg      60
aatctg                                                                  66

<210>  497
<211>  66
<212>  DNA
<213>  Artificial Sequence

<220>
<223>  primer

<400>  497
gtgactggag ttcagacgtg tgctcttccg atctagctta tgtctggtta ctgccagttg      60
aatctg                                                                  66

<210>  498

<211>  66
<212>  DNA
<213>  Artificial Sequence

<220>
<223>  primer


<400>  498
gtgactggag ttcagacgtg tgctcttccg atctataacg actctggtta ctgccagttg        60
aatctg                                                                    66



<210>  499
<211>  66
<212>  DNA
<213>  Artificial Sequence

<220>
<223>  primer


<400>  499
gtgactggag ttcagacgtg tgctcttccg atctgatgga gttctggtta ctgccagttg        60
aatctg                                                                    66



<210>  500
<211>  66
<212>  DNA
<213>  Artificial Sequence

<220>
<223>  primer


<400>  500
gtgactggag ttcagacgtg tgctcttccg atcttgatct aatctggtta ctgccagttg        60
aatctg                                                                    66



<210>  501
<211>  66
<212>  DNA
<213>  Artificial Sequence

<220>
<223>  primer


<400>  501
gtgactggag ttcagacgtg tgctcttccg atctagctat attctggtta ctgccagttg        60
aatctg                                                                    66



<210>  502
<211>  66
<212>  DNA
<213>  Artificial Sequence

<220>
<223>  primer


<400>  502
gtgactggag ttcagacgtg tgctcttccg atctagcaga gatctggtta ctgccagttg      60
aatctg                                                                  66




<210>  503
<211>  66
<212>  DNA
<213>  Artificial Sequence

<220>
<223>  primer


<400>  503
gtgactggag ttcagacgtg tgctcttccg atctgacgta agtctggtta ctgccagttg      60
aatctg                                                                  66




<210>  504
<211>  66
<212>  DNA
<213>  Artificial Sequence

<220>
<223>  primer


<400>  504
gtgactggag ttcagacgtg tgctcttccg atctaggcct gatctggtta ctgccagttg      60
aatctg                                                                  66




<210>  505
<211>  66
<212>  DNA
<213>  Artificial Sequence

<220>
<223>  primer


<400>  505
gtgactggag ttcagacgtg tgctcttccg atcttaagat tatctggtta ctgccagttg      60
aatctg                                                                  66




<210>  506
<211>  66
<212>  DNA
<213>  Artificial Sequence

<220>
<223>  primer

<400>  506
gtgactggag ttcagacgtg tgctcttccg atctaggcga attctggtta ctgccagttg        60
aatctg                                                                    66

<210>  507
<211>  66
<212>  DNA
<213>  Artificial Sequence

<220>
<223>  primer

<400>  507
gtgactggag ttcagacgtg tgctcttccg atctttgttc tttctggtta ctgccagttg        60
aatctg                                                                    66

<210>  508
<211>  66
<212>  DNA
<213>  Artificial Sequence

<220>
<223>  primer

<400>  508
gtgactggag ttcagacgtg tgctcttccg atcttactcc tgtctggtta ctgccagttg        60
aatctg                                                                    66

<210>  509
<211>  66
<212>  DNA
<213>  Artificial Sequence

<220>
<223>  primer

<400>  509
gtgactggag ttcagacgtg tgctcttccg atctgactgg cgtctggtta ctgccagttg        60
aatctg                                                                    66

<210>  510
<211>  66
<212>  DNA
<213>  Artificial Sequence

<220>
<223>  primer

<400>  510

gtgactggag ttcagacgtg tgctcttccg atctatatat actctggtta ctgccagttg 60
aatctg 66

<210> 511
<211> 66
<212> DNA
<213> Artificial Sequence

<220>
<223> primer

<400> 511
gtgactggag ttcagacgtg tgctcttccg atcttagatc ggtctggtta ctgccagttg 60
aatctg 66

<210> 512
<211> 66
<212> DNA
<213> Artificial Sequence

<220>
<223> primer

<400> 512
gtgactggag ttcagacgtg tgctcttccg atctaggcaa gctctggtta ctgccagttg 60
aatctg 66

<210> 513
<211> 66
<212> DNA
<213> Artificial Sequence

<220>
<223> primer

<400> 513
gtgactggag ttcagacgtg tgctcttccg atcttccagg tatctggtta ctgccagttg 60
aatctg 66

<210> 514
<211> 66
<212> DNA
<213> Artificial Sequence

<220>
<223> primer

<400> 514
gtgactggag ttcagacgtg tgctcttccg atctaggtca agtctggtta ctgccagttg 60
aatctg 66

<210>  515
<211>  66
<212>  DNA
<213>  Artificial Sequence

<220>
<223>  primer


<400>  515
gtgactggag ttcagacgtg tgctcttccg atcttacact ggtctggtta ctgccagttg      60
aatctg                                                                  66




<210>  516
<211>  66
<212>  DNA
<213>  Artificial Sequence

<220>
<223>  primer


<400>  516
gtgactggag ttcagacgtg tgctcttccg atcttccttg cgtctggtta ctgccagttg      60
aatctg                                                                  66




<210>  517
<211>  66
<212>  DNA
<213>  Artificial Sequence

<220>
<223>  primer


<400>  517
gtgactggag ttcagacgtg tgctcttccg atctaggacc tctctggtta ctgccagttg      60
aatctg                                                                  66




<210>  518
<211>  66
<212>  DNA
<213>  Artificial Sequence

<220>
<223>  primer


<400>  518
gtgactggag ttcagacgtg tgctcttccg atcttaagca tttctggtta ctgccagttg      60
aatctg                                                                  66




<210>  519

<211>  66
<212>  DNA
<213>  Artificial Sequence

<220>
<223>  primer


<400>  519
gtgactggag ttcagacgtg tgctcttccg atctaggata tttctggtta ctgccagttg        60
aatctg                                                                    66



<210>  520
<211>  66
<212>  DNA
<213>  Artificial Sequence

<220>
<223>  primer


<400>  520
gtgactggag ttcagacgtg tgctcttccg atctaatcgt tatctggtta ctgccagttg        60
aatctg                                                                    66



<210>  521
<211>  66
<212>  DNA
<213>  Artificial Sequence

<220>
<223>  primer


<400>  521
gtgactggag ttcagacgtg tgctcttccg atctgtctac attctggtta ctgccagttg        60
aatctg                                                                    66



<210>  522
<211>  66
<212>  DNA
<213>  Artificial Sequence

<220>
<223>  primer


<400>  522
gtgactggag ttcagacgtg tgctcttccg atctcgctgc tctctggtta ctgccagttg        60
aatctg                                                                    66



<210>  523
<211>  66
<212>  DNA
<213>  Artificial Sequence

<220>
<223>  primer

<400>  523
gtgactggag ttcagacgtg tgctcttccg atctgatcaa catctggtta ctgccagttg      60
aatctg                                                                  66

<210>  524
<211>  66
<212>  DNA
<213>  Artificial Sequence

<220>
<223>  primer

<400>  524
gtgactggag ttcagacgtg tgctcttccg atctcgaagg actctggtta ctgccagttg      60
aatctg                                                                  66

<210>  525
<211>  66
<212>  DNA
<213>  Artificial Sequence

<220>
<223>  primer

<400>  525
gtgactggag ttcagacgtg tgctcttccg atctgatgcc ggtctggtta ctgccagttg      60
aatctg                                                                  66

<210>  526
<211>  66
<212>  DNA
<213>  Artificial Sequence

<220>
<223>  primer

<400>  526
gtgactggag ttcagacgtg tgctcttccg atctctacga agtctggtta ctgccagttg      60
aatctg                                                                  66

<210>  527
<211>  66
<212>  DNA
<213>  Artificial Sequence

<220>
<223>  primer

<400>  527
gtgactggag ttcagacgtg tgctcttccg atctgatgcg tctctggtta ctgccagttg        60
aatctg                                                                    66

<210>  528
<211>  66
<212>  DNA
<213>  Artificial Sequence

<220>
<223>  primer

<400>  528
gtgactggag ttcagacgtg tgctcttccg atctctacgg catctggtta ctgccagttg        60
aatctg                                                                    66

<210>  529
<211>  66
<212>  DNA
<213>  Artificial Sequence

<220>
<223>  primer

<400>  529
gtgactggag ttcagacgtg tgctcttccg atctgattcc tttctggtta ctgccagttg        60
aatctg                                                                    66

<210>  530
<211>  66
<212>  DNA
<213>  Artificial Sequence

<220>
<223>  primer

<400>  530
gtgactggag ttcagacgtg tgctcttccg atctctactc gatctggtta ctgccagttg        60
aatctg                                                                    66

<210>  531
<211>  66
<212>  DNA
<213>  Artificial Sequence

<220>
<223>  primer

<400>  531

gtgactggag ttcagacgtg tgctcttccg atctgattcg agtctggtta ctgccagttg 60
aatctg 66

<210> 532
<211> 66
<212> DNA
<213> Artificial Sequence

<220>
<223> primer

<400> 532
gtgactggag ttcagacgtg tgctcttccg atctaatcgg cgtctggtta ctgccagttg 60
aatctg 66

<210> 533
<211> 66
<212> DNA
<213> Artificial Sequence

<220>
<223> primer

<400> 533
gtgactggag ttcagacgtg tgctcttccg atctttcgcc gatctggtta ctgccagttg 60
aatctg 66

<210> 534
<211> 66
<212> DNA
<213> Artificial Sequence

<220>
<223> primer

<400> 534
gtgactggag ttcagacgtg tgctcttccg atctctggcc tctctggtta ctgccagttg 60
aatctg 66

<210> 535
<211> 66
<212> DNA
<213> Artificial Sequence

<220>
<223> primer

<400> 535
gtgactggag ttcagacgtg tgctcttccg atctgaactt attctggtta ctgccagttg 60
aatctg 66

<210>  536
<211>  66
<212>  DNA
<213>  Artificial Sequence

<220>
<223>  primer


<400>  536
gtgactggag ttcagacgtg tgctcttccg atctcgtatt ggtctggtta ctgccagttg      60
aatctg                                                                  66




<210>  537
<211>  66
<212>  DNA
<213>  Artificial Sequence

<220>
<223>  primer


<400>  537
gtgactggag ttcagacgtg tgctcttccg atctgaagca catctggtta ctgccagttg      60
aatctg                                                                  66




<210>  538
<211>  66
<212>  DNA
<213>  Artificial Sequence

<220>
<223>  primer


<400>  538
gtgactggag ttcagacgtg tgctcttccg atctcttaat actctggtta ctgccagttg      60
aatctg                                                                  66




<210>  539
<211>  66
<212>  DNA
<213>  Artificial Sequence

<220>
<223>  primer


<400>  539
gtgactggag ttcagacgtg tgctcttccg atctgaagtc tttctggtta ctgccagttg      60
aatctg                                                                  66




<210>  540

<211>  66
<212>  DNA
<213>  Artificial Sequence

<220>
<223>  primer


<400>  540
gtgactggag ttcagacgtg tgctcttccg atctgaagag gctctggtta ctgccagttg        60
aatctg                                                                    66




<210>  541
<211>  66
<212>  DNA
<213>  Artificial Sequence

<220>
<223>  primer


<400>  541
gtgactggag ttcagacgtg tgctcttccg atctcggata actctggtta ctgccagttg        60
aatctg                                                                    66




<210>  542
<211>  66
<212>  DNA
<213>  Artificial Sequence

<220>
<223>  primer


<400>  542
gtgactggag ttcagacgtg tgctcttccg atctgaatct ggtctggtta ctgccagttg        60
aatctg                                                                    66




<210>  543
<211>  66
<212>  DNA
<213>  Artificial Sequence

<220>
<223>  primer


<400>  543
gtgactggag ttcagacgtg tgctcttccg atctctgatt gatctggtta ctgccagttg        60
aatctg                                                                    66




<210>  544
<211>  66
<212>  DNA
<213>  Artificial Sequence

<220>
<223>  primer


<400>  544
gtgactggag ttcagacgtg tgctcttccg atctaatccg tttctggtta ctgccagttg        60
aatctg                                                                    66




<210>  545
<211>  66
<212>  DNA
<213>  Artificial Sequence

<220>
<223>  primer


<400>  545
gtgactggag ttcagacgtg tgctcttccg atcttgcgta catctggtta ctgccagttg        60
aatctg                                                                    66




<210>  546
<211>  66
<212>  DNA
<213>  Artificial Sequence

<220>
<223>  primer


<400>  546
gtgactggag ttcagacgtg tgctcttccg atctgaatca attctggtta ctgccagttg        60
aatctg                                                                    66




<210>  547
<211>  66
<212>  DNA
<213>  Artificial Sequence

<220>
<223>  primer


<400>  547
gtgactggag ttcagacgtg tgctcttccg atcttgagtc agtctggtta ctgccagttg        60
aatctg                                                                    66




<210>  548
<211>  66
<212>  DNA
<213>  Artificial Sequence

<220>
<223>  primer

<400>  548
gtgactggag ttcagacgtg tgctcttccg atctgaatgc tctctggtta ctgccagttg        60
aatctg                                                                    66

<210>  549
<211>  66
<212>  DNA
<213>  Artificial Sequence

<220>
<223>  primer

<400>  549
gtgactggag ttcagacgtg tgctcttccg atctgaatat cctctggtta ctgccagttg        60
aatctg                                                                    66

<210>  550
<211>  66
<212>  DNA
<213>  Artificial Sequence

<220>
<223>  primer

<400>  550
gtgactggag ttcagacgtg tgctcttccg atctcttatg aatctggtta ctgccagttg        60
aatctg                                                                    66

<210>  551
<211>  66
<212>  DNA
<213>  Artificial Sequence

<220>
<223>  primer

<400>  551
gtgactggag ttcagacgtg tgctcttccg atcttcggca cctctggtta ctgccagttg        60
aatctg                                                                    66

<210>  552
<211>  66
<212>  DNA
<213>  Artificial Sequence

<220>
<223>  primer

<400>  552

gtgactggag ttcagacgtg tgctcttccg atctaagaag cgtctggtta ctgccagttg 60
aatctg 66

<210> 553
<211> 66
<212> DNA
<213> Artificial Sequence

<220>
<223> primer

<400> 553
gtgactggag ttcagacgtg tgctcttccg atctctcacg attctggtta ctgccagttg 60
aatctg 66

<210> 554
<211> 66
<212> DNA
<213> Artificial Sequence

<220>
<223> primer

<400> 554
gtgactggag ttcagacgtg tgctcttccg atcttcggtc gatctggtta ctgccagttg 60
aatctg 66

<210> 555
<211> 66
<212> DNA
<213> Artificial Sequence

<220>
<223> primer

<400> 555
gtgactggag ttcagacgtg tgctcttccg atcttcggta agtctggtta ctgccagttg 60
aatctg 66

<210> 556
<211> 66
<212> DNA
<213> Artificial Sequence

<220>
<223> primer

<400> 556
gtgactggag ttcagacgtg tgctcttccg atctaagata catctggtta ctgccagttg 60
aatctg 66

<210>  557
<211>  66
<212>  DNA
<213>  Artificial Sequence

<220>
<223>  primer


<400>  557
gtgactggag ttcagacgtg tgctcttccg atctgtcgct gttctggtta ctgccagttg        60
aatctg                                                                    66




<210>  558
<211>  66
<212>  DNA
<213>  Artificial Sequence

<220>
<223>  primer


<400>  558
gtgactggag ttcagacgtg tgctcttccg atcttcggat gttctggtta ctgccagttg        60
aatctg                                                                    66




<210>  559
<211>  66
<212>  DNA
<213>  Artificial Sequence

<220>
<223>  primer


<400>  559
gtgactggag ttcagacgtg tgctcttccg atctcgagcc ggtctggtta ctgccagttg        60
aatctg                                                                    66




<210>  560
<211>  66
<212>  DNA
<213>  Artificial Sequence

<220>
<223>  primer


<400>  560
gtgactggag ttcagacgtg tgctcttccg atctcgatta tctctggtta ctgccagttg        60
aatctg                                                                    66




<210>  561

<211>  66
<212>  DNA
<213>  Artificial Sequence

<220>
<223>  primer


<400>  561
gtgactggag ttcagacgtg tgctcttccg atcttcgaag cttctggtta ctgccagttg        60
aatctg                                                                    66



<210>  562
<211>  66
<212>  DNA
<213>  Artificial Sequence

<220>
<223>  primer


<400>  562
gtgactggag ttcagacgtg tgctcttccg atctctatca tttctggtta ctgccagttg        60
aatctg                                                                    66



<210>  563
<211>  66
<212>  DNA
<213>  Artificial Sequence

<220>
<223>  primer


<400>  563
gtgactggag ttcagacgtg tgctcttccg atctaagtta tctctggtta ctgccagttg        60
aatctg                                                                    66



<210>  564
<211>  66
<212>  DNA
<213>  Artificial Sequence

<220>
<223>  primer


<400>  564
gtgactggag ttcagacgtg tgctcttccg atctcgaacg gatctggtta ctgccagttg        60
aatctg                                                                    66



<210>  565
<211>  66
<212>  DNA
<213>  Artificial Sequence

<220>
<223>  primer

<400>  565
gtgactggag ttcagacgtg tgctcttccg atctctactg actctggtta ctgccagttg      60
aatctg                                                                  66

<210>  566
<211>  66
<212>  DNA
<213>  Artificial Sequence

<220>
<223>  primer

<400>  566
gtgactggag ttcagacgtg tgctcttccg atcttcttaa gttctggtta ctgccagttg      60
aatctg                                                                  66

<210>  567
<211>  66
<212>  DNA
<213>  Artificial Sequence

<220>
<223>  primer

<400>  567
gtgactggag ttcagacgtg tgctcttccg atctttagag tctctggtta ctgccagttg      60
aatctg                                                                  66

<210>  568
<211>  66
<212>  DNA
<213>  Artificial Sequence

<220>
<223>  primer

<400>  568
gtgactggag ttcagacgtg tgctcttccg atctaagacg aatctggtta ctgccagttg      60
aatctg                                                                  66

<210>  569
<211>  66
<212>  DNA
<213>  Artificial Sequence

<220>
<223>  primer

<400>  569
gtgactggag ttcagacgtg tgctcttccg atctttatta tgtctggtta ctgccagttg        60
aatctg                                                                    66

<210>  570
<211>  66
<212>  DNA
<213>  Artificial Sequence

<220>
<223>  primer

<400>  570
gtgactggag ttcagacgtg tgctcttccg atctcgctat tatctggtta ctgccagttg        60
aatctg                                                                    66

<210>  571
<211>  66
<212>  DNA
<213>  Artificial Sequence

<220>
<223>  primer

<400>  571
gtgactggag ttcagacgtg tgctcttccg atcttctatc agtctggtta ctgccagttg        60
aatctg                                                                    66

<210>  572
<211>  66
<212>  DNA
<213>  Artificial Sequence

<220>
<223>  primer

<400>  572
gtgactggag ttcagacgtg tgctcttccg atctcggtgg tatctggtta ctgccagttg        60
aatctg                                                                    66

<210>  573
<211>  66
<212>  DNA
<213>  Artificial Sequence

<220>
<223>  primer

<400>  573

gtgactggag ttcagacgtg tgctcttccg atcttcacca attctggtta ctgccagttg 60
aatctg 66

<210> 574
<211> 66
<212> DNA
<213> Artificial Sequence

<220>
<223> primer

<400> 574
gtgactggag ttcagacgtg tgctcttccg atctctggaa gctctggtta ctgccagttg 60
aatctg 66

<210> 575
<211> 66
<212> DNA
<213> Artificial Sequence

<220>
<223> primer

<400> 575
gtgactggag ttcagacgtg tgctcttccg atctcgtaag agtctggtta ctgccagttg 60
aatctg 66

<210> 576
<211> 66
<212> DNA
<213> Artificial Sequence

<220>
<223> primer

<400> 576
gtgactggag ttcagacgtg tgctcttccg atctaagaga gctctggtta ctgccagttg 60
aatctg 66

<210> 577
<211> 66
<212> DNA
<213> Artificial Sequence

<220>
<223> primer

<400> 577
gtgactggag ttcagacgtg tgctcttccg atcttcaacg agtctggtta ctgccagttg 60
aatctg 66

<210>  578
<211>  66
<212>  DNA
<213>  Artificial Sequence

<220>
<223>  primer


<400>  578
gtgactggag ttcagacgtg tgctcttccg atcttgcgag actctggtta ctgccagttg      60
aatctg                                                                  66




<210>  579
<211>  66
<212>  DNA
<213>  Artificial Sequence

<220>
<223>  primer


<400>  579
gtgactggag ttcagacgtg tgctcttccg atctcctggt gttctggtta ctgccagttg      60
aatctg                                                                  66




<210>  580
<211>  66
<212>  DNA
<213>  Artificial Sequence

<220>
<223>  primer


<400>  580
gtgactggag ttcagacgtg tgctcttccg atctaagtaa gttctggtta ctgccagttg      60
aatctg                                                                  66




<210>  581
<211>  66
<212>  DNA
<213>  Artificial Sequence

<220>
<223>  primer


<400>  581
gtgactggag ttcagacgtg tgctcttccg atcttgactg aatctggtta ctgccagttg      60
aatctg                                                                  66




<210>  582

<211>  66
<212>  DNA
<213>  Artificial Sequence

<220>
<223>  primer


<400>  582
gtgactggag ttcagacgtg tgctcttccg atctaagact gttctggtta ctgccagttg        60
aatctg                                                                    66




<210>  583
<211>  66
<212>  DNA
<213>  Artificial Sequence

<220>
<223>  primer


<400>  583
gtgactggag ttcagacgtg tgctcttccg atctcaatga tgtctggtta ctgccagttg        60
aatctg                                                                    66




<210>  584
<211>  66
<212>  DNA
<213>  Artificial Sequence

<220>
<223>  primer


<400>  584
gtgactggag ttcagacgtg tgctcttccg atctcacagt aatctggtta ctgccagttg        60
aatctg                                                                    66




<210>  585
<211>  66
<212>  DNA
<213>  Artificial Sequence

<220>
<223>  primer


<400>  585
gtgactggag ttcagacgtg tgctcttccg atcttggtca tttctggtta ctgccagttg        60
aatctg                                                                    66




<210>  586
<211>  66
<212>  DNA
<213>  Artificial Sequence

<220>
<223>  primer

<400>  586
gtgactggag ttcagacgtg tgctcttccg atctcaaccg tgtctggtta ctgccagttg      60
aatctg                                                                  66

<210>  587
<211>  66
<212>  DNA
<213>  Artificial Sequence

<220>
<223>  primer

<400>  587
gtgactggag ttcagacgtg tgctcttccg atcttggtgc actctggtta ctgccagttg      60
aatctg                                                                  66

<210>  588
<211>  66
<212>  DNA
<213>  Artificial Sequence

<220>
<223>  primer

<400>  588
gtgactggag ttcagacgtg tgctcttccg atctccacaa tgtctggtta ctgccagttg      60
aatctg                                                                  66

<210>  589
<211>  66
<212>  DNA
<213>  Artificial Sequence

<220>
<223>  primer

<400>  589
gtgactggag ttcagacgtg tgctcttccg atcttgtgtg cctctggtta ctgccagttg      60
aatctg                                                                  66

<210>  590
<211>  66
<212>  DNA
<213>  Artificial Sequence

<220>
<223>  primer

<400>  590
gtgactggag ttcagacgtg tgctcttccg atctcaccac ggtctggtta ctgccagttg        60
aatctg                                                                    66

<210>  591
<211>  66
<212>  DNA
<213>  Artificial Sequence

<220>
<223>  primer

<400>  591
gtgactggag ttcagacgtg tgctcttccg atcttgtgtt aatctggtta ctgccagttg        60
aatctg                                                                    66

<210>  592
<211>  63
<212>  DNA
<213>  Artificial Sequence

<220>
<223>  primer

<400>  592
gtgactggag ttcagacgtg tgctcttccg atctagcttg gttgtagcac gattgcagca        60
ttg                                                                       63

<210>  593
<211>  63
<212>  DNA
<213>  Artificial Sequence

<220>
<223>  primer

<400>  593
gtgactggag ttcagacgtg tgctcttccg atctagctta tgtgtagcac gattgcagca        60
ttg                                                                       63

<210>  594
<211>  63
<212>  DNA
<213>  Artificial Sequence

<220>
<223>  primer

<400>  594

gtgactggag ttcagacgtg tgctcttccg atctataacg actgtagcac gattgcagca     60
ttg                                                                   63

<210>  595
<211>  63
<212>  DNA
<213>  Artificial Sequence

<220>
<223>  primer

<400>  595
gtgactggag ttcagacgtg tgctcttccg atctgatgga gttgtagcac gattgcagca     60
ttg                                                                   63

<210>  596
<211>  63
<212>  DNA
<213>  Artificial Sequence

<220>
<223>  primer

<400>  596
gtgactggag ttcagacgtg tgctcttccg atcttgatct aatgtagcac gattgcagca     60
ttg                                                                   63

<210>  597
<211>  63
<212>  DNA
<213>  Artificial Sequence

<220>
<223>  primer

<400>  597
gtgactggag ttcagacgtg tgctcttccg atctagctat attgtagcac gattgcagca     60
ttg                                                                   63

<210>  598
<211>  63
<212>  DNA
<213>  Artificial Sequence

<220>
<223>  primer

<400>  598
gtgactggag ttcagacgtg tgctcttccg atctagcaga gatgtagcac gattgcagca     60
ttg                                                                   63

<210>  599
<211>  63
<212>  DNA
<213>  Artificial Sequence

<220>
<223>  primer


<400>  599
gtgactggag ttcagacgtg tgctcttccg atctgacgta agtgtagcac gattgcagca      60
ttg                                                                    63




<210>  600
<211>  63
<212>  DNA
<213>  Artificial Sequence

<220>
<223>  primer


<400>  600
gtgactggag ttcagacgtg tgctcttccg atctaggcct gatgtagcac gattgcagca      60
ttg                                                                    63




<210>  601
<211>  63
<212>  DNA
<213>  Artificial Sequence

<220>
<223>  primer


<400>  601
gtgactggag ttcagacgtg tgctcttccg atcttaagat tatgtagcac gattgcagca      60
ttg                                                                    63




<210>  602
<211>  63
<212>  DNA
<213>  Artificial Sequence

<220>
<223>  primer


<400>  602
gtgactggag ttcagacgtg tgctcttccg atctaggcga attgtagcac gattgcagca      60
ttg                                                                    63




<210>  603

<211>  63
<212>  DNA
<213>  Artificial Sequence

<220>
<223>  primer


<400>  603
gtgactggag ttcagacgtg tgctcttccg atctttgttc tttgtagcac gattgcagca      60
ttg                                                                    63



<210>  604
<211>  63
<212>  DNA
<213>  Artificial Sequence

<220>
<223>  primer


<400>  604
gtgactggag ttcagacgtg tgctcttccg atcttactcc tgtgtagcac gattgcagca      60
ttg                                                                    63



<210>  605
<211>  63
<212>  DNA
<213>  Artificial Sequence

<220>
<223>  primer


<400>  605
gtgactggag ttcagacgtg tgctcttccg atctgactgg cgtgtagcac gattgcagca      60
ttg                                                                    63



<210>  606
<211>  63
<212>  DNA
<213>  Artificial Sequence

<220>
<223>  primer


<400>  606
gtgactggag ttcagacgtg tgctcttccg atctatatat actgtagcac gattgcagca      60
ttg                                                                    63



<210>  607
<211>  63
<212>  DNA
<213>  Artificial Sequence

<220>
<223>  primer

<400>  607
gtgactggag ttcagacgtg tgctcttccg atcttagatc ggtgtagcac gattgcagca      60
ttg                                                                    63

<210>  608
<211>  63
<212>  DNA
<213>  Artificial Sequence

<220>
<223>  primer

<400>  608
gtgactggag ttcagacgtg tgctcttccg atctaggcaa gctgtagcac gattgcagca      60
ttg                                                                    63

<210>  609
<211>  63
<212>  DNA
<213>  Artificial Sequence

<220>
<223>  primer

<400>  609
gtgactggag ttcagacgtg tgctcttccg atcttccagg tatgtagcac gattgcagca      60
ttg                                                                    63

<210>  610
<211>  63
<212>  DNA
<213>  Artificial Sequence

<220>
<223>  primer

<400>  610
gtgactggag ttcagacgtg tgctcttccg atctaggtca agtgtagcac gattgcagca      60
ttg                                                                    63

<210>  611
<211>  63
<212>  DNA
<213>  Artificial Sequence

<220>
<223>  primer

<400>  611
gtgactggag ttcagacgtg tgctcttccg atcttacact ggtgtagcac gattgcagca      60
ttg                                                                    63

<210>  612
<211>  63
<212>  DNA
<213>  Artificial Sequence

<220>
<223>  primer

<400>  612
gtgactggag ttcagacgtg tgctcttccg atcttccttg cgtgtagcac gattgcagca      60
ttg                                                                    63

<210>  613
<211>  63
<212>  DNA
<213>  Artificial Sequence

<220>
<223>  primer

<400>  613
gtgactggag ttcagacgtg tgctcttccg atctaggacc tctgtagcac gattgcagca      60
ttg                                                                    63

<210>  614
<211>  63
<212>  DNA
<213>  Artificial Sequence

<220>
<223>  primer

<400>  614
gtgactggag ttcagacgtg tgctcttccg atcttaagca tttgtagcac gattgcagca      60
ttg                                                                    63

<210>  615
<211>  63
<212>  DNA
<213>  Artificial Sequence

<220>
<223>  primer

<400>  615

gtgactggag ttcagacgtg tgctcttccg atctaggata tttgtagcac gattgcagca 60
ttg 63

<210> 616
<211> 63
<212> DNA
<213> Artificial Sequence

<220>
<223> primer

<400> 616
gtgactggag ttcagacgtg tgctcttccg atctaatcgt tatgtagcac gattgcagca 60
ttg 63

<210> 617
<211> 63
<212> DNA
<213> Artificial Sequence

<220>
<223> primer

<400> 617
gtgactggag ttcagacgtg tgctcttccg atctgtctac attgtagcac gattgcagca 60
ttg 63

<210> 618
<211> 63
<212> DNA
<213> Artificial Sequence

<220>
<223> primer

<400> 618
gtgactggag ttcagacgtg tgctcttccg atctcgctgc tctgtagcac gattgcagca 60
ttg 63

<210> 619
<211> 63
<212> DNA
<213> Artificial Sequence

<220>
<223> primer

<400> 619
gtgactggag ttcagacgtg tgctcttccg atctgatcaa catgtagcac gattgcagca 60
ttg 63

<210>  620
<211>  63
<212>  DNA
<213>  Artificial Sequence

<220>
<223>  primer


<400>  620
gtgactggag ttcagacgtg tgctcttccg atctcgaagg actgtagcac gattgcagca      60
ttg                                                                    63




<210>  621
<211>  63
<212>  DNA
<213>  Artificial Sequence

<220>
<223>  primer


<400>  621
gtgactggag ttcagacgtg tgctcttccg atctgatgcc ggtgtagcac gattgcagca      60
ttg                                                                    63




<210>  622
<211>  63
<212>  DNA
<213>  Artificial Sequence

<220>
<223>  primer


<400>  622
gtgactggag ttcagacgtg tgctcttccg atctctacga agtgtagcac gattgcagca      60
ttg                                                                    63




<210>  623
<211>  63
<212>  DNA
<213>  Artificial Sequence

<220>
<223>  primer


<400>  623
gtgactggag ttcagacgtg tgctcttccg atctgatgcg tctgtagcac gattgcagca      60
ttg                                                                    63




<210>  624

<211>  63
<212>  DNA
<213>  Artificial Sequence

<220>
<223>  primer


<400>  624
gtgactggag ttcagacgtg tgctcttccg atctctacgg catgtagcac gattgcagca      60
ttg                                                                    63




<210>  625
<211>  63
<212>  DNA
<213>  Artificial Sequence

<220>
<223>  primer


<400>  625
gtgactggag ttcagacgtg tgctcttccg atctgattcc tttgtagcac gattgcagca      60
ttg                                                                    63




<210>  626
<211>  63
<212>  DNA
<213>  Artificial Sequence

<220>
<223>  primer


<400>  626
gtgactggag ttcagacgtg tgctcttccg atctctactc gatgtagcac gattgcagca      60
ttg                                                                    63




<210>  627
<211>  63
<212>  DNA
<213>  Artificial Sequence

<220>
<223>  primer


<400>  627
gtgactggag ttcagacgtg tgctcttccg atctgattcg agtgtagcac gattgcagca      60
ttg                                                                    63




<210>  628
<211>  63
<212>  DNA
<213>  Artificial Sequence

<220>
<223>  primer

<400>  628
gtgactggag ttcagacgtg tgctcttccg atctaatcgg cgtgtagcac gattgcagca      60
ttg                                                                    63

<210>  629
<211>  63
<212>  DNA
<213>  Artificial Sequence

<220>
<223>  primer

<400>  629
gtgactggag ttcagacgtg tgctcttccg atctttcgcc gatgtagcac gattgcagca      60
ttg                                                                    63

<210>  630
<211>  63
<212>  DNA
<213>  Artificial Sequence

<220>
<223>  primer

<400>  630
gtgactggag ttcagacgtg tgctcttccg atctctggcc tctgtagcac gattgcagca      60
ttg                                                                    63

<210>  631
<211>  63
<212>  DNA
<213>  Artificial Sequence

<220>
<223>  primer

<400>  631
gtgactggag ttcagacgtg tgctcttccg atctgaactt attgtagcac gattgcagca      60
ttg                                                                    63

<210>  632
<211>  63
<212>  DNA
<213>  Artificial Sequence

<220>
<223>  primer

<400>  632
gtgactggag ttcagacgtg tgctcttccg atctcgtatt ggtgtagcac gattgcagca      60
ttg                                                                    63

<210>  633
<211>  63
<212>  DNA
<213>  Artificial Sequence

<220>
<223>  primer

<400>  633
gtgactggag ttcagacgtg tgctcttccg atctgaagca catgtagcac gattgcagca      60
ttg                                                                    63

<210>  634
<211>  63
<212>  DNA
<213>  Artificial Sequence

<220>
<223>  primer

<400>  634
gtgactggag ttcagacgtg tgctcttccg atctcttaat actgtagcac gattgcagca      60
ttg                                                                    63

<210>  635
<211>  63
<212>  DNA
<213>  Artificial Sequence

<220>
<223>  primer

<400>  635
gtgactggag ttcagacgtg tgctcttccg atctgaagtc tttgtagcac gattgcagca      60
ttg                                                                    63

<210>  636
<211>  63
<212>  DNA
<213>  Artificial Sequence

<220>
<223>  primer

<400>  636

gtgactggag ttcagacgtg tgctcttccg atctgaagag gctgtagcac gattgcagca 60
ttg 63

<210> 637
<211> 63
<212> DNA
<213> Artificial Sequence

<220>
<223> primer

<400> 637
gtgactggag ttcagacgtg tgctcttccg atctcggata actgtagcac gattgcagca 60
ttg 63

<210> 638
<211> 63
<212> DNA
<213> Artificial Sequence

<220>
<223> primer

<400> 638
gtgactggag ttcagacgtg tgctcttccg atctgaatct ggtgtagcac gattgcagca 60
ttg 63

<210> 639
<211> 63
<212> DNA
<213> Artificial Sequence

<220>
<223> primer

<400> 639
gtgactggag ttcagacgtg tgctcttccg atctctgatt gatgtagcac gattgcagca 60
ttg 63

<210> 640
<211> 63
<212> DNA
<213> Artificial Sequence

<220>
<223> primer

<400> 640
gtgactggag ttcagacgtg tgctcttccg atctaatccg tttgtagcac gattgcagca 60
ttg 63

<210>  641
<211>  63
<212>  DNA
<213>  Artificial Sequence

<220>
<223>  primer


<400>  641
gtgactggag ttcagacgtg tgctcttccg atcttgcgta catgtagcac gattgcagca      60
ttg                                                                    63




<210>  642
<211>  63
<212>  DNA
<213>  Artificial Sequence

<220>
<223>  primer


<400>  642
gtgactggag ttcagacgtg tgctcttccg atctgaatca attgtagcac gattgcagca      60
ttg                                                                    63




<210>  643
<211>  63
<212>  DNA
<213>  Artificial Sequence

<220>
<223>  primer


<400>  643
gtgactggag ttcagacgtg tgctcttccg atcttgagtc agtgtagcac gattgcagca      60
ttg                                                                    63




<210>  644
<211>  63
<212>  DNA
<213>  Artificial Sequence

<220>
<223>  primer


<400>  644
gtgactggag ttcagacgtg tgctcttccg atctgaatgc tctgtagcac gattgcagca      60
ttg                                                                    63




<210>  645

<211>  63
<212>  DNA
<213>  Artificial Sequence

<220>
<223>  primer


<400>  645
gtgactggag ttcagacgtg tgctcttccg atctgaatat cctgtagcac gattgcagca      60
ttg                                                                    63



<210>  646
<211>  63
<212>  DNA
<213>  Artificial Sequence

<220>
<223>  primer


<400>  646
gtgactggag ttcagacgtg tgctcttccg atctcttatg aatgtagcac gattgcagca      60
ttg                                                                    63



<210>  647
<211>  63
<212>  DNA
<213>  Artificial Sequence

<220>
<223>  primer


<400>  647
gtgactggag ttcagacgtg tgctcttccg atcttcggca cctgtagcac gattgcagca      60
ttg                                                                    63



<210>  648
<211>  63
<212>  DNA
<213>  Artificial Sequence

<220>
<223>  primer


<400>  648
gtgactggag ttcagacgtg tgctcttccg atctaagaag cgtgtagcac gattgcagca      60
ttg                                                                    63



<210>  649
<211>  63
<212>  DNA
<213>  Artificial Sequence

<220>
<223>  primer

<400>  649
gtgactggag ttcagacgtg tgctcttccg atctctcacg attgtagcac gattgcagca      60
ttg                                                                    63

<210>  650
<211>  63
<212>  DNA
<213>  Artificial Sequence

<220>
<223>  primer

<400>  650
gtgactggag ttcagacgtg tgctcttccg atcttcggtc gatgtagcac gattgcagca      60
ttg                                                                    63

<210>  651
<211>  63
<212>  DNA
<213>  Artificial Sequence

<220>
<223>  primer

<400>  651
gtgactggag ttcagacgtg tgctcttccg atcttcggta agtgtagcac gattgcagca      60
ttg                                                                    63

<210>  652
<211>  63
<212>  DNA
<213>  Artificial Sequence

<220>
<223>  primer

<400>  652
gtgactggag ttcagacgtg tgctcttccg atctaagata catgtagcac gattgcagca      60
ttg                                                                    63

<210>  653
<211>  63
<212>  DNA
<213>  Artificial Sequence

<220>
<223>  primer

<400>  653
gtgactggag ttcagacgtg tgctcttccg atctgtcgct gttgtagcac gattgcagca      60
ttg                                                                    63

<210>  654
<211>  63
<212>  DNA
<213>  Artificial Sequence

<220>
<223>  primer

<400>  654
gtgactggag ttcagacgtg tgctcttccg atcttcggat gttgtagcac gattgcagca      60
ttg                                                                    63

<210>  655
<211>  63
<212>  DNA
<213>  Artificial Sequence

<220>
<223>  primer

<400>  655
gtgactggag ttcagacgtg tgctcttccg atctcgagcc ggtgtagcac gattgcagca      60
ttg                                                                    63

<210>  656
<211>  63
<212>  DNA
<213>  Artificial Sequence

<220>
<223>  primer

<400>  656
gtgactggag ttcagacgtg tgctcttccg atctcgatta tctgtagcac gattgcagca      60
ttg                                                                    63

<210>  657
<211>  63
<212>  DNA
<213>  Artificial Sequence

<220>
<223>  primer

<400>  657

gtgactggag ttcagacgtg tgctcttccg atcttcgaag cttgtagcac gattgcagca 60
ttg 63

<210> 658
<211> 63
<212> DNA
<213> Artificial Sequence

<220>
<223> primer

<400> 658
gtgactggag ttcagacgtg tgctcttccg atctctatca tttgtagcac gattgcagca 60
ttg 63

<210> 659
<211> 63
<212> DNA
<213> Artificial Sequence

<220>
<223> primer

<400> 659
gtgactggag ttcagacgtg tgctcttccg atctaagtta tctgtagcac gattgcagca 60
ttg 63

<210> 660
<211> 63
<212> DNA
<213> Artificial Sequence

<220>
<223> primer

<400> 660
gtgactggag ttcagacgtg tgctcttccg atctcgaacg gatgtagcac gattgcagca 60
ttg 63

<210> 661
<211> 63
<212> DNA
<213> Artificial Sequence

<220>
<223> primer

<400> 661
gtgactggag ttcagacgtg tgctcttccg atctctactg actgtagcac gattgcagca 60
ttg 63

<210>  662
<211>  63
<212>  DNA
<213>  Artificial Sequence

<220>
<223>  primer


<400>  662
gtgactggag ttcagacgtg tgctcttccg atcttcttaa gttgtagcac gattgcagca      60
ttg                                                                    63




<210>  663
<211>  63
<212>  DNA
<213>  Artificial Sequence

<220>
<223>  primer


<400>  663
gtgactggag ttcagacgtg tgctcttccg atctttagag tctgtagcac gattgcagca      60
ttg                                                                    63




<210>  664
<211>  63
<212>  DNA
<213>  Artificial Sequence

<220>
<223>  primer


<400>  664
gtgactggag ttcagacgtg tgctcttccg atctaagacg aatgtagcac gattgcagca      60
ttg                                                                    63




<210>  665
<211>  63
<212>  DNA
<213>  Artificial Sequence

<220>
<223>  primer


<400>  665
gtgactggag ttcagacgtg tgctcttccg atctttatta tgtgtagcac gattgcagca      60
ttg                                                                    63




<210>  666

<211>  63
<212>  DNA
<213>  Artificial Sequence

<220>
<223>  primer


<400>  666
gtgactggag ttcagacgtg tgctcttccg atctcgctat tatgtagcac gattgcagca      60
ttg                                                                    63




<210>  667
<211>  63
<212>  DNA
<213>  Artificial Sequence

<220>
<223>  primer


<400>  667
gtgactggag ttcagacgtg tgctcttccg atcttctatc agtgtagcac gattgcagca      60
ttg                                                                    63




<210>  668
<211>  63
<212>  DNA
<213>  Artificial Sequence

<220>
<223>  primer


<400>  668
gtgactggag ttcagacgtg tgctcttccg atctcggtgg tatgtagcac gattgcagca      60
ttg                                                                    63




<210>  669
<211>  63
<212>  DNA
<213>  Artificial Sequence

<220>
<223>  primer


<400>  669
gtgactggag ttcagacgtg tgctcttccg atcttcacca attgtagcac gattgcagca      60
ttg                                                                    63




<210>  670
<211>  63
<212>  DNA
<213>  Artificial Sequence

<220>
<223>  primer

<400>  670
gtgactggag ttcagacgtg tgctcttccg atctctggaa gctgtagcac gattgcagca      60
ttg                                                                    63

<210>  671
<211>  63
<212>  DNA
<213>  Artificial Sequence

<220>
<223>  primer

<400>  671
gtgactggag ttcagacgtg tgctcttccg atctcgtaag agtgtagcac gattgcagca      60
ttg                                                                    63

<210>  672
<211>  63
<212>  DNA
<213>  Artificial Sequence

<220>
<223>  primer

<400>  672
gtgactggag ttcagacgtg tgctcttccg atctaagaga gctgtagcac gattgcagca      60
ttg                                                                    63

<210>  673
<211>  63
<212>  DNA
<213>  Artificial Sequence

<220>
<223>  primer

<400>  673
gtgactggag ttcagacgtg tgctcttccg atcttcaacg agtgtagcac gattgcagca      60
ttg                                                                    63

<210>  674
<211>  63
<212>  DNA
<213>  Artificial Sequence

<220>
<223>  primer

<400>  674
gtgactggag ttcagacgtg tgctcttccg atcttgcgag actgtagcac gattgcagca      60
ttg                                                                    63

<210>  675
<211>  63
<212>  DNA
<213>  Artificial Sequence

<220>
<223>  primer

<400>  675
gtgactggag ttcagacgtg tgctcttccg atctcctggt gttgtagcac gattgcagca      60
ttg                                                                    63

<210>  676
<211>  63
<212>  DNA
<213>  Artificial Sequence

<220>
<223>  primer

<400>  676
gtgactggag ttcagacgtg tgctcttccg atctaagtaa gttgtagcac gattgcagca      60
ttg                                                                    63

<210>  677
<211>  63
<212>  DNA
<213>  Artificial Sequence

<220>
<223>  primer

<400>  677
gtgactggag ttcagacgtg tgctcttccg atcttgactg aatgtagcac gattgcagca      60
ttg                                                                    63

<210>  678
<211>  63
<212>  DNA
<213>  Artificial Sequence

<220>
<223>  primer

<400>  678

gtgactggag ttcagacgtg tgctcttccg atctaagact gttgtagcac gattgcagca 60
ttg 63

<210> 679
<211> 63
<212> DNA
<213> Artificial Sequence

<220>
<223> primer

<400> 679
gtgactggag ttcagacgtg tgctcttccg atctcaatga tgtgtagcac gattgcagca 60
ttg 63

<210> 680
<211> 63
<212> DNA
<213> Artificial Sequence

<220>
<223> primer

<400> 680
gtgactggag ttcagacgtg tgctcttccg atctcacagt aatgtagcac gattgcagca 60
ttg 63

<210> 681
<211> 63
<212> DNA
<213> Artificial Sequence

<220>
<223> primer

<400> 681
gtgactggag ttcagacgtg tgctcttccg atcttggtca tttgtagcac gattgcagca 60
ttg 63

<210> 682
<211> 63
<212> DNA
<213> Artificial Sequence

<220>
<223> primer

<400> 682
gtgactggag ttcagacgtg tgctcttccg atctcaaccg tgtgtagcac gattgcagca 60
ttg 63

<210>  683
<211>  63
<212>  DNA
<213>  Artificial Sequence

<220>
<223>  primer


<400>  683
gtgactggag ttcagacgtg tgctcttccg atcttggtgc actgtagcac gattgcagca      60
ttg                                                                    63




<210>  684
<211>  63
<212>  DNA
<213>  Artificial Sequence

<220>
<223>  primer


<400>  684
gtgactggag ttcagacgtg tgctcttccg atctccacaa tgtgtagcac gattgcagca      60
ttg                                                                    63




<210>  685
<211>  63
<212>  DNA
<213>  Artificial Sequence

<220>
<223>  primer


<400>  685
gtgactggag ttcagacgtg tgctcttccg atcttgtgtg cctgtagcac gattgcagca      60
ttg                                                                    63




<210>  686
<211>  63
<212>  DNA
<213>  Artificial Sequence

<220>
<223>  primer


<400>  686
gtgactggag ttcagacgtg tgctcttccg atctcaccac ggtgtagcac gattgcagca      60
ttg                                                                    63




<210>  687

<211>  63
<212>  DNA
<213>  Artificial Sequence

<220>
<223>  primer


<400>  687
gtgactggag ttcagacgtg tgctcttccg atcttgtgtt aatgtagcac gattgcagca      60
ttg                                                                    63




<210>  688
<211>  64
<212>  DNA
<213>  Artificial Sequence

<220>
<223>  primer


<400>  688
gtgactggag ttcagacgtg tgctcttccg atctagcttg gtggcaaatg ctttcgctct      60
ggtc                                                                   64




<210>  689
<211>  64
<212>  DNA
<213>  Artificial Sequence

<220>
<223>  primer


<400>  689
gtgactggag ttcagacgtg tgctcttccg atctagctta tgggcaaatg ctttcgctct      60
ggtc                                                                   64




<210>  690
<211>  64
<212>  DNA
<213>  Artificial Sequence

<220>
<223>  primer


<400>  690
gtgactggag ttcagacgtg tgctcttccg atctataacg acggcaaatg ctttcgctct      60
ggtc                                                                   64




<210>  691
<211>  64
<212>  DNA
<213>  Artificial Sequence

<220>
<223>  primer

<400>  691
gtgactggag ttcagacgtg tgctcttccg atctgatgga gtggcaaatg ctttcgctct      60
ggtc                                                                    64

<210>  692
<211>  64
<212>  DNA
<213>  Artificial Sequence

<220>
<223>  primer

<400>  692
gtgactggag ttcagacgtg tgctcttccg atcttgatct aaggcaaatg ctttcgctct      60
ggtc                                                                    64

<210>  693
<211>  64
<212>  DNA
<213>  Artificial Sequence

<220>
<223>  primer

<400>  693
gtgactggag ttcagacgtg tgctcttccg atctagctat atggcaaatg ctttcgctct      60
ggtc                                                                    64

<210>  694
<211>  64
<212>  DNA
<213>  Artificial Sequence

<220>
<223>  primer

<400>  694
gtgactggag ttcagacgtg tgctcttccg atctagcaga gaggcaaatg ctttcgctct      60
ggtc                                                                    64

<210>  695
<211>  64
<212>  DNA
<213>  Artificial Sequence

<220>
<223>  primer

<400>  695
gtgactggag ttcagacgtg tgctcttccg atctgacgta agggcaaatg ctttcgctct      60
ggtc                                                                  64

<210>  696
<211>  64
<212>  DNA
<213>  Artificial Sequence

<220>
<223>  primer

<400>  696
gtgactggag ttcagacgtg tgctcttccg atctaggcct gaggcaaatg ctttcgctct      60
ggtc                                                                  64

<210>  697
<211>  64
<212>  DNA
<213>  Artificial Sequence

<220>
<223>  primer

<400>  697
gtgactggag ttcagacgtg tgctcttccg atcttaagat taggcaaatg ctttcgctct      60
ggtc                                                                  64

<210>  698
<211>  64
<212>  DNA
<213>  Artificial Sequence

<220>
<223>  primer

<400>  698
gtgactggag ttcagacgtg tgctcttccg atctaggcga atggcaaatg ctttcgctct      60
ggtc                                                                  64

<210>  699
<211>  64
<212>  DNA
<213>  Artificial Sequence

<220>
<223>  primer

<400>  699

gtgactggag ttcagacgtg tgctcttccg atctttgttc ttggcaaatg ctttcgctct 60
ggtc 64

<210> 700
<211> 64
<212> DNA
<213> Artificial Sequence

<220>
<223> primer

<400> 700
gtgactggag ttcagacgtg tgctcttccg atcttactcc tgggcaaatg ctttcgctct 60
ggtc 64

<210> 701
<211> 64
<212> DNA
<213> Artificial Sequence

<220>
<223> primer

<400> 701
gtgactggag ttcagacgtg tgctcttccg atctgactgg cgggcaaatg ctttcgctct 60
ggtc 64

<210> 702
<211> 64
<212> DNA
<213> Artificial Sequence

<220>
<223> primer

<400> 702
gtgactggag ttcagacgtg tgctcttccg atctatatat acggcaaatg ctttcgctct 60
ggtc 64

<210> 703
<211> 64
<212> DNA
<213> Artificial Sequence

<220>
<223> primer

<400> 703
gtgactggag ttcagacgtg tgctcttccg atcttagatc ggggcaaatg ctttcgctct 60
ggtc 64

<210>  704
<211>  64
<212>  DNA
<213>  Artificial Sequence

<220>
<223>  primer


<400>  704
gtgactggag ttcagacgtg tgctcttccg atctaggcaa gcggcaaatg ctttcgctct      60
ggtc                                                                    64




<210>  705
<211>  64
<212>  DNA
<213>  Artificial Sequence

<220>
<223>  primer


<400>  705
gtgactggag ttcagacgtg tgctcttccg atcttccagg taggcaaatg ctttcgctct      60
ggtc                                                                    64




<210>  706
<211>  64
<212>  DNA
<213>  Artificial Sequence

<220>
<223>  primer


<400>  706
gtgactggag ttcagacgtg tgctcttccg atctaggtca agggcaaatg ctttcgctct      60
ggtc                                                                    64




<210>  707
<211>  64
<212>  DNA
<213>  Artificial Sequence

<220>
<223>  primer


<400>  707
gtgactggag ttcagacgtg tgctcttccg atcttacact ggggcaaatg ctttcgctct      60
ggtc                                                                    64




<210>  708

<211>  64
<212>  DNA
<213>  Artificial Sequence

<220>
<223>  primer


<400>  708
gtgactggag ttcagacgtg tgctcttccg atcttccttg cgggcaaatg ctttcgctct      60
ggtc                                                                  64



<210>  709
<211>  64
<212>  DNA
<213>  Artificial Sequence

<220>
<223>  primer


<400>  709
gtgactggag ttcagacgtg tgctcttccg atctaggacc tcggcaaatg ctttcgctct      60
ggtc                                                                  64



<210>  710
<211>  64
<212>  DNA
<213>  Artificial Sequence

<220>
<223>  primer


<400>  710
gtgactggag ttcagacgtg tgctcttccg atcttaagca ttggcaaatg ctttcgctct      60
ggtc                                                                  64



<210>  711
<211>  64
<212>  DNA
<213>  Artificial Sequence

<220>
<223>  primer


<400>  711
gtgactggag ttcagacgtg tgctcttccg atctaggata ttggcaaatg ctttcgctct      60
ggtc                                                                  64



<210>  712
<211>  64
<212>  DNA
<213>  Artificial Sequence

<220>
<223>  primer

<400>  712
gtgactggag ttcagacgtg tgctcttccg atctaatcgt taggcaaatg ctttcgctct      60
ggtc                                                                    64

<210>  713
<211>  64
<212>  DNA
<213>  Artificial Sequence

<220>
<223>  primer

<400>  713
gtgactggag ttcagacgtg tgctcttccg atctgtctac atggcaaatg ctttcgctct      60
ggtc                                                                    64

<210>  714
<211>  64
<212>  DNA
<213>  Artificial Sequence

<220>
<223>  primer

<400>  714
gtgactggag ttcagacgtg tgctcttccg atctcgctgc tcggcaaatg ctttcgctct      60
ggtc                                                                    64

<210>  715
<211>  64
<212>  DNA
<213>  Artificial Sequence

<220>
<223>  primer

<400>  715
gtgactggag ttcagacgtg tgctcttccg atctgatcaa caggcaaatg ctttcgctct      60
ggtc                                                                    64

<210>  716
<211>  64
<212>  DNA
<213>  Artificial Sequence

<220>
<223>  primer

<400>  716
gtgactggag ttcagacgtg tgctcttccg atctcgaagg acggcaaatg ctttcgctct      60
ggtc                                                                    64

<210>  717
<211>  64
<212>  DNA
<213>  Artificial Sequence

<220>
<223>  primer

<400>  717
gtgactggag ttcagacgtg tgctcttccg atctgatgcc ggggcaaatg ctttcgctct      60
ggtc                                                                    64

<210>  718
<211>  64
<212>  DNA
<213>  Artificial Sequence

<220>
<223>  primer

<400>  718
gtgactggag ttcagacgtg tgctcttccg atctctacga agggcaaatg ctttcgctct      60
ggtc                                                                    64

<210>  719
<211>  64
<212>  DNA
<213>  Artificial Sequence

<220>
<223>  primer

<400>  719
gtgactggag ttcagacgtg tgctcttccg atctgatgcg tcggcaaatg ctttcgctct      60
ggtc                                                                    64

<210>  720
<211>  64
<212>  DNA
<213>  Artificial Sequence

<220>
<223>  primer

<400>  720

gtgactggag ttcagacgtg tgctcttccg atctctacgg caggcaaatg ctttcgctct 60
ggtc 64

<210> 721
<211> 64
<212> DNA
<213> Artificial Sequence

<220>
<223> primer

<400> 721
gtgactggag ttcagacgtg tgctcttccg atctgattcc ttggcaaatg ctttcgctct 60
ggtc 64

<210> 722
<211> 64
<212> DNA
<213> Artificial Sequence

<220>
<223> primer

<400> 722
gtgactggag ttcagacgtg tgctcttccg atctctactc gaggcaaatg ctttcgctct 60
ggtc 64

<210> 723
<211> 64
<212> DNA
<213> Artificial Sequence

<220>
<223> primer

<400> 723
gtgactggag ttcagacgtg tgctcttccg atctgattcg agggcaaatg ctttcgctct 60
ggtc 64

<210> 724
<211> 64
<212> DNA
<213> Artificial Sequence

<220>
<223> primer

<400> 724
gtgactggag ttcagacgtg tgctcttccg atctaatcgg cgggcaaatg ctttcgctct 60
ggtc 64

<210>  725
<211>  64
<212>  DNA
<213>  Artificial Sequence

<220>
<223>  primer


<400>  725
gtgactggag ttcagacgtg tgctcttccg atctttcgcc gaggcaaatg ctttcgctct      60
ggtc                                                                    64




<210>  726
<211>  64
<212>  DNA
<213>  Artificial Sequence

<220>
<223>  primer


<400>  726
gtgactggag ttcagacgtg tgctcttccg atctctggcc tcggcaaatg ctttcgctct      60
ggtc                                                                    64




<210>  727
<211>  64
<212>  DNA
<213>  Artificial Sequence

<220>
<223>  primer


<400>  727
gtgactggag ttcagacgtg tgctcttccg atctgaactt atggcaaatg ctttcgctct      60
ggtc                                                                    64




<210>  728
<211>  64
<212>  DNA
<213>  Artificial Sequence

<220>
<223>  primer


<400>  728
gtgactggag ttcagacgtg tgctcttccg atctcgtatt ggggcaaatg ctttcgctct      60
ggtc                                                                    64




<210>  729

<211>  64
<212>  DNA
<213>  Artificial Sequence

<220>
<223>  primer


<400>  729
gtgactggag ttcagacgtg tgctcttccg atctgaagca caggcaaatg ctttcgctct      60
ggtc                                                                    64




<210>  730
<211>  64
<212>  DNA
<213>  Artificial Sequence

<220>
<223>  primer


<400>  730
gtgactggag ttcagacgtg tgctcttccg atctcttaat acggcaaatg ctttcgctct      60
ggtc                                                                    64




<210>  731
<211>  64
<212>  DNA
<213>  Artificial Sequence

<220>
<223>  primer


<400>  731
gtgactggag ttcagacgtg tgctcttccg atctgaagtc ttggcaaatg ctttcgctct      60
ggtc                                                                    64




<210>  732
<211>  64
<212>  DNA
<213>  Artificial Sequence

<220>
<223>  primer


<400>  732
gtgactggag ttcagacgtg tgctcttccg atctgaagag gcggcaaatg ctttcgctct      60
ggtc                                                                    64




<210>  733
<211>  64
<212>  DNA
<213>  Artificial Sequence

<220>
<223>  primer


<400>  733
gtgactggag ttcagacgtg tgctcttccg atctcggata acggcaaatg ctttcgctct      60
ggtc                                                                    64




<210>  734
<211>  64
<212>  DNA
<213>  Artificial Sequence

<220>
<223>  primer


<400>  734
gtgactggag ttcagacgtg tgctcttccg atctgaatct ggggcaaatg ctttcgctct      60
ggtc                                                                    64




<210>  735
<211>  64
<212>  DNA
<213>  Artificial Sequence

<220>
<223>  primer


<400>  735
gtgactggag ttcagacgtg tgctcttccg atctctgatt gaggcaaatg ctttcgctct      60
ggtc                                                                    64




<210>  736
<211>  64
<212>  DNA
<213>  Artificial Sequence

<220>
<223>  primer


<400>  736
gtgactggag ttcagacgtg tgctcttccg atctaatccg ttggcaaatg ctttcgctct      60
ggtc                                                                    64




<210>  737
<211>  64
<212>  DNA
<213>  Artificial Sequence

<220>
<223>  primer

<400>  737
gtgactggag ttcagacgtg tgctcttccg atcttgcgta caggcaaatg ctttcgctct      60
ggtc                                                                    64

<210>  738
<211>  64
<212>  DNA
<213>  Artificial Sequence

<220>
<223>  primer

<400>  738
gtgactggag ttcagacgtg tgctcttccg atctgaatca atggcaaatg ctttcgctct      60
ggtc                                                                    64

<210>  739
<211>  64
<212>  DNA
<213>  Artificial Sequence

<220>
<223>  primer

<400>  739
gtgactggag ttcagacgtg tgctcttccg atcttgagtc agggcaaatg ctttcgctct      60
ggtc                                                                    64

<210>  740
<211>  64
<212>  DNA
<213>  Artificial Sequence

<220>
<223>  primer

<400>  740
gtgactggag ttcagacgtg tgctcttccg atctgaatgc tcggcaaatg ctttcgctct      60
ggtc                                                                    64

<210>  741
<211>  64
<212>  DNA
<213>  Artificial Sequence

<220>
<223>  primer

<400>  741

gtgactggag ttcagacgtg tgctcttccg atctgaatat ccggcaaatg ctttcgctct 60
ggtc 64

<210> 742
<211> 64
<212> DNA
<213> Artificial Sequence

<220>
<223> primer

<400> 742
gtgactggag ttcagacgtg tgctcttccg atctcttatg aaggcaaatg ctttcgctct 60
ggtc 64

<210> 743
<211> 64
<212> DNA
<213> Artificial Sequence

<220>
<223> primer

<400> 743
gtgactggag ttcagacgtg tgctcttccg atcttcggca ccggcaaatg ctttcgctct 60
ggtc 64

<210> 744
<211> 64
<212> DNA
<213> Artificial Sequence

<220>
<223> primer

<400> 744
gtgactggag ttcagacgtg tgctcttccg atctaagaag cgggcaaatg ctttcgctct 60
ggtc 64

<210> 745
<211> 64
<212> DNA
<213> Artificial Sequence

<220>
<223> primer

<400> 745
gtgactggag ttcagacgtg tgctcttccg atctctcacg atggcaaatg ctttcgctct 60
ggtc 64

<210>  746
<211>  64
<212>  DNA
<213>  Artificial Sequence

<220>
<223>  primer


<400>  746
gtgactggag ttcagacgtg tgctcttccg atcttcggtc gaggcaaatg ctttcgctct      60
ggtc                                                                    64




<210>  747
<211>  64
<212>  DNA
<213>  Artificial Sequence

<220>
<223>  primer


<400>  747
gtgactggag ttcagacgtg tgctcttccg atcttcggta agggcaaatg ctttcgctct      60
ggtc                                                                    64




<210>  748
<211>  64
<212>  DNA
<213>  Artificial Sequence

<220>
<223>  primer


<400>  748
gtgactggag ttcagacgtg tgctcttccg atctaagata caggcaaatg ctttcgctct      60
ggtc                                                                    64




<210>  749
<211>  64
<212>  DNA
<213>  Artificial Sequence

<220>
<223>  primer


<400>  749
gtgactggag ttcagacgtg tgctcttccg atctgtcgct gtggcaaatg ctttcgctct      60
ggtc                                                                    64




<210>  750

<211>  64
<212>  DNA
<213>  Artificial Sequence

<220>
<223>  primer


<400>  750
gtgactggag ttcagacgtg tgctcttccg atcttcggat gtggcaaatg ctttcgctct        60
ggtc                                                                      64




<210>  751
<211>  64
<212>  DNA
<213>  Artificial Sequence

<220>
<223>  primer


<400>  751
gtgactggag ttcagacgtg tgctcttccg atctcgagcc ggggcaaatg ctttcgctct        60
ggtc                                                                      64




<210>  752
<211>  64
<212>  DNA
<213>  Artificial Sequence

<220>
<223>  primer


<400>  752
gtgactggag ttcagacgtg tgctcttccg atctcgatta tcggcaaatg ctttcgctct        60
ggtc                                                                      64




<210>  753
<211>  64
<212>  DNA
<213>  Artificial Sequence

<220>
<223>  primer


<400>  753
gtgactggag ttcagacgtg tgctcttccg atcttcgaag ctggcaaatg ctttcgctct        60
ggtc                                                                      64




<210>  754
<211>  64
<212>  DNA
<213>  Artificial Sequence

<220>
<223>  primer

<400>  754
gtgactggag ttcagacgtg tgctcttccg atctctatca ttggcaaatg ctttcgctct      60
ggtc                                                                    64

<210>  755
<211>  64
<212>  DNA
<213>  Artificial Sequence

<220>
<223>  primer

<400>  755
gtgactggag ttcagacgtg tgctcttccg atctaagtta tcggcaaatg ctttcgctct      60
ggtc                                                                    64

<210>  756
<211>  64
<212>  DNA
<213>  Artificial Sequence

<220>
<223>  primer

<400>  756
gtgactggag ttcagacgtg tgctcttccg atctcgaacg gaggcaaatg ctttcgctct      60
ggtc                                                                    64

<210>  757
<211>  64
<212>  DNA
<213>  Artificial Sequence

<220>
<223>  primer

<400>  757
gtgactggag ttcagacgtg tgctcttccg atctctactg acggcaaatg ctttcgctct      60
ggtc                                                                    64

<210>  758
<211>  64
<212>  DNA
<213>  Artificial Sequence

<220>
<223>  primer

<400>  758
gtgactggag ttcagacgtg tgctcttccg atcttcttaa gtggcaaatg ctttcgctct      60
ggtc                                                                    64

<210>  759
<211>  64
<212>  DNA
<213>  Artificial Sequence

<220>
<223>  primer

<400>  759
gtgactggag ttcagacgtg tgctcttccg atctttagag tcggcaaatg ctttcgctct      60
ggtc                                                                    64

<210>  760
<211>  64
<212>  DNA
<213>  Artificial Sequence

<220>
<223>  primer

<400>  760
gtgactggag ttcagacgtg tgctcttccg atctaagacg aaggcaaatg ctttcgctct      60
ggtc                                                                    64

<210>  761
<211>  64
<212>  DNA
<213>  Artificial Sequence

<220>
<223>  primer

<400>  761
gtgactggag ttcagacgtg tgctcttccg atctttatta tgggcaaatg ctttcgctct      60
ggtc                                                                    64

<210>  762
<211>  64
<212>  DNA
<213>  Artificial Sequence

<220>
<223>  primer

<400>  762

gtgactggag ttcagacgtg tgctcttccg atctcgctat taggcaaatg ctttcgctct 60
ggtc 64

<210> 763
<211> 64
<212> DNA
<213> Artificial Sequence

<220>
<223> primer

<400> 763
gtgactggag ttcagacgtg tgctcttccg atcttctatc agggcaaatg ctttcgctct 60
ggtc 64

<210> 764
<211> 64
<212> DNA
<213> Artificial Sequence

<220>
<223> primer

<400> 764
gtgactggag ttcagacgtg tgctcttccg atctcggtgg taggcaaatg ctttcgctct 60
ggtc 64

<210> 765
<211> 64
<212> DNA
<213> Artificial Sequence

<220>
<223> primer

<400> 765
gtgactggag ttcagacgtg tgctcttccg atcttcacca atggcaaatg ctttcgctct 60
ggtc 64

<210> 766
<211> 64
<212> DNA
<213> Artificial Sequence

<220>
<223> primer

<400> 766
gtgactggag ttcagacgtg tgctcttccg atctctggaa gcggcaaatg ctttcgctct 60
ggtc 64

<210>  767
<211>  64
<212>  DNA
<213>  Artificial Sequence

<220>
<223>  primer


<400>  767
gtgactggag ttcagacgtg tgctcttccg atctcgtaag agggcaaatg ctttcgctct      60
ggtc                                                                    64




<210>  768
<211>  64
<212>  DNA
<213>  Artificial Sequence

<220>
<223>  primer


<400>  768
gtgactggag ttcagacgtg tgctcttccg atctaagaga gcggcaaatg ctttcgctct      60
ggtc                                                                    64




<210>  769
<211>  64
<212>  DNA
<213>  Artificial Sequence

<220>
<223>  primer


<400>  769
gtgactggag ttcagacgtg tgctcttccg atcttcaacg agggcaaatg ctttcgctct      60
ggtc                                                                    64




<210>  770
<211>  64
<212>  DNA
<213>  Artificial Sequence

<220>
<223>  primer


<400>  770
gtgactggag ttcagacgtg tgctcttccg atcttgcgag acggcaaatg ctttcgctct      60
ggtc                                                                    64




<210>  771

<211>  64
<212>  DNA
<213>  Artificial Sequence

<220>
<223>  primer


<400>  771
gtgactggag ttcagacgtg tgctcttccg atctcctggt gtggcaaatg ctttcgctct        60
ggtc                                                                      64




<210>  772
<211>  64
<212>  DNA
<213>  Artificial Sequence

<220>
<223>  primer


<400>  772
gtgactggag ttcagacgtg tgctcttccg atctaagtaa gtggcaaatg ctttcgctct        60
ggtc                                                                      64




<210>  773
<211>  64
<212>  DNA
<213>  Artificial Sequence

<220>
<223>  primer


<400>  773
gtgactggag ttcagacgtg tgctcttccg atcttgactg aaggcaaatg ctttcgctct        60
ggtc                                                                      64




<210>  774
<211>  64
<212>  DNA
<213>  Artificial Sequence

<220>
<223>  primer


<400>  774
gtgactggag ttcagacgtg tgctcttccg atctaagact gtggcaaatg ctttcgctct        60
ggtc                                                                      64




<210>  775
<211>  64
<212>  DNA
<213>  Artificial Sequence

<220>
<223>  primer

<400>  775
gtgactggag ttcagacgtg tgctcttccg atctcaatga tgggcaaatg ctttcgctct      60
ggtc                                                                    64

<210>  776
<211>  64
<212>  DNA
<213>  Artificial Sequence

<220>
<223>  primer

<400>  776
gtgactggag ttcagacgtg tgctcttccg atctcacagt aaggcaaatg ctttcgctct      60
ggtc                                                                    64

<210>  777
<211>  64
<212>  DNA
<213>  Artificial Sequence

<220>
<223>  primer

<400>  777
gtgactggag ttcagacgtg tgctcttccg atcttggtca ttggcaaatg ctttcgctct      60
ggtc                                                                    64

<210>  778
<211>  64
<212>  DNA
<213>  Artificial Sequence

<220>
<223>  primer

<400>  778
gtgactggag ttcagacgtg tgctcttccg atctcaaccg tgggcaaatg ctttcgctct      60
ggtc                                                                    64

<210>  779
<211>  64
<212>  DNA
<213>  Artificial Sequence

<220>
<223>  primer

<400>  779
gtgactggag ttcagacgtg tgctcttccg atcttggtgc acggcaaatg ctttcgctct      60
ggtc                                                                    64

<210>  780
<211>  64
<212>  DNA
<213>  Artificial Sequence

<220>
<223>  primer

<400>  780
gtgactggag ttcagacgtg tgctcttccg atctccacaa tgggcaaatg ctttcgctct      60
ggtc                                                                    64

<210>  781
<211>  64
<212>  DNA
<213>  Artificial Sequence

<220>
<223>  primer

<400>  781
gtgactggag ttcagacgtg tgctcttccg atcttgtgtg ccggcaaatg ctttcgctct      60
ggtc                                                                    64

<210>  782
<211>  64
<212>  DNA
<213>  Artificial Sequence

<220>
<223>  primer

<400>  782
gtgactggag ttcagacgtg tgctcttccg atctcaccac ggggcaaatg ctttcgctct      60
ggtc                                                                    64

<210>  783
<211>  64
<212>  DNA
<213>  Artificial Sequence

<220>
<223>  primer

<400>  783

gtgactggag ttcagacgtg tgctcttccg atcttgtgtt aaggcaaatg ctttcgctct 60
ggtc 64

<210> 784
<211> 65
<212> DNA
<213> Artificial Sequence

<220>
<223> primer

<220>
<221> misc_feature
<222> 51
<223> n is a, c, g, or t

<400> 784
gtgactggag ttcagacgtg tgctcttccg atctagcttg gttggacaaa ncgtctacgc 60
tgcag 65

<210> 785
<211> 65
<212> DNA
<213> Artificial Sequence

<220>
<223> primer

<220>
<221> misc_feature
<222> 51
<223> n is a, c, g, or t

<400> 785
gtgactggag ttcagacgtg tgctcttccg atctagctta tgtggacaaa ncgtctacgc 60
tgcag 65

<210> 786
<211> 65
<212> DNA
<213> Artificial Sequence

<220>
<223> primer

<220>
<221> misc_feature
<222> 51
<223> n is a, c, g, or t

<400> 786
gtgactggag ttcagacgtg tgctcttccg atctataacg actggacaaa ncgtctacgc 60
tgcag 65

<210>  787
<211>  65
<212>  DNA
<213>  Artificial Sequence

<220>
<223>  primer


<220>
<221>  misc_feature
<222>  51
<223>  n is a, c, g, or t

<400>  787
gtgactggag ttcagacgtg tgctcttccg atctgatgga gttggacaaa ncgtctacgc      60
tgcag                                                                  65


<210>  788
<211>  65
<212>  DNA
<213>  Artificial Sequence

<220>
<223>  primer


<220>
<221>  misc_feature
<222>  51
<223>  n is a, c, g, or t

<400>  788
gtgactggag ttcagacgtg tgctcttccg atcttgatct aatggacaaa ncgtctacgc      60
tgcag                                                                  65


<210>  789
<211>  65
<212>  DNA
<213>  Artificial Sequence

<220>
<223>  primer


<220>
<221>  misc_feature
<222>  51
<223>  n is a, c, g, or t

<400>  789
gtgactggag ttcagacgtg tgctcttccg atctagctat attggacaaa ncgtctacgc      60
tgcag                                                                  65


<210>  790

<211>  65
<212>  DNA
<213>  Artificial Sequence

<220>
<223>  primer


<220>
<221>  misc_feature
<222>  51
<223>  n is a, c, g, or t

<400>  790
gtgactggag ttcagacgtg tgctcttccg atctagcaga gatggacaaa ncgtctacgc        60
tgcag                                                                    65



<210>  791
<211>  65
<212>  DNA
<213>  Artificial Sequence

<220>
<223>  primer


<220>
<221>  misc_feature
<222>  51
<223>  n is a, c, g, or t

<400>  791
gtgactggag ttcagacgtg tgctcttccg atctgacgta agtggacaaa ncgtctacgc        60
tgcag                                                                    65



<210>  792
<211>  65
<212>  DNA
<213>  Artificial Sequence

<220>
<223>  primer


<220>
<221>  misc_feature
<222>  51
<223>  n is a, c, g, or t

<400>  792
gtgactggag ttcagacgtg tgctcttccg atctaggcct gatggacaaa ncgtctacgc        60
tgcag                                                                    65



<210>  793
<211>  65
<212>  DNA
<213>  Artificial Sequence

<220>
<223>  primer


<220>
<221>  misc_feature
<222>  51
<223>  n is a, c, g, or t

<400>  793
gtgactggag ttcagacgtg tgctcttccg atcttaagat tatggacaaa ncgtctacgc      60
tgcag                                                                  65



<210>  794
<211>  65
<212>  DNA
<213>  Artificial Sequence

<220>
<223>  primer


<220>
<221>  misc_feature
<222>  51
<223>  n is a, c, g, or t

<400>  794
gtgactggag ttcagacgtg tgctcttccg atctaggcga attggacaaa ncgtctacgc      60
tgcag                                                                  65



<210>  795
<211>  65
<212>  DNA
<213>  Artificial Sequence

<220>
<223>  primer


<220>
<221>  misc_feature
<222>  51
<223>  n is a, c, g, or t

<400>  795
gtgactggag ttcagacgtg tgctcttccg atctttgttc tttggacaaa ncgtctacgc      60
tgcag                                                                  65



<210>  796
<211>  65
<212>  DNA
<213>  Artificial Sequence

<220>
<223>  primer

<220>
<221>  misc_feature
<222>  51
<223>  n is a, c, g, or t

<400>  796
gtgactggag ttcagacgtg tgctcttccg atcttactcc tgtggacaaa ncgtctacgc      60
tgcag                                                                  65



<210>  797
<211>  65
<212>  DNA
<213>  Artificial Sequence

<220>
<223>  primer


<220>
<221>  misc_feature
<222>  51
<223>  n is a, c, g, or t

<400>  797
gtgactggag ttcagacgtg tgctcttccg atctgactgg cgtggacaaa ncgtctacgc      60
tgcag                                                                  65



<210>  798
<211>  65
<212>  DNA
<213>  Artificial Sequence

<220>
<223>  primer


<220>
<221>  misc_feature
<222>  51
<223>  n is a, c, g, or t

<400>  798
gtgactggag ttcagacgtg tgctcttccg atctatatat actggacaaa ncgtctacgc      60
tgcag                                                                  65



<210>  799
<211>  65
<212>  DNA
<213>  Artificial Sequence

<220>
<223>  primer


<220>

<221>  misc_feature
<222>  51
<223>  n is a, c, g, or t

<400>  799
gtgactggag ttcagacgtg tgctcttccg atcttagatc ggtggacaaa ncgtctacgc      60
tgcag                                                                  65

<210>  800
<211>  65
<212>  DNA
<213>  Artificial Sequence

<220>
<223>  primer

<220>
<221>  misc_feature
<222>  51
<223>  n is a, c, g, or t

<400>  800
gtgactggag ttcagacgtg tgctcttccg atctaggcaa gctggacaaa ncgtctacgc      60
tgcag                                                                  65

<210>  801
<211>  65
<212>  DNA
<213>  Artificial Sequence

<220>
<223>  primer

<220>
<221>  misc_feature
<222>  51
<223>  n is a, c, g, or t

<400>  801
gtgactggag ttcagacgtg tgctcttccg atcttccagg tatggacaaa ncgtctacgc      60
tgcag                                                                  65

<210>  802
<211>  65
<212>  DNA
<213>  Artificial Sequence

<220>
<223>  primer

<220>
<221>  misc_feature
<222>  51
<223>  n is a, c, g, or t

<400>  802
gtgactggag ttcagacgtg tgctcttccg atctaggtca agtggacaaa ncgtctacgc      60
tgcag                                                                  65

<210>  803
<211>  65
<212>  DNA
<213>  Artificial Sequence

<220>
<223>  primer

<220>
<221>  misc_feature
<222>  51
<223>  n is a, c, g, or t

<400>  803
gtgactggag ttcagacgtg tgctcttccg atcttacact ggtggacaaa ncgtctacgc      60
tgcag                                                                  65

<210>  804
<211>  65
<212>  DNA
<213>  Artificial Sequence

<220>
<223>  primer

<220>
<221>  misc_feature
<222>  51
<223>  n is a, c, g, or t

<400>  804
gtgactggag ttcagacgtg tgctcttccg atcttccttg cgtggacaaa ncgtctacgc      60
tgcag                                                                  65

<210>  805
<211>  65
<212>  DNA
<213>  Artificial Sequence

<220>
<223>  primer

<220>
<221>  misc_feature
<222>  51
<223>  n is a, c, g, or t

<400>  805
gtgactggag ttcagacgtg tgctcttccg atctaggacc tctggacaaa ncgtctacgc      60

tgcag                                                                 65

<210>  806
<211>  65
<212>  DNA
<213>  Artificial Sequence

<220>
<223>  primer

<220>
<221>  misc_feature
<222>  51
<223>  n is a, c, g, or t

<400>  806
gtgactggag ttcagacgtg tgctcttccg atcttaagca tttggacaaa ncgtctacgc      60
tgcag                                                                 65

<210>  807
<211>  65
<212>  DNA
<213>  Artificial Sequence

<220>
<223>  primer

<220>
<221>  misc_feature
<222>  51
<223>  n is a, c, g, or t

<400>  807
gtgactggag ttcagacgtg tgctcttccg atctaggata tttggacaaa ncgtctacgc      60
tgcag                                                                 65

<210>  808
<211>  65
<212>  DNA
<213>  Artificial Sequence

<220>
<223>  primer

<220>
<221>  misc_feature
<222>  51
<223>  n is a, c, g, or t

<400>  808
gtgactggag ttcagacgtg tgctcttccg atctaatcgt tatggacaaa ncgtctacgc      60
tgcag                                                                 65

<210>  809
<211>  65
<212>  DNA
<213>  Artificial Sequence

<220>
<223>  primer


<220>
<221>  misc_feature
<222>  51
<223>  n is a, c, g, or t

<400>  809
gtgactggag ttcagacgtg tgctcttccg atctgtctac attggacaaa ncgtctacgc      60
tgcag                                                                  65


<210>  810
<211>  65
<212>  DNA
<213>  Artificial Sequence

<220>
<223>  primer


<220>
<221>  misc_feature
<222>  51
<223>  n is a, c, g, or t

<400>  810
gtgactggag ttcagacgtg tgctcttccg atctcgctgc tctggacaaa ncgtctacgc      60
tgcag                                                                  65


<210>  811
<211>  65
<212>  DNA
<213>  Artificial Sequence

<220>
<223>  primer


<220>
<221>  misc_feature
<222>  51
<223>  n is a, c, g, or t

<400>  811
gtgactggag ttcagacgtg tgctcttccg atctgatcaa catggacaaa ncgtctacgc      60
tgcag                                                                  65


<210>  812
<211>  65

<212>  DNA
<213>  Artificial Sequence

<220>
<223>  primer


<220>
<221>  misc_feature
<222>  51
<223>  n is a, c, g, or t

<400>  812
gtgactggag ttcagacgtg tgctcttccg atctcgaagg actggacaaa ncgtctacgc      60
tgcag                                                                  65



<210>  813
<211>  65
<212>  DNA
<213>  Artificial Sequence

<220>
<223>  primer


<220>
<221>  misc_feature
<222>  51
<223>  n is a, c, g, or t

<400>  813
gtgactggag ttcagacgtg tgctcttccg atctgatgcc ggtggacaaa ncgtctacgc      60
tgcag                                                                  65



<210>  814
<211>  65
<212>  DNA
<213>  Artificial Sequence

<220>
<223>  primer


<220>
<221>  misc_feature
<222>  51
<223>  n is a, c, g, or t

<400>  814
gtgactggag ttcagacgtg tgctcttccg atctctacga agtggacaaa ncgtctacgc      60
tgcag                                                                  65



<210>  815
<211>  65
<212>  DNA
<213>  Artificial Sequence

<220>
<223>  primer


<220>
<221>  misc_feature
<222>  51
<223>  n is a, c, g, or t

<400>  815
gtgactggag ttcagacgtg tgctcttccg atctgatgcg tctggacaaa ncgtctacgc      60
tgcag                                                                  65



<210>  816
<211>  65
<212>  DNA
<213>  Artificial Sequence

<220>
<223>  primer


<220>
<221>  misc_feature
<222>  51
<223>  n is a, c, g, or t

<400>  816
gtgactggag ttcagacgtg tgctcttccg atctctacgg catggacaaa ncgtctacgc      60
tgcag                                                                  65



<210>  817
<211>  65
<212>  DNA
<213>  Artificial Sequence

<220>
<223>  primer


<220>
<221>  misc_feature
<222>  51
<223>  n is a, c, g, or t

<400>  817
gtgactggag ttcagacgtg tgctcttccg atctgattcc tttggacaaa ncgtctacgc      60
tgcag                                                                  65



<210>  818
<211>  65
<212>  DNA
<213>  Artificial Sequence

<220>
<223>  primer

<220>
<221>  misc_feature
<222>  51
<223>  n is a, c, g, or t

<400>  818
gtgactggag ttcagacgtg tgctcttccg atctctactc gatggacaaa ncgtctacgc      60
tgcag                                                                  65

<210>  819
<211>  65
<212>  DNA
<213>  Artificial Sequence

<220>
<223>  primer

<220>
<221>  misc_feature
<222>  51
<223>  n is a, c, g, or t

<400>  819
gtgactggag ttcagacgtg tgctcttccg atctgattcg agtggacaaa ncgtctacgc      60
tgcag                                                                  65

<210>  820
<211>  65
<212>  DNA
<213>  Artificial Sequence

<220>
<223>  primer

<220>
<221>  misc_feature
<222>  51
<223>  n is a, c, g, or t

<400>  820
gtgactggag ttcagacgtg tgctcttccg atctaatcgg cgtggacaaa ncgtctacgc      60
tgcag                                                                  65

<210>  821
<211>  65
<212>  DNA
<213>  Artificial Sequence

<220>
<223>  primer

<220>
<221>  misc_feature

<222>  51
<223>  n is a, c, g, or t

<400>  821
gtgactggag ttcagacgtg tgctcttccg atctttcgcc gatggacaaa ncgtctacgc      60
tgcag                                                                  65

<210>  822
<211>  65
<212>  DNA
<213>  Artificial Sequence

<220>
<223>  primer

<220>
<221>  misc_feature
<222>  51
<223>  n is a, c, g, or t

<400>  822
gtgactggag ttcagacgtg tgctcttccg atctctggcc tctggacaaa ncgtctacgc      60
tgcag                                                                  65

<210>  823
<211>  65
<212>  DNA
<213>  Artificial Sequence

<220>
<223>  primer

<220>
<221>  misc_feature
<222>  51
<223>  n is a, c, g, or t

<400>  823
gtgactggag ttcagacgtg tgctcttccg atctgaactt attggacaaa ncgtctacgc      60
tgcag                                                                  65

<210>  824
<211>  65
<212>  DNA
<213>  Artificial Sequence

<220>
<223>  primer

<220>
<221>  misc_feature
<222>  51
<223>  n is a, c, g, or t

<400>  824
gtgactggag ttcagacgtg tgctcttccg atctcgtatt ggtggacaaa ncgtctacgc      60
tgcag                                                                  65

<210>  825
<211>  65
<212>  DNA
<213>  Artificial Sequence

<220>
<223>  primer

<220>
<221>  misc_feature
<222>  51
<223>  n is a, c, g, or t

<400>  825
gtgactggag ttcagacgtg tgctcttccg atctgaagca catggacaaa ncgtctacgc      60
tgcag                                                                  65

<210>  826
<211>  65
<212>  DNA
<213>  Artificial Sequence

<220>
<223>  primer

<220>
<221>  misc_feature
<222>  51
<223>  n is a, c, g, or t

<400>  826
gtgactggag ttcagacgtg tgctcttccg atctcttaat actggacaaa ncgtctacgc      60
tgcag                                                                  65

<210>  827
<211>  65
<212>  DNA
<213>  Artificial Sequence

<220>
<223>  primer

<220>
<221>  misc_feature
<222>  51
<223>  n is a, c, g, or t

<400>  827
gtgactggag ttcagacgtg tgctcttccg atctgaagtc tttggacaaa ncgtctacgc      60
tgcag                                                                  65

<210>  828
<211>  65
<212>  DNA
<213>  Artificial Sequence

<220>
<223>  primer


<220>
<221>  misc_feature
<222>  51
<223>  n is a, c, g, or t

<400>  828
gtgactggag ttcagacgtg tgctcttccg atctgaagag gctggacaaa ncgtctacgc        60
tgcag                                                                    65



<210>  829
<211>  65
<212>  DNA
<213>  Artificial Sequence

<220>
<223>  primer


<220>
<221>  misc_feature
<222>  51
<223>  n is a, c, g, or t

<400>  829
gtgactggag ttcagacgtg tgctcttccg atctcggata actggacaaa ncgtctacgc        60
tgcag                                                                    65



<210>  830
<211>  65
<212>  DNA
<213>  Artificial Sequence

<220>
<223>  primer


<220>
<221>  misc_feature
<222>  51
<223>  n is a, c, g, or t

<400>  830
gtgactggag ttcagacgtg tgctcttccg atctgaatct ggtggacaaa ncgtctacgc        60
tgcag                                                                    65

<210>  831
<211>  65
<212>  DNA
<213>  Artificial Sequence

<220>
<223>  primer


<220>
<221>  misc_feature
<222>  51
<223>  n is a, c, g, or t

<400>  831
gtgactggag ttcagacgtg tgctcttccg atctctgatt gatggacaaa ncgtctacgc      60
tgcag                                                                  65



<210>  832
<211>  65
<212>  DNA
<213>  Artificial Sequence

<220>
<223>  primer


<220>
<221>  misc_feature
<222>  51
<223>  n is a, c, g, or t

<400>  832
gtgactggag ttcagacgtg tgctcttccg atctaatccg tttggacaaa ncgtctacgc      60
tgcag                                                                  65



<210>  833
<211>  65
<212>  DNA
<213>  Artificial Sequence

<220>
<223>  primer


<220>
<221>  misc_feature
<222>  51
<223>  n is a, c, g, or t

<400>  833
gtgactggag ttcagacgtg tgctcttccg atcttgcgta catggacaaa ncgtctacgc      60
tgcag                                                                  65



<210>  834
<211>  65
<212>  DNA

<213>  Artificial Sequence

<220>
<223>  primer


<220>
<221>  misc_feature
<222>  51
<223>  n is a, c, g, or t

<400>  834
gtgactggag ttcagacgtg tgctcttccg atctgaatca attggacaaa ncgtctacgc      60
tgcag                                                                  65



<210>  835
<211>  65
<212>  DNA
<213>  Artificial Sequence

<220>
<223>  primer


<220>
<221>  misc_feature
<222>  51
<223>  n is a, c, g, or t

<400>  835
gtgactggag ttcagacgtg tgctcttccg atcttgagtc agtggacaaa ncgtctacgc      60
tgcag                                                                  65



<210>  836
<211>  65
<212>  DNA
<213>  Artificial Sequence

<220>
<223>  primer


<220>
<221>  misc_feature
<222>  51
<223>  n is a, c, g, or t

<400>  836
gtgactggag ttcagacgtg tgctcttccg atctgaatgc tctggacaaa ncgtctacgc      60
tgcag                                                                  65



<210>  837
<211>  65
<212>  DNA
<213>  Artificial Sequence

<220>

<223>  primer

<220>
<221>  misc_feature
<222>  51
<223>  n is a, c, g, or t

<400>  837
gtgactggag ttcagacgtg tgctcttccg atctgaatat cctggacaaa ncgtctacgc      60
tgcag                                                                  65

<210>  838
<211>  65
<212>  DNA
<213>  Artificial Sequence

<220>
<223>  primer

<220>
<221>  misc_feature
<222>  51
<223>  n is a, c, g, or t

<400>  838
gtgactggag ttcagacgtg tgctcttccg atctcttatg aatggacaaa ncgtctacgc      60
tgcag                                                                  65

<210>  839
<211>  65
<212>  DNA
<213>  Artificial Sequence

<220>
<223>  primer

<220>
<221>  misc_feature
<222>  51
<223>  n is a, c, g, or t

<400>  839
gtgactggag ttcagacgtg tgctcttccg atcttcggca cctggacaaa ncgtctacgc      60
tgcag                                                                  65

<210>  840
<211>  65
<212>  DNA
<213>  Artificial Sequence

<220>
<223>  primer

<220>
<221>  misc_feature
<222>  51
<223>  n is a, c, g, or t

<400>  840
gtgactggag ttcagacgtg tgctcttccg atctaagaag cgtggacaaa ncgtctacgc      60
tgcag                                                                  65



<210>  841
<211>  65
<212>  DNA
<213>  Artificial Sequence

<220>
<223>  primer


<220>
<221>  misc_feature
<222>  51
<223>  n is a, c, g, or t

<400>  841
gtgactggag ttcagacgtg tgctcttccg atctctcacg attggacaaa ncgtctacgc      60
tgcag                                                                  65



<210>  842
<211>  65
<212>  DNA
<213>  Artificial Sequence

<220>
<223>  primer


<220>
<221>  misc_feature
<222>  51
<223>  n is a, c, g, or t

<400>  842
gtgactggag ttcagacgtg tgctcttccg atcttcggtc gatggacaaa ncgtctacgc      60
tgcag                                                                  65



<210>  843
<211>  65
<212>  DNA
<213>  Artificial Sequence

<220>
<223>  primer


<220>
<221>  misc_feature
<222>  51

<223>  n is a, c, g, or t

<400>  843
gtgactggag ttcagacgtg tgctcttccg atcttcggta agtggacaaa ncgtctacgc      60
tgcag                                                                  65

<210>  844
<211>  65
<212>  DNA
<213>  Artificial Sequence

<220>
<223>  primer

<220>
<221>  misc_feature
<222>  51
<223>  n is a, c, g, or t

<400>  844
gtgactggag ttcagacgtg tgctcttccg atctaagata catggacaaa ncgtctacgc      60
tgcag                                                                  65

<210>  845
<211>  65
<212>  DNA
<213>  Artificial Sequence

<220>
<223>  primer

<220>
<221>  misc_feature
<222>  51
<223>  n is a, c, g, or t

<400>  845
gtgactggag ttcagacgtg tgctcttccg atctgtcgct gttggacaaa ncgtctacgc      60
tgcag                                                                  65

<210>  846
<211>  65
<212>  DNA
<213>  Artificial Sequence

<220>
<223>  primer

<220>
<221>  misc_feature
<222>  51
<223>  n is a, c, g, or t

<400>  846

gtgactggag ttcagacgtg tgctcttccg atcttcggat gttggacaaa ncgtctacgc 60
tgcag 65

<210> 847
<211> 65
<212> DNA
<213> Artificial Sequence

<220>
<223> primer

<220>
<221> misc_feature
<222> 51
<223> n is a, c, g, or t

<400> 847
gtgactggag ttcagacgtg tgctcttccg atctcgagcc ggtggacaaa ncgtctacgc 60
tgcag 65

<210> 848
<211> 65
<212> DNA
<213> Artificial Sequence

<220>
<223> primer

<220>
<221> misc_feature
<222> 51
<223> n is a, c, g, or t

<400> 848
gtgactggag ttcagacgtg tgctcttccg atctcgatta tctggacaaa ncgtctacgc 60
tgcag 65

<210> 849
<211> 65
<212> DNA
<213> Artificial Sequence

<220>
<223> primer

<220>
<221> misc_feature
<222> 51
<223> n is a, c, g, or t

<400> 849
gtgactggag ttcagacgtg tgctcttccg atcttcgaag cttggacaaa ncgtctacgc 60
tgcag 65

<210>  850
<211>  65
<212>  DNA
<213>  Artificial Sequence

<220>
<223>  primer


<220>
<221>  misc_feature
<222>  51
<223>  n is a, c, g, or t

<400>  850
gtgactggag ttcagacgtg tgctcttccg atctctatca tttggacaaa ncgtctacgc      60
tgcag                                                                  65


<210>  851
<211>  65
<212>  DNA
<213>  Artificial Sequence

<220>
<223>  primer


<220>
<221>  misc_feature
<222>  51
<223>  n is a, c, g, or t

<400>  851
gtgactggag ttcagacgtg tgctcttccg atctaagtta tctggacaaa ncgtctacgc      60
tgcag                                                                  65


<210>  852
<211>  65
<212>  DNA
<213>  Artificial Sequence

<220>
<223>  primer


<220>
<221>  misc_feature
<222>  51
<223>  n is a, c, g, or t

<400>  852
gtgactggag ttcagacgtg tgctcttccg atctcgaacg gatggacaaa ncgtctacgc      60
tgcag                                                                  65


<210>  853

<211>  65
<212>  DNA
<213>  Artificial Sequence

<220>
<223>  primer


<220>
<221>  misc_feature
<222>  51
<223>  n is a, c, g, or t

<400>  853
gtgactggag ttcagacgtg tgctcttccg atctctactg actggacaaa ncgtctacgc        60
tgcag                                                                    65



<210>  854
<211>  65
<212>  DNA
<213>  Artificial Sequence

<220>
<223>  primer


<220>
<221>  misc_feature
<222>  51
<223>  n is a, c, g, or t

<400>  854
gtgactggag ttcagacgtg tgctcttccg atcttcttaa gttggacaaa ncgtctacgc        60
tgcag                                                                    65



<210>  855
<211>  65
<212>  DNA
<213>  Artificial Sequence

<220>
<223>  primer


<220>
<221>  misc_feature
<222>  51
<223>  n is a, c, g, or t

<400>  855
gtgactggag ttcagacgtg tgctcttccg atctttagag tctggacaaa ncgtctacgc        60
tgcag                                                                    65



<210>  856
<211>  65
<212>  DNA
<213>  Artificial Sequence

<220>
<223>  primer


<220>
<221>  misc_feature
<222>  51
<223>  n is a, c, g, or t

<400>  856
gtgactggag ttcagacgtg tgctcttccg atctaagacg aatggacaaa ncgtctacgc        60
tgcag                                                                    65



<210>  857
<211>  65
<212>  DNA
<213>  Artificial Sequence

<220>
<223>  primer


<220>
<221>  misc_feature
<222>  51
<223>  n is a, c, g, or t

<400>  857
gtgactggag ttcagacgtg tgctcttccg atctttatta tgtggacaaa ncgtctacgc        60
tgcag                                                                    65



<210>  858
<211>  65
<212>  DNA
<213>  Artificial Sequence

<220>
<223>  primer


<220>
<221>  misc_feature
<222>  51
<223>  n is a, c, g, or t

<400>  858
gtgactggag ttcagacgtg tgctcttccg atctcgctat tatggacaaa ncgtctacgc        60
tgcag                                                                    65



<210>  859
<211>  65
<212>  DNA
<213>  Artificial Sequence

<220>
<223>  primer

<220>
<221>  misc_feature
<222>  51
<223>  n is a, c, g, or t

<400>  859
gtgactggag ttcagacgtg tgctcttccg atcttctatc agtggacaaa ncgtctacgc      60
tgcag                                                                  65

<210>  860
<211>  65
<212>  DNA
<213>  Artificial Sequence

<220>
<223>  primer

<220>
<221>  misc_feature
<222>  51
<223>  n is a, c, g, or t

<400>  860
gtgactggag ttcagacgtg tgctcttccg atctcggtgg tatggacaaa ncgtctacgc      60
tgcag                                                                  65

<210>  861
<211>  65
<212>  DNA
<213>  Artificial Sequence

<220>
<223>  primer

<220>
<221>  misc_feature
<222>  51
<223>  n is a, c, g, or t

<400>  861
gtgactggag ttcagacgtg tgctcttccg atcttcacca attggacaaa ncgtctacgc      60
tgcag                                                                  65

<210>  862
<211>  65
<212>  DNA
<213>  Artificial Sequence

<220>
<223>  primer

<220>

<221>  misc_feature
<222>  51
<223>  n is a, c, g, or t

<400>  862
gtgactggag ttcagacgtg tgctcttccg atctctggaa gctggacaaa ncgtctacgc      60
tgcag                                                                  65

<210>  863
<211>  65
<212>  DNA
<213>  Artificial Sequence

<220>
<223>  primer

<220>
<221>  misc_feature
<222>  51
<223>  n is a, c, g, or t

<400>  863
gtgactggag ttcagacgtg tgctcttccg atctcgtaag agtggacaaa ncgtctacgc      60
tgcag                                                                  65

<210>  864
<211>  65
<212>  DNA
<213>  Artificial Sequence

<220>
<223>  primer

<220>
<221>  misc_feature
<222>  51
<223>  n is a, c, g, or t

<400>  864
gtgactggag ttcagacgtg tgctcttccg atctaagaga gctggacaaa ncgtctacgc      60
tgcag                                                                  65

<210>  865
<211>  65
<212>  DNA
<213>  Artificial Sequence

<220>
<223>  primer

<220>
<221>  misc_feature
<222>  51
<223>  n is a, c, g, or t

<400>  865
gtgactggag ttcagacgtg tgctcttccg atcttcaacg agtggacaaa ncgtctacgc        60
tgcag                                                                    65

<210>  866
<211>  65
<212>  DNA
<213>  Artificial Sequence

<220>
<223>  primer

<220>
<221>  misc_feature
<222>  51
<223>  n is a, c, g, or t

<400>  866
gtgactggag ttcagacgtg tgctcttccg atcttgcgag actggacaaa ncgtctacgc        60
tgcag                                                                    65

<210>  867
<211>  65
<212>  DNA
<213>  Artificial Sequence

<220>
<223>  primer

<220>
<221>  misc_feature
<222>  51
<223>  n is a, c, g, or t

<400>  867
gtgactggag ttcagacgtg tgctcttccg atctcctggt gttggacaaa ncgtctacgc        60
tgcag                                                                    65

<210>  868
<211>  65
<212>  DNA
<213>  Artificial Sequence

<220>
<223>  primer

<220>
<221>  misc_feature
<222>  51
<223>  n is a, c, g, or t

<400>  868
gtgactggag ttcagacgtg tgctcttccg atctaagtaa gttggacaaa ncgtctacgc        60

tgcag                                                                 65

<210>  869
<211>  65
<212>  DNA
<213>  Artificial Sequence

<220>
<223>  primer

<220>
<221>  misc_feature
<222>  51
<223>  n is a, c, g, or t

<400>  869
gtgactggag ttcagacgtg tgctcttccg atcttgactg aatggacaaa ncgtctacgc     60
tgcag                                                                 65

<210>  870
<211>  65
<212>  DNA
<213>  Artificial Sequence

<220>
<223>  primer

<220>
<221>  misc_feature
<222>  51
<223>  n is a, c, g, or t

<400>  870
gtgactggag ttcagacgtg tgctcttccg atctaagact gttggacaaa ncgtctacgc     60
tgcag                                                                 65

<210>  871
<211>  65
<212>  DNA
<213>  Artificial Sequence

<220>
<223>  primer

<220>
<221>  misc_feature
<222>  51
<223>  n is a, c, g, or t

<400>  871
gtgactggag ttcagacgtg tgctcttccg atctcaatga tgtggacaaa ncgtctacgc     60
tgcag                                                                 65

<210>  872
<211>  65
<212>  DNA
<213>  Artificial Sequence

<220>
<223>  primer


<220>
<221>  misc_feature
<222>  51
<223>  n is a, c, g, or t

<400>  872
gtgactggag ttcagacgtg tgctcttccg atctcacagt aatggacaaa ncgtctacgc      60
tgcag                                                                  65


<210>  873
<211>  65
<212>  DNA
<213>  Artificial Sequence

<220>
<223>  primer


<220>
<221>  misc_feature
<222>  51
<223>  n is a, c, g, or t

<400>  873
gtgactggag ttcagacgtg tgctcttccg atcttggtca tttggacaaa ncgtctacgc      60
tgcag                                                                  65


<210>  874
<211>  65
<212>  DNA
<213>  Artificial Sequence

<220>
<223>  primer


<220>
<221>  misc_feature
<222>  51
<223>  n is a, c, g, or t

<400>  874
gtgactggag ttcagacgtg tgctcttccg atctcaaccg tgtggacaaa ncgtctacgc      60
tgcag                                                                  65


<210>  875
<211>  65

<212>  DNA
<213>  Artificial Sequence

<220>
<223>  primer


<220>
<221>  misc_feature
<222>  51
<223>  n is a, c, g, or t

<400>  875
gtgactggag ttcagacgtg tgctcttccg atcttggtgc actggacaaa ncgtctacgc      60
tgcag                                                                  65



<210>  876
<211>  65
<212>  DNA
<213>  Artificial Sequence

<220>
<223>  primer


<220>
<221>  misc_feature
<222>  51
<223>  n is a, c, g, or t

<400>  876
gtgactggag ttcagacgtg tgctcttccg atctccacaa tgtggacaaa ncgtctacgc      60
tgcag                                                                  65



<210>  877
<211>  65
<212>  DNA
<213>  Artificial Sequence

<220>
<223>  primer


<220>
<221>  misc_feature
<222>  51
<223>  n is a, c, g, or t

<400>  877
gtgactggag ttcagacgtg tgctcttccg atcttgtgtg cctggacaaa ncgtctacgc      60
tgcag                                                                  65



<210>  878
<211>  65
<212>  DNA
<213>  Artificial Sequence

<220>
<223>  primer

<220>
<221>  misc_feature
<222>  51
<223>  n is a, c, g, or t

<400>  878
gtgactggag ttcagacgtg tgctcttccg atctcaccac ggtggacaaa ncgtctacgc      60
tgcag                                                                  65

<210>  879
<211>  65
<212>  DNA
<213>  Artificial Sequence

<220>
<223>  primer

<220>
<221>  misc_feature
<222>  51
<223>  n is a, c, g, or t

<400>  879
gtgactggag ttcagacgtg tgctcttccg atcttgtgtt aatggacaaa ncgtctacgc      60
tgcag                                                                  65

<210>  880
<211>  60
<212>  DNA
<213>  Artificial Sequence

<220>
<223>  primer

<400>  880
gtgactggag ttcagacgtg tgctcttccg atctagcttg gtgtcgctgt ttggagacac      60

<210>  881
<211>  60
<212>  DNA
<213>  Artificial Sequence

<220>
<223>  primer

<400>  881
gtgactggag ttcagacgtg tgctcttccg atctagctta tggtcgctgt ttggagacac      60

<210>  882
<211>  60
<212>  DNA
<213>  Artificial Sequence

<220>
<223>  primer


<400>  882
gtgactggag ttcagacgtg tgctcttccg atctataacg acgtcgctgt ttggagacac        60



<210>  883
<211>  60
<212>  DNA
<213>  Artificial Sequence

<220>
<223>  primer


<400>  883
gtgactggag ttcagacgtg tgctcttccg atctgatgga gtgtcgctgt ttggagacac        60



<210>  884
<211>  60
<212>  DNA
<213>  Artificial Sequence

<220>
<223>  primer


<400>  884
gtgactggag ttcagacgtg tgctcttccg atcttgatct aagtcgctgt ttggagacac        60



<210>  885
<211>  60
<212>  DNA
<213>  Artificial Sequence

<220>
<223>  primer


<400>  885
gtgactggag ttcagacgtg tgctcttccg atctagctat atgtcgctgt ttggagacac        60



<210>  886
<211>  60
<212>  DNA
<213>  Artificial Sequence

<220>
<223>  primer

<400>  886
gtgactggag ttcagacgtg tgctcttccg atctagcaga gagtcgctgt ttggagacac        60

<210>  887
<211>  60
<212>  DNA
<213>  Artificial Sequence

<220>
<223>  primer

<400>  887
gtgactggag ttcagacgtg tgctcttccg atctgacgta aggtcgctgt ttggagacac        60

<210>  888
<211>  60
<212>  DNA
<213>  Artificial Sequence

<220>
<223>  primer

<400>  888
gtgactggag ttcagacgtg tgctcttccg atctaggcct gagtcgctgt ttggagacac        60

<210>  889
<211>  60
<212>  DNA
<213>  Artificial Sequence

<220>
<223>  primer

<400>  889
gtgactggag ttcagacgtg tgctcttccg atcttaagat tagtcgctgt ttggagacac        60

<210>  890
<211>  60
<212>  DNA
<213>  Artificial Sequence

<220>
<223>  primer

<400>  890
gtgactggag ttcagacgtg tgctcttccg atctaggcga atgtcgctgt ttggagacac        60

<210>  891
<211>  60
<212>  DNA
<213>  Artificial Sequence

<220>
<223>  primer


<400>  891
gtgactggag ttcagacgtg tgctcttccg atctttgttc ttgtcgctgt ttggagacac        60



<210>  892
<211>  60
<212>  DNA
<213>  Artificial Sequence

<220>
<223>  primer


<400>  892
gtgactggag ttcagacgtg tgctcttccg atcttactcc tggtcgctgt ttggagacac        60



<210>  893
<211>  60
<212>  DNA
<213>  Artificial Sequence

<220>
<223>  primer


<400>  893
gtgactggag ttcagacgtg tgctcttccg atctgactgg cggtcgctgt ttggagacac        60



<210>  894
<211>  60
<212>  DNA
<213>  Artificial Sequence

<220>
<223>  primer


<400>  894
gtgactggag ttcagacgtg tgctcttccg atctatatat acgtcgctgt ttggagacac        60



<210>  895
<211>  60
<212>  DNA
<213>  Artificial Sequence

<220>
<223>  primer

<400>  895
gtgactggag ttcagacgtg tgctcttccg atcttagatc gggtcgctgt ttggagacac        60

<210>  896
<211>  60
<212>  DNA
<213>  Artificial Sequence

<220>
<223>  primer

<400>  896
gtgactggag ttcagacgtg tgctcttccg atctaggcaa gcgtcgctgt ttggagacac        60

<210>  897
<211>  60
<212>  DNA
<213>  Artificial Sequence

<220>
<223>  primer

<400>  897
gtgactggag ttcagacgtg tgctcttccg atcttccagg tagtcgctgt ttggagacac        60

<210>  898
<211>  60
<212>  DNA
<213>  Artificial Sequence

<220>
<223>  primer

<400>  898
gtgactggag ttcagacgtg tgctcttccg atctaggtca aggtcgctgt ttggagacac        60

<210>  899
<211>  60
<212>  DNA
<213>  Artificial Sequence

<220>
<223>  primer

<400>  899
gtgactggag ttcagacgtg tgctcttccg atcttacact gggtcgctgt ttggagacac        60

<210>  900
<211>  60
<212>  DNA
<213>  Artificial Sequence

<220>
<223>  primer


<400>  900
gtgactggag ttcagacgtg tgctcttccg atcttccttg cggtcgctgt ttggagacac        60



<210>  901
<211>  60
<212>  DNA
<213>  Artificial Sequence

<220>
<223>  primer


<400>  901
gtgactggag ttcagacgtg tgctcttccg atctaggacc tcgtcgctgt ttggagacac        60



<210>  902
<211>  60
<212>  DNA
<213>  Artificial Sequence

<220>
<223>  primer


<400>  902
gtgactggag ttcagacgtg tgctcttccg atcttaagca ttgtcgctgt ttggagacac        60



<210>  903
<211>  60
<212>  DNA
<213>  Artificial Sequence

<220>
<223>  primer


<400>  903
gtgactggag ttcagacgtg tgctcttccg atctaggata ttgtcgctgt ttggagacac        60



<210>  904
<211>  60
<212>  DNA
<213>  Artificial Sequence

<220>
<223>  primer

<400>  904
gtgactggag ttcagacgtg tgctcttccg atctaatcgt tagtcgctgt ttggagacac        60

<210>  905
<211>  60
<212>  DNA
<213>  Artificial Sequence

<220>
<223>  primer

<400>  905
gtgactggag ttcagacgtg tgctcttccg atctgtctac atgtcgctgt ttggagacac        60

<210>  906
<211>  60
<212>  DNA
<213>  Artificial Sequence

<220>
<223>  primer

<400>  906
gtgactggag ttcagacgtg tgctcttccg atctcgctgc tcgtcgctgt ttggagacac        60

<210>  907
<211>  60
<212>  DNA
<213>  Artificial Sequence

<220>
<223>  primer

<400>  907
gtgactggag ttcagacgtg tgctcttccg atctgatcaa cagtcgctgt ttggagacac        60

<210>  908
<211>  60
<212>  DNA
<213>  Artificial Sequence

<220>
<223>  primer

<400>  908
gtgactggag ttcagacgtg tgctcttccg atctcgaagg acgtcgctgt ttggagacac        60

<210>  909
<211>  60
<212>  DNA
<213>  Artificial Sequence

<220>
<223>  primer


<400>  909
gtgactggag ttcagacgtg tgctcttccg atctgatgcc gggtcgctgt ttggagacac        60



<210>  910
<211>  60
<212>  DNA
<213>  Artificial Sequence

<220>
<223>  primer


<400>  910
gtgactggag ttcagacgtg tgctcttccg atctctacga aggtcgctgt ttggagacac        60



<210>  911
<211>  60
<212>  DNA
<213>  Artificial Sequence

<220>
<223>  primer


<400>  911
gtgactggag ttcagacgtg tgctcttccg atctgatgcg tcgtcgctgt ttggagacac        60



<210>  912
<211>  60
<212>  DNA
<213>  Artificial Sequence

<220>
<223>  primer


<400>  912
gtgactggag ttcagacgtg tgctcttccg atctctacgg cagtcgctgt ttggagacac        60



<210>  913
<211>  60
<212>  DNA
<213>  Artificial Sequence

<220>
<223>  primer

<400>  913
gtgactggag ttcagacgtg tgctcttccg atctgattcc ttgtcgctgt ttggagacac        60

<210>  914
<211>  60
<212>  DNA
<213>  Artificial Sequence

<220>
<223>  primer

<400>  914
gtgactggag ttcagacgtg tgctcttccg atctctactc gagtcgctgt ttggagacac        60

<210>  915
<211>  60
<212>  DNA
<213>  Artificial Sequence

<220>
<223>  primer

<400>  915
gtgactggag ttcagacgtg tgctcttccg atctgattcg aggtcgctgt ttggagacac        60

<210>  916
<211>  60
<212>  DNA
<213>  Artificial Sequence

<220>
<223>  primer

<400>  916
gtgactggag ttcagacgtg tgctcttccg atctaatcgg cggtcgctgt ttggagacac        60

<210>  917
<211>  60
<212>  DNA
<213>  Artificial Sequence

<220>
<223>  primer

<400>  917
gtgactggag ttcagacgtg tgctcttccg atctttcgcc gagtcgctgt ttggagacac        60

<210>  918
<211>  60
<212>  DNA
<213>  Artificial Sequence

<220>
<223>  primer


<400>  918
gtgactggag ttcagacgtg tgctcttccg atctctggcc tcgtcgctgt ttggagacac        60



<210>  919
<211>  60
<212>  DNA
<213>  Artificial Sequence

<220>
<223>  primer


<400>  919
gtgactggag ttcagacgtg tgctcttccg atctgaactt atgtcgctgt ttggagacac        60



<210>  920
<211>  60
<212>  DNA
<213>  Artificial Sequence

<220>
<223>  primer


<400>  920
gtgactggag ttcagacgtg tgctcttccg atctcgtatt gggtcgctgt ttggagacac        60



<210>  921
<211>  60
<212>  DNA
<213>  Artificial Sequence

<220>
<223>  primer


<400>  921
gtgactggag ttcagacgtg tgctcttccg atctgaagca cagtcgctgt ttggagacac        60



<210>  922
<211>  60
<212>  DNA
<213>  Artificial Sequence

<220>
<223>  primer

<400>  922
gtgactggag ttcagacgtg tgctcttccg atctcttaat acgtcgctgt ttggagacac        60

<210>  923
<211>  60
<212>  DNA
<213>  Artificial Sequence

<220>
<223>  primer

<400>  923
gtgactggag ttcagacgtg tgctcttccg atctgaagtc ttgtcgctgt ttggagacac        60

<210>  924
<211>  60
<212>  DNA
<213>  Artificial Sequence

<220>
<223>  primer

<400>  924
gtgactggag ttcagacgtg tgctcttccg atctgaagag gcgtcgctgt ttggagacac        60

<210>  925
<211>  60
<212>  DNA
<213>  Artificial Sequence

<220>
<223>  primer

<400>  925
gtgactggag ttcagacgtg tgctcttccg atctcggata acgtcgctgt ttggagacac        60

<210>  926
<211>  60
<212>  DNA
<213>  Artificial Sequence

<220>
<223>  primer

<400>  926
gtgactggag ttcagacgtg tgctcttccg atctgaatct gggtcgctgt ttggagacac        60

<210>  927
<211>  60
<212>  DNA
<213>  Artificial Sequence

<220>
<223>  primer


<400>  927
gtgactggag ttcagacgtg tgctcttccg atctctgatt gagtcgctgt ttggagacac        60



<210>  928
<211>  60
<212>  DNA
<213>  Artificial Sequence

<220>
<223>  primer


<400>  928
gtgactggag ttcagacgtg tgctcttccg atctaatccg ttgtcgctgt ttggagacac        60



<210>  929
<211>  60
<212>  DNA
<213>  Artificial Sequence

<220>
<223>  primer


<400>  929
gtgactggag ttcagacgtg tgctcttccg atcttgcgta cagtcgctgt ttggagacac        60



<210>  930
<211>  60
<212>  DNA
<213>  Artificial Sequence

<220>
<223>  primer


<400>  930
gtgactggag ttcagacgtg tgctcttccg atctgaatca atgtcgctgt ttggagacac        60



<210>  931
<211>  60
<212>  DNA
<213>  Artificial Sequence

<220>
<223>  primer

<400>  931
gtgactggag ttcagacgtg tgctcttccg atcttgagtc aggtcgctgt ttggagacac        60

<210>  932
<211>  60
<212>  DNA
<213>  Artificial Sequence

<220>
<223>  primer

<400>  932
gtgactggag ttcagacgtg tgctcttccg atctgaatgc tcgtcgctgt ttggagacac        60

<210>  933
<211>  60
<212>  DNA
<213>  Artificial Sequence

<220>
<223>  primer

<400>  933
gtgactggag ttcagacgtg tgctcttccg atctgaatat ccgtcgctgt ttggagacac        60

<210>  934
<211>  60
<212>  DNA
<213>  Artificial Sequence

<220>
<223>  primer

<400>  934
gtgactggag ttcagacgtg tgctcttccg atctcttatg aagtcgctgt ttggagacac        60

<210>  935
<211>  60
<212>  DNA
<213>  Artificial Sequence

<220>
<223>  primer

<400>  935
gtgactggag ttcagacgtg tgctcttccg atcttcggca ccgtcgctgt ttggagacac        60

<210>  936
<211>  60
<212>  DNA
<213>  Artificial Sequence

<220>
<223>  primer


<400>  936
gtgactggag ttcagacgtg tgctcttccg atctaagaag cggtcgctgt ttggagacac        60



<210>  937
<211>  60
<212>  DNA
<213>  Artificial Sequence

<220>
<223>  primer


<400>  937
gtgactggag ttcagacgtg tgctcttccg atctctcacg atgtcgctgt ttggagacac        60



<210>  938
<211>  60
<212>  DNA
<213>  Artificial Sequence

<220>
<223>  primer


<400>  938
gtgactggag ttcagacgtg tgctcttccg atcttcggtc gagtcgctgt ttggagacac        60



<210>  939
<211>  60
<212>  DNA
<213>  Artificial Sequence

<220>
<223>  primer


<400>  939
gtgactggag ttcagacgtg tgctcttccg atcttcggta aggtcgctgt ttggagacac        60



<210>  940
<211>  60
<212>  DNA
<213>  Artificial Sequence

<220>
<223>  primer

<400>  940
gtgactggag ttcagacgtg tgctcttccg atctaagata cagtcgctgt ttggagacac        60

<210>  941
<211>  60
<212>  DNA
<213>  Artificial Sequence

<220>
<223>  primer

<400>  941
gtgactggag ttcagacgtg tgctcttccg atctgtcgct gtgtcgctgt ttggagacac        60

<210>  942
<211>  60
<212>  DNA
<213>  Artificial Sequence

<220>
<223>  primer

<400>  942
gtgactggag ttcagacgtg tgctcttccg atcttcggat gtgtcgctgt ttggagacac        60

<210>  943
<211>  60
<212>  DNA
<213>  Artificial Sequence

<220>
<223>  primer

<400>  943
gtgactggag ttcagacgtg tgctcttccg atctcgagcc gggtcgctgt ttggagacac        60

<210>  944
<211>  60
<212>  DNA
<213>  Artificial Sequence

<220>
<223>  primer

<400>  944
gtgactggag ttcagacgtg tgctcttccg atctcgatta tcgtcgctgt ttggagacac        60

<210>  945
<211>  60
<212>  DNA
<213>  Artificial Sequence

<220>
<223>  primer


<400>  945
gtgactggag ttcagacgtg tgctcttccg atcttcgaag ctgtcgctgt ttggagacac        60




<210>  946
<211>  60
<212>  DNA
<213>  Artificial Sequence

<220>
<223>  primer


<400>  946
gtgactggag ttcagacgtg tgctcttccg atctctatca ttgtcgctgt ttggagacac        60




<210>  947
<211>  60
<212>  DNA
<213>  Artificial Sequence

<220>
<223>  primer


<400>  947
gtgactggag ttcagacgtg tgctcttccg atctaagtta tcgtcgctgt ttggagacac        60




<210>  948
<211>  60
<212>  DNA
<213>  Artificial Sequence

<220>
<223>  primer


<400>  948
gtgactggag ttcagacgtg tgctcttccg atctcgaacg gagtcgctgt ttggagacac        60




<210>  949
<211>  60
<212>  DNA
<213>  Artificial Sequence

<220>
<223>  primer

<400>  949
gtgactggag ttcagacgtg tgctcttccg atctctactg acgtcgctgt ttggagacac        60

<210>  950
<211>  60
<212>  DNA
<213>  Artificial Sequence

<220>
<223>  primer

<400>  950
gtgactggag ttcagacgtg tgctcttccg atcttcttaa gtgtcgctgt ttggagacac        60

<210>  951
<211>  60
<212>  DNA
<213>  Artificial Sequence

<220>
<223>  primer

<400>  951
gtgactggag ttcagacgtg tgctcttccg atctttagag tcgtcgctgt ttggagacac        60

<210>  952
<211>  60
<212>  DNA
<213>  Artificial Sequence

<220>
<223>  primer

<400>  952
gtgactggag ttcagacgtg tgctcttccg atctaagacg aagtcgctgt ttggagacac        60

<210>  953
<211>  60
<212>  DNA
<213>  Artificial Sequence

<220>
<223>  primer

<400>  953
gtgactggag ttcagacgtg tgctcttccg atctttatta tggtcgctgt ttggagacac        60

<210>  954
<211>  60
<212>  DNA
<213>  Artificial Sequence

<220>
<223>  primer


<400>  954
gtgactggag ttcagacgtg tgctcttccg atctcgctat tagtcgctgt ttggagacac        60



<210>  955
<211>  60
<212>  DNA
<213>  Artificial Sequence

<220>
<223>  primer


<400>  955
gtgactggag ttcagacgtg tgctcttccg atcttctatc aggtcgctgt ttggagacac        60



<210>  956
<211>  60
<212>  DNA
<213>  Artificial Sequence

<220>
<223>  primer


<400>  956
gtgactggag ttcagacgtg tgctcttccg atctcggtgg tagtcgctgt ttggagacac        60



<210>  957
<211>  60
<212>  DNA
<213>  Artificial Sequence

<220>
<223>  primer


<400>  957
gtgactggag ttcagacgtg tgctcttccg atcttcacca atgtcgctgt ttggagacac        60



<210>  958
<211>  60
<212>  DNA
<213>  Artificial Sequence

<220>
<223>  primer

<400>  958
gtgactggag ttcagacgtg tgctcttccg atctctggaa gcgtcgctgt ttggagacac        60

<210>  959
<211>  60
<212>  DNA
<213>  Artificial Sequence

<220>
<223>  primer

<400>  959
gtgactggag ttcagacgtg tgctcttccg atctcgtaag aggtcgctgt ttggagacac        60

<210>  960
<211>  60
<212>  DNA
<213>  Artificial Sequence

<220>
<223>  primer

<400>  960
gtgactggag ttcagacgtg tgctcttccg atctaagaga gcgtcgctgt ttggagacac        60

<210>  961
<211>  60
<212>  DNA
<213>  Artificial Sequence

<220>
<223>  primer

<400>  961
gtgactggag ttcagacgtg tgctcttccg atcttcaacg aggtcgctgt ttggagacac        60

<210>  962
<211>  60
<212>  DNA
<213>  Artificial Sequence

<220>
<223>  primer

<400>  962
gtgactggag ttcagacgtg tgctcttccg atcttgcgag acgtcgctgt ttggagacac        60

<210>  963
<211>  60
<212>  DNA
<213>  Artificial Sequence

<220>
<223>  primer


<400>  963
gtgactggag ttcagacgtg tgctcttccg atctcctggt gtgtcgctgt ttggagacac        60



<210>  964
<211>  60
<212>  DNA
<213>  Artificial Sequence

<220>
<223>  primer


<400>  964
gtgactggag ttcagacgtg tgctcttccg atctaagtaa gtgtcgctgt ttggagacac        60



<210>  965
<211>  60
<212>  DNA
<213>  Artificial Sequence

<220>
<223>  primer


<400>  965
gtgactggag ttcagacgtg tgctcttccg atcttgactg aagtcgctgt ttggagacac        60



<210>  966
<211>  60
<212>  DNA
<213>  Artificial Sequence

<220>
<223>  primer


<400>  966
gtgactggag ttcagacgtg tgctcttccg atctaagact gtgtcgctgt ttggagacac        60



<210>  967
<211>  60
<212>  DNA
<213>  Artificial Sequence

<220>
<223>  primer

<400>  967
gtgactggag ttcagacgtg tgctcttccg atctcaatga tggtcgctgt ttggagacac        60

<210>  968
<211>  60
<212>  DNA
<213>  Artificial Sequence

<220>
<223>  primer

<400>  968
gtgactggag ttcagacgtg tgctcttccg atctcacagt aagtcgctgt ttggagacac        60

<210>  969
<211>  60
<212>  DNA
<213>  Artificial Sequence

<220>
<223>  primer

<400>  969
gtgactggag ttcagacgtg tgctcttccg atcttggtca ttgtcgctgt ttggagacac        60

<210>  970
<211>  60
<212>  DNA
<213>  Artificial Sequence

<220>
<223>  primer

<400>  970
gtgactggag ttcagacgtg tgctcttccg atctcaaccg tggtcgctgt ttggagacac        60

<210>  971
<211>  60
<212>  DNA
<213>  Artificial Sequence

<220>
<223>  primer

<400>  971
gtgactggag ttcagacgtg tgctcttccg atcttggtgc acgtcgctgt ttggagacac        60

<210>  972
<211>  60
<212>  DNA
<213>  Artificial Sequence

<220>
<223>  primer


<400>  972
gtgactggag ttcagacgtg tgctcttccg atctccacaa tggtcgctgt ttggagacac        60




<210>  973
<211>  60
<212>  DNA
<213>  Artificial Sequence

<220>
<223>  primer


<400>  973
gtgactggag ttcagacgtg tgctcttccg atcttgtgtg ccgtcgctgt ttggagacac        60




<210>  974
<211>  60
<212>  DNA
<213>  Artificial Sequence

<220>
<223>  primer


<400>  974
gtgactggag ttcagacgtg tgctcttccg atctcaccac gggtcgctgt ttggagacac        60




<210>  975
<211>  60
<212>  DNA
<213>  Artificial Sequence

<220>
<223>  primer


<400>  975
gtgactggag ttcagacgtg tgctcttccg atcttgtgtt aagtcgctgt ttggagacac        60




<210>  976
<211>  65
<212>  DNA
<213>  Artificial Sequence

<220>
<223>  primer

<400>  976
gtgactggag ttcagacgtg tgctcttccg atctagcgct cggaaacacg gacacccaaa      60
gtagt                                                                  65

<210>  977
<211>  65
<212>  DNA
<213>  Artificial Sequence

<220>
<223>  primer

<400>  977
gtgactggag ttcagacgtg tgctcttccg atcttgcgct tcgaaacacg gacacccaaa      60
gtagt                                                                  65

<210>  978
<211>  65
<212>  DNA
<213>  Artificial Sequence

<220>
<223>  primer

<400>  978
gtgactggag ttcagacgtg tgctcttccg atctagcttg gtgaaacacg gacacccaaa      60
gtagt                                                                  65

<210>  979
<211>  65
<212>  DNA
<213>  Artificial Sequence

<220>
<223>  primer

<400>  979
gtgactggag ttcagacgtg tgctcttccg atctagctta tggaaacacg gacacccaaa      60
gtagt                                                                  65

<210>  980
<211>  65
<212>  DNA
<213>  Artificial Sequence

<220>
<223>  primer

<400>  980

gtgactggag ttcagacgtg tgctcttccg atctataacg acgaaacacg gacacccaaa 60
gtagt 65

<210> 981
<211> 65
<212> DNA
<213> Artificial Sequence

<220>
<223> primer

<400> 981
gtgactggag ttcagacgtg tgctcttccg atctgatgga gtgaaacacg gacacccaaa 60
gtagt 65

<210> 982
<211> 65
<212> DNA
<213> Artificial Sequence

<220>
<223> primer

<400> 982
gtgactggag ttcagacgtg tgctcttccg atcttgatct aagaaacacg gacacccaaa 60
gtagt 65

<210> 983
<211> 65
<212> DNA
<213> Artificial Sequence

<220>
<223> primer

<400> 983
gtgactggag ttcagacgtg tgctcttccg atctagctat atgaaacacg gacacccaaa 60
gtagt 65

<210> 984
<211> 65
<212> DNA
<213> Artificial Sequence

<220>
<223> primer

<400> 984
gtgactggag ttcagacgtg tgctcttccg atctagcaga gagaaacacg gacacccaaa 60
gtagt 65

<210>  985
<211>  65
<212>  DNA
<213>  Artificial Sequence

<220>
<223>  primer


<400>  985
gtgactggag ttcagacgtg tgctcttccg atctgacgta aggaaacacg gacacccaaa      60
gtagt                                                                  65




<210>  986
<211>  65
<212>  DNA
<213>  Artificial Sequence

<220>
<223>  primer


<400>  986
gtgactggag ttcagacgtg tgctcttccg atctaggcct gagaaacacg gacacccaaa      60
gtagt                                                                  65




<210>  987
<211>  65
<212>  DNA
<213>  Artificial Sequence

<220>
<223>  primer


<400>  987
gtgactggag ttcagacgtg tgctcttccg atcttaagat tagaaacacg gacacccaaa      60
gtagt                                                                  65




<210>  988
<211>  65
<212>  DNA
<213>  Artificial Sequence

<220>
<223>  primer


<400>  988
gtgactggag ttcagacgtg tgctcttccg atctaggcga atgaaacacg gacacccaaa      60
gtagt                                                                  65




<210>  989

<211>  65
<212>  DNA
<213>  Artificial Sequence

<220>
<223>  primer

<400>  989
gtgactggag ttcagacgtg tgctcttccg atctttgttc ttgaaacacg gacacccaaa      60
gtagt                                                                  65

<210>  990
<211>  65
<212>  DNA
<213>  Artificial Sequence

<220>
<223>  primer

<400>  990
gtgactggag ttcagacgtg tgctcttccg atcttactcc tggaaacacg gacacccaaa      60
gtagt                                                                  65

## Description

The present invention relates to the field of parallel nucleic acid analytics.

### Background of the invention

Within just a few months the newly emerged coronavirus Sars-CoV2 caused the worldwide pandemic of Covid-19. While the world is awaiting an effective and scalable vaccination and an antiviral therapy, Covid-19 must be contained as good as possible. To this end two measures are available, namely social distancing e.g. via lockdown measures and molecular testing for contact tracing and random surveillance. However, the molecular method to detect active disease, i.e. the presence of viral RNA from swab, gargle, sputum, or saliva, is currently costly and hindered by shortage of equipment and supply of reagents. Large scale testing can be implemented by PCR detection in pooled RNA extracts (Ben-Ami et al., Clinical Microbiology and Infection 2020, 26: 1248-1253). However, such pooling approaches may need retesting to identify the individual positive sample in a positively tested pool. In order to identify individual samples in a multiplex approach, sample-derived DNA is barcoded by a specific nucleotide sequence (Dao Thi et al., Sci. Transl. Med. 2020; 12: eabc7075). The barcode sequence is determined together with the viral sequence, thereby creating the connection of viral presence within the respective sample. In general, however, multiplexing reduces the sensitivity of the assay and/or increases the limit of detection for viral genetic material (Visseaux et al., J Clin Micr 2020, 58(8): e00630-20).

Yet there still remains the need to further increase testing capacity while at the same time reducing testing costs and preferably also maintain or increase test sensitivity. A goal of the present invention is to improve large scale testing capacities to address these needs.

### Summary of the invention

The present invention provides a method for detecting a nucleic acid of interest in a plurality of samples, comprising the steps of a) providing analyte nucleic acids from a plurality of samples in separate containers for each sample, wherein the containers are arranged into an array of subsets, wherein the array comprises two or more subsets; b) amplifying nucleic acids by a primer extension reaction using at least one pair of primers hybridized to the analyte nucleic acids, wherein a pair of primers comprises a forward and a reverse primer, wherein the forward and reverse primers both comprise an adaptor sequence, an sample identifier sequence and a binding sequence for hybridization to the analyte nucleic acids, respectively; c) combining the amplified nucleic acids of step b) of containers of two or more subsets to an array of combined containers, wherein containers of one subset, but not of another subset, are combined to a combined container; d) amplifying nucleic acids by a primer extension reaction using at least one pair of further primers hybridized to the amplified nucleic acids of combined containers of step c), wherein a pair of further primers comprises a further forward and a further reverse primer, wherein the further forward and further reverse primers both comprise a subset identifier sequence and a sequence for hybridization to the adaptor sequence; e) determining the sequences of the amplified nucleic acids of step d); f) assigning a determined sequence of a nucleic acid of interest of step e) to a sample through association to a subset and container with the subset identifier sequences and the sample identifier sequences.

The invention further provides a set of primers suitable for a method of the invention. Such a set may comprise at least 10 different primers A which comprise the sequence, from 5' to 3': an adaptor A sequence, an identifier sequence of at least 4 nt in length and a target binding sequence, wherein the identifier sequence is different within the at least 10 different primers A, optionally with a sequence distance of a Hamming distance of at least 1 or a Levenshtein distance of at least 1; at least 10 different primers B which comprise the sequence, from 5' to 3': an adaptor B sequence, an identifier sequence of at least 4 nt in length and a target binding sequence, wherein the identifier sequence is different within the at least 10 different primers B, optionally with a sequence distance of a Hamming distance of at least 1 or a Levenshtein distance of at least 1; at least 10 different primers C which comprise the sequence, from 5' to 3': an adaptor C sequence, an identifier sequence of at least 4 nt in length and a binding sequence that binds to the adaptor A sequence, wherein the identifier sequence is different within the at least 10 different primers C, optionally with a sequence distance of a Hamming distance of at least 1 or a Levenshtein distance of at least 1; and at least 10 different primers D which comprise the sequence, from 5' to 3': an adaptor D sequence, an identifier sequence of at least 4 nt in length and a binding sequence that binds to the adaptor B sequence, wherein the identifier sequence is different within the at least 10 different primers D, optionally with a sequence distance of a Hamming distance of at least 1 or a Levenshtein distance of at least 1.

In preferred embodiments the nucleic acid of interest is RNA, such as from an RNA virus. The method incudes a conversion of the RNA to cDNA in step a). As such the inventive method can also be written as method for detecting a nucleic acid of interest, including ribonucleic acid (RNA), in a plurality of samples, comprising the steps of a) providing analyte nucleic acids from a plurality of samples in separate containers for each sample, wherein the containers are arranged into an array of subsets, wherein the array comprises two or more subsets; comprising reverse transcribing RNA, thereby generating a complementary DNA of the RNA as analyte nucleic acids; b) amplifying nucleic acids, in particular DNA, by a primer extension reaction using at least one pair of primers hybridized to the analyte nucleic acids, wherein a pair of primers comprises a forward and a reverse primer, wherein the forward and reverse primers both comprise an adaptor sequence, an sample identifier sequence and a binding sequence for hybridization to the analyte nucleic acids, respectively; wherein the amplification is preferably DNA specific and/or with a DNA polymerase; c) combining the amplified nucleic acids of step b) of containers of two or more subsets to an array of combined containers, wherein containers of one subset, but not of another subset, are combined to a combined container; d) amplifying nucleic acids by a primer extension reaction using at least one pair of further primers hybridized to the amplified nucleic acids of combined containers of step c), wherein a pair of further primers comprises a further forward and a further reverse primer, wherein the further forward and further reverse primers both comprise a subset identifier sequence and a sequence for hybridization to the adaptor sequence; e) determining the sequences of the amplified nucleic acids of step d); f) assigning a determined sequence of a nucleic acid of interest of step e) to a sample through association to a subset and container with the subset identifier sequences and the sample identifier sequences.

The disclosure of the invention relates to all aspects of the invention likewise, e.g. descriptions of the method also relate to the set as a suitability or a use of the set; likewise, descriptions of the set also relate to its components that can be used in the method. In particular, primers A and B may be used as forward and as reverse primer; primers C and D may be used as further forward and as further reverse primers.

### Detailed description of the invention

The present invention provides a method for detecting a nucleic acid of interest in a plurality of samples. Such a nucleic acid of interest can e.g. be from a pathogen, such as a virus, bacteria or fungi, and thus be used to detected said pathogen via its nucleic acid. This allows the method to be used in the diagnosis of diseases that are associated with the presence of the pathogen.

Usually the nucleic acid of interest is preselected, i.e. by using primers that bind to the nucleic acid of intertest. As such, the inventive method can be used to detect the presence of specific pathogens of interest.

The inventive method is usually performed in vitro, i.e. a sample is analysed according to the invention ex vivo and/or outside of a subject from whom the sample has been previously obtained.

The subject from whom the sample is obtained can e.g. be a human or a non-human animal, e.g. a mammal, a bird or reptile. Preferably the subject is a mammal, in particular a human.

The sample can be from or contain a biological sample such as a body fluid, such as a sputum, saliva, nasal mucus, bronchoalveolar lavage, urine, a serum or blood sample. Such a sample containing sputum, saliva, or nasal mucus may be obtained from a nasopharyngeal swab, an oropharyngeal swab, a nasal swab, a gargle solution, or providing sputum into a container. More generally the sample may be a solid sample, liquid sample, or aerosols carried in gaseous exhale.

The nucleic acid of interest can be released from the biological sample, cell or encapsulation (e.g. viral encapsulation) so that it is accessible in solution for further treatment. Such a release may include a removal from the pathogenic source, e.g. by the disassembly of the pathogen, which may include virus disassembly or lysis or porosis of bacteria or fungi. Optionally the nucleic acid of interest is stabilized by e.g. removing and/or inactivating any enzymes that would degrade or digest the nucleic acid of interest. E.g. a DNase inhibitor can be added if the nucleic acid of interest is or comprises DNA or a RNase inhibitor can be added if the nucleic acid of interest is or comprises RNA. In combination or as an alternative, the sample may be treated by elevated temperature or chemicals, such as e.g. at an intensity and for a time sufficient to inactivate such unwanted enzymes like DNases and/or RNases.

As said, the nucleic acid of interest can be DNA or RNA, preferably it comprises or consists of RNA.

The nucleic acids of interest, e.g. as released from a biological sample, cell or encapsulation as described above, may be used as the analyte nucleic acids in the inventive methods. In further embodiments, the nucleic acids may be further treated, copied or converted into another type of nucleic acid, such as for example creating a cDNA from an RNA of a biological sample. The cDNA may be generated by reverse transcription. The cDNA may then be used as analyte nucleic acid in the inventive method. A further modification may be fragmentation of a nucleic acid from a sample.

In preferred embodiments of the invention viral RNA is a nucleic acid of interest. Such viral RNA is released from virus capsules, such as capsids or envelopes, and converted into cDNA by performing a reverse transcription. In particular, the invention can comprise in step a) providing RNA from a sample and generating a cDNA of said RNA by reverse transcription, wherein the nucleic acids that are amplified in step b) comprise said cDNA. Reverse transcription can be performed by reverse transcriptase, such as a M-MLV reverse transcriptase, wild type or modified, such as a superscript^{™} reverse transcriptase, e.g. Superscript^{™} III reverse transcriptase or superscript^{™} IV reverse transcriptase. Such modified M-MLV reverse transcriptase preferably has reduced RNase H activity and/or increased thermal stability as compared to wild type M-MLV reverse transcriptase under operative conditions (ambient pressure at 1 bar, 50°C). Example primers for reverse transcription can e.g. random primers, such as random hexamers, which is a collection of various primers with variable sequences at a given length. Example lengths of primers are 5, 6, 7, 8, 9, 10, 11, 12, 13, 14 or more nucleotides in length, preferably 6 (hexamers) or preferably 8-12 for target specific primers. If performed, preferably no barcodes or "identifier sequences" are added during reverse transcription. It is preferred to add such "identifier sequence" in the inventive method later during step b) and d), especially when treating DNA (e.g. cDNA) as analyte nucleic acids. Such barcodes or "identifier sequences" may be later used downstream to connect a determined nucleic acid sequence to a subject from whom the sample originated (and who may thus have an infection with a pathogen).

A container to hold the nucleic acid of a sample may be any holding means suitable for the inventive method. Example containers may be selected from a flask, vial, bag, syringe, or a well, including a microwell on a well-plate, or a droplet. In particular wells on well-plates are preferred as they allow easy organisation, parallel pipetting, easy handling and automation during the inventive method. Before being put into the preferred container to be used in the inventive method, a sample may have been handled, transported and/or treated in another container, e.g. to facilitate transportation from the patient to the facility performing the inventive method.

Each sample that is distinguished according to the plurality of samples is at least initially in a separate container. Of course, the invention does include using pooled biological samples (e.g. from more than one subject) to be treated as one sample but it is preferred that each sample is associated to only one subject for individual association with the nucleic acid of interest.

The plurality of samples may include 10 or more samples, e.g. 20, 50, 75, 100, 150, 200, 250, 300, 400, 500, 600, 700, 800, 900, 1000, 1200, 1400, 1600, 1800, 2000, 2500, 3000, 4000, 5000, 6000, 7000, 8000, 9000, 9500, 10000, 12000, or more samples and any range in between these values. For example, the plurality of samples is 20 to 1000000 samples, e.g. 40 to 500000 samples or 90 to 100000 samples.

In step a) of the inventive method, analyte nucleic acids from a plurality of samples are provided in separate containers for each sample. The analyte nucleic acids may be RNA or DNA; DNA is preferred, as such the analyte nucleic acids may comprises or consists of DNA. If the nucleic acid of interest, e.g. from a pathogen, is RNA, it is preferred to generate a cDNA therefrom to be used as the analyte nucleic acid, as discussed above. The generation of cDNA may be done in the same containers that are later used during the inventive method. Enzymes, like a reverse transcriptase may be inactivated, such as by elevated temperature (e.g. 70°C or more for a superscript^{™} reverse transcriptase), before continuing with step b).

The containers are arranged into an array of subsets, wherein the array comprises two or more subsets. "array of subsets" means that the containers (with the analyte nucleic acids) are grouped in at least two groups ("array comprises two or more subsets"). This arrangement can be an information available to the user who performs the inventive method and/or a special grouping from which the subset arrangement is apparent and/or a labelling or marking of the containers. For example, the containers may be arranged into an array of subsets and the subsets comprise the containers at a coordinate. That coordinate may be noted and information of its container may be stored by the user. Coordinates may be of a 2D or 3D arrangement, e.g. rows, columns and/or stacks of one or more carrier(s) with the containers. Such a carrier may be a plate, e.g. with depressions as containers, such as a well plate. Of course, any other form of coordinate may be used to identify a container of a subset and/or of a sample from a particular subject, including water-based droplets in oil. Containers may have walls or not, such as in the case of droplets. In a preferred embodiment, the containers are arranged into an array of subsets and the subsets comprise the containers at a coordinate. The containers with a corresponding coordinate for each subset are combined in step c).

An example is using two or more well-plates. All wells (containers) in one well-plate are a subset with each well-plate forming a subset of its wells (containers). According to this example, a plurality of two or more well-plates would thus contain an array of two or more subsets with the subset amount being the number of well-plates and each subset (individual well-plate) consisting of the number of wells (containers) per plate or less, if not all wells contain an analyte nucleic acid. In fact, in preferred embodiments such a well-plate (subset) may use 1-10 of its wells (containers) for controls, so that in case of 96-well plates fewer than 96 wells may be used for analyte nucleic acids. Irrespective of this example, in any embodiment of the invention, each subset may comprise one or more control nucleic acids or empty controls without nucleic acids in a container, that are treated similarly throughout the inventive method as containers with analyte nucleic acids.

In preferred embodiments of the invention, the two or more subsets are 8 or more subsets, 10 or more subsets, such as 20 or more subsets, e.g. 30 or more subsets, 40 or more subsets, 50 or more subsets, 60 or more subsets, 70 or more subsets, 80 or more subsets, or 90 or more subsets. An example for 8 subsets are 8 well plates of PCR strips.

In equally preferred embodiments, a subset comprises 10 or more containers, preferably 20 or more containers, or 30 or more containers, or 40 or more containers, or 50 or more containers, or 60 or more containers, or 70 or more containers, or 80 or more containers, or 90 or more containers.

Examples of such number for subset or container arrays are well plates such as 96-, 384- or 1536-well plates. Any such number as mentioned above (at least 2, 10, 20, etc.) of wells can be used as containers or members of subsets of the present invention. Remaining wells on a plate may or may not be used, e.g. as controls. With the subset representing plates or in general any such array, the number of subsets represents the number of plates or arrays. When subsets are combined in step c), their combined nucleic acids may also be stored in a container, with each combined nucleic acids of a subset being in a separate container. Again, in some examples, well plates may be used, with e.g. one or more wells containing the combined nucleic acids of a subset. In preferred embodiments, the method uses 96 containers (subset size) and 96 subsets; in further options the method uses 384 containers (subset size) and 96 or 384 subsets. This would make full use of 96- or 384 well plates. Also 1536 well plates can be used for the containers or the subsets. In any of these embodiments, the number of containers to accommodate samples or subsets (when combined in the step d) can be lower to accommodate controls as mentioned above.

In step b) of the inventive method, nucleic acids are amplified by a primer extension reaction using at least one pair of primers hybridized to the analyte nucleic acids, wherein a pair of primers comprises a forward and a reverse primer, wherein the forward and reverse primers both comprise an adaptor sequence, a sample identifier sequence ("sample index") and a binding sequence for hybridization to the analyte nucleic acids, respectively.

As mentioned above, the analyte nucleic acids are preferably DNA - either because DNA has been provided by the sample or after conversion of RNA into cDNA with the cDNA being used as analyte nucleic acids in step b). Primer extension reactions are conventional in the art and include binding the primes to the analyte nucleic acids and extending the primers with further nucleotides in a template specific fashion, with the template being the analyte nucleic acid. Accordingly, a complementary strand is generated if the sample contains the appropriate template. Such a reaction is preferably performed with a DNA polymerase. A second strand can be generated likewise with another primer. Both primers are referred to as forward and reverse primers for first and second strand generation as is common in the art. An example of a primer extension reaction is PCR.

Alternatively, the analyte nucleic acid is RNA. With RNA as template, it is preferred to generate a complementary DNA strand (also termed copy DNA or cDNA). As such the (forward) primer preferably is DNA with the extended nucleotides also being DNA. Such a reaction is for example a reverse transcription, which can be facilitated by a reverse transcriptase. Second strand generation with the reverse primer is then a DNA templated DNA polymerisation, preferably with a DNA polymerase as described above. Further rounds or cycles of amplification can then be entirely DNA-based, including primer extension reactions using the forward primer, which in a further round or cycle may use the generated complementary strand of the reverse primers' amplification product as template. The difference with the previous paragraph - according to this embodiment - would be that in the previous paragraph, both forward and reverse primers add their sample identifier in entirely DNA-based amplification procedures, whereas the second alternative adds primers with the sample identifier already in a reverse transcription reaction (or generally in a RNA-based amplification reaction). It is preferred according to the invention to add the sample identifier only using DNA templates since DNA-based primer extension reactions are more robust with fewer side reactions but of course using RNA templates is also a possibility, in particular given that the invention uses two sample identifiers (on both, the forward and reverse primers) to compensate and even add further reliability to the method, thereby increasing specificity.

As said, according to the invention, the forward and reverse primers both comprise an adaptor sequence, a sample identifier sequence and a binding sequence for hybridization to the analyte nucleic acids, respectively.

The binding sequence for hybridization to the analyte nucleic acids comprises a complementary sequence to the analyte nucleic acid (or its complementary sequence in case of the reverse primer) so that the primer specifically binds to the analyte nucleic acid (or its complementary sequence in case of the reverse primer) by hybridisation under reaction conditions. Reaction conditions for hybridization may vary according to the enzymes and/or buffers used and may e.g. comprise heating to 58°C in a PCR buffer comprising 750 mM Tris-HCl pH 8.3, 200 mM (NH₄)₂SO₄, 1% Triton X-100, which is a preferred embodiment of the invention. Also variations of the buffers can be used. E.g. a buffer may comprise a pH of 7 to 9, preferably about 8.3. Preferably sulphate ions are present; also preferred a crowding agent such as Triton X-100 is used; preferably in a concentration of 0.3 to 3 %, e.g. about 1%, (all w/w-%). Preferably the complementary sequence to the analyte nucleic acid comprises 8 or more, e.g. 9, 10, 11, 12, 13, 14, 15, 16, 17, 18, 19, 20, 21, 22 or even more nucleotides, e.g. 8-30 nucleotides that are complementary with the analyte nucleic acid (or its complementary sequence in case of the reverse primer). The number of complementary nucleotides of the forward and reverse primer can be selected independently. Through the binding sequence, the primers are specific to an analyte nucleic acid sequence of interest. These analyte sequences can e.g. be from a pathogen as will be elaborated in more detail below.

The adaptor sequences on the forward and reverse primers are sequences that are used downstream of the inventive method to bind further forward and reverse primers in step d) to the amplified nucleic acids given that the adaptor sequences are preserved in the amplification products. Such adaptor sequences are usually artificial and usually do not bind to expected nucleic acids in the sample. Suitable adaptor sequences are known in the art and have e.g. been deposited in databases (e.g. the Illumina Adapter Sequences 1000000002694 v14 of July 2020). Any such adaptors can be used according to the invention. Generally, almost any sequence is possible, but of course complementary sequences are used in the further forward and reverse primers of step d), which thus form interrelated primers with the forward and reverse primers of step b). Of course, only one primer sequence in the amplified nucleic acids is bound by each sequence for hybridization to the adaptor sequence in the further forward and reverse primers. For example, the sequence for hybridization to the adaptor sequence in the further forward primer binds to the adaptor sequence of the forward primer but not of the reverse primer; the sequence for hybridization to the adaptor sequence of the further reverse primer binds to the adaptor sequence of the reverse primer but not of the forward primer; or both vice-versa. The adaptor sequence of the forward and reverse primer, selected independently, can be 8 or more, e.g. 9, 10, 11, 12, 13, 14, 15, 16, 17, 18, 19, 20, 21, 22 or even more nucleotides, e.g. 8-30 nucleotides in length.

The sample identifier sequence is a label or barcode that identifies the amplification product of a sample and is introduced in said amplification product via the primers. The sample identifier sequences are usually selected to identify a sample within a subset. Since further identifier sequences are introduced in step d), it is not needed to uniquely label each sample of the entire array. The sample identifier sequences can be shared between different types of analyte nucleic acids/binding sequence for hybridization to the analyte nucleic ac-ids. Nucleic acids of different subsets may be labelled with sample identifier sequences that are selected from the same pool of sample identifier sequences and/or may comprise nucleic acids that are labelled by the same sample identifier sequences. Since both the forward and reverse primers comprise sample identifier sequences - selected independently - the inventive method introduces high specificity. In a preferred embodiment, the sample identifier sequences of the forward and reverse primers of a given container/sample should not be identical or at least not selected to be the same with identities being only random occurrences that should be below 5% or below 1% of the containers or samples. The inventors have noticed with this method that the incidence of false positive results can be significantly reduced, which otherwise occurs when high numbers of amplification cycles are used. Unwanted amplification reactions may lead to a product despite the absence of the nucleic acid of interest in a sample, thus leading to a false positive result - if no further control steps are incurred.

Preferably in step b) each sample identifier sequence of the forward primers is different from sample identifier sequence of other forward primers for a given binding sequence for hybridization to the analyte nucleic acids. In other words, the sample identifier sequences are each unique for a given analyte nucleic acid or binding sequence for hybridization to the analyte nucleic acid - or in the final amplification product - unique for a given amplicon. For different binding sequences for hybridization to the analyte nucleic acid/amplicons the same sample identifier sequences may be used. In similar preferred embodiments, in step b) each sample identifier sequence of the reverse primers is different from sample identifier sequence of other reverse primers for a given binding sequence for hybridization to the analyte nucleic acids. These two preferred embodiments can of course be combined; i.e. the sample identifier sequences of the forward and reverse primers are unique for a given binding sequences for hybridization to the analyte nucleic acid/amplicons. In other embodiments, the sample identifier sequences may also be unique among the combined group of forward and reverse primers, but this is not required but of course optional - optionally only for a given binding sequences for hybridization to the analyte nucleic acid/amplicon or not only for a given binding sequences for hybridization to the analyte nucleic acid/amplicon.

The inventive method is based on repeated amplifications of analyte nucleic acids and then of complementary nucleic acids ("amplification cycles"). Each amplification initiated by at least either a forward or reverse primer is considered an amplification cycle. Thus, for illustration, an amplification reaction of both the forward and reverse primer but no further amplification reaction is considered as a reaction with 2 amplification cycles. 2 amplification cycles is thus the minimum for both the forward and reverse primer to get incorporated into the amplification product, for labelling with the sample identifiers. If both forward and reverse primers are present as well as templates for both the forward and the reverse primer, then both primers will result in an amplification product in one amplification cycle. This is usually the case for amplification cycle number 2 onwards. In experimental practice, an amplification cycle is one round of heating and cooling. High numbers of amplification cycles increase the number of erroneous results. However, large numbers of amplification cycles may be needed to detect a nucleic acid of interest or analyte nucleic acid that is present in low amounts. Standardized conditions for all samples/containers are usually used, i.e. the same number of amplification cycles for all containers/samples. In some containers high numbers of analyte nucleic acids may be present, whereas in other containers low numbers are present. Prior to the invention, this caused sub-optimal conditions for some samples leading to false positive or false negative results. The present invention has improved on such methods to allow sufficient sensitivity and selectivity to detect various samples in one multiplexed reaction, as e.g. shown in the examples. The invention works with various amplification cycles for step b), such as 2-80 amplification cycles, preferably 5 to 75, 10 to 70, 20 to 65, 25 to 60, 30 to 55, 35 to 50, or 40 to 45 amplification cycles, or any ranges between these values. In preferred embodiments the amplification of step b) is an endpoint amplification. According to this embodiment that is preferred in all aspects of the invention as it strives towards equalizing the number of amplified nucleic acids in each container regardless of initial nucleic acid amount. This greatly helps in detecting nucleic acids of interest at various concentration and amount ranges in the inventive parallel detection method. Endpoint amplification means that the amplification reaction is carried out until no further amplification can occur (in each container). This can be caused by in fact that the required nucleotides for a further amplification cycle are consumed and/or the primers for another amplification cycle have been consumed. A container with fewer nucleic acids that are amplified will take more amplification cycles than a container with a higher number of nucleic acids that are amplified. Accordingly, since all containers are treated similarly, i.e. with the same number of amplification cycles, a number of amplification cycles is selected that will amplify a container with a minimum amount of nucleic acids of interest that are to be detected. Of course, an empty container will not produce any amplification products regardless of the number of amplification cycles and amount of nucleotides and/or primers. Primer and/or nucleotide consumption during amplification can be due to analyte nucleic acids (if present), controls and/or spike-ins. If no controls and spike-ins are present, then a detection limit may be chosen for a minimum number of analyte nucleic acids that lead in a given number of cycles to the endpoint (no further complete amplification possible). Such a minimum number of analyte nucleic acids can be e.g. 10 or 100 or 500 or 1000 analyte nucleic acids. The selected number of amplification cycles shall take into account the amount of nucleotides and primers that are added, which determine the cycle number until they are consumed (amplification to saturation). Accordingly, in preferred embodiments of the invention, the number of amplification cycles in step b) is selected so that no further amplification of an analyte nucleic acid occurs in the containers. Under normal circumstances 40 or more, e.g. 45 or more cycles are sufficient for an endpoint amplification. A skilled practitioner can choose a suitable amplification cycle number for the sample at hand. In general, if low amounts of nucleic acids of interest are expected to be in a sample, then higher numbers of amplification cycles will be selected. With the present invention, it was possible to detect nucleic acids of interest that had high differences in sample abundance, with samples differing by a factor of up to x10⁷ copies of nucleic acids of interest per sample. It was quite surprising that such differing samples could be processed in one multiplexed reaction at the same conditions for all samples.

In a preferred structure of the forward and/or the reverse primer the binding sequence for hybridization to the analyte nucleic acids is preferably on the 3' end of the forward and/or reverse primer. This allows an efficient extension reaction in 5' to 3' direction. Preferably the sample identifier sequence is between the binding sequence and the adaptor sequence. The adaptor sequence may be at or near the 5' end of the forward and/or reverse primer. Accordingly, a preferred primer structure for the forward and/or reverse primer comprises in 5' to 3' direction: [adaptor sequence] - [sample identifier sequence] - [binding sequence].

Step c) comprises combining the amplified nucleic acids of step b) of containers of two or more subsets to an array of combined containers, wherein containers of one subset, but not of another subset, are combined to a combined container. Accordingly, the containers of one subset are combined to the combined container. This is done for more than two subsets, thereby creating two or more combined containers, each corresponding to one subset. Given that the analyte nucleic acids have been amplified to create amplification products that are labelled by the sample identifier in step b), each amplified nucleic acid carries the sample/original container information despite the combination into one pool of multiple different amplification products of different analyte nucleic acids in the combined container.

In preferred embodiments of the invention, the two or more subsets that are combined (for each subset separately of course) are preferably 10 or more subsets, such as 20 or more subsets, e.g. 30 or more subsets, 40 or more subsets, 50 or more subsets, 60 or more subsets, 70 or more subsets, 80 or more subsets, or 90 or more subsets. In preferred embodiments 96 subsets, 384 or 1536 subsets are combined to fully use 96-, 384-, or 1536-well plates with each well containing a combined subset. The number of containers to accommodate subsets can be lower to accommodate controls as mentioned above.

The combined containers may be physically similar to the containers described above, but of course contain various amplified nucleic acids. Example combined containers may be selected from a flask, vial, bag, syringe, or a well, including a microwell on a well-plate. In particular, a well-plate is preferred with different wells being or containing different combined containers.

Step d) comprises amplifying nucleic acids by a primer extension reaction using at least one pair of further primers hybridized to the amplified nucleic acids of combined containers of step c), wherein a pair of further primers comprises a further forward and a further reverse primer, wherein the further forward and further reverse primers both comprise a subset identifier sequence ("subset index") and a sequence for hybridization to the adaptor sequence. Step d) is similar to step b), wherein now amplification products are amplified instead of analyte nucleic acids, the primers are referred to as "further primers", and the label is now used to identify the subset (or combined container) instead of the analyte. Adaptors for a further amplification reaction or other uses, such as a sequencing adaptor sequence, are now optional and may or may not be present. The further primers bind to the adaptor sequences that have been incorporated into the amplification products through the primers of step b) as described above.

In a preferred structure of the further forward and/or the reverse primer the sequence for hybridization to the adaptor sequence is preferably on the 3' end of the forward and/or reverse primer. This allows an efficient extension reaction in 5' to 3' direction. Preferably the subset identifier sequence is in 5' direction from the sequence for hybridization to the adaptor sequence.

In preferred embodiments, in step d) each subset identifier sequence of the further forward primers is different from subset identifier sequence of other further forward primers; and/or in step d) each subset identifier sequence of the further reverse primers is different from subset identifier sequence of other further reverse primers. As said above with regard to the uniqueness of the forward and reverse primers in step b), also the further forward and/or reverse primers are unique among each group, i.e. subset identifier sequences of further forward primers differ from other subset identifier sequences of other further forward primers. The same applies for the subset identifier sequence of further reverse primers. Accordingly, in this preferred embodiment the subset identifier sequences are unique within the respective groups of further forward primers and the further reverse primers. In other embodiments, subset identifier sequences may also be unique among the combined group of further forward and further reverse primers, but this is not required but of course optional.

In preferred embodiments, the further forward and/or the reverse primer may comprise a further adaptor sequence. Such a further adapter sequence may allow further primer binding reaction or binding to probes during a sequencing reaction, as such the further primer is also referred to as a "sequencing adaptor sequence". When such a further adaptor is present, it is preferred that the subset identifier sequence is between the sequence for hybridization to the adaptor sequence and the sequencing or further adaptor sequence. The further or sequencing adaptor sequence may be at or near the 5' end of the further forward and/or reverse primer. Accordingly, a preferred primer structure for the further forward and/or reverse primer comprises in 5' to 3' direction: [sequencing adaptor sequence] - [subset identifier sequence] - [sequence for hybridization to the adaptor sequence].

In preferred embodiments, the main amplification leading to a substantial increase in amplification products is in step b). Further amplification can be achieved in step d). However, it is preferred to leave the amplification focus on step b). Accordingly, in this preferred embodiment, only few amplification cycles are done in step d). At least two amplification cycles should be done to have a reaction of both the further forward primer and of the further reverse primer. The invention works with few but also several amplification cycles for step d), such as up to 35 amplification cycles or up to 20 amplification cycles. In preferred embodiments 2 to 14 amplification cycles, preferably 3 to 13, 4 to 12, 5 to 11, 6 to 10, 7 to 9, or 8 amplification cycles, or any ranges between these values are used. In preferred embodiments, amplifying nucleic acids in step d) is restricted to at most 12 amplification cycles, preferably at most 10 amplification cycles.

In preferred embodiments, the amplification product as obtained in step b) is DNA. Also in step d), preferably DNA amplification products are obtained. A DNA polymerase can be used as described above for step b). Likewise, also a PCR reaction is preferred for step d).

In further preferred embodiments, in step d) staggered primers are used to amplify nucleic acids. "Staggered primers" means that multiple primers are used, wherein the primers have 0 to 10, such as 1, 2, 3, 4, 5, 6, 7, 8, 9, or 10, preferably 1 to 4, inserted nucleotides as distancing nucleotides between the "further adaptor sequence" (e.g. "sequencing adaptor") and the subset identifier sequence. This embodiment applies to both or one of the further forward and/or further reverse primers. Usually only one of the further primers, either the further forward or further reverse primer, has these distancing nucleotides to have their benefits, when sequencing of amplification products is from one end of the amplification products. Preferably the further forward primers comprise the distancing nucleotides. The distancing nucleotides cause an offset during sequencing of different amplified nucleic acids in step e) so that a specific nucleotide of the subset identifier sequence is at a different distance to the further adaptor sequence and is thus read at a different time-point or instance of the sequencing reaction. The distancing nucleates are preferably random, i.e. mixtures of A, G, C, T at a given position in the primer. Having various distances reduces competitive reactions and/or optimize the gain of the sequencer when sequencing the same nucleotide position, which without the staggering would be the same nucleated type (e.g. G, A, C, T) at the same time or instance. A distancing nucleotide can be any nucleotide type, preferably at a given position (distance from further adaptor sequence) different distancing nucleotides are used in different primers to avoid creating such competitive reactions with the distancing nucleotides. Such staggered primers (mixtures of primers with different distancing nucleotides) are used per container/subset. A particular container may have a constant number of distancing nucleotides, whereas other containers have other numbers of distancing nucleotides.

Step e) comprises determining the sequences of the amplified nucleic acids of step d). Sequence determination can be done in the way as known in the art, e.g. by Sanger sequencing or next generation sequencing. Preferably, for sequencing, the amplified nucleic acids of step d) are immobilized on a solid phase, such as on a column or on beads. An example is flow cell sequencing. For such immobilization binding to probes on the solid phase is possible, e.g. by the sequencing adaptors mentioned above. Sequencing adaptors may be Illumina P5 and/or P7 adaptors. Either only one end of the amplified nucleic acids of step d) is immobilized or both ends of the amplified nucleic acids of step d) are immobilized, thereby forming a loop with the solid phase. For one end immobilization only one of the further forward primer and further reverse primers may have a sequencing adaptor; for both end immobilization both of the further forward primer and further reverse primers may have a sequencing adaptor.

The sequences that are determined in step e) are the subset identifier sequences as introduced by the further forward and further reverse primers into the amplified nucleic acids, the sample identifier sequences as introduced by the forward and reverse primers into the amplified nucleic acids and the analyte sequence that corresponds to the sequence between the region that is hybridized in the analyte nucleic acid by the binding sequences for hybridization to the analyte nucleic acids of the forward and reverse primers. The analyte sequence corresponds to and identifies the nucleic acid of interest and the sample identifier sequences and the subset identifier sequences correspond to and identify a sample.

Preferably the binding sequences for hybridization to the analyte nucleic acids in the forward and reverse primers are selected so that costs for sequencing are limited, i.e. the region between these corresponding regions on the analyte molecule - leading to the analyte sequence (amplicon) - has a length of at least 10 and/or up to 400 nucleotides, preferably 20 to 300 nucleotides, even more preferred 30 to 200 nucleotides, such as 40 to 150 nucleotides, even more preferred 45 to 100 nucleotides, or about 70 nucleotides.

In preferred embodiments, any amplicons for analyte nucleic acids, spike-ins, internal and added controls are all within these ranges, in particular preferred are approximately the same, such as within 30% of their length (+/-30% of the longest amplicon length in nucleotides). Having similar lengths minimized competitive reactions.

As a data processing step that follows from the information generated in step e), in step f) a determined sequence of a nucleic acid of interest of step e) is assigned to a sample through association to a subset and container with the subset identifier sequences and the sample identifier sequences. In other embodiments, step f) can be performed with technical means, such as on a computer. The computer may receive sample identification information, such as the location of its container, e.g. in the array, the subset identifier sequences and the sample identifier sequences and the determined sequences and present the information of a detected nucleic acid of interest, via its analyte sequence, in a sample. Preferably the information given in step f) includes a yes or no information of the nucleotide acid of interest being or not being in a sample. The computer, or the inventive set or kit, may comprise a computer readable data memory device, such as a flash memory or a hard disk, to store instructions for performing such a method. In some embodiments of the invention, step f) may be omitted or outsourced to another service provider, who is not performing the method of steps a) to e).

In preferred embodiments of the inventive method, at least one of the forward and reverse primers of step b) is depleted during and/or after step b) but before step c). Alternatively or in combination, preferably in combination also free nucleotides (substrate in an amplification reaction) are depleted. Such a depletion reduces the risk of adverse reaction based on priming by the forward and reverse primers during step d). Such a depletion can e.g. be a physical removal, such as by binding to a solid phase, such as beads and removing the beads with the primers. The solid phase may comprise complementary sequences that bind single stranded primers but not double stranded amplified products. Preferably the primers and/or nucleotides are enzymatically depleted, e.g. by a nuclease or phosphatase, which removes phosphates from primers and/or nucleotides 3' end so that they are unsuitable for further extension reactions. An example is the enzyme mixture ExoStar^{™}. Preferably, exonuclease 1 and/or alkaline phosphatase are used for enzymatic depletion of primers and/or nucleotides.

In preferred embodiments of the invention with a reverse transcription of RNA nucleic acids of interest in step a) as mentioned above, it is also preferred to deplete primers (RT primers) and/or nucleotides before step b) in order to avoid adverse reactions. Said depletion can be performed mechanically or enzymatically as described above, e.g. by purification, absorption, e.g. on a solid phase, or enzymatic activity, such as by a phosphatase or nuclease.

Another method of depletion would be amplification of a spike-in nucleic acid with the forward and/or reverse primer. In this embodiment, the forward and reverse primers bind to the spike-in nucleic acid (or just "spike-in"), that has a complementary nucleic acid sequence to the binding sequence for hybridization to the analyte nucleic acids of the primers but a different sequence than the analyte nucleic acids between said complementary sequences so as to not confuse the spike-ins with nucleic acids of interest. The spike-in nucleic acids thus create a competitive amplification reaction to the analyte nucleic acids and thereby deplete the primers. Since such a competitive reaction may reduce sensitivity of analyte nucleic acids, it is preferred to deplete the primers, e.g. physically or enzymatically. However, it was surprisingly found that in most instances of the inventive method such a competitive reaction does not hinder the detection of analyte nucleic acids - see examples.

An enzymatic removal can e.g. be with a single strand specific nuclease. This is one of the preferred embodiments. Thus, the inventive method may comprise a depletion of the forward and reverse primers, comprising treatment with a single strand specific nuclease.

Spike-ins or any other added control nucleic acids, that is/are added to a container and are amplified and labelled like an analyte nucleic acid in the inventive method, are used in preferred embodiments for all containers. Such spike-ins and controls can be used to identify problems in the inventive detection method, e.g. if an inhibitor of any reaction was present. Spike-ins and added control nucleic acids should produce a detectable product at step e) regardless of sample quality.

The inventive method may further comprise amplifying and processing through steps a) to d), preferably also steps e) and or f), an internal control of the sample, which is a nucleic acid or gene or its coding region that is expected to exist in a sample regardless of the nucleic acid of interest. Such internal controls may e.g. be from constitutively expressed genes of a subject, e.g. a human. In preferred embodiments, the internal control may be a ribosomal gene or coding sequence. A primer pair that binds to a ribosomal gene or coding sequence or any other internal control of a nucleic acid that is expected to exist in a sample may be used in step a) (in case of RNA) or step b) (in particular in case of DNA). RNA internal controls are preferred. A ribosomal gene or coding region or other internal control may be amplified in parallel to the analyte nucleic acids and its amplification products in step b) and d) as a control. The control reaction may be contained in a separate container without analyte nucleic acids or in a container with analyte nucleic acids.

In particular the comparison of spike-ins' and added controls' products of step d) can be compared with step d)'s products of internal controls of a sample. This comparison can be an indicator of problems in the inventive method (when the added controls do not produce an expected product or amount thereof) or provide an indicator if the sample was not isolated or processed properly (if the added control or spike-in produces an expected product in step d) but not the internal control). Such problems can e.g. be improper gargling and other improper sample procurement methods.

The nucleic acid of interest can be a pathogen nucleic acid. Such a pathogen nucleic acid may be characteristic of a particular pathogen. By identifying the pathogen nucleic acid, the presence of at least parts of a pathogen in the sample can be inferred. Preferably the pathogen nucleic acid is a viral nucleic acid.

In a particular preferred embodiment of the invention, the nucleic acid of interest is selected from a SARS-CoV-2 nucleic acid, an influenza nucleic acid, a parainfluenza nucleic acid, a respiratory syncytial virus nucleic acid, a rhinovirus nucleic acid, or a combination thereof. When referring to combinations of the nucleic acid of interest two or more nucleic acids of interest are detected in parallel in the inventive method. Accordingly, more primer pairs are included in step b) that are specific for the analyte nucleic acids corresponding to the two or more nucleic acids of interest, via their binding sequence for hybridization to the analyte nucleic acids. Also, pathogen specific primers may optionally be used in step a), in a reverse transcription, or in step b) to increase sensitivity of the method.

In preferred embodiments of the invention two or more nucleic acids of interest are selected for detection in the inventive method, preferably wherein the two or more nucleic acids of interest are from a virus. The virus can be the same virus or a different virus for two or more nucleic acids of interest. In preferred embodiments in step b) forward and reverse primers with binding sequences for hybridization to the two or more analyte nucleic acids are used. The two or more nucleic acids of interest can be searched for in step f) and assigned to a sample.

In preferred embodiments, 1, 2, 3, 4, 5, 6, 7, 8 or more nucleic acids of interest, preferably two or more nucleic acids of interest, are detected in parallel. In preferred embodiments the two or more nucleic acids of interest are detected from 1, 2, 3, 4, 5, 6, 7, 8 or more organisms, such as pathogens. E.g. it is possible to detect two or more different nucleic acids of interest from the same organism, such as a pathogen, or from differing organisms, e.g. pathogens. In preferred embodiments, the choice of amplicons also includes an internal control directed towards host RNA as sample quality control.

The pathogen may be a virus, a bacterium, or a fungal pathogen.

Example viruses include enterovirus, metapneumovirus, adenovirus, influenza A, B, C, D virus, respiratory syncytial virus (RSV), SARS-associated coronavirus, including SARS-CoV-1, SARS-CoV-2, MERS-CoV, rubeola virus, varicella zoster virus, no-rovirus, rotavirus, enterovirus, among others. Preferably the virus is an RNA virus.

Bacterial pathogens may be selected from Neisseria meningitidis, Mycoplasma pneumoniae, Bordetella pertussis, Streptococcus spp., e.g. Streptococcus pneumoniae, a group A streptococcus, Staphylococcus aureus, e.g. Methicillin-resistant Staphylococcus aureus, Mycobacterium tuberculosis, among others.

A fungal pathogen is e.g. selected from Aspergillus spp. (in particular spores thereof).

The pathogen may be a respiratory pathogen, which e.g. can be found in sputum, saliva, nasal mucus, bronchoalveolar lavage. Such body fluids could be collected as samples and pathogens therein could be detected.

Furthermore, the inventive method could be used to detect DNA with a biomarker of a disease as nucleic acid of interest, such as a KrasG12D mutation in DNA shed from lung cancer nodules.

The inventive method can be used to detect any such pathogen or biomarker. The inventive set may comprise primers with a binding sequence for hybridization to the analyte nucleic acids comprising such pathogen nucleic acids or nucleic acids with the biomarker.

In preferments, the sample identifier sequences of the forward primers comprise a sequence distance, preferably a Hamming distance or a Levenshtein distance, to other sample identifier sequences of forward primers of at least 1, preferably of at least 2 or at least 3.

In further preferments, the sample identifier sequences of the reverse primers comprise a sequence distance, preferably a Hamming distance or a Levenshtein distance, to other sample identifier sequences of reverse primers of at least 1, preferably of at least 2 or at least 3.

In preferments, the subset identifier sequences of the further forward primers comprise a sequence distance, preferably a Hamming distance or a Levenshtein distance, to other subset identifier sequences of further forward primers of at least 1, preferably of at least 2 or at least 3.

In further preferments, the subset identifier sequences of the further reverse primers comprise a sequence distance, preferably a Hamming distance or a Levenshtein distance, to other subset identifier sequences of further reverse primers of at least 1, preferably of at least 2 or at least 3.

As mentioned above, the identifier's sequences can be independently selected from the same sequence pools. The nature of being an identifier sequence in the amplified products of step d) being related to the forward, reverse, further forward, or further reverse primer is apparent on the position of the identifier sequence within the amplified products. Amplified products will usually have the structure: [subset identifier from the further forward primer] - [sample identifier sequence from the forward primer] - [analyte sequence] - [sample identifier sequence from the reverse primer] - [subset identifier sequence from the further reverse primer], or any complementary sequence to any part or the entirety of this structure. In dependence of alternate adaptor use, the structure may have the inner part from analyte to sample identifier sequence reversed, such as: [subset identifier from the further forward primer] - [sample identifier sequence from the reverse primer] - [analyte sequence] - [sample identifier sequence from the forward primer] - [subset identifier sequence from the further reverse primer], or any complementary sequence to any part or the entirety of this structure. As such, the position of the identifier sequences can be known and thus using the same pool of sequences for selecting any one of the identifier sequences is non-problematic. Of course, within a group of identifier sequences, no double sequences should occur that would label two different samples identically, or similarly, such as by requiring a sequence distance as mentioned above within a group of identifier sequences. A sequence distance within a group of identifier sequences reduces misassigning in step f), e.g. due to sequencing errors.

In further preferred embodiments, selected independently any or all of the identifier sequences have a length of at least 4, preferably 5, more preferred at least 6 nucleotides, or at least 7 or at least 8, nucleotides. In particular, the sample identifier sequences of the forward primers may have a length of at least 4, preferably at least 5, even more preferred at least 6 nucleotides (nt), preferably at least 7 nucleotides or at least 8 nucleotides, such as 4-16 nucleotides in length; the sample identifier sequences of the reverse primers may have a length of at least 4, preferably at least 5, even more preferred at least 6 nucleotides, preferably at least 7 nucleotides or at least 8 nucleotides, such as 4-16 nucleotides in length; the subset identifier sequences of the further forward primers may have a length of at least 4, preferably at least 5, even more preferred at least 6 nucleotides, preferably at least 7 nucleotides or at least 8 nucleotides, such as 4-16 nucleotides in length; the subset identifier sequences of the further reverse primers may have a length of at least 4, preferably at least 5, even more preferred at least 6 nucleotides, preferably at least 7 nucleotides or at least 8 nucleotides, such as 4-16 nucleotides in length. The length can be scaled according to the numbers of sample processed in parallel in the inventive method in order to maintain distinguishability between the samples and subsets.

The present invention further comprises a set of primers suitable for a method of the invention. The set may comprise or consist of at least 10 different primers A which comprise the sequence, from 5' to 3': an adaptor A sequence, an identifier sequence of at least 4, preferably at least 5, even more preferred at least 6, nt in length and a target binding sequence, wherein the identifier sequence is different within the at least 10 different primers A, preferably with a sequence distance of a Hamming distance of at least 1 or a Levenshtein distance of at least 1, preferably of at least 2 or at least 3. Everything disclosed above regarding to forward primers applies to primers A of the set.

The set may further comprise at least 10 different primers B which comprise the sequence, from 5' to 3': an adaptor B sequence, an identifier sequence of at least 4 nt in length, preferably at least 5, even more preferred at least 6, nt in length, and a target binding sequence, wherein the identifier sequence is different within the at least 10 different primers B, preferably with a sequence distance of a Hamming distance of at least 1, preferably of at least 2 or at least 3, or a Levenshtein distance of at least 1, preferably of at least 2 or at least 3. Everything disclosed above regarding reverse primers applies to primers B of the set.

The set may further comprise at least 10 different primers C which comprise the sequence, from 5' to 3': an adaptor C sequence, an identifier sequence of at least 4 nt in length, preferably at least 5, even more preferred at least 6, nt in length, and a binding sequence that binds to the adaptor A sequence, wherein the identifier sequence is different within the at least 10 different primers C, preferably with a sequence distance of a Hamming distance of at least 1, preferably of at least 2 or at least 3, or a Levenshtein distance of at least 1, preferably of at least 2 or at least 3. Everything disclosed above regarding further forward primers applies to primers C of the set.

The set may further comprise at least 10 different primers D which comprise the sequence, from 5' to 3': an adaptor D sequence, an identifier sequence of at least 4 nt in length, preferably at least 5, even more preferred at least 6, nt in length, and a binding sequence that binds to the adaptor B sequence, wherein the identifier sequence is different within the at least 10 different primers D, preferably with a sequence distance of a Hamming distance of at least 1, preferably of at least 2 or at least 3, or a Levenshtein distance of at least 1, preferably of at least 2 or at least 3. Everything disclosed above regarding further reverse primers applies to primers D of the set.

Primers A may be used as forward primers in the inventive method; primers B may be used as reverse primers in the inventive method; primers C may be used as further forward primers in the inventive method; primers D may be used as further reverse primers in the inventive method.

In preferred embodiments, the target binding sequences of the primers A (or of the forward primers) and primers B (or of the reverse primers) comprise a sequence or antisense-sequence, respectively, of a viral gene. Both primer have a target binding sequence that allows binding of primers to a target nucleotide comprising the target sequence or a complementary sequence thereto (depending if the sense or anti-sense strand is bound). The target binding sequence is also referred to as binding sequence for hybridization to the analyte nucleic acids herein. In the inventive methods, the analyte nucleic acids comprise the target binding sequence or a complementary sequence thereto.

The viral gene preferably is of a SARS-CoV-2 N, M, E or S gene or coding sequence thereof, influenza PA, PB1, PB2, PA, HA, NP, NA, M1, M2, NS1 or NEP gene or coding sequence thereof, a parainfluenza HN, F, M, NP, P or L gene or coding sequence thereof, a rhinovirus VP1, VP2, VP3, VP4, 2A, 2B, 2C, 3A, 3B, 3C or 3D gene or coding sequence; a human respiratory syncytial virus A (HRSV-A) GA1, GA2, GA3, GA4, GA5, GA6, GA7, SAA1, NA1, or NA2 gene or coding sequence; a human respiratory syncytial virus B (HRSV-B) GB1, GA2, GA3, GB4, SAB1, SAB2, SAB3, BA1, BA2, BA3, BA4, BA5, or BA6 gene or coding sequence. These genes or coding regions are also preferred nucleic acids of interest and can be hybridized in step b) of the inventive method with the forward and reverse primers. In particular preferred embodiments of all aspects of the invention, the nucleic acids of interest or viral genes targeted by the primers, are the N gene of SARS-CoV-2, preferably bound or targeted by two different primer pairs and the influenza M1 or M2 genes.

The set may further comprise a primer pair that binds to a ribosomal gene or coding sequence or any other internal control of a nucleic acid that is expected to exist in a sample. Such a ribosomal gene or coding region or other internal control may be amplified as a control in parallel to the analyte nucleic acids and its amplification products in step b) and d). The control reaction may be contained in a separate container without analyte nucleic acids or in a container with analyte nucleic acids.

The inventive set may be provided in a kit. Such a kit may be provided in a packaging container comprising the contents of the kit. The kit may also comprise instruction for performing the inventive method.

Furthermore, the kit may comprise any components needed in the inventive method, such as a polymerase, optionally a reverse transcriptase, buffers, nucleotides, a single strand specific nuclease such as a single strand specific RNA nuclease and/or a single strand specific DNA nuclease.

Throughout the present disclosure, the articles "a," "an," and "the" are used herein to refer to one or to more than one (i.e., to at least one) of the grammatical object of the article.

As used herein, words of approximation such as, without limitation, "about", "substantial" or "substantially" refer to a condition that when so modified is understood to not necessarily be absolute or perfect but would be considered close enough to those of ordinary skill in the art to warrant designating the condition as being present. The extent to which the description may vary will depend on how great a change can be instituted and still have one of ordinary skill in the art recognize the modified feature as still having the required characteristics and capabilities of the unmodified feature. In general, but subject to the preceding discussion, a numerical value herein that is modified by a word of approximation such as "about" may vary from the stated value by e.g. ±10%.

As used herein, the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps. The "comprising" expressions when used on an element in combination with a numerical range of a certain value of that element means that the element is limited to that range and "comprising" relates to the optional presence of other elements. E.g. the element with a range may be subject to an implicit proviso excluding the presence of that element in an amount outside of that range. As used herein, the phrase "consisting essentially of" requires the specified integer(s) or steps as well as those that do not materially affect the character or function of the claimed invention. As used herein, the closed term "consisting" is used to indicate the presence of the recited elements only.

The present invention will be further described by the figures and examples, without being necessarily limited to these embodiments of the invention.

### Figures

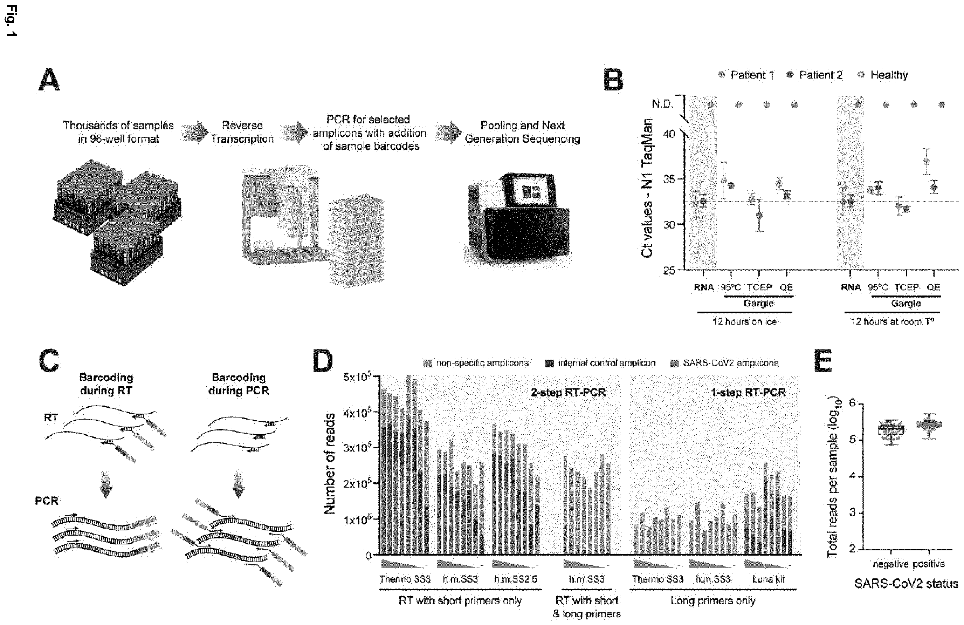

**Figure 1****: Setup of a next generation sequencing (NGS) based pipeline for the detection of Sars-CoV2 from crude respiratory samples.** A. Overview scheme to illustrate the envisioned analysis pipeline. B. Comparative analysis of SARS-CoV2 detection following different RNA extraction treatments by TaqMan qRT-PCR on the N1 amplicon. Gargle samples from two SARS-CoV2 positive and one negative person were collected in HBSS. RNA was purified (without concentrating) or crude lysates were produced using either heat inactivation for 30 min (REF), TCEP/EDTA (HUDSON buffer), or QuickExtract. C. Illustration of amplicon indexing either during RT or PCR. Colored boxes symbolize different indexes to be used in individual samples for detection during NGS. Arrows illustrate oligonucleotides hybridizing to RNA during RT or DNA in PCR. D. Comparison of amplicons obtained upon indexing in RT or PCR. Long primers containing indexes and complementary sequences of >20 bp and thus compatible with PCR were either included as RT primers already, or RT was performed with short primers, i.e., random hexamers and two N gene-specific primers binding the 3' of the SARS-CoV2 amplicon. In 1-step PCR, hot start Taq polymerase was already present during RT, while in 2-step PCR hot start Taq polymerase was added subsequent to RT. Grey triangle symbolizes a dilution series of synthetic template of 3645, 1215, 405, 135, 45, 15, 5, or 0 molecules. Thermo SS3: Superscript III enzyme provided by Thermofisher; h.m. SS3: homemade superscript III like enzyme; h.m. SS2.5: homemade advanced superscript II like enzyme; **E.** Comparison of total read counts obtained from a total of 192 samples either negative of positive for SARS-CoV2 at various concentrations.

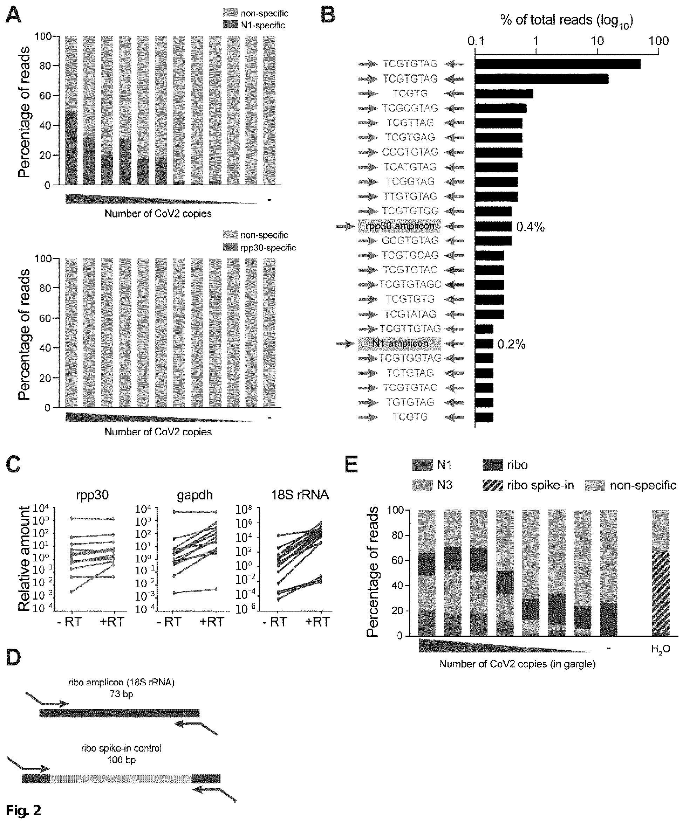

**Figure 2****: A control primer pair targeting 18S rRNA provides better specificity than the widely used RPP30 primers. A.** NGS read counts obtained with the SARS-CoV2 N gene-specific primer pair N1 as well as the human RNAse P (RPP30)-specific primer pair designed by the CDC. RT-PCR was performed on inactivated gargle-QE samples spiked with 5120, 2560, 1280, ..., 5 molecules of synthetic template in a twofold dilution series as well as 0 molecules. Non-specific amplicons are defined as amplicons generated by the respective matched primer pairs but with incorrect sequence in between. **B.** Analysis of all amplicons generated in the pool of conditions shown in A. Unspecific amplicons typically incorporated a short stretch of sequence complementary to primer sequence and were almost exclusively generated by at least one RPP30-specific primer. Specific amplicons add up to <1% of reads in this condition. **C.** SYBR Green qPCR analysis of RPP30 primers and two alternative internal control primer pairs in the presence and absence of RT on RNA purified from gargle of 16 individuals. Ct values are transformed to arbitrary units. **D.,E.** Illustration of an RT control (RTC) spike-in; An amplicon with identical primer binding sites to the ribosomal internal control yet different and longer intermediate sequence was synthesized, cloned, and T7 transcribed. It was added to the reverse transcription at 1000 molecules/reaction. The ratio of ribosomal versus RTC reads serves as a sample quality measurement, reads for neither the ribosome nor the spike-in point towards an inhibited/failed PCR reaction for the specific sample.

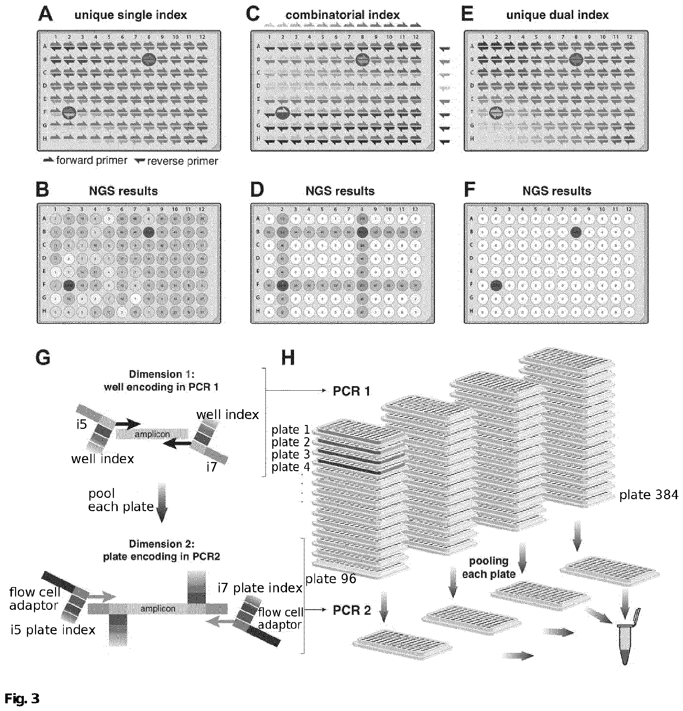

**Figure 3****: Two-dimensional redundant dual indexing allows scaling to population-level testing. A.** Scheme depicting 96 well-based indices. These can be incorporated by forward primers or reverse primers. For the latter they can be incorporated during RT or PCR. Red circles highlight the positions into which synthetic template was loaded to test performance of the indexing strategy. **B.** N3 amplicon reads obtained from next generation sequencing mapped to wells based on indices incorporated by reverse primers during PCR as in A. Forward primer indices were disregarded in this analysis. Note the frequent mis-assignment to incorrect positions. **C.** Scheme depicting combinatorial indexing. Each well is identified as a unique combination of forward and reverse index. **D.** N3 NGS reads mapped to wells based on combinatorial indexing as in C. To simulate combinatorial indexing the identical dataset as in B and F was used and primers were treated in pools pointing to columns or rows. **E.** Unique dual indexing is a redundant indexing method that encodes e.g. wells both by forward and reverse primers. Thus, illegitimate recombination products of reads can be bioinformatically rejected. **F.** NGS resuit for the same dataset as in B and D analyzed with dual indices as in E successfully filtered away all misassigned reads. G. Two-step PCR strategy to integrate redundant dual indices pointing to wells and subsequently plates. Colored boxes represent indices. H. Illustration of the PCR workflow. RT and PCR 1 are performed on all samples individually. Subsequently each plate is pooled to one well position, the amplicon is treated with Exostar to remove excess primers, and a second PCR is done. Our currently used and validated barcode set allows pooling of up to 36,864 samples. All PCR 2 amplicons are pooled, gel purified, and sequenced on any Illumina platform.

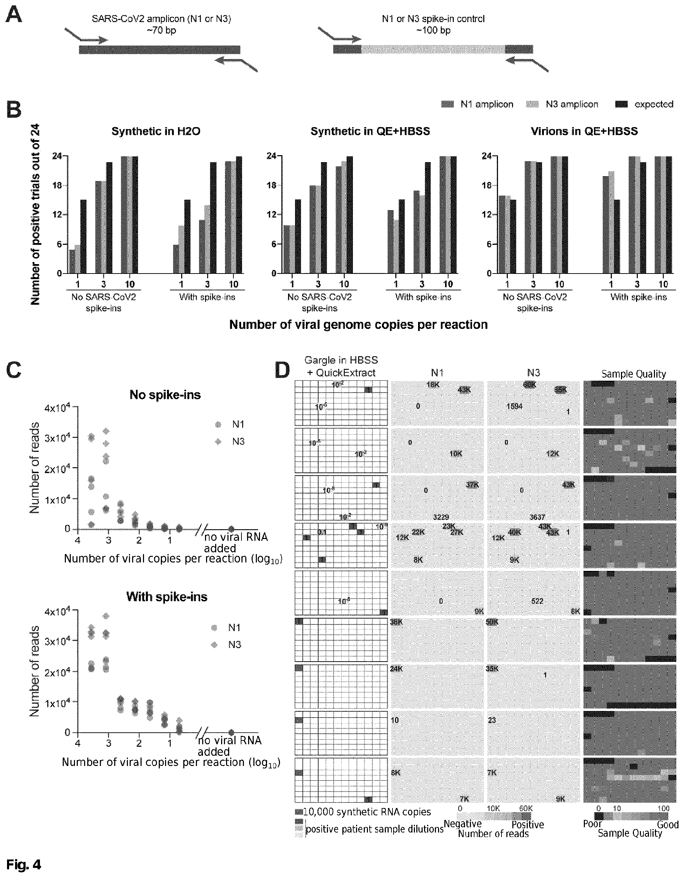

**Figure 4****: SARSeq is specific and sensitive when tested on a large set of gargle samples. A.** Schematic illustration of RNA spike-ins to scavenge N1 and N3 primers during PCR 1 in SARS-CoV2 negative samples. Analogous to the RTC, primer binding sites are identical to N1/3 amplicons, but intermediate sequence is distinct and longer so as not to compete with virus-derived amplicons. **B.** Frequency of detection of SARS-CoV2 at minimal template concentration. Number of detected cases of 24 is depicted. Note that the expected frequency of detection is only 63% and 95% for one and three molecules assuming a Poisson distribution of molecules in wells. **C.** Read counts of quadruplicates in dependency of synthetic SARS-CoV2 template. Note the reduced variability in the presence of N1/3 spike-ins. **D.** SARSeq performance on a test pool of 864 samples. Gargle samples collected in HBSS for our in-house testing pipeline of personnel were spiked with synthetic RNA or a dilution series generated from a positive sample presenting undiluted with a Ct of 30. All missed samples were 1:100,000 dilutions of that probe and thus presumably negative for SARS-CoV2. No negative positions presented with >1 read. Sample quality is assessed by the ratio or ribosomal reads to ribosomal spike-in.

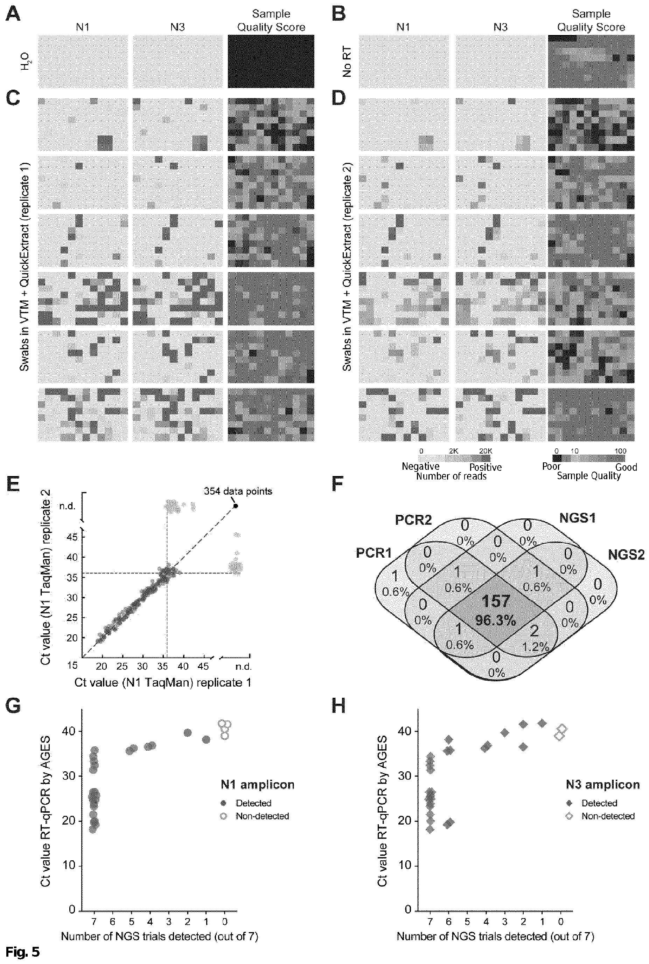

**Figure 5****: Validation of SARSeq on patient samples. A.** H₂O control plate of SARSeq with three primer pairs, namely N1, N3, and ribosome in the presence of RTC as well as N1 and N3 scavenger spike-ins. **B.** SARSeq on a gargle-QuickExtract plate in the absence of reverse transcriptase. No false positive wells were detected. Sample QC scores high due to the absence of RTC reads while DNA templated ribosomal amplicon is observed. **C.** Analysis of 576 samples obtained by swab and collected in VTM. **D.** Independent replicate of C based on the same inactivated patient swab. Color codes depict read counts and sample quality score for C and D. **E.** Two independent N1 TaqMan qPCR runs on samples as in C and D. Ct values of both runs are plotted against each other. Color code: red: sample scoring positive in both qPCR replicates, orange: sample scoring once, black: sample scoring negative. Stochastic and latest detection of SARS-CoV2 at cycle 36. **F.** Venn diagram of reproducibility for all samples scoring positive with Ct 36 or less for at least one qPCR replicate. **G, H.** Comparison of NGS results by SARSeq to Ct values obtained by diagnostic qPCR. A set of 90 samples (including swabs and gargle in different buffers) was used for RNA extraction and qPCR measurements and in parallel aliquots of these samples were mixed either with QuickExtract or TCEP/EDTA and measured by SARSeq in triplicates and quadruplicates, respectively. We report the number of replicates in which we called a sample positive by NGS (with the N1 or N3 amplicon) relative to the qPCR Ct values. Not shown are 63 samples that were negative by qPCR and NGS.

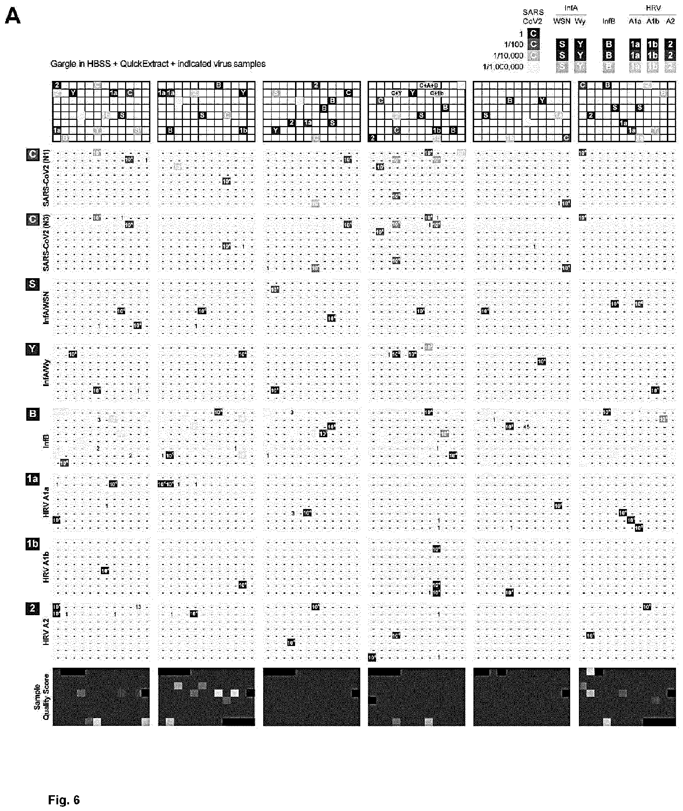

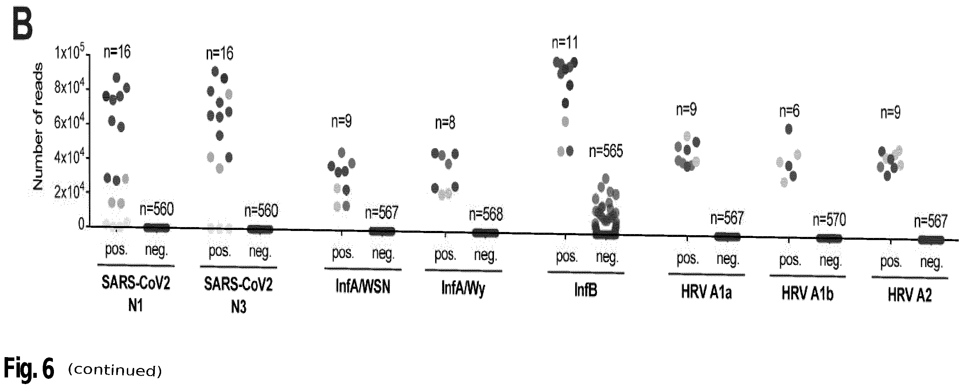

**Figure 6****: Multiplexed analysis of several respiratory viruses. A.** Six 96 well plates filled with gargle in HBSS and inactivated in QuickExtract from our in-house pipeline were spiked with RNA from various respiratory viruses. For SARS-CoV2, a positive gargle sample with Ct value of 30 was diluted as indicated. RNA for all other viruses was obtained from HEK cells 48 h after infection with the virus. Dilution indicated by voluminometric ratio. SARSeq was performed with six primer pairs in one reaction, namely N1 and N3 for SARS-CoV2, Influenza A virus, Influenza B virus, human rhinovirus (HRV), and the ribosome. Influenza A substrains and HRV substrains were distinguished based on amplicon sequence variants. **B.** Analysis of false positive and false negative rate of the experiment in panel A. As expected, 1:100,000 dilutions of the SARS-CoV2 sample with a Ct of 30 were missed. All other positive samples were detected for all viral spike-ins. False positive samples were detected for Influenza B virus presumably due to sporadic contamination of reagents or equipment with purified RNA also in the H₂0 control plate (not shown). False positive read counts did not reach numbers observed in truly positive samples. No false positive samples above threshold were detected for any other virus.

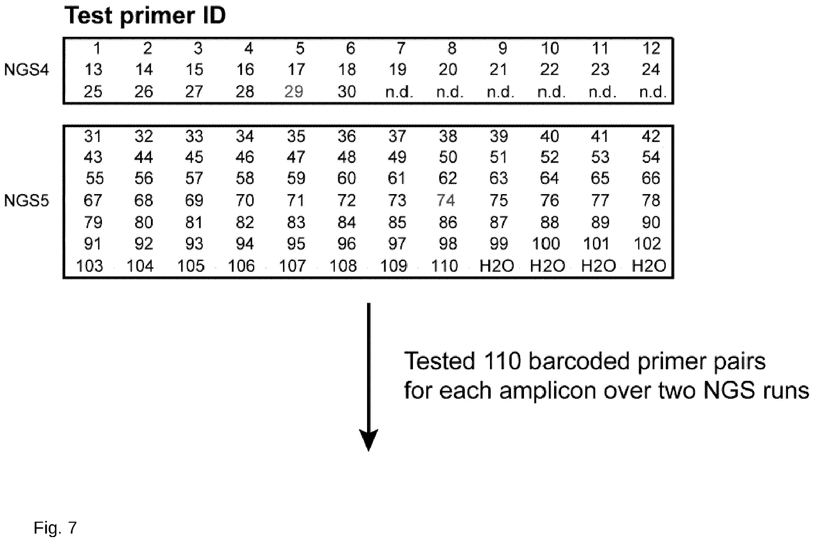

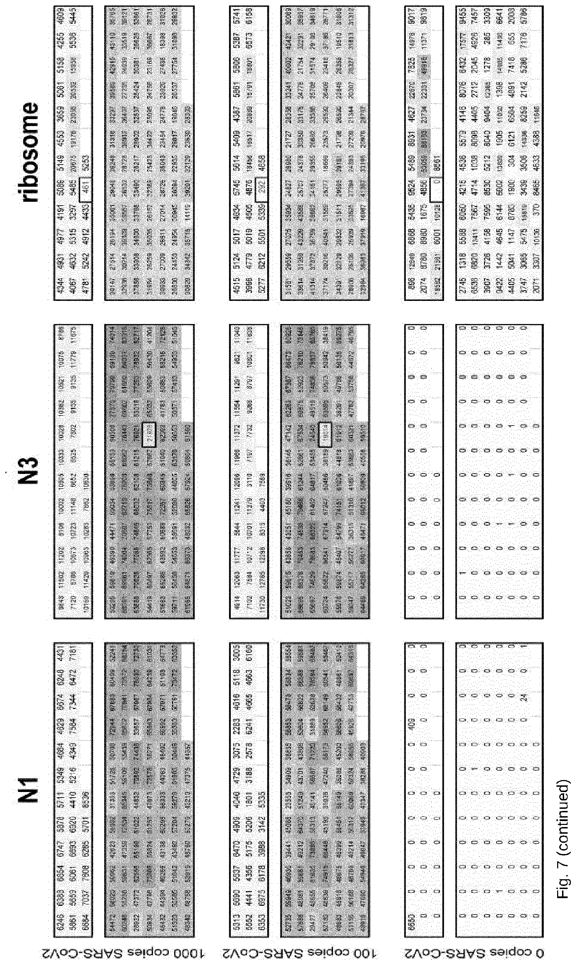

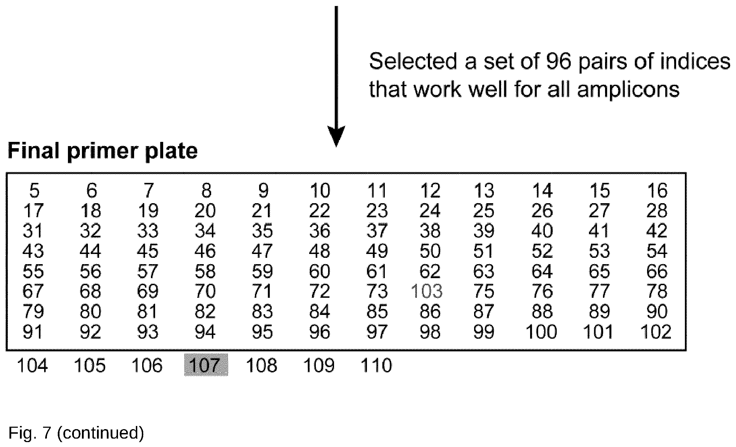

**Figure 7****:** Testing of primer pairs for three amplicons (SARS-CoV2 N1 and N3, and human ribosome) carrying extensions with 110 unique dual indexes. Primer pairs with indices #29 were excluded as they did not efficiently produce the ribosome amplicon; primer pairs with indices #74 were excluded as they did not efficiently produce the N3 amplicon. Primer pairs 1-4 can be used efficiently but were not included in the final set because these indices were frequently used in the lab during initial setup of the method and we wanted to eliminate all possible sources of cross contamination.

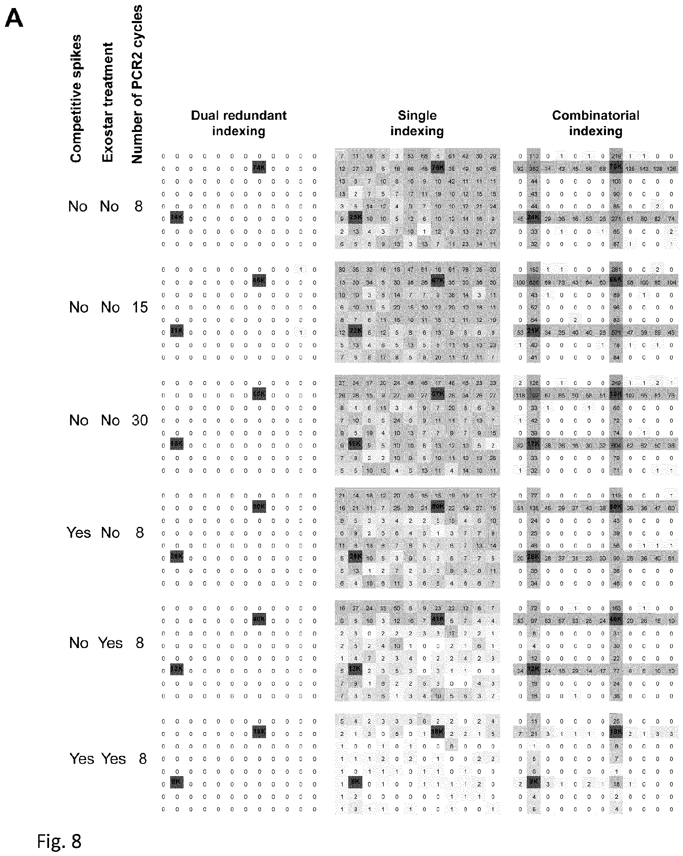

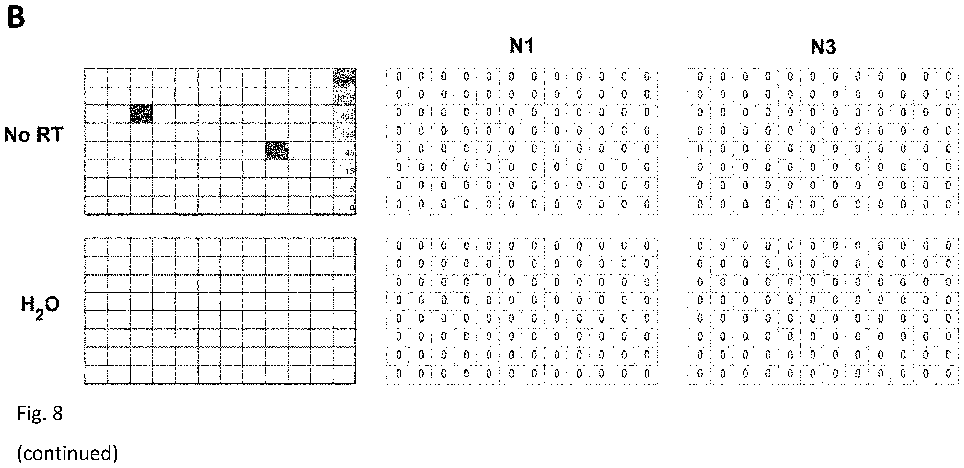

**Figure 8****: A.** Contribution of the different measures taken to completely suppress mis-assignment of indices to incorrect samples (and in that way avoid false positives). SARS-CoV2 synthetic RNA was added to positions B8 and F2. Assigning sample identities based on single indices or combinatorial indices produces a substantial amount of misassignments. These can be reduced with three independent measures: addition of a competitive spike-in and treatment with Exostar to remove any left-over primers after PCR1, and limiting the number of cycles in PCR2 to prevent recombination between amplicons and their indices. Whereas all these measures help reduce misassignments, adding dual redundant indices (or unique dual indices) completely suppresses this. **B.** Shown are N1 and N3 reads for two negative control plates (-RT and H₂O) that were run together with numerous other positive plates in the same run. The fact that we detect 0 reads shows that there is no misassignment of well identities across plates either. This is because each plate is also encoded by redundant/unique dual indices.

### Examples

The invention provides a sensitive and specific method to detect SARS-CoV2 RNAs and other nucleic acids that could be scaled to tens of thousands of parallel tests. The gold standard to detect SARS-CoV2 are RT-qPCR-based assays, which measure the abundance of short RNA fragments characteristic of SARS-CoV2 by means of DNA amplification. As PCR reactions can amplify incorrect fragments (unspecific amplicons), despite the use of specific primer pairs, RT-qPCR assays typically increase specificity by the use of fluorescently labeled TaqMan probes. This typically means that only a few different amplicons can be detected per reaction, which - together with the need for specific qPCR machines - severely limits the scalability and throughput of these assays (especially if sample-pooling is not desirable).

Next-Generation-Sequencing (NGS) can in principle allow the detection of specific amplicons by sequencing and computational analyses and is not limited in the number of different amplicons it can detect: as the amplicon identity is revealed during sequencing, multiple different fragments (viral and cellular controls) can be amplified in the same reaction if the primer pair used for the different amplicons are compatible. In addition to detecting multiple different amplicons per sample in parallel, individual samples can be uniquely labeled with characteristic sequence-barcodes, allowing the subsequent pooling and pooled sequencing. The advantages of detecting multiple amplicons per sample and processing tens of thousands of samples in parallel mean that NGS-protocols offer huge cost-saving potential and are thus highly attractive for large-scale testing.

However, while NGS-protocols are conceptually simple and indeed several different protocols have been proposed, their efficient implementation is challenged at various steps and these challenges directly impact general feasibility, costs and potential cost savings.

Here, we describe a robust high-throughput protocol based on a redundant, dual and two-dimensional barcoding strategy that achieves perfect sample-recall by redundant dual indexing while scaling to tens-of-thousands of samples by combinatorial indexing along two dimensions, termed SARSeq (Saliva Analysis by RNA Sequencing, according to one of its preferred uses). Example protocols further use a 2-step endpoint RT-PCR and using NGS-compatible amplicons and primer pairs. The protocols were validated on samples with synthetic RNAs and on patient samples, demonstrating that the protocols' utility extends to the simultaneous detection of SARS-CoV2, influenza and HRV viruses from the same sample in a single experiment. Overall, the pipeline can be efficiently combined with high-throughput sample collection in e.g. 96- or 384-well formats, robotics and NGS to detect SARS-CoV2 and other viruses in tens of thousands of samples per experiment with a turn-around time of about 1 day (Figure 1A).

### Example 1: Input sample preparation

The pipeline we describe can start from a variety of different input samples. The types of tested samples:
- Purified RNA from gargle samples
- Gargle samples mixed with QuickExtract solution form Lucigen (1:1 ratio)
- Swabs in VTM mixed with QuickExtract (1:1 ratio)
Samples mixed directly with QuickExtract were incubated for 5 min at 95°C for inactivation and used directly or stored frozen at -80°C. We did not observe a decline in positive signals upon freezing and thawing (even after two cycles of freeze-thaw).

The samples were arrayed in 96-well plates. The described reaction setup uses up to 5 µL of any of the above described samples.

### Example 2: Reverse transcription

Reverse transcription was performed with Superscript III reverse transcriptase and a primer mix containing random hexamers as well as two 12-mer oligonucleotides that prime on the SARS-CoV2 N gene.

A master mix containing all components listed below was prepared and distributed to 96-well plates (20 µL per well). Using a liquid-handling robot (or multi-channel pipettes), 5 µL of each sample were transferred to individual wells containing the RT reaction mix. RT reactions were set up at room temperature. Plates were sealed with aluminum sealing foil (It facilitates easy removal after RT reaction that reduces vibrations in wells avoiding generation of aerosols which may cause cross contamination between samples) and incubated in a thermocycler following conditions listed below.

### Master mix composition per reaction/well (volumes in µL):

| | |
|---|---|
| 10x RT Buffer^{A} | 2.5 |
| 25 mM each dNTPs | 0.5 |
| 1M DTT | 0.1 |
| RT primer mix^{B} | 2 |
| RNAse Inhibitor | 0.5 |
| Superscript III | 0.5 |
| Water | 13.8 |

Wherever mentioned, for each reaction 1000 copies of Ribosome synthetic RNA spike-in and 50 copies of each N1 and N3 RNA synthetic spike-in is included in the RT master mix. Also, wherever mentioned Thermofisher/Invitrogen^{™} SuperScript^{™} III or Luna Universal One-Step RT-qPCR Kit (NEB) or Superscript 2.5 is used for reverse transcription. In all other experiments Superscript III is used for reverse transcription.

### Thermocycler program:

5 min at 25°C (primer annealing)
15 min at 55°C (reverse transcription)
3 min at 95°C (RT inactivation)
Cool down to 12°C (removing the plate while it's still hot will cause bending of the plastic, making further pipetting and sealing more difficult)

### ^{A}10x RT Buffer composition:

200 mM Tris-HCl pH 8.3
500 mM KCl
50 mM MgCl₂
200 mM (NH₄)₂SO₄
1% Triton X-100

*^{B}RT primer mix composition:* 12.5 mM of each random hexamers, N-gene specific 12-mer #1 and N-gene specific 12-mer #2 (final concentration in the complete 25 µL RT reaction is 1 mM each)

### Example 3: In vitro transcription of spike-in synthetic RNA templates

Ribo, N1 and N3 spike-in DNA fragments with unique identification sequences in the middle and away from primer binding sites are ordered from IDT or Microsynth. Spike-in templates are cloned into pCR2.1 plasmid by topo cloning. Spike-in template containing plasmid clones are confirmed with Sanger sequencing. For efficient in vitro transcription, plasmids are linearized downstream of the T7 promoter and spike-in template by cutting with a unique restriction enzyme. *In vitro* transcription is carried out using NEB HiScribe^{™} kit according to manufacturer's instructions. Transcribed reaction was treated with Turbo DNAse/Thermofisher for 1 hr and RNA is purified using Zymo RNA clean and concentrator spin columns. RNA is aliquoted and stored at -80C.

### Example 4: First PCR

A first PCR was performed for amplification of cDNA with addition of sample indexes as dual barcodes.

A master mix containing all components listed below, including HotStart Taq Polymerase and Uracil DNA glycosylase (Antarctic Thermolabile UDG from NEB/M0372L) was prepared and distributed to a deep-well 96-well plate. The 96 primer pair combinations^{C} containing dual well barcodes were also arrayed in 96-well plates (multiple primer plates can be prepared simultaneously and stored frozen at -20°C). Using a liquid-handling robot, the 96 sets of barcoded primers were added to the PCR master mix and mixed thoroughly. 25 µL of this complete 2x PCR mix were added to the 25 µL RT reactions prepared as above. Plates were sealed with aluminum sealing foil and incubated in a thermocycler following conditions listed below.

All components were kept at room temperature during reaction set up; together with the first step in the thermocycler, a 10 min incubation at 30°C, this provides the right conditions for UDG to act on Uracil-containing amplification products of previous PCR reactions, thereby removing spurious carry over contaminants. After UDG heat inactivation, the subsequent PCR reaction was again carried out in the presence of UTP to prevent carry over contamination in following runs.

### Forward primers:

1^{st} set for the SarsCoV2 N gene (N1): SEQ ID NO: 1 to 96
2^{nd} set for the SarsCoV2 N gene (N3): SEQ ID NO: 97 to 192
Set for rRNA: SEQ ID NO: 193 to 288
Set for influenza A: SEQ ID NO 289 to 384
Set for influenza B: SEQ ID NO: 385 to 480
Set for human rhino virus: SEQ ID NO: 481 to 495

All forward primers contain the same 5' part containing the adaptor sequence (CTACACGACGCTCTTCCGATCT - nt 1 to 22 of any of SEQ ID NO: 1 to 495) followed be the staggering nucleotides, i.e. 1 to 4 N, which are randomly selected from A, C, G, or T, followed by the sample identifier sequence, which is different for all primers within a given amplicon-specific set, here a 8-mer for all of SEQ ID NO: 1 to 495 (e.g. CCAATACT, nt 24-31 of SEQ ID NO: 1), followed by a binding sequence for hybridization to the analyte nucleic acids; e.g. for N1: gaccccaaaatcagcgaaat (nt 32 to 51 of SEQ ID NO: 1); for N3: gggagccttgaatacaccaaaa (nt 32 to 53 of SEQ ID NO: 97); for rRNA: ggcattcgtattgcgccgcta (nt 32 to 52 of SEQ ID NO: 193); for influenza A: CTCATGGART-GGCTAAAG (nt 32 to 49 of SEQ ID NO: 289); for influenza B: GCCTTCTCCATCTTCTG (nt 32 to 48 of SEQ ID NO: 385); for human rhino virus: TCCTCCGGCCCCTGAAT (nt 32 to 48 of SEQ ID NO: 481).

### Reverse primers:

1^{st} set for the SarsCoV2 N gene (N1): SEQ ID NO: 496 to 591
2^{nd} set for the SarsCoV2 N gene (N3): SEQ ID NO: 592 to 687
Set for rRNA: SEQ ID NO: 688 to 783
Set for influenza A: SEQ ID NO 784 to 879
Set for influenza B: SEQ ID NO: 880 to 975
Set for human rhino virus: SEQ ID NO: 976 to 990

All reverse primers contain the same 5' part containing the adaptor sequence (GTGACTGGAGTTCAGACGTGTGCTCTTCCGATCT - nt 1 to 34 of any of SEQ ID NO: 496 to 990); followed by the sample identifier sequence, which is different for all primers within a given amplicon-specific set, here a 8-mer for all of SEQ ID NO: 1 to 495 (e.g. AGCTTGGT, nt 35-42 of SEQ ID NOs: 496 to 990), followed by the binding sequence for hybridization to the analyte nucleic acids; e.g. for N1: TCTGGTTACTGCCAGTTGAATCTG (nt 43 to 66 of SEQ ID NO: 496); for N3: tgtagcacgattgcagcattg (nt 43 to 63 of SEQ ID NO: 592); for rRNA: GGCAAATGCTTTCGCTCTGGTC (nt 43 to 64 of SEQ ID NO: 688); for influenza A; TGGACAAANCGTCTAC-GCTGCAG (nt 43 to 65 of SEQ ID NO: 784); for influenza B: GTCGCTGTTTGGAGACAC (nt 43 to 60 of SEQ ID NO: 880); for human rhino virus: GAAACACGGACACCCAAAGTAGT (nt 43 to 65 of SEQ ID NO: 976) .

Staggering distancing nucleotides are only used for the forward but not the reverse primer since their effect is only needed on one side of the amplifications products to have their effects during sequencing.

### Master mix composition per reaction/well (volumes in µL)

| | |
|---|---|
| 10x PCR top up Buffer^{D} | 2.5 |
| 100 mM dUTP(NEB) | 0.07 |
| HotStart Taq Polymerase | 0.5 |
| Antarctic thermolabile UDG | 0.5 |
| Water | 16.43 |

### Thermocycler program:

10 min at 30°C (for high UDG activity)
3 min at 95°C (UDG inactivation and HotStart Taq activation)
45 cycles of: 20 sec at 95°C, 30 sec at 58°C, 20 sec at 72°C
2 min at 72°C
Cool down to 12°C (removing the plate while it's still hot will cause bending of the plastic, making further pipetting and sealing more difficult)

*^{c}PCR primer mix composition:* 2 mM of each forward and reverse primer, for all viral amplicons and 1 mM of each forward and reverse primer for the rRNA amplicon (final concentration of each primer pair in the complete 50 µL reaction is 200 and 100 nM, respectively)

### ^{D}10x PCR Top Up Buffer composition:

750 mM Tris-HCl pH 8.3
200 mM (NH₄)₂SO₄
1% Triton X-100

### Example 5: Plate pooling

All well-barcoded PCR products from a single 96-well plate were pooled, typically 20 µL of each reaction was combined in a plastic reservoir using a multi-channel pipette, and after mixing thoroughly 1 mL was transferred to an Eppendorf tube. This was repeated for every PCR plate. 5 µL from each plate pool were re-arrayed in a new 96-well plate and treated with 2 µL of il-lustra ExoProStar 1-step for 30 min at 37°C followed by 15 min at 80°C to remove any left-over primer.

### Example 6: Second PCR

Subset indices are added (plate barcodes) with sequencing adaptors. A master mix with all components listed below was distributed across a 96-well plate (37.5 pL/well). To each we added 10 µL of unique dual-indexed i5/i7 primer pairs (Custom synthesized index primers with Nextflex barcodes, arrayed in 96-well plates) and 2.5 µL of Exostar-treated PCR1 pool. The reactions were run for 8 cycles to add sequencing adaptors with plate barcodes.

### Master mix composition per reaction/well (volumes in µL)

| | |
|---|---|
| 10x Sequencing-ready PCR Buffer^{E} | 5 |
| 25 mM each dNTPs | 0.5 |
| 100 mM dUTP | 0.07 |
| Hot start Taq Polymerase | 0.5 |
| Water | 31.43 |

### Thermocycler program:

3 min at 95°C
8 cycles of: 15 sec at 95°C, 30 sec at 65°C, 30 sec at 72°C
2 min at 72°C
Cool down to 12°C

### ^{E}10x Sequencing-ready PCR Buffer composition:

750 mM Tris-HCl pH 8.3
200 mM (NH₄)₂SO₄
20 mM MgCl₂
0.1% Tween 20

### Example 7: Pooling and preparation for sequencing

All samples from a 96-well plate (20 µL from each well) were pooled and 250 µL of pooled sample is resolved on 2% agarose gel and 220 bp to 260 bp size amplicons are excised and gel purified using Qiagen gel extraction kit.

In order to ensure fast turnaround, the preparation of libraries for Illumina sequencing was optimized empirically. In the first four sequencing runs, standard quality control of the library, including Qubit measurement, a size analysis and qPCR, was performed. A correlation between the concentration measurement by Qubit and the qPCR was detected. In every case the molarity determined by qPCR was 10x higher than the concentration measured by Qubit. Thus, we can omit the size analysis and the qPCR, which are both time consuming. Eventually the library concentration is determined by 3 independent Qubit measurements, the obtained value in ng/µL is multiplied by 10 and used as the molarity of the sample in nanomolar. This procedure enables us to start the sequencer within a 15 min time-frame after receiving the sequencing library. Final preparation of the sequencing run happens according to Illumina's guidelines, including denaturation of the sample, neutralization and final dilution for sequencing.

### Example 8: Sequencing

Depending on the sequencer type, the following concentrations were used for sequencing: 10 pM for MiSeq V2 chemistry, 15 pM for MiSeq V3 chemistry, 2.2 pM for NextSeq550 high output and 1.3 pM for NextSeq550 medium output. In every sequencing run 10% of PhiX library were spiked-in to increase complexity. To avoid contaminations with barcodes from previous sequencing runs, the sequencers were washed with bleach according to Illumina's guidelines before every run.

### Example 9: Data analysis

The NGS data (fastq.gz files) were mapped in a single pass to sets of expected amplicon sequences and to sets of expected well- and plate indices. While any standard NGS workflow and read-mapping software (e.g. bowtie or similar) would be suitable, we used dedicated shell and awk scripts based on stringhashing that allows for 0 or 1 mismatch per amplicon and index. The i5 and i7 indices signified the plate-indices (subset index) and parts of the forward read (and the reverse read in the case of paired-end sequencing) signified the well-indices (sample index); we required the correct redundant encoding of plate and well. As the well-index on the forward read started at random offsets, we first determined the amplicon identity and position, then inferred the position of the well index, and finally compared the well index to the intended well indices.

### Example 10: Direct sample processing without RNA extraction

Many current pipelines for detection of viral RNA begin with purification of RNA from biological material (swabs, saliva, gargle, etc). However, to lower costs and match the throughput of an NGS-based pipeline, RNA extraction was bypassed. Typically, samples are collected in viral transport medium (VTM). Additionally, saliva represents an ideal viral transport medium and is now frequently used as input for SARS-CoV2 testing pipelines. We found that gargling of 10 ml HBSS is compatible with self-sampling. Gargle samples enable similar sensitivity to swabs collected by medical staff and are preferred to pure saliva as samples become more uniform in viscosity and are thus easier to pipette, making the samples better suited for automation **(****Figure 1A****).** Several approaches to stabilize viral RNA from respiratory specimens, circumventing RNA purification and enabling the required scalability are known (swab-seq (Bloom et al., doi.org/10.1101/2020.08.04.20167874), Salivadirect (Vogels et al., doi.org/10.1101/2020.08.03.20167791), Quick extract (Ladha et al., doi.org/10.1101/2020.05.07.20055947), HUDSON buffer (Lalli et al., doi.org/10.1101/2020.05.07.20093542)). To compare these methods, we mixed retroviral particles packaged in cell culture into gargle samples and then treated the samples according to the different protocols: i) 30 min at 95°C (swab-seq), ii) 5 min at 95 after addition of Proteinase K (Saliva-direct), iii) 1:1 mixture with QuickExtract and 5 min at 95°C, and iv) lysis and RNA extraction. We then assessed efficiency of RNA unmasking and its stability before and after treatment, by performing quantitative RT-PCR of a virus specific amplicon. We confirmed that all these methods generate stable RNA that are compatible with RT-PCR. DNA QuickExtract, however, showed the least precipitation of VTM upon boiling and was thus used for most experiments unless otherwise stated **(****Figure 1B****).**

### Example 11: 2-step RT-PCR has increased specificity compared to 1-step RT-PCR

The amplification of viral genome fragments for sequencing consists of RT and PCR steps. Indexes pointing towards individual samples can be incorporated during the reverse transcription (RT) step as well as during PCR **(****Figure 1C****).** However, we found that introduction of sample barcodes in the RT step resulted in an increased fraction of non-specific PCR products as compared to their introduction at the PCR step, presumably because the higher temperature of PCRs constitute more restrictive and thus specific conditions **(****Figure 1D****).** Given the large but limited sequence space on NGS flow cells, such lack of specificity means that many unspecific amplicons would be sequence, which interferes with upscaling to very large sample numbers. We therefore settled on integration of positional indexes during PCR only. Specifically, individual samples are arrayed in individual wells of 96-well plates in which the RNA from each sample is first subjected to reverse transcription (RT) and then to amplification by PCR to produce diverse desired viral and control amplicons. Priming in the RT step is performed with random hexamers as well as two gene specific twelve-mers that increase sensitivity for Sars-CoV2.

### Example 12: Compression of dynamic range by 45-cycle end-point-PCR enables sensitive detection of samples with low viral titers

One of the hurdles towards establishing a pooled NGS-based assay for samples from virus-infected individuals derives from the fact that viral loads can differ by many orders of magnitude such that high-titer samples would dominate NGS. TaqMan RT-qPCR reports differences in Ct values of 20-25 cycles, which translate into 2²⁵=33.5 million-fold differences in viral titers. Therefore, if samples with low virus titer are to be robustly identified as positives, e.g. with >100 virus-derived amplicon reads, the samples with high virus titers required 3.3x10⁹ reads, which is prohibitive. Therefore, the dynamic range needs to be compressed, in order to dampen the signal from a highly positive sample at the expense of providing sufficient sensitivity to detect samples with lower titers. In order to achieve this compression of signal, we run the first PCR reaction for 45 cycles until each individual sample reaches saturation. This results in the generation of similar numbers of amplicons per well independent of the initial viral titer **(****Figure 1D,E**). In summary, using crude respiratory specimens as input, a 2-step RT-PCR generates high specificity and even representation of characteristic amplicons across samples to enable pooling of many samples for analysis by NGS.

### Example 13: Selection of viral and human control amplicons

In addition to the very large dynamic range of viral titers between patients, nonspecific PCR amplicons can severely impact the detection of viral amplicons by NGS, because the number of NGS reads is inherently limited (and directly proportional to the total costs). For example, the parallel analysis of approximately 40K (96x384) samples means that each sample can receive a total of only ∼500 reads on a MiSeq, ∼2K on a HiSeq, and ∼10K on a NextSeq platform. If a substantial fraction of these reads were spent on sequencing nonspecific amplicons, assay sensitivity would be severely impacted. It is thus pivotal to select amplicons and primer pairs that i) show high sensitivity ii) generate amplicons of comparable short size iii) generate few non-specific amplicons alone or in combination with any other primer pairs present in the same reaction, which is of particular importance when using primers with long extensions (here: sample-identifying barcode sequences and primer-binding sites for a 2nd PCR as discussed below).

### Example 14: 18S-rRNA-specific primer pair to control for sample presence and quality

We tested several published Sars-CoV2 specific primer pairs for the performance with our DNA overhangs. The Sars-CoV2 N-gene-specific primers N1 and N3 proposed by the Center for Disease Control (CDC), both being specific for the SARS-CoV2 N gene, performed best in SYBR-Green qPCR (which does not control for amplicon identity) as well as in initial sequencing runs, and had an ideal amplicon length of ∼70. We also tested the N1 amplicon together with the widely used internal control primer pair targeting RPP30 (RNAse P). While the N1 primers showed a fair amount of correct amplicons in dependence of the amount of synthetically spiked in template, the fraction of specific amplicons for RPP30 was only 0.06-1.5% **(****Figure 2A****).** When analyzing all observed amplicons across conditions, we detected various short sequences that together used up >99% of all NGS reads. The vast majority was generated by the RPP30 specific primers **(Figure 2B),** suggesting that these primers are not compatible with multiplexed PCR and NGS. We therefore set out to establish a new control primer pair that would produce much fewer nonspecific amplicons. We tested several primer sets on gargle samples obtained from 16 individuals, yet only a single primer pair, specific for 18S ribosomal RNA, was detected in all samples and showed a strong dependency on the presence of reverse transcriptase **(****Figure 2C****).** We therefore chose this 18S ribosomal RNA specific primer pair to control for sample quality. Of note, ribosomal amplicons were detected at Ct values of 15-45 and therefore in a comparable range as viral amplicons in patients. We therefore tested ribosomal RNA as host control in the SARSeq panel. Indeed, the ribosomal primer pair/amplicon selection performed well and neither generated abundant unspecific amplicons alone, nor in combination with N1 or N3 primer pairs **(****Figure 2E****)** .

### Example 15: Synthetic spike-in RNA controls for RT-PCR performance

Since the 18S amplicon - like the RPP30 amplicon - does not span an intron it cannot discriminate against genomic DNA templates abundant in respiratory samples. In particular, QuickExtract processed gargle did not show a dominant RNA based signal even with ribosomal primers **(****Figure 2C****).** This is maybe expected since very few living cells will shed during the gargle procedure and released RNA will also not survive for extended periods of sample storage. In contrast, viral particles with genomic RNA are shed efficiently and maintained well in particular in buffered isotonic conditions such as viral transport medium (VTM), saliva, and gargle in HBSS to protect viral RNA from very abundant RNAse activity in respiratory specimen REF. To integrate an additional internal RNA control to assess successful reverse transcription in all probes, we therefore included RNA with identical primer binding sites as the ribosomal amplicon, yet unrelated sequence in between. This in vitro transcribed RNA was spiked into the reverse transcription mix at a concentration of 1000 molecules/reaction **(****Figure 2D****).** Using the RT- and PCR buffers (Examples 2, 3 and 6) SARSeq reached a very good amplicon specificity with the majority of amplicons **(****Figure 2E****).** The ratio of reads for the ribosomal amplicon and spike in serves as a very good assessment of sample quality. The high specificity of amplicons set the stage to develop a high throughput indexing strategy that allows to analyze tens of thousands of samples in parallel.

### Example 16 - comparative example: Simple sample indexing strategies don't retain sample identity

To exploit the high-throughput nature of NGS, we designed a sample barcoding strategy that allows multiplexing of tens of thousands of samples for a single sequencing run while retaining perfect sample specificity, i.e. suppressing misassignment of reads to incorrect samples. Retaining perfect sample specificity is crucial in the context of samples from healthy individuals (no viruses) and from patients with the abovementioned very wide range of virus titers. Several strategies for sample indexing are currently available that make use of the possibility that NGS can not only determine the amplicon identity but any other information on the amplicon, including sample-specific barcode sequences or indices. First, samples can be individually indexed by a sample-specific barcode in one of the two primers, i.e. in the forward or in the reverse primer **(****Figure** 3A). In such a setup, sample-specific primers incorporate sample-indexes into the amplicons of each sample that can be detected in NGS (REF e.g. the 19000RT barcodes and swab seq 2.0). This strategy however does not scale well as it requires distinct primer pairs for each amplicon and sample (linear/additive scaling). More importantly, it cannot retain perfect sample identity due to template-switching PCR artifacts and index-hopping on flow cells, which can lead to random associations between amplicon- and index sequences. This problem is of particular relevance when high-titer samples are analyzed in parallel with samples from healthy individuals as we demonstrated by spiking synthetic Sars-CoV2 template into two wells of a 96 well plate (wells B8 and F2) in which all other wells are negative (**Figure 3B**). Simple combinatorial indexing with a column barcode on one primer and a row barcode on the other primer can overcome the scalability problem (swab seq 1.0) (combinatorial/multiplicative scaling; **Figure** 3C), but suffers from the same inability to retain perfect sample identity, which in this case leads to a characteristic cross-shaped pattern along the rows and columns of the positive samples **(****Figure** 3D).

### Example 17: Two-dimensional redundant dual indexing is scalable and retains sample identity

We implemented redundant, dual indexing (also referred to as "unique dual") that retains perfect sample identity by redundantly encoding sample identity on both ends of the amplicon, thereby eliminating illegitimate index combinations **(****Figure 3E****,F,G).** We achieve this by two indices introduced through forward and reverse primers that both point to the same sample/position (Figure 3E, G). Such an approach requires two indices (=unique primers) per sample **(****Figure 3E****)** and therefore does not scale well when a single PCR is used (1 dimension). We therefore use a two-dimensional indexing strategy by adding a second PCR step that again uses redundant, dual indexing, yet allows fully combinatorial indexing between dimension 1 and 2 (combinatorial/multiplicative; **Figure 3G****).** This strategy of two-dimensional redundant dual indexing has only a modest requirement to increase indexing primer numbers for very large panels of parallel samples and allows the encoding of 96x96 or 96x384 samples with 2x96 amplicon-specific primers (2 per amplicon; 1st dimension) plus 2x96 or 2x384 global primers (irrespective of amplicon; 2nd dimension), respectively, when using standard 96-and 384-well plates, for example. Greater number of indexes/primers are easily scalable due to the multiplicity of the 2-step index strategy, which presents a robust and scalable indexing strategy.

### Example 18: A set of 96 validated primer pairs per amplicon for the 1st dimension

In practice, we extended the amplicon-specific primers for the 1st PCR (1st dimension - sample indexing) at their 5' ends to include a sample-specific barcode and i5/i7 sequences as primer-binding sites for the 2nd PCR. To ensure sufficiently complex sequences of the NGS forward reads for cluster identification and stable sequencing, we staggered the sample-barcode and the amplicon-specific sequence by a random offset of 1-4 base pairs in the forward primers - see "n" nucleotides in SEQ ID NO: 1 to 495 **(see** **Figure 3G** **and Example 4).** We tested a total of 110 primer pairs per amplicon to establish a set of 96 primer pairs that show good amplification behaviour for all amplicons **(****Figure 7****).** Importantly, all amplicons within one well obtain the identical stagger and index to also prevent recombination between amplicons during PCR. Pre-prepared primer-plates and robotic pipetting pipelines allow us to process thousands of samples in parallel.

### Example 19: Perfectly retaining sample identity across the two dimensions

After the indexing of individual samples (=wells of a 96-well plate; 1st dimension), all samples of one plate are pooled to one position in a new 96-well plate, and in a second PCR, a plate specific index is added (2nd dimension). We implemented four measures to ensure that sample identity is perfectly retained between the 1st and 2nd PCR. i) To avoid that primers from the 1st PCR are present during the 2nd PCR, we included an RNA template with N1 and N3 primer binding sites similar to our RT control and the denominator used in the Swab-Seq pipeline to suppress stochastic contaminations. In addition, we ii) treated the pools with DNA exonuclease to enzymatically degrade all single-stranded DNA and thus all remaining primers especially from Sars-CoV2 negative wells. iii) We keep the cycle number for PCR 2 at a minimum to avoid amplicon recombination during PCR and use a PCR protocol that prevents premature termination of an extension step. Indeed, the measures synergistically contributed to the robustness of read assignment **(****Fig. 8A****).** iv) In each dimension we use dual and redundant indices with a Lowenstein distance of at least three mismatches. This pipeline also suppressed read misassignment across plates, such that the positions of Figure 3A, C, E were not detected on other plates run in the same experiment **(****Fig. 8B****).**

We thus established a scalable and robust indexing for Sars-CoV2 by setting up a PCR-based indexing-strategy, that incorporates two sets of redundant indices and we bioinformatically filter to only allow legitimate combinations of those four indices. The ability to encode 96 well barcodes and 384 plate barcodes means this can be used to investigate 36,864 individual samples simultaneously. To illustrate scalability of the approach, a 4-fold increase in each dimension for example by using 384-well plates would enable multiplexing of >145,000 samples. Sequencing capacity is thus no longer a bottleneck to frequent population wide testing for many countries and the sequencing price is negligible. This indexing systems allows to filter against any DNA remnants in sequencing flow cells, that can result in read misassignment between independent analysis runs. In summary, dual redundant indexing introduced during PCR 1 ensures sample identity specificity and two-dimensional indexing allows scalability while preventing any spill of reads from positive to negative samples even across multiple orders of magnitude in signal intensity. We termed this direct analysis of respiratory samples using next generation sequencing SARSeq for "Saliva Analysis by RNA Sequencing".

### Example 20: Specificity and Sensitivity of SARSeq

To test sensitivity, specificity and scalability of SARSeq we set out to run large sample cohorts. To assay under close to real-life conditions we prepared samples plates and processed them in parallel using a robotic pipetting platform. We also tested the effect of spike-ins with identical primer binding sites to the N1/N3 amplicons but different sequences in between, as introduced previously.

Initially we tested sensitivity of the SARSeq pipeline by diluting viral templates to 1, 3, or 10 copies per 5ul probe volume (2-0.2 copies/ul). To account for the contribution of Quick Extract as input buffer to the pipeline as well as to test RNA extraction with this method we tested above dilutions with synthetic RNA in H₂O as well as Quick Extract mixed to HBSS and also assayed viral particles in Quick extract. Using H₂O for dilution we were able to detect Sars-CoV2 in 5 and 6 of 24 tested cases for N1 and N3 respectively at a dilution of in average 1 amplicon per well **(****Figure 4A****).** At such dilution 63% of wells are expected to contain one or more viral templates assuming a Poisson distribution pointing towards a detection efficiency of 0.3-0.4 per molecule. Quick Extract increased that efficiency to 0.7 while detection straight from viral particles was at 1.1 per molecule **(****Figure 4B****).** Presence of N1 and N3 denominator spike-ins even showed slightly better detection efficiencies. It is assumed that infectious Covid-19 patients show with detectable viral titers of >10³/µL. Our detection limit is thus at least 100 times more sensitive than required for mitigation of the current Sars-CoV2 pandemic. Next, we tested the effect of spike-ins on the quantitative behavior of SARSeq **(****Figure 4C****).** Due to the endpoint PCR we perform, SARSeq intentionally only returns semiquantitative results. However, when testing a dilution series of synthetic spikes in quadruplicate we noticed an improved reproducibility in presence of these spike-ins and therefore included them in the final setup.

To challenge SARSeq with hundreds of real samples omitting RNA purification, we next generated sample plates from gargle samples collected in HBSS. Such crude samples may contain reagents inhibitory to the RT or PCR step. To this end, we generated a dilution series of synthetic template and also spiked a dilution series of a subject or patient sample presenting with Ct=30 in a taqman Q-PCR assay into several positions. Indeed, when testing six 96-well plates, we were able to detect all patients previously detected with an RNA purification followed by a Taqman-based qPCR. In addition, we saw all patients and synthetic spike-ins that we had added prior to running the pipeline. Overlap between N1 and N3 amplicon-based results was 100% **(****Figure 4D****).**

Another critical parameter when testing very large numbers of patients is the false positive rate. We were therefore extremely pleased to see that our indexing strategy and pipeline delivered typically zero and very rarely 1 read indicative of SARS-CoV2 for gargle of persons previously tested negative by Q-PCR as well as all H₂O controls. This binary result showcased an unambiguous assessment of infection status by SARSeq. Due to the absence of false positive results we did not need to use our denominator amplicons to set a threshold for signal strength for positive results. However, we envision that they could be a powerful method to reduce stochastic reads if incorporated at sufficiently high concentration to reduce sensitivity.

### Examle 21: Performance of SARSeq on large panels of patient samples

To test if SARSeq would robustly detect real SARS-CoV2 virus in VTM samples collected in clinical diagnostic pipelines of many independent patients, we tested a set of 564 samples with SARSeq in duplicate. While we did not detect N1 or N3 amplicons in H₂O or -RT conditions **(****Figure 5A, B**) we frequently obtained SarsCoV2 reads across all plates with good correspondence between amplicons and replicates. **(****Figure 5C, D****).** To assay correspondence to the standard test, we also measured the samples in a Taqman Q-PCR assay in duplicates. Expectedly we found a very good correspondence of both replicates for Ct values below 36 **(****Figure 5E****, red dots)** and a stochastic behavior at the detection border **(****Figure 5E****, orange dots)** with either one or two replicates scoring positive. No Sars-CoV2 was detected in an additional 354 samples. We analyzed the overlap between detection in qPCR- and NGS-based assays and found that 157 or 96.3% of samples that scored with a Ct value of <36 in at least one qPCR in fact scored positive in all four assays while six samples were detected with one or two assays only **(****Figure 5F****).** We hypothesized that the stochastic behavior at detection border is due to presence or absence of a single viral genome in the reaction. If that was true, then correspondence between amplicons of a single replicate is expected to be better than correspondence per amplicon between replicates even though N1 and N3 might show different sensitivities. Indeed, of the samples detected with 1-3 assays, 48 and 43 samples show detection of Sars-CoV2 with both amplicons within one replicate while only 18 and 25 samples are detected twice independently with N1 or N3, respectively. We thus conclude that SARSeq is sensitive down to single viral genomes. We plotted read counts of both SARSeq replicates against each other to visualize read count reproducibility **(****Figure 5G, H****).** As anticipated, we see a lower correlation than with qPCR, some samples undetected in NGS but present in one qPCR run at any Ct, as well as the detection of some samples missed by one or both Q-PCRs. We also observe a distinct upper end of N1/N3 read counts attributed to the intentional blunting of reads in end point PCR to distribute sequencing space evenly across samples (see also Figure 1E). Taken together, SARSeq was able to reproducibly identify Sars-CoV2 across a patient cohort of 564 samples with high reproducibility and a sensitivity to single molecule level.

### Example 22: Broader applicability to detection of respiratory infectious agents

Multiple infectious agents cause disease with overlapping clinical symptoms, foremost Influenza A and B. Moreover, it is expected that particularly in the winter season, various respiratory symptoms will cause concerns for patients (even if clinical symptoms are not strictly overlapping) and thereby dramatically increase the demand for reliable/sensitive Sars-CoV2 tests. Most prominently, we anticipate that rhinoviral infections will remain frequent even in measures of social distancing as a typical winter season brings several episodes of rhinoviral infection per person. For SARSeq additional amplicons come at no extra cost if it doesn't increase sequencing depth requirements. Therefore, we can further multiplex SARSeq to detect in addition other common respiratory viruses found in the same type of sample as SARS-CoV2.

To accommodate for the aforementioned RNA viruses, we sought to optimize primers for influenza A, B, and rhinovirus to be combined with our Sars-CoV2 specific SARSeq pipeline. To this end we selected primers based on qPCR performance, amplicon length, and an NGS pilot. For a pan-influenza A amplicon we settled on combining a degenerated forward primer from Bose et al. (J Clin Microbiol 47(9), 2009: 2779-2786) with a degenerate WHO reverse primer, both targeting the M gene. For pan-influenza B we selected a primers (see example 4) of SEQ ID NO: SEQ ID NO: 385 to 480 (forward primers) and SEQ ID NO: 880 to 975 (reverse primers) to the M gene. Rhinovirus is detected using a primer pair of Do et al. (J Mol Diagn. 2010; 12(1): 102-108).

To test performance across a large number of specimen we took gargle-Quick extract samples and spiked in purified RNA obtained from HEK293T cells infected with respective virus strains 48 h prior purification at a voluminometric ratio of 1:100 or dilutions thereof. Samples were processed using the robotic pipeline. We performed PCR1 in the presence of six primer pairs, two against Sars-CoV2, and one each for ribosomal control, influenza A, influenza B, and rhinovirus. Upon pooling and PCR2, samples were sequenced by paired end sequencing to account for the presence of longer amplicons, and reads were mapped to wells/samples **(****Figure 6B****).** Of note, by pipetting spike-in RNA prior to setting up the RT reaction we apparently generated an RNA contamination of influenza B that was stochastically distributed across all reactions (including H₂O controls but not -RT controls) and obtained reproducibly lower read counts than spike in samples. This event emphasizes the importance of a local separation of reaction mix preparation and sample handling. All post-PCR steps were always performed in a different lab from the beginning and thus DNA amplicons did not generate detectable contamination of our pipeline. Nevertheless, we implemented incorporation of UTP and a UDG digestion step prior to PCR in our protocol to protect against contamination risk with amplicons. Importantly, however, our multiplexes pipeline to detect RNA of various viral respiratory diseases in parallel performed robust-ly across six 96-well plates. We obtained no false negative samples besides expectedly the 1/1 mio dilutions of a Sars-CoV2 sample with Ct30. Aside from the apparent influenza B sample contamination, we did also not see any other false positive events. Moreover, the amplicon sequence of influenza A allowed us to distinguish between the two spike-in strains we had used, namely A/WSN and A/Wy. This is of particular interest since A/Wy like B/Lee used as an Influenza B spike-in is contained in the current vaccination as it is anticipated to circulate in the northern hemisphere 2020/21 flu season. Similarly, we were able to distinguish between the three rhinoviral strains used as spike-in, namely HRV A1a, A1b and A2, by polymorphism in respective amplicons. Taken together our pipeline in its current setup thus differentially detects seven different viral respiratory agents in a single reaction, contains various internal controls, a sample quality control, and by design has a particular sensitivity for Sars-CoV2. SARSeq thus represents a multiplexed, massively parallelized assay for the detection of respiratory infections by means of RT-PCR and next generation sequencing.

### Summary

The global pandemic caused by Sars-CoV2 has majorly impacted on our societies and economies worldwide. It has demonstrated that most countries are not prepared to fight such challenges despite our progress in modern medicine as well as our conceptual understanding of infection cascades. Social distancing measures to interfere with further spread of Sars-CoV2 are likewise affecting almost all aspects of human life. To minimize such measures, it is required to implement them as directed as possible, for which data of infection events are needed. Importantly, such screening efforts serve a different goal than testing symptomatic individuals for Sars-CoV2. While the latter requires gold-standard methods to advise on the optimal treatment, screening for mitigation does allow for some ambiguity especially if the alternative is not to test. However, mass testing for mitigation comes with major practical challenges: i) Sample collection requires major investment into logistics. Amongst them are supply chain issues, personnel, the need for digital data management, and often legal hurdles. Also, communication to improve compliance has been identified as an increasing challenge. ii) Tests must be highly specific for Sars-CoV2 and almost devoid of false positives to prevent isolation of people based on erroneous results to build trust. NGS based detection of Sars-CoV2 can supply such results by detecting genome fragments at a precision even more specific than Taqman probes can be. In addition, the detection of two fragments in parallel represents an independent detection. iii) Costs for mass testing must be as low as possible to reasonably enable scaling. SARSeq as presented here relies on enzymes that can be purchased at scale and produced in-house. Buffers are made from standard salts. iv) Mass testing must not interfere or be confused with the tests performed in medical/diagnostic facilities. It should thus neither be carried out at the same facilities nor compete for supply required to diagnose symptomatic patients. SARSeq relies exclusively on equipment available in sufficient quantity in classical molecular biology facilities such as universities, institutes, biotech, and pharma. v) Tests must scale massively. We have set up SARSeq for a maximum analysis of 36K samples in parallel and tested up to 20K samples in a single sequencing run. Therefore, NGS capacities do not represent a practical limitation of this method. SARSeq is limited by sample supply as well as the throughput of PCRs that can be implemented.

Beyond Sars-CoV2, the pipeline can be rapidly adapted to additional amplicons for the detection of other infectious agents such as newly emerging viruses. We already implemented parallelized screening for Sars-CoV2, Influenza A and B, as well as HRV and aim to extend it to further respiratory infections with overlapping clinical manifestations such as RSV. Moreover, any newly emerging virus can be added to the panel. Also, SARSeq is not limited to respiratory specimen but the pipeline can be extended to other human samples or even monitoring pipelines such as waste-water monitoring.

A limitation of SARSeq is the time requirement of the assay. Two PCR reactions must be performed followed by sequencing (NGS) and analysis, so the theoretical time requirement is around 15h. Therefore, SARSeq is not ideally suited for situations where immediate results are required. In such cases, antigen tests or RT-LAMP are superior methods. Rather, it is ideally implemented for regular (e.g. weekly) surveillance of infections at very large scale. Also, SARSeq is currently only semiquantitative. If exact viral titers are required e.g. to terminate quarantine it might be preferable to use non-multiplexed qPCR.

## Claims

1. A method for detecting a nucleic acid of interest in a plurality of samples, comprising the steps of
a) providing analyte nucleic acids from a plurality of samples in separate containers for each sample, wherein the containers are arranged into an array of subsets, wherein the array comprises two or more subsets;
b) amplifying nucleic acids by a primer extension reaction using at least one pair of primers hybridized to the analyte nucleic acids, wherein a pair of primers comprises a forward and a reverse primer, wherein the forward and reverse primers both comprise an adaptor sequence, a sample identifier sequence and a binding sequence for hybridization to the analyte nucleic acids, respectively;
c) combining the amplified nucleic acids of step b) of containers of two or more subsets to an array of combined containers, wherein containers of one subset, but not of another subset, are combined to a combined container;
d) amplifying nucleic acids by a primer extension reaction using at least one pair of further primers hybridized to the amplified nucleic acids of combined containers of step c), wherein a pair of further primers comprises a further forward and a further reverse primer, wherein the further forward and further reverse primers both comprise a subset identifier sequence and a sequence for hybridization to the adaptor sequence;
e) determining the sequences of the amplified nucleic acids of step d);
f) assigning a determined sequence of a nucleic acid of interest of step e) to a sample through association to a subset and container with the subset identifier sequences and the sample identifier sequences.

2. The method of claim 1, wherein i) in step b) each sample identifier sequence of the forward primers is different from sample identifier sequence of other forward primers for a given binding sequence for hybridization to the analyte nucleic acids; and/or
ii) in step b) each sample identifier sequence of the reverse primers is different from sample identifier sequence of other reverse primers for a given binding sequence for hybridization to the analyte nucleic acids;
and/or
iii) in step d) each subset identifier sequence of the further forward primers is different from subset identifier sequence of other further forward primers;
and/or
iv) in step d) each subset identifier sequence of the further reverse primers is different from subset identifier sequence of other further reverse primers;
preferably a combination of i), ii), iii) and iv).

3. The method of claim 1 or 2, wherein the further primers of step d) comprise a sequencing adaptor sequence.

4. The method of any one of claims 1 to 3, wherein the step a) comprises providing a RNA from a sample and generating a cDNA of said RNA by reverse transcription, wherein the nucleic acids that are amplified in step b) comprise said cDNA.

5. The method of any one of claims 1 to 4, wherein the at least one of the forward and reverse primers of step b) is depleted during and/or after step b) but before step c); preferably wherein the depletion is during step b) and comprises amplification of a spike-in nucleic acid with the forward and/or reverse primer; and/or preferably wherein the depletion of the forward and reverse primers comprises treatment with a single-strand specific nuclease.

6. The method of any one of claims 1 to 5, wherein amplifying nucleic acids in step d) is restricted to at most 35 amplification cycles, preferably at most 20 amplification cycles.

7. The method of any one of claims 1 to 6, wherein the plurality of samples comprises 4000 or more samples and/or the two or more subsets are 40 or more subsets and/or wherein a subset comprises 40 or more containers.

8. The method of any one of claims 1 to 7, wherein the nucleic acid of interest is a pathogen nucleic acid, preferably a viral nucleic acid.

9. The method of claim 8, wherein the nucleic acid of interest is selected from a coronavirus nucleic acid, preferably a SARS-CoV-2 nucleic acid, an influenza nucleic acid, a parainfluenza nucleic acid, a rhinovirus nucleic acid, or a combination thereof.

10. The method of any one of claims 1 to 9, wherein two or more nucleic acids of interest are selected, preferably wherein the two or more nucleic acids of interest are from a virus, which is the same virus or a different virus for two or more nucleic acids of interest, and/or preferably wherein in step b) forward and reverse primers with binding sequences for hybridization to the two or more analyte nucleic acids are used.

11. The method of any one of claims 1 to 10, wherein the sample identifier sequence of the forward primers, the sample identifier sequence of the reverse primers, the subset identifier sequence of the further forward primers, and the subset identifier sequence of the further reverse primers, respectively, comprise a sequence distance, preferably a Hamming distance or a Levenshtein distance, to other sample identifier sequences of forward primers, sample identifier sequences of the reverse primers, subset identifier sequences of further forward primers, and subset identifier sequences of further reverse primers, respectively, of at least 2, preferably of at least 3.

12. The method of any one of claims 1 to 11, wherein the sample identifier sequence of the forward primers, the sample identifier sequence of the reverse primers, the subset identifier sequence of the further forward primers, and the subset identifier sequence of the further reverse primers, respectively, each selected independently, have a length of at least 4 nucleotides.

13. A set of primers suitable for a method of any one of claims 1 to 12,
at least 10 different primers A which comprise the sequence, from 5' to 3': an adaptor A sequence, an identifier sequence of at least 4 nt in length and a target binding sequence, wherein the identifier sequence is different within the at least 10 different primers A, preferably with a sequence distance of a Hamming distance of at least 1 or a Levenshtein distance of at least 1;
at least 10 different primers B which comprise the sequence, from 5' to 3': an adaptor B sequence, an identifier sequence of at least 4 nt in length and a target binding sequence, wherein the identifier sequence is different within the at least 10 different primers B, preferably with a sequence distance of a Hamming distance of at least 1 or a Levenshtein distance of at least 1;
at least 10 different primers C which comprise the sequence, from 5' to 3': an adaptor C sequence, an identifier sequence of at least 4 nt in length and a binding sequence that binds to the adaptor A sequence, wherein the identifier sequence is different within the at least 10 different primers C, preferably with a sequence distance of a Hamming distance of at least 1 or a Levenshtein distance of at least 1; and
at least 10 different primers D which comprise the sequence, from 5' to 3': an adaptor D sequence, an identifier sequence of at least 4 nt in length and a binding sequence that binds to the adaptor B sequence, wherein the identifier sequence is different within the at least 10 different primers D, preferably with a sequence distance of a Hamming distance of at least 1 or a Levenshtein distance of at least 1.

14. The set of primers according to claim 13, wherein the target binding sequences of the primers A and primers B comprise a sequence or antisense-sequence, respectively, of a viral gene, preferably of a SARS-CoV-2 N, M, E or S gene or coding sequence thereof, Influenza PA, PB1, PB2, PA, HA, NP, NA, M1, M2, NS1 or NEP gene or coding sequence thereof, a parainfluenza HN, F, M, NP, P or L gene or coding sequence thereof, a rhinovirus VP1, VP2, VP3, VP4, 2A, 2B, 2C, 3A, 3B, 3C or 3D gene or coding sequence; a human respiratory syncytial virus A (HRSV-A) GA1, GA2, GA3, GA4, GA5, GA6, GA7, SAA1, NA1, or NA2 gene or coding sequence; a human respiratory syncytial virus B (HRSV-B) GB1, GA2, GA3, GB4, SAB1, SAB2, SAB3, BA1, BA2, BA3, BA4, BA5, or BA6 gene or coding sequence.

15. The set of primers according to claim 13 or 14, further comprising a primer pair that binds to a ribosomal gene or coding sequence.
